# EUROPÄISCHE PATENTSCHRIFT

(11) **EP 2 411 363 B1**
(45) Veröffentlichungstag und Bekanntmachung des Hinweises auf die Patenterteilung: **07.10.2015**
(21) Anmeldenummer: 10708146.5
(22) Anmeldetag: 12.03.2010
(51) Int. Cl.: C07D 211/42, C07D 211/56, C07D 211/60, C07D 211/62, C07D 401/06, C07D 405/06, C07D 405/12, C07D 409/12, C07D 413/12, C07D 417/06, A61K 31/451, A61K 31/4525, A61K 31/4535, A61K 31/454, A61K 31/4545, A61K 9/20, A61K 9/08, A61K 9/10

(54) **SUBSTITUIERTE PIPERIDINE ALS PAR-1 ANTAGONISTEN**
SUBSTITUTED PIPERIDINES AS PAR-1 ANTAGONISTS
PIPÉRIDINES SUBSTITUÉES EN TANT QU'ANTAGONISTES DE PAR-1

(30) Priorität: 23.03.2009 DE 102009014484
(43) Veröffentlichungstag der Anmeldung: 01.02.2012
(73) Patentinhaber: Bayer Intellectual Property GmbH, 40789 Monheim (DE)
(72) Erfinder: JESKE, Mario, 42699 Solingen (DE); HEIMBACH, Dirk, 40549 Düsseldorf (DE); RÖHRIG, Susanne, 40724 Hilden (DE); CANCHO GRANDE, Yolanda, 51373 Leverkusen (DE); SCHNEIDER, Dirk, 42115 Wuppertal (DE); RESTER, Ulrich, 42115 Wuppertal (DE); BENDER, Eckhard, 40764 Langenfeld (DE); MEININGHAUS, Mark, 42327 Wuppertal (DE); ZIMMERMANN, Katja, 40489 Düsseldorf (DE); ZUBOV, Dmitry, 42857 Remscheid (DE); BUCHMÜLLER, Anja, 45259 Essen (DE); VON DEGENFELD, Georges, 51373 Leverkusen (DE); GERDES, Christoph, 51063 Köln (DE); GERISCH, Michael, 42329 Wuppertal (DE); GNOTH, Mark, Jean, 40822 Mettmann (DE); GERICKE, Kersten, Matthias, 42115 Wuppertal (DE)
(74) Vertreter: BIP Patents
(86) Internationale Anmeldenummer: PCT/EP2010/001567
(87) Internationale Veröffentlichungsnummer: WO 2010/108608

(56) Entgegenhaltungen:
- WO-A2-2006/012226
- WO-A2-2006/032342
- US-A1- 2001 044 454
- DIAZ J L ET AL: "Fast and efficient access to a family of multifunctional 1,3,5-trisubstituted piperidines" SYNTHETIC COMMUNICATIONS 200801 US LNKD- DOI:10.1080/00397910701877608, Bd. 38, Nr. 16, Januar 2008 (2008-01), Seiten 2799-2813, XP009133531 ISSN: 0039-7911
- CHACKALAMANNIL SAMUEL: "Thrombin receptor (protease activated receptor-1) antagonists as potent antithrombotic agents with strong antiplatelet effects." JOURNAL OF MEDICINAL CHEMISTRY 7 SEP 2006 LNKD- PUBMED:16942011, Bd. 49, Nr. 18, 7. September 2006 (2006-09-07), Seiten 5389-5403, XP002582198 ISSN: 0022-2623

## Beschreibung

Die Erfindung betrifft neue substituierte Piperidine, Verfahren zu ihrer Herstellung, solche Verbindungen zur Verwendung in der Behandlung und/oder Prophylaxe von Krankheiten sowie ihre Verwendung zur Herstellung von Arzneimitteln zur Behandlung und/oder Prophylaxe von Krankheiten, insbesondere von Herz-Kreislauf-Erkrankungen und von Tumorerkrankungen.

Thrombozyten (Blutplättchen) sind ein wesentlicher Faktor sowohl in der physiologischen Blutstillung (Hämostase) als auch bei thromboembolischen Erkrankungen. Insbesondere im arteriellen System kommt Thrombozyten eine zentrale Bedeutung in der komplexen Interaktion zwischen Blutkomponenten und Gefäßwand zu. Unerwünschte Thrombozytenaktivierung kann durch Bildung plättchenreicher Thromben zu thromboembolischen Erkrankungen und thrombotischen Komplikationen mit lebensbedrohlichen Zuständen führen.

Einer der potentesten Plättchenaktivatoren ist die Blutgerinnungsprotease Thrombin, die an verletzten Blutgefäßwänden gebildet wird und neben der Fibrinbildung zur Aktivierung von Thrombozyten, Endothelzellen und mesenchymalen Zellen führt (Vu TKH, Hung DT, Wheaton VI, Coughlin SR, Cell 1991, 64, 1057-1068). An Thrombozyten *in vitro* und in Tiermodellen hemmen Thrombin-Inhibitoren die Plättchenaggregation bzw. die Bildung plättchenreicher Thromben. Beim Menschen können arterielle Thrombosen erfolgreich mit Inhibitoren der Thrombozytenfunktion sowie Thrombin-Inhibitoren verhindert oder behandelt werden (Bhatt DL, Topol EJ, Nat. Rev. Drug Discov. 2003, 2, 15-28). Deshalb besteht eine hohe Wahrscheinlichkeit, dass Antagonisten der Thrombinwirkung auf Blutplättchen die Bildung von Thromben und das Auftreten von klinischen Folgen wie Herzinfarkt und Schlaganfall vermindern. Weitere zelluläre Thrombinwirkungen, z.B. auf Gefäßendothel- und -glattmuskelzellen, Leukozyten und Fibroblasten, sind möglicherweise für entzündliche und proliferative Erkrankungen verantwortlich. Die zellulären Effekte von Thrombin werden zumindest teilweise über eine Familie G-Proteingekoppelter Rezeptoren (Protease Activated Receptors, PARs) vermittelt, deren Prototyp der PAR-1-Rezeptor darstellt. PAR-1 wird durch Bindung von Thrombin und proteolytische Spaltung seines extrazellulär liegenden N-Terminus aktiviert. Durch die Proteolyse wird ein neuer N-Terminus mit der Aminosäurensequenz SFLLRN... freigelegt, der als Agonist ("Tethered Ligand") zur intramolekularen Rezeptoraktivierung und Übertragung intrazellulärer Signale führt. Von der Tethered-Ligand Sequenz abgeleitete Peptide können als Agonisten des Rezeptors eingesetzt werden und führen auf Thrombozyten zur Aktivierung und Aggregation. Andere Proteasen sind ebenfalls in der Lage, PAR-1 zu aktivieren, hierunter z.B. Plasmin, Faktor VIIa, Faktor Xa, Trypsin, aktiviertes Protein C (aPC), Tryptase, Cathepsin G, Proteinase 3, Granzyme A, Elastase und Matrixmetalloprotease 1 (MMP-1).

Im Gegensatz zur Inhibition der Proteaseaktivität von Thrombin mit direkten Thrombin-Inhibitoren sollte eine Blockade des PAR-1 zur Hemmung der Thrombozytenaktivierung ohne Verminderung der Gerinnungsfähigkeit des Blutes (Antikoagulation) führen.

Antikörper und andere selektive PAR-1-Antagonisten hemmen die Thrombin-induzierte Aggregation von Thrombozyten *in vitro* bei niedrigen bis mittleren Thrombinkonzentrationen (Kahn ML, Nakanishi-Matsui M, Shapiro MJ, Ishihara H, Coughlin SR, J. Clin. Invest. 1999, 103, 879-887). Ein weiterer Thrombinrezeptor mit möglicher Bedeutung für die Pathophysiologie thrombotischer Prozesse, PAR-4, wurde auf humanen und tierischen Thrombozyten identifiziert. In experimentellen Thrombosen an Tieren mit einem dem Menschen vergleichbaren PAR-Expressionsmuster reduzieren PAR-1-Antagonisten die Bildung plättchenreicher Thromben (Derian CK, Damiano BP, Addo MF, Darrow AL, D'Andrea MR, Nedelman M, Zhang H-C, MaryanoffBE, Andrade-Gordon P, J. Pharmacol. Exp. Ther. 2003, 304, 855-861).

In den letzten Jahren wurde eine Vielzahl von Substanzen auf ihre plättchenfunktionshemmende Wirkung geprüft, in der Praxis haben sich aber nur wenige Plättchenfunktionshemmer bewährt. Es besteht daher ein Bedarf an Pharmazeutika, die spezifisch eine gesteigerte Plättchenreaktion hemmen ohne das Blutungsrisiko erheblich zu erhöhen und damit das Risiko von thromboembolischen Komplikationen vermindern.

Effekte von Thrombin, die über den Rezeptor PAR-1 vermittelt werden, haben Auswirkungen auf den Krankheitsverlauf während und nach operativer koronarer Bypassanlage (CABG) sowie anderer Operationen und insbesondere Operationen mit extrakorporalem Kreislauf (z. B. Herz-Lungenmaschine). Im Verlauf der Operation kann es aufgrund der prä- oder intraoperativen Medikation mit gerinnungshemmenden und/oder plättchenhemmenden Substanzen zu Blutungskomplikationen kommen. Aus diesem Grunde muss beispielsweise eine Medikation mit Clopidogrel mehrere Tage vor einer CABG pausiert werden. Außerdem kann es wie erwähnt (z.B. aufgrund des ausgedehnten Kontaktes zwischen Blut und künstlichen Oberflächen bei Einsatz einer extrakorporalen Zirkulation oder bei Bluttransfusionen) zur Ausbildung einer disseminierten intravaskulären Gerinnung oder Verbrauchskoagulopathie (DIC) kommen, die sekundär zu Blutungskomplikationen führen kann. Im weiteren Verlauf kommt es häufig zur Restenose der angelegten venösen oder arteriellen Bypässe (bis hin zum Verschluß) aufgrund von Thrombose, Intimafibrose, Arteriosklerose, Angina pectoris, Myokardinfarkt, Herzinsuffizienz, Arrhythmien, transitorische ischämische Attacke (TIA) und/oder Schlaganfall.

Der Rezeptor PAR-1 wird im Menschen auch auf anderen Zellen exprimiert, hierunter z.B. Endothelzellen, glatte Gefäßmuskelzellen und Tumorzellen. Bösartige Tumorerkrankungen (Krebs) haben eine hohe Inzidenz und sind im Allgemeinen mit einer hohen Sterblichkeit verbunden. Gegenwärtige Therapien erreichen nur in einem Bruchteil der Patienten eine Vollremission und sind typischerweise mit schweren Nebenwirkungen verbunden. Daher besteht ein hoher Bedarf an effektiveren und sichereren Therapien. Der PAR-1-Rezeptor trägt zur Entstehung, dem Wachstum, der Invasivität und der Metastasierung von Krebs bei. Außerdem vermittelt auf Endothelzellen exprimiertes PAR-1 Signale, die in Gefäßwachstum münden ("Angiogenese"), ein Vorgang, der zur Ermöglichung von Tumorwachstum über ca. 1 mm³ hinaus unerläßlich ist. Angiogenese trägt auch zur Enstehung oder Verschlimmerung anderer Erkrankungen bei, hierunter z.B. hämatopoetische Krebserkrankungen, die zur Erblindung führende Makuladegeneration und diabetische Retinopathie, entzündliche Erkrankungen wie rheumatoide Arthritis und Colitis.

Sepsis (oder Septikämie) ist eine häufige Erkrankung mit hoher Letalität. Anfängliche Symptome der Sepsis sind typischerweise unspezifisch (z.B. Fieber, reduziertes Allgemeinbefinden), im weiteren Verlauf kann es jedoch zur generalisierten Aktivierung des Gerinnungssystems kommen ("Disseminated Intravascular coagulation" oder "Verbrauchskoagulopathie" (DIC)) mit Mikrothrombosierung in verschiedenen Organen und sekundärer Blutungskomplikationen. DIC kann auch unabhängig von einer Sepsis auftreten, z.B. im Rahmen von Operationen oder bei Tumorerkrankungen.

Die Therapie der Sepsis besteht einerseits in der konsequenten Beseitigung der infektiösen Ursache, z.B. durch operative Herdsanierung und Antibiose. Andererseits besteht sie in der temporären intensivmedizinischen Unterstützung der beeinträchtigten Organsysteme. Therapien der verschiedenen Stadien dieser Erkrankung sind z.B. in folgender Publikation beschrieben (Dellinger et al., Crit. Care Med. 2004, 32, 858-873). Für die DIC existieren keine erwiesenermaßen effektive Therapien.

Eine Aufgabe der vorliegenden Erfindung ist es daher, neue PAR-1-Antagonisten zur Behandlung von Erkrankungen, wie z. B. Herz-Kreislauf-Erkrankungen und thromboembolischen Erkrankungen, sowie Tumorerkrankungen bei Menschen und Tieren zur Verfügung zu stellen.

WO 2006/012226, WO 2006/020598, WO 2007/038138, WO 2007/130898, WO 2007/101270 und US 2006/0004049 beschreiben strukturell ähnliche Piperidine als 11-β HSD1 Inhibitoren zur Behandlung von unter anderem Diabetes, thromboembolischen Erkrankungen und Schlaganfall.

DIAZ J L ET AL: "Fast and efficient access to a family of multifunctional 1,3,5-trisubstituted piperidines", SYNTHETIC COMMUNICATIONS, Bd. 38, Nr. 16, Seiten 2799-2813, offenbart zwei Synthesezwischenstufen, die durch Disclaimer von den Ansprüchen ausgenommen sind.

WO2116/032342 und US2001/044454 offenbaren Antagonisten des Thrombinrezeptors (PAR-1), von denen sich die vorliegenden Verbindungen unter anderem in der Gruppe R1 unterscheiden.

Eine Übersicht über Antagonisten des Thrombinrezeptors (PAR-1) findet sich in CHACKALAMANNIL SAMUEL: "Thrombin receptor (protease activated receptor-1) antagonists as potent antithrombotic agents with strong antiplatelet effects.", JOURNAL OF MEDICINAL CHEMISTRY, Bd. 49, Nr. 18, Seiten 5389-5403.

Gegenstand der Erfindung sind Verbindungen der Formel 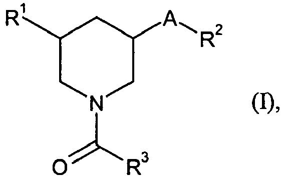 in welcher
- A: für eine Gruppe der Formel 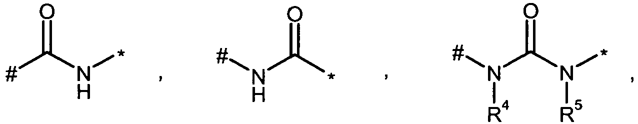 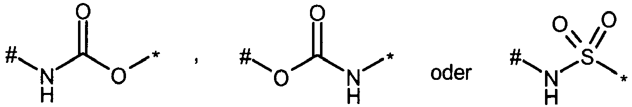 steht,
wobei
# die Anknüpfstelle an den Piperidinring ist,
* die Anknüpfstelle an R² ist,
R⁴ für Wasserstoff oder C₁-C₃-Alkyl steht, und
R⁵ für Wasserstoff oder C₁-C₃-Alkyl steht,
- R¹: für Phenyl steht,
wobei Phenyl substituiert sein kann mit 1 bis 3 Substituenten, unabhängig voneinander ausgewählt aus der Gruppe bestehend aus Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, Methylsulfonyl, C₁-C₄-Alkyl, C₁-C₄-Alkoxy und C₁-C₄-Alkoxycarbonyl,
- R²: für C₁-C₆-Alkyl, C₃-C₆-Cycloalkyl, 4- bis 6-gliedriges Heterocyclyl, Phenyl, 2,2-Difluor-1,3-benzodioxolyl oder 5- oder 6-gliedriges Heteroaryl steht,
wobei Cycloalkyl, Heterocyclyl, Phenyl und Heteroaryl substituiert sein können mit 1 bis 3 Substituenten, unabhängig voneinander ausgewählt aus der Gruppe bestehend aus Halogen, Cyano, Hydroxy, Amino, Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, C₁-C₄-Alkyl, C₁-C₄-Alkoxy, C₁-C₆-Alkylamino und Phenyl,
worin Phenyl substituiert sein kann mit 1 bis 3 Substituenten, unabhängig voneinander ausgewählt aus der Gruppe bestehend aus Halogen und Trifluormethyl,
und
wobei C₁-C₄-Alkyl substituiert sein kann mit einem Substituenten ausgewählt aus der Gruppe bestehend aus C₃-C₆-Cycloalkyl und Phenyl,
worin Cycloalkyl und Phenyl substituiert sein können mit 1 bis 3 Substituenten, unabhängig voneinander ausgewählt aus der Gruppe bestehend aus Halogen, Cyano, Monofluormethyl, Difluormethyl, Trifluormethyl, Monofluormethoxy, Difluormethoxy, Trifluormethoxy, C₁-C₄-Alkyl und C₁-C₄-Alkoxy,
- R³: für C₁-C₆-Alkyl, C₂-C₆-Alkenyl, C₂-C₆-Alkinyl, C₁-C₆-Alkoxy, C₁-C₆-Alkylamino, C₃-C₇-Cycloalkyl, 4- bis 7-gliedriges Heterocyclyl, Phenyl, 5- oder 6-gliedriges Heteroaryl, C₃-C₇-Cycloalkyloxy, C₃-C₇-Cycloalkylamino, 4- bis 7-gliedriges Heterocyclylamino, Phenylamino oder 5- oder 6-gliedriges Heteroarylamino steht,
wobei Alkyl, C₂-C₆-Alkoxy und Alkylamino substituiert sein können mit einem Substituenten ausgewählt aus der Gruppe bestehend aus Halogen, Hydroxy, Amino, Cyano, C₁-C₄-Alkoxy, C₁-C₄-Alkoxycarbonyl, C₃-C₇-Cycloalkyl, 4- bis 6-gliedriges Heterocyclyl, Phenyl und 5- oder 6-gliedriges Heteroaryl,
worin Alkoxy substituiert sein kann mit einem Substituenten C₁-C₄-Alkoxy,
und
wobei Cycloalkyl, Heterocyclyl, Phenyl, Heteroaryl, Cycloalkyloxy, Cycloalkylamino, Heterocyclylamino, Phenylamino und Heteroarylamino substituiert sein können mit 1 bis 3 Substituenten, unabhängig voneinander ausgewählt aus der Gruppe bestehend aus Halogen, Cyano, Oxo, Hydroxy, Amino, Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, Hydroxycarbonyl, Aminocarbonyl, C₁-C₄-Alkyl, C₁-C₄-Alkoxy, C₁-C₆-Alkylamino, C₁-C₄-Alkoxycarbonyl, C₁-C₄-Alkylaminocarbonyl und Cyclopropyl,
worin Alkyl substituiert sein kann mit einem Substituenten Hydroxy,
und ihre Salze, ihre Solvate und die Solvate ihrer Salze.

Erfindungsgemäße Verbindungen sind die Verbindungen der Formel (I) und deren Salze, Solvate und Solvate der Salze; die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Gegenstand der Erfindung sind nicht die Verbindungen trans-tert-Butyl-3-{[(benzyloxy)carbonyl]amino}-5-phenylpiperin-1-carboxylat und cis-tert-Butyl-3-{[(benzyloxy)carbonyl]amino}-5-phenylpiperin-1-carboxylat als solche.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methyl-morpholin, Arginin, Lysin, Ethylendiamin, *N*-Methylpiperidin und Cholin.

Als Solvate werden im Rahmen der Erfindung solche Formen der erfindungsgemäßen Verbindungen bezeichnet, welche in festem oder flüssigem Zustand durch Koordination mit Lösungs-mittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:
Alkyl per se und "Alk" und "Alkyl," in Alkoxy, Alkylamino, Alkoxycarbonyl und Alkylaminocarbonyl stehen für einen linearen oder verzweigten Alkylrest mit 1 bis 6 Kohlenstoffatomen, beispielhaft und vorzugsweise für Methyl, Ethyl, n-Propyl, iso-Propyl, n-Butyl, tert-Butyl, n-Pentyl und n-Hexyl.
Alkenyl steht für einen linearen oder verzweigten Alkenylrest mit 2 bis 6 Kohlenstoffatomen. Bevorzugt ist ein linearer oder verzweigter Alkenylrest mit 2 bis 4, besonders bevorzugt mit 2 bis 3 Kohlenstoffatomen. Beispielsweise und vorzugsweise seien genannt: Vinyl, Allyl, n-Prop-1-en-1-yl und n-But-2-en-1-yl.
Alkinyl steht für einen linearen oder verzweigten Alkinylrest mit 2 bis 6 Kohlenstoffatomen. Bevorzugt ist ein linearer oder verzweigter Alkinylrest mit 2 bis 4, besonders bevorzugt mit 2 bis 3 Kohlenstoffatomen. Beispielsweise und vorzugsweise seien genannt: Ethinyl, n-Prop-2-in-1-yl und n-But-3-en-1-yl.
Alkoxy steht beispielhaft und vorzugsweise für Methoxy, Ethoxy, n-Propoxy, iso-Propoxy, n-Butoxy, tert-Butoxy, n-Pentoxy und n-Hexoxy.
Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, iso-Propylamino, tert-Butylamino, *N,N*-Dimethylamino, *N,N*-Diethylamino, *N*-Ethyl-*N-*methylamino, *N*-Methyl-*N*-n-propylamino, *N*-iso-Propyl-*N*-n-propylamino und *N*-tert-Butyl-*N-*methylamino. C₁-C₄-Alkylarnino steht beilspielsweise für eisen Monualkylaminorest mit 1 bis 4 Kohlenstoffatomen oder für einen Dialkylaminorest mit jeweils 1 bis 4 Kohlenstoffatomen pro Alkylsubstituent.
Alkoxycarbonyl steht beispielhaft und vorzugsweise für Methoxycarbonyl, Ethoxycarbonyl, n-Propoxycarbonyl, iso-Propoxycarbonyl, n-Butoxycarbonyl, tert-Butoxycarbonyl, n-Pentoxycarbonyl und n-Hexoxycarbonyl.
Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, iso-Propylaminocarbonyl, tert-Butylaminocarbonyl, *N,N*-Dimethylaminocarbonyl, *N,N*-Diethylaminocarbonyl, *N*-Ethyl-*N-*methylaminocarbonyl, *N-*Methyl-*N-*n-propylaminocarbonyl, *N*-iso-Propyl-*N*-n-propylaminocarbonyl und *N*-tert-Butyl-*N-*methylaminocarbonyl. C₁-C₄-Alkylaminocarbonyl steht beispielsweise für einen Monoalkylaminocarbonylrest mit 1 bis 4 Kohlenstoffatomen oder für einen Dialkylaminocarbonylrest mit jeweils 1 bis 4 Kohlenstoffatomen pro Alkylsubstituent.
Cycloalkyl steht für eine monocyclische Cycloalkylgruppe mit in der Regel 3 bis 7, bevorzugt 5 oder 6 Kohlenstoffatomen, beispielhaft und vorzugsweise für Cycloalkyl seien genannt Cyclopropyl, Cyclobutyl, Cyclopentyl, Cyclohexyl und Cycloheptyl.
Cycloalkyloxy steht für eine monocyclische Cycloalkyloxygruppe mit in der Regel 3 bis 7, bevorzugt 5 oder 6 Kohlenstoffatomen, beispielhaft und vorzugsweise für Cycloalkyloxy seien genannt Cyclopropyloxy, Cyclobutyloxy, Cyclopentyloxy und Cyclohexyloxy.
Cycloalkylamino steht für eine monocyclische Cycloalkylaminogruppe mit in der Regel 3 bis 7, bevorzugt 3 oder 4 Kohlenstoffatomen, beispielhaft und vorzugsweise für Cycloalkylamino seien genannt Cyclopropylamino, Cyclobutylamino, Cyclopentylamino und Cyclohexylamino.
Heterocyclyl steht für einen monocyclischen oder bicyclischen, heterocyclischen Rest mit 4 bis 7 Ringatomen und bis zu 3, vorzugsweise bis zu 2 Heteroatomen und/oder Heterogruppen aus der Reihe N, O, S, SO, SO₂, wobei ein Stickstoffatom auch ein N-Oxid bilden kann. Die HeterocyclylReste können gesättigt oder teilweise ungesättigt sein. Bevorzugt sind 5- oder 6-gliedrige, monocyclische gesättigte Heterocyclylreste mit bis zu zwei Heteroatomen aus der Reihe O, N und S, beispielhaft und vorzugsweise für Oxetanyl, Azetidinyl, Pyrrolidin-2-yl, Pyrrolidin-3-yl, Pyrrolinyl, Tetrahydrofuranyl, Tetrahydrothienyl, Pyranyl, Piperidin-1-yl, Piperidin-2-yl, Piperidin-3-yl, Piperidin-4-yl, 1,2,5,6-Tetrahydropyridin-3-yl, 1,2,5,6-Tetrahydropyridin-4-yl, Thiopyranyl, Morpholin-1-yl, Morpholin-2-yl, Morpholin-3-yl, Piperazin-1-yl, Piperazin-2-yl.
Heterocyclylamino steht für einen monocyclischen oder bicyclischen, heterocyclischen Heterocyclylamino-Rest mit 4 bis 7 Ringatomen und bis zu 3, vorzugsweise bis zu 2 Heteroatomen und/oder Heterogruppen aus der Reihe N, O, S, SO, SO₂, wobei ein Stickstoffatom auch ein N-Oxid bilden kann. Die Heterocyclyl-Reste können gesättigt oder teilweise ungesättigt sein. Bevorzugt sind 5- oder 6-gliedrige, monocyclische gesättigte Heterocyclylreste mit bis zu zwei Heteroatomen aus der Reihe O, N und S, beispielhaft und vorzugsweise für Oxetanylamino, Azetidinylamino, Pyrrolidin-2-yl-amino, Pyrrolidin-3-yl-amino, Tetrahydrofuranylamino, Tetrahydrothienylamino, Pyranylamino, Piperidin-2-yl-amino, Piperidin-3-yl-amino, Piperidin-4-yl-amino, 1,2,5,6-Tetrahydropyridin-3-yl-amino, 1,2,5,6-Tetrahydropyridin-4-yl-amino, Thiopyranylamino, Morpholin-2-yl-amino, Morpholin-3-yl-amino, Piperazin-2-yl-amino.
Heteroaryl steht für einen aromatischen, monocyclischen Rest mit in der Regel 5 oder 6 Ringatomen und bis zu 4 Heteroatomen aus der Reihe S, O und N, wobei ein Stickstoffatom auch ein N-Oxid bilden kann, beispielhaft und vorzugsweise für Thienyl, Furyl, Pyrrolyl, Thiazolyl, Oxazolyl, Isoxazolyl, Oxadiazolyl, Pyrazolyl, Imidazolyl, Triazolyl, Pyridyl, Pyrimidyl, Pyridazinyl, Pyrazinyl.
Heteroarylamino steht für einen aromatischen, monocyclischen Heteroarylamino-Rest mit in der Regel 5 oder 6 Ringatomen und bis zu 4 Heteroatomen aus der Reihe S, O und N, wobei ein Stickstoffatom auch ein N-Oxid bilden kann, beispielhaft und vorzugsweise für Thienylamino, Furylamino, Pyrrolylamino, Thiazolylamino, Oxazolylamino, Isoxazolylamino, Oxadiazolylamino, Pyrazolylamino, Imidazolylamino, Pyridylamino, Pyrimidylamino, Pyridazinylamino, Pyrazinylamino.
Halogen steht für Fluor, Chlor, Brom und Jod, vorzugsweise für Fluor und Chlor.

In der Formel der Gruppe, die für A stehen kann, steht der Endpunkt der Linie, neben der ein # oder ein * steht, nicht für ein Kohlenstoffatom beziehungsweise eine CH₂-Gruppe, sondern ist Bestandteil der Bindung zu dem Atom, an das A gebunden ist.

Bevorzugt sind Verbindungen der Formel (I), in welcher
- A: für eine Gruppe der Formel 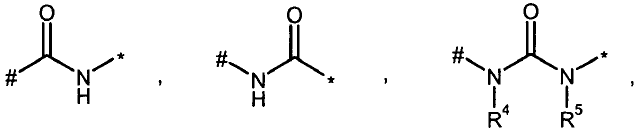 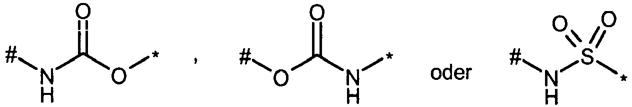 steht,
wobei
# die Anknüpfstelle an den Piperidinring ist,
* die Anknüpfstelle an R² ist,
R⁴ für Wasserstoff oder C₁-C₃-Alkyl steht,
und
R⁵ für Wasserstoff oder C₁-C₃-Alkyl steht,
- R¹: für Phenyl steht,
wobei Phenyl substituiert ist mit 1 bis 3 Substituenten, unabhängig voneinander ausgewählt aus der Gruppe bestehend aus Trifluormethyl, Trifluormethoxy, C₁-C₄-Alkyl, C₁-C₄-Alkoxy und C₁-C₄-Alkoxycarbonyl,
- R²: für C₁-C₆-Alkyl, C₃-C₆-Cycloalkyl, 4- bis 6-gliedriges Heterocyclyl, Phenyl, 2,2-Difluor-1,3-benzodioxolyl oder 5- oder 6-gliedriges Heteroaryl steht,
wobei Cycloalkyl, Heterocyclyl, Phenyl und Heteroaryl substituiert sein können mit 1 bis 3 Substituenten, unabhängig voneinander ausgewählt aus der Gruppe bestehend aus Halogen, Cyano, Hydroxy, Amino, Trifluormethyl, Difluormethoxy, Trifluormethoxy, Methyl, Ethyl, Methoxy, Ethoxy und Phenyl,
worin Phenyl substituiert sein kann mit 1 bis 3 Substituenten, unabhängig voneinander ausgewählt aus der Gruppe bestehend aus Halogen und Trifluormethyl,
   wobei C₁-C₂-Alkyl substituiert sein kann mit einem Substituenten Phenyl,
   worin Phenyl substituiert sein kann mit 1 bis 3 Substituenten, unabhängig voneinander ausgewählt aus der Gruppe bestehend aus Halogen, Cyano, Trifluormethyl, Trifluormethoxy, Methyl, Ethyl, Methoxy und Ethoxy,
- R³: für C₁-C₆-Alkyl, C₂-C₆-Alkenyl, C₂-C₆-Alkinyl, C₁-C₆-Alkoxy, C₁-C₆-Alkylamino, C₃-C₇-Cycloalkyl, 4- bis 7-gliedriges Heterocyclyl, Phenyl, 5- oder 6-gliedriges Heteroaryl, C₃-C₇-Cycloalkyloxy, C₃-C₇-Cycloalkylamino, 4- bis 7-gliedriges Heterocyclylamino, Phenylamino oder 5- oder 6-gliedriges Heteroarylamino steht,
wobei Alkyl, C₂-C₆-Alkoxy und Alkylamino substituiert sein können mit einem Substituenten ausgewählt aus der Gruppe bestehend aus Halogen, Hydroxy, Amino, Cyano, C₁-C₄-Alkoxy, C₁-C₄-Alkoxycarbonyl, C₃-C₇-Cycloalkyl, 4- bis 6-gliedriges Heterocyclyl, Phenyl und 5- oder 6-gliedriges Heteroaryl,
worin Alkoxy substituiert sein kann mit einem Substituenten C₁-C₄-Alkoxy,
und
wobei Cycloalkyl, Heterocyclyl, Phenyl, Heteroaryl, Cycloalkyloxy, Cycloalkylamino, Heterocyclylamino, Phenylamino und Heteroarylamino substituiert sein können mit 1 bis 3 Substituenten, unabhängig voneinander ausgewählt aus der Gruppe bestehend aus Halogen, Cyano, Oxo, Hydroxy, Amino, Trifluormethyl, Difluormethoxy, Trifluormethoxy, Hydroxycarbonyl, Aminocarbonyl, Methyl, Ethyl, Methoxy, Ethoxy, Dimethylamino, Methoxycarbonyl, Ethoxycarbonyl, Dimethylaminocarbonyl und Cyclopropyl,
worin Methyl und Ethyl substituiert sein können mit einem Substituenten Hydroxy,
und ihre Salze, ihre Solvate und die Solvate ihrer Salze.

Bevorzugt sind auch Verbindungen der Formel (I), in welcher
- A: für eine Gruppe der Formel 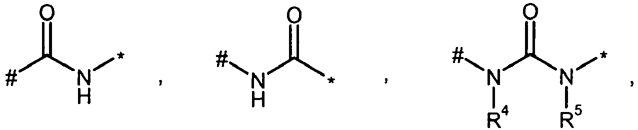 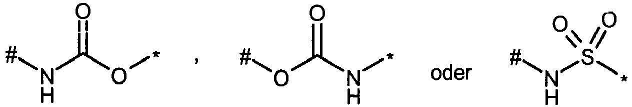 steht,
wobei
# die Anknüpfstelle an den Piperidinring ist,
* die Anknüpfstelle an R² ist,
R⁴ für Wasserstoff oder Methyl steht,
und
R⁵ für Wasserstoff oder Methyl steht,
- R¹: für Phenyl steht,
wobei Phenyl substituiert ist mit 1 bis 2 Substituenten, unabhängig voneinander ausgewählt aus der Gruppe bestehend aus Trifluormethyl, Trifluormethoxy, Methyl, Ethyl, Isopropyl, Methoxy und Ethoxycarbonyl,
- R²: für Methyl, Ethyl, Isopropyl, tert-Butyl, Cyclopropyl, Cyclopentyl, Pyrrolidinyl, Piperazinyl, Phenyl, 2,2-Difluor-1,3-benzodioxolyl, Thienyl, Thiazolyl oder Pyridyl steht,
wobei Cyclopentyl, Piperazinyl, Phenyl, Thienyl, Thiazolyl und Pyridyl substituiert sein können mit 1 bis 2 Substituenten, unabhängig voneinander ausgewählt aus der Gruppe bestehend aus Halogen, Cyano, Hydroxy, Trifluormethyl, Difluormethoxy, Trifluormethoxy, Methyl, Ethyl, Methoxy, Ethoxy und Phenyl,
worin Phenyl substituiert sein kann mit 1 bis 2 Substituenten, unabhängig voneinander ausgewählt aus der Gruppe bestehend aus Halogen und Trifluormethyl,
und
wobei Methyl substituiert sein kann mit einem Substituenten Phenyl,
- R³: für Methyl, Ethyl, Isopropyl, tert-Butyl, Ethoxy, Ethylamino, tert-Butylamino, N-Methyl-N-ethylamino, Diethylamino, Cyclopropyl, Cyclobutyl, Cyclopentyl, Cyclohexyl, Tetrahydrofuranyl, Tetrahydropyranyl, Morpholin-4-yl, Thiomorpholin-4-yl, 1,1-Dioxidothiomorpholin-4-yl, Azetidiny-1-yl, Pyrrolidin-1-yl, Piperidin-1-yl, Piperazin-1-yl, Phenyl, Pyrrolyl, Furyl, Thiazolyl, Pyrazolyl, Pyridyl, Cyclopentyloxy, Cyclohexylamino, Phenylamino oder Pyridylamino steht,
wobei Methyl, Ethyl, Isopropyl, tert-Butyl, Ethoxy und Ethylamino substituiert sein können mit einem Substituenten ausgewählt aus der Gruppe bestehend aus Halogen, Hydroxy, Methoxy, Cyclopropyl, Phenyl, Furyl, Thienyl und Pyrazolyl,
und
wobei Cyclopropyl, Cyclobutyl, Cyclohexyl, Tetrahydrofuranyl, Morpholin-4-yl, Azetidiny-1-yl, Pyrrolidin-1-yl, Piperidin-1-yl, Piperazin-1-yl, Phenyl, Furyl, Thiazolyl, Pyrazolyl, Pyridyl, Cyclohexylamino und Phenylamino substituiert sein können mit 1 bis 2 Substituenten, unabhängig voneinander ausgewählt aus der Gruppe bestehend aus Halogen, Cyano, Oxo, Hydroxy, Amino, Trifluormethyl, Difluormethoxy, Trifluormethoxy, Hydroxycarbonyl, Aminocarbonyl, Methyl, Ethyl, Methoxy, Ethoxy, Dimethylamino, Methoxycarbonyl, Ethoxycarbonyl, Dimethylaminocarbonyl und Cyclopropyl,
worin Methyl und Ethyl substituiert sein können mit einem Substituenten Hydroxy,
und ihre Salze, ihre Solvate und die Solvate ihrer Salze.

Bevorzugt sind auch Verbindungen der Formel (I), in welcher
- A: für eine Gruppe der Formel 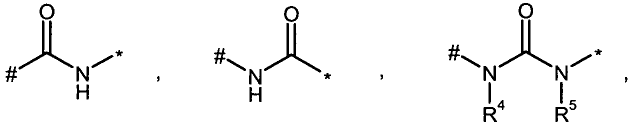 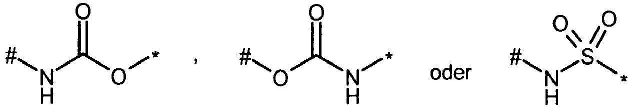 steht,
wobei
# die Anknüpfstelle an den Piperidinring ist,
* die Anknüpfstelle an R² ist,
R⁴ für Wasserstoff oder Methyl steht,
und
R⁵ für Wasserstoff oder Methyl steht,
- R¹: für Phenyl steht,
wobei Phenyl substituiert ist mit 1 bis 2 Substituenten, unabhängig voneinander ausgewählt aus der Gruppe bestehend aus Trifluormethyl, Trifluormethoxy, Methyl und Ethyl,
- R²: für Methyl, Ethyl, Isopropyl, tert-Butyl, Cyclopropyl, Cyclopentyl, Phenyl, Thienyl oder Pyridyl steht,
wobei Cyclopentyl, Phenyl, Thienyl und Pyridyl substituiert sein können mit 1 bis 2 Substituenten, unabhängig voneinander ausgewählt aus der Gruppe bestehend aus Halogen, Cyano, Trifluormethyl, Trifluormethoxy, Methyl, Methoxy und Phenyl,
worin Phenyl substituiert sein kann mit 1 bis 2 Substituenten, unabhängig voneinander ausgewählt aus der Gruppe bestehend aus Chlor, Fluor und Trifluormethyl,
und
wobei Methyl substituiert sein kann mit einem Substituenten Phenyl,
- R³: für Morpholin-4-yl, 1,1-Dioxidothiomorpholin-4-yl, 3-Hydroxyazetidiny-1-yl, 3-Hydroxypyrrolidin-1-yl, 4-Cyanopiperidin-1-yl oder 4-Hydroxypiperidin-1-yl steht,
und ihre Salze, ihre Solvate und die Solvate ihrer Salze.

Bevorzugt sind auch Verbindungen der Formel (I), in welcher
- A: für eine Gruppe der Formel 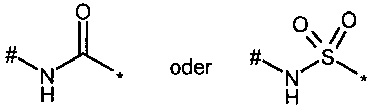 steht,
wobei
# die Anknüpfstelle an den Piperidinring ist,
* die Anknüpfstelle an R² ist,
- R¹: für Phenyl steht,
wobei Phenyl substituiert ist mit 1 bis 2 Substituenten, unabhängig voneinander ausgewählt aus der Gruppe bestehend aus Trifluormethyl, Trifluormethoxy, Methyl und Ethyl,
- R²: für Methyl, Ethyl, Isopropyl, tert-Butyl, Cyclopropyl, Cyclopentyl, Phenyl, Thienyl oder Pyridyl steht,
wobei Cyclopentyl, Phenyl, Thienyl und Pyridyl substituiert sein können mit 1 bis 2 Substituenten, unabhängig voneinander ausgewählt aus der Gruppe bestehend aus Halogen, Cyano, Trifluormethyl, Trifluormethoxy, Methyl, Methoxy und Phenyl,
worin Phenyl substituiert sein kann mit 1 bis 2 Substituenten, unabhängig voneinander ausgewählt aus der Gruppe bestehend aus Chlor, Fluor und Trifluormethyl,
und
wobei Methyl substituiert sein kann mit einem Substituenten Phenyl,
- R³: für Morpholin-4-yl, 1,1-Dioxidothiomorpholin-4-yl, 3-Hydroxyazetidiny-1-yl, 3-Hydroxypyrrolidin-1-yl, 4-Cyanopiperidin-1-yl oder 4-Hydroxypiperidin-1-yl steht,
und ihre Salze, ihre Solvate und die Solvate ihrer Salze.

Bevorzugt sind auch Verbindungen der Formel (I), in welcher die Substituenten -R¹ und -A-R² in cis-Position zueinander stehen.

Bevorzugt sind auch Verbindungen der Formel (I), in welcher
- A: für eine Gruppe der Formel 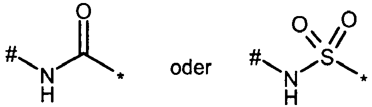 steht,
wobei
# die Anknüpfstelle an den Piperidinring ist,
* die Anknüpfstelle an R² ist.

Bevorzugt sind auch Verbindungen der Formel (I), in welcher
- A: für eine Gruppe der Formel 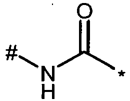 steht,
wobei
# die Anknüpfstelle an den Piperidinring ist,
* die Anknüpfstelle an R² ist.

Bevorzugt sind auch Verbindungen der Formel (I), in welcher
- A: für eine Gruppe der Formel 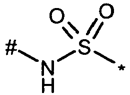 steht,
wobei
# die Anknüpfstelle an den Piperidinring ist,
* die Anknüpfstelle an R² ist.

Bevorzugt sind auch Verbindungen der Formel (I), in welcher
- A: für eine Gruppe der Formel 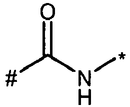 steht,
wobei
# die Anknüpfstelle an den Piperidinring ist,
* die Anknüpfstelle an R² ist.

Bevorzugt sind auch Verbindungen der Formel (I), in welcher
- A: für eine Gruppe der Formel 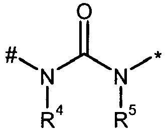 steht,
wobei
# die Anknüpfstelle an den Piperidinring ist,
* die Anknüpfstelle an R² ist,
R⁴ für Wasserstoff oder Methyl steht,
und
R⁵ für Wasserstoff oder Methyl steht.

Bevorzugt sind auch Verbindungen der Formel (I), in welcher
- A: für eine Gruppe der Formel 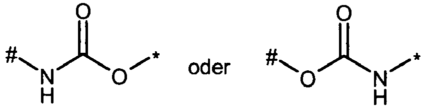 steht,
wobei
# die Anknüpfstelle an den Piperidinring ist,
* die Anknüpfstelle an R² ist.

Bevorzugt sind auch Verbindungen der Formel (I), in welcher R¹ für Phenyl steht, wobei Phenyl substituiert ist mit einem Substituenten in para-Position zur Verknüpfungsstelle an den Piperidinring, ausgewählt aus der Gruppe bestehend aus Trifluormethyl, Trifluormethoxy und Ethyl.

Bevorzugt sind auch Verbindungen der Formel (I), in welcher R¹ für Phenyl steht, wobei Phenyl substituiert ist mit einem Substituenten Ethyl in para-Position zur Verknüpfungsstelle an den Piperidinring.

Bevorzugt sind auch Verbindungen der Formel (I), in welcher
- R²: für Methyl, Ethyl, Isopropyl, tert-Butyl, Cyclopropyl, Cyclopentyl, Phenyl, Thienyl oder Pyridyl steht,
wobei Cyclopentyl, Phenyl, Thienyl und Pyridyl substituiert sein können mit 1 bis 2 Substituenten, unabhängig voneinander ausgewählt aus der Gruppe bestehend aus Halogen, Cyano, Trifluormethyl, Trifluormethoxy, Methyl, Methoxy und Phenyl,
worin Phenyl substituiert sein kann mit 1 bis 2 Substituenten, unabhängig voneinander ausgewählt aus der Gruppe bestehend aus Chlor, Fluor und Trifluormethyl,
und
wobei Methyl substituiert sein kann mit einem Substituenten Phenyl.

Bevorzugt sind auch Verbindungen der Formel (I), in welcher R² für Phenyl steht.

Bevorzugt sind auch Verbindungen der Formel (I), in welcher R³ für Morpholin-4-yl, 1,1-Dioxidothiomorpholin-4-yl, 3-Hydroxyazetidiny-1-yl, 3-Hydroxypyrrolidin-1-yl, 4-Cyanopiperidin-1-yl oder 4-Hydroxypiperidin-1-yl steht.

Bevorzugt sind auch Verbindungen der Formel (I), in welcher R³ für Morpholin-4-yl steht.

Bevorzugt sind auch Verbindungen der Formel (I), in welcher R³ für 4-Hydroxypiperidin-1-yl steht.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Gegenstand der Erfindung ist weiterhin ein Verfahren zur Herstellung der Verbindungen der Formel (I), oder ihrer Salze, ihrer Solvate oder der Solvate ihrer Salze, wobei entweder
[A] Verbindungen der Formel 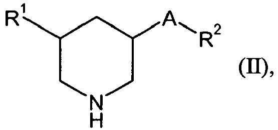 in welcher
   A, R¹ und R² die oben angegebene Bedeutung aufweisen,
   mit Verbindungen der Formel 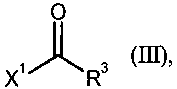 in welcher
   R³ die oben angegebene Bedeutung aufweist, und
   - X¹: für Halogen, bevorzugt Brom oder Chlor, oder Hydroxy steht,
   umgesetzt werden
   oder
[B] Verbindungen der Formel (II) mit Verbindungen der Formel 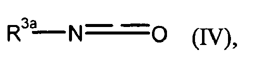 in welcher
   - R^{3a}: für C₁-C₆-Alkyl, C₃-C₇-Cycloalkyl, 4- bis 7-gliedriges Heterocyclyl, Phenyl oder 5- oder 6-gliedriges Heteroaryl steht,
   wobei Alkyl substituiert sein kann mit einem Substituenten ausgewählt aus der Gruppe bestehend aus Halogen, Hydroxy, Amino, Cyano, C₁-C₄-Alkoxy, C₁-C₄-Alkoxycarbonyl, C₃-C₇-Cycloalkyl, 4- bis 6-gliedriges Heterocyclyl, Phenyl und 5- oder 6-gliedriges Heteroaryl,
   worin Alkoxy substituiert sein kann mit einem Substituenten C₁-C₄-Alkoxy,
   und
   wobei Cycloalkyl, Heterocyclyl, Phenyl und Heteroaryl substituiert sein können mit 1 bis 3 Substituenten, unabhängig voneinander ausgewählt aus der Gruppe bestehend aus Halogen, Cyano, Oxo, Hydroxy, Amino, Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, Hydroxycarbonyl, Aminocarbonyl, C₁-C₄-Alkyl, C₁-C₄-Alkoxy, C₁-C₆-Alkylanüno, C₁-C₄-Alkoxycarbonyl, C₁-C₄-Alkylaminocarbonyl und Cyclopropyl,
   worin Alkyl substituiert sein kann mit einem Substituenten Hydroxy,
   zu Verbindungen der Formel 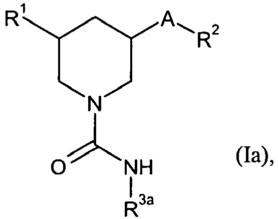 in welcher
   A, R¹, R² und R^{3a} die oben angegebene Bedeutung aufweisen,
   umgesetzt werden
   oder
[C] Verbindungen der Formel 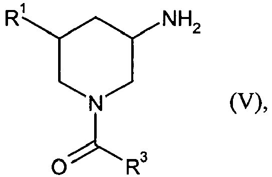 in welcher
   R¹ und R³ die oben angegebene Bedeutung aufweisen,
   mit Verbindungen der Formel 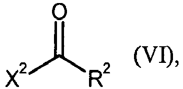 in welcher
   R² die oben angegebene Bedeutung aufweist, und
   X² für Halogen, bevorzugt Brom oder Chlor, oder Hydroxy steht,
   zu Verbindungen der Formel 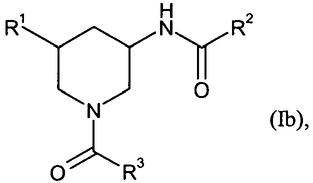 in welcher
   R¹, R² und R³ die oben angegebene Bedeutung aufweisen, umgesetzt werden
   oder
[D] Verbindungen der Formel (V) mit Verbindungen der Formel 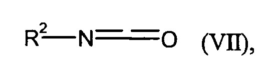 in welcher
   R² die oben angegebene Bedeutung aufweist,
   zu Verbindungen der Formel 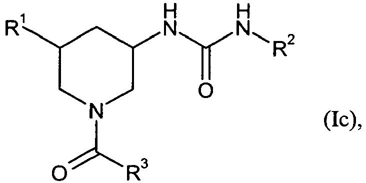 in welcher
   R¹, R² und R³ die oben angegebene Bedeutung aufweisen,
   umgesetzt werden
   oder
[E] Verbindungen der Formel (V) mit Verbindungen der Formel 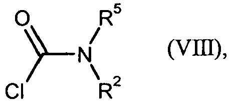 in welcher
   R² und R⁵ die oben angegebene Bedeutung aufweisen,
   zu Verbindungen der Formel 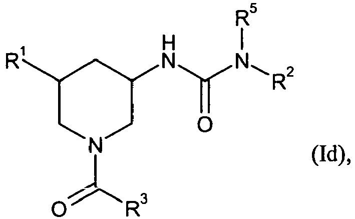 in welcher
   R¹, R², R³ und R⁵ die oben angegebene Bedeutung aufweisen,
   umgesetzt werden
   oder
[F] Verbindungen der Formel (Id) mit Verbindungen der Formel 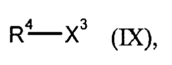 in welcher
   R⁴ die oben angegebene Bedeutung aufweist, und
   X³ für Halogen, bevorzugt Iod, Brom oder Chlor, steht,
   zu Verbindungen der Formel 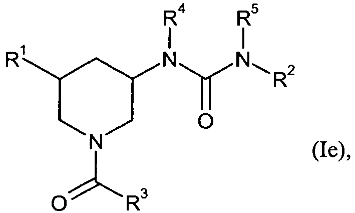 in welcher
   R¹, R², R³, R⁴ und R⁵ die oben angegebene Bedeutung aufweisen,
   umgesetzt werden
   oder
[G] Verbindungen der Formel (V) mit Verbindungen der Formel 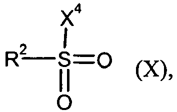 in welcher
   R² die oben angegebene Bedeutung aufweist, und
   X⁴ für Chlor oder Hydroxy steht,
   zu Verbindungen der Formel 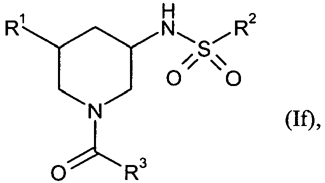 in welcher
   R¹, R² und R³ die oben angegebene Bedeutung aufweisen,
   umgesetzt werden
   oder
[H] Verbindungen der Formel 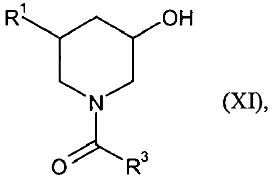 in welcher
   R¹ und R³ die oben angegebene Bedeutung aufweisen,
   zunächst mit Disuccinimidylcarbonat und anschließend mit Verbindungen der Formel

   H₂N-R² (XII),

   in welcher
   R² die oben angegebene Bedeutung aufweist,
   zu Verbindungen der Formel 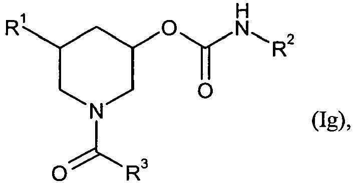 in welcher
   R¹, R² und R³ die oben angegebene Bedeutung aufweisen,
   umgesetzt werden.

Die Umsetzung nach Verfahren [A] erfolgt, wenn X² für Halogen steht, im Allgemeinen in inerten Lösungsmitteln, gegebenenfalls in Gegenwart einer Base, bevorzugt in einem Temperaturbereich von -30°C bis 50°C bei Normaldruck.

Inerte Lösungsmittel sind beispielsweise Tetrahydrofuran, Methylenchlorid, Pyridin, Dioxan oder Dimethylformamid, bevorzugt ist Tetrahydrofuran.

Basen sind beispielsweise Triethylamin, Diisopropylethylamin oder *N*-Methylmorpholin, bevorzugt ist Triethylamin oder Diisopropylethylamin.

Die Umsetzung nach Verfahren [A] erfolgt, wenn X² für Hydroxy steht, im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Als Dehydratisierungsreagenzien eignen sich hierbei beispielsweise Carbodiimide wie z.B. *N,N'-*Diethyl-, *N*,*N*,*'*-Dipropyl-, *N,N'*-Diisopropyl-, *N,N'*-Dicyclohexylcarbodiimid, *N*-(3-Di-methylaminoisopropyl)-*N'*-ethylcarbodiimid-hydrochlorid (EDC), *N*-Cyclohexylcarbodiimid-*N'-*propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder *2-tert-*Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)-phosphoniumhexafluorophosphat, oder *O*-(Benzotriazol-1-yl)-*N,N,N',N*'-tetra-methyluronium-hexafluorophosphat (HBTU), 2-(2-Oxo-1-(2H)-pyridyl)-1,1,3,3-tetramethyluroniumtetrafluoro-borat (TPTU) oder *O*-(7-Azabenzotriazol-1-yl)-*N,N,N'N'*-tetramethyl-uroniumhexafluorophosphat (HATU), oder 1-Hydroxybenzotriazol (HOBt), oder Benzotriazol-1-yloxytris(dimethylamino)-phosphoniumhexafluorophosphat (BOP), oder *N*-Hydroxysuccinimid, oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin.

Vorzugsweise wird die Kondensation mit HATU oder mit EDC in Gegenwart von HOBt durchgeführt.

Die Verbindungen der Formel (III) sind bekannt oder lassen sich nach bekannten Verfahren aus den entsprechenden Ausgangsverbindungen synthetisieren.

Die Umsetzung nach Verfahren [B] erfolgt im Allgemeinen in inerten Lösungsmitteln, gegebenenfalls in Gegenwart einer Base, bevorzugt in einem Temperaturbereich von 0°C bis 50°C bei Normaldruck.

Inerte Lösungsmittel sind beispielsweise Tetrahydrofuran, Methylenchlorid, Dioxan oder Dimethylformamid, bevorzugt ist Tetrahydrofuran.

Basen sind beispielsweise Triethylamin, Diisopropylethylamin oder *N*-Methylmorpholin, bevorzugt ist Triethylamin oder Diisopropylethylamin.

Die Verbindungen der Formel (IV) sind bekannt oder lassen sich nach bekannten Verfahren aus den entsprechenden Ausgangsverbindungen synthetisieren.

Die Umsetzung nach Verfahren [C] erfolgt wie für Verfahrens [A] beschrieben.

Die Verbindungen der Formel (VI) sind bekannt oder lassen sich nach bekannten Verfahren aus den entsprechenden Ausgangsverbindungen synthetisieren.

Die Umsetzung nach Verfahren [D] erfolgt wie für Verfahrens [B] beschrieben.

Die Verbindungen der Formel (VII) sind bekannt oder lassen sich nach bekannten Verfahren aus den entsprechenden Ausgangsverbindungen synthetisieren.

Die Umsetzung nach Verfahren [E] erfolgt wie für Verfahrens [B] beschrieben.

Die Verbindungen der Formel (VIII) sind bekannt oder lassen sich nach bekannten Verfahren aus den entsprechenden Ausgangsverbindungen synthetisieren.

Die Umsetzung nach Verfahren [F] erfolgt im Allgemeinen in inerten Lösungsmitteln, in Gegenwart einer Base, gegebenenfalls in Gegenwart von Kaliumiodid, bevorzugt in einem Temperaturbereich von Raumtemperatur bis zum Rückfluß der Lösungsmittel bei Normaldruck.

Inerte Lösungsmittel sind beispielsweise Halogenkohlenwasserstoffe wie Methylenchlorid, Trichlormethan oder 1,2-Dichlorethan, Ether wie Dioxan, Tetrahydrofuran oder 1,2-Dimethoxyethan, oder andere Lösemittel wie Aceton, Dimethylformamid, Dimethylacetamid, 2-Butanon oder Acetonitril, bevorzugt ist Dimethylformamid.

Basen sind beispielsweise Alkalicarbonate wie Cäsiumcarbonat, Natrium- oder Kaliumcarbonat, oder Natrium- oder Kaliummethanolat, oder Natrium- oder Kaliumethanolat oder Kalium-tert.-butylat, oder Amide wie Natriumamid, Lithium-bis-(trimethylsilyl)amid oder Lithiumdiisopropylamid, oder metallorganische Verbindungen wie Butyllithium oder Phenyllithium, oder andere Basen wie Natriumhydrid, DBU, bevorzugt ist Natriumhydrid.

Die Verbindungen der Formel (IX) sind bekannt oder lassen sich nach bekannten Verfahren aus den entsprechenden Ausgangsverbindungen synthetisieren.

Die Umsetzung nach Verfahren [G] erfolgt X² für Halogen steht, im Allgemeinen in inerten Lösungsmitteln, gegebenenfalls in Gegenwart einer Base, bevorzugt in einem Temperaturbereich von 0°C bis 50°C bei Normaldruck.

Inerte Lösungsmittel sind beispielsweise Tetrahydrofuran, Methylenchlorid, Pyridin, Dioxan oder Dimethylformamid, bevorzugt ist Methylenchlorid.

Basen sind beispielsweise Triethylamin, Diisopropylethylamin oder *N*-Methylmorpholin, bevorzugt ist Triethylamin oder Diisopropylethylamin.

Die Umsetzung nach Verfahren [G] erfolgt, wenn X² für Hydroxy steht, im Allgemeinen in inerten Lösungsmitteln, in Gegenwart von 4-Dimethylaminopyridin, gegebenenfalls in Gegenwart einer Base, bevorzugt in einem Temperaturbereich von 0°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Basen sind beispielsweise organische Basen wie Trialkylamine z.B. Triethylamin, *N*-Methylmorpholin, *N*-Methylpiperidin, 4-Dimethylaminopyridin oder Diisopropylethylamin, bevorzugt ist Diisopropylethylamin.

Die Verbindungen der Formel (X) sind bekannt oder lassen sich nach bekannten Verfahren aus den entsprechenden Ausgangsverbindungen synthetisieren.

Die Umsetzung nach Verfahren [H] erfolgt im Allgemeinen in inerten Lösungsmitteln, gegebenenfalls in Gegenwart einer Base, bevorzugt in einem Temperaturbereich von 0°C bis 50°C bei Normaldruck.

Basen sind beispielsweise organische Basen wie Trialkylamine z.B. Triethylamin, *N*-Methylmorpholin, *N*-Methylpiperidin, 4-Dimethylaminopyridin oder Diisopropylethylamin, bevorzugt ist Diisopropylethylamin.

Die Verbindungen der Formel (XII) sind bekannt oder lassen sich nach bekannten Verfahren aus den entsprechenden Ausgangsverbindungen synthetisieren.

Die Verbindungen der Formel (II) sind bekannt oder können hergestellt werden, indem Verbindungen der Formel 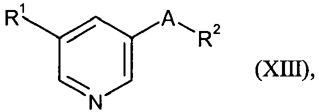 in welcher
A, R¹ und R² die oben angegebene Bedeutung aufweisen,
hydriert werden.

Die Hydrierung erfolgt im Allgemeinen mit einem Reduktionsmittel in inerten Lösungsmitteln, gegebenenfalls unter Zusatz von Säure wie Mineralsäuren und Carbonsäuren, bevorzugt Essigsäure, bevorzugt in einem Temperaturbereich von Raumtemperatur bis zum Rückfluss der Lösungsmittel und in einem Druckbereich von Normaldruck bis 100 bar, bevorzugt bei 50-80 bar.

Als Reduktionsmittel ist bevorzugt Wasserstoff mit Palladium auf Aktivkohle, mit Rhodium auf Aktivkohle, mit Ruthenium auf Aktivkohle oder daraus gemischte Katalysatom, oder Wasserstoff mit Palladium auf Aluminiumoxid oder mit Rhodium auf Aluminiumoxid, bevorzugt ist Wasserstoff mit Palladium auf Aktivkohle oder mit Rhodium auf Aktivkohle.

Inerte Lösungsmittel sind beispielsweise Alkohole wie Methanol, Ethanol, n-Propanol, iso-Propanol, n-Butanol oder tert-Butanol, bevorzugt ist Methanol oder Ethanol.

Die Verbindungen der Formel (XIII) sind bekannt oder können hergestellt werden, indem Verbindungen der Formel 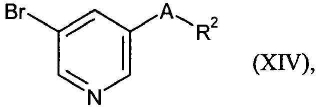 in welcher
A und R² die oben angegebene Bedeutung aufweisen,
mit Verbindungen der Formel 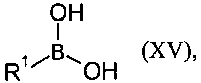 in welcher
R¹ die oben angegebene Bedeutung aufweist,
umgesetzt werden.

Die Umsetzung erfolgt im Allgemeinen in inerten Lösungsmitteln, in Gegenwart eines Katalysators, gegebenenfalls in Gegenwart eines Zusatzreagenzes, bevorzugt in einem Temperaturbereich von Raumtemperatur bis zum Rückfluss des Lösungsmittels bei Normaldruck. Inerte Lösungsmittel sind beispielsweise Ether wie Dioxan, Tetrahydrofuran oder 1,2-Dimethoxyethan, Kohlenwasserstoffe wie Benzol, Xylol oder Toluol, oder andere Lösungsmittel wie Nitrobenzol, Dimethylformamid, Dimethylacetamid, Dimethylsulfoxid oder *N-*Methylpyrrolidon, gegebenenfalls wird diesen Lösungsmitteln etwas Wasser zugesetzt. Bevorzugt ist Toluol mit Wasser oder eine Mischung aus 1,2-Dimethoxyethan, Dimethylformamid und Wasser.

Katalysatoren sind beispielsweise für Suzuki-Reaktionsbedingungen übliche Palladium-Katalysatoren, bevorzugt sind Katalysatoren wie z.B. Dichlorbis(triphenylphosphin)-palladium, Tetrakistriphenylphosphinpalladium(0), Palladium(II)acetat oder Bis-(diphenylphosphan-ferrocenyl)-palladium-(II)-chlorid.

Zusatzreagenzien sind beispielsweise Kaliumacetat, Cäsium-, Kalium- oder Natriumcarbonat, Bariumhydroxid, Kalium-tert-butylat, Cäsiumfluorid, Kaliumfluorid oder Kaliumphosphat, bevorzugt sind Kaliumfluorid oder Natriumcarbonat.

Die Verbindungen der Formeln (XIV) und (XV) sind bekannt oder lassen sich nach bekannten Verfahren aus den entsprechenden Ausgangsverbindungen synthetisieren.

Die Verbindungen der Formel 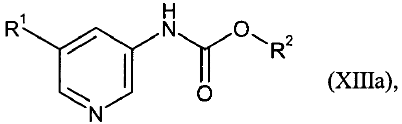 in welcher
R¹ und R² die oben angegebene Bedeutung aufweisen,
sind bekannt oder können hergestellt werden, indem Verbindungen der Formel 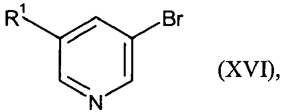 in welcher
R¹ die oben angegebene Bedeutung aufweist,
mit Verbindungen der Formel 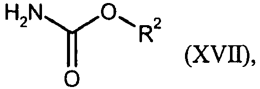 in welcher
R² die oben angegebene Bedeutung aufweist,
umgesetzt werden.

Die Umsetzung erfolgt im Allgemeinen in inerten Lösungsmitteln, in Gegenwart eines Katalysators, in Gegenwart eines Liganden, gegebenenfalls in Gegenwart eines Zusatzreagenzes, bevorzugt in einem Temperaturbereich von Raumtemperatur bis zum Rückfluss des Lösungsmittels bei Normaldruck.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, oder andere Lösungsmittel wie Nitrobenzol, Dimethylformamid, Dimethylacetamid, Dimethylsulfoxid oder *N-*Methylpyrrolidon, gegebenenfalls wird diesen Lösungsmitteln etwas Wasser zugesetzt. Bevorzugt ist Dioxan.

Katalysatoren sind beispielsweise für Buchwald-Hartwig-Kreuzkupplungen übliche Palladium-Katalysatoren, bevorzugt sind Katalysatoren wie z.B. Dichlorbis(triphenylphosphin)-palladium, Tetrakistriphenylphosphinpalladium(0), Tris(dibenzylidenaceton)dipalladium, Palladium(II)acetat oder Bis-(diphenylphosphan-ferrocenyl)-palladium-(II)-chlorid, bevorzugt ist Tris(dibenzyliden-aceton)dipalladium.

Liganden sind beispielsweise 4,5-Bis(diphenylphosphino)-9,9'-dimethylxanthen, 1,2-Bis(diphenylphosphino)ethan, 2,2'-Bis(diphenylphosphino)-1,1'-binaphtyl (BINAP) und 1,1'-Bis(diphenylphosphanyl)ferrocen, bevorzugt ist 4,5-Bis(diphenylphosphino)-9,9'-dimethylxanthen.

Zusatzreagenzien sind beispielsweise Kaliumacetat, Cäsium-, Kalium- oder Natriumcarbonat, Bariumhydroxid, Kalium-tert-butylat, Cäsiumfluorid, Kaliumfluorid oder Kaliumphosphat, bevorzugt Cäsiumcarbonat.

Die Verbindungen der Formel (XVI) sind bekannt oder lassen sich nach bekannten Verfahren aus den entsprechenden Ausgangsverbindungen synthetisieren. Die Verbindungen der Formel (XVI) können auch nach dem für die Umsetzung von Verbindungen der Formel (XIV) mit Verbindungen der Formel (XV) beschriebenen Verfahren aus den entsprechenden Ausgangsverbindungen synthetisieren werden.

Die Verbindungen der Formel (XVII) sind bekannt oder lassen sich nach bekannten Verfahren aus den entsprechenden Ausgangsverbindungen synthetisieren.

Die Verbindungen der Formel (V) sind bekannt oder können hergestellt werden, indem Verbindungen der Formel 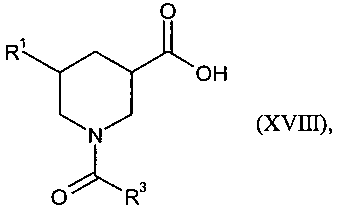 in welcher
R¹ und R³ die oben angegebene Bedeutung aufweisen,
mit Diphenylphosphorazidat umgesetzt werden und die Umsetzung anschließend gegebenenfalls unter Zugabe von Säure aufgearbeitet wird.

Die Umsetzung erfolgt im Allgemeinen in Lösungsmitteln, in Gegenwart einer Base, gegebenenfalls in Gegenwart von Di-tert-butyldicarbonat, gegebenenfalls in Gegenwart von Molsieb, bevorzugt in einem Temperaturbereich von Raumtemperatur bis zum Rückfluss der Lösungsmittel bei Normaldruck.

Lösungsmittel sind beispielsweise Alkohole wie Methanol, Ethanol, n-Propanol, iso-Propanol, n-Butanol oder tert-Butanol, oder Gemische dieser Alkohole mit Kohlenwasserstoffen wie Benzol, Xylol oder Toluol, bevorzugt ist ein Gemisch aus tert-Butanol und Toluol.

Basen sind beispielsweise organische Basen wie Trialkylamine z.B. Triethylamin, *N*-Methylmorpholin, *N*-Methylpiperidin, 4-Dimethylaminopyridin oder Diisopropylethylamin, bevorzugt ist Triethylamin.

Säuren sind beispielsweise Trifluoressigsäure, Trifluoressigsäure in Dichlormethan oder konzentierte Salzsäure.

Die Verbindungen der Formel (XVIII) sind bekannt oder können hergestellt werden, indem Verbindungen der Formel 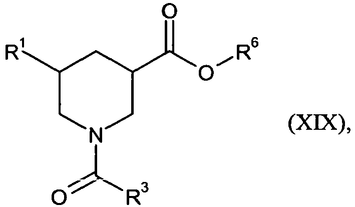 in welcher
R¹ und R³ die oben angegebene Bedeutung aufweisen, und
R⁶ für Methyl oder Ethyl steht,
mit einer Base umgesetzt werden.

Die Umsetzung erfolgt im Allgemeinen in inerten Lösungsmitteln, in Gegenwart einer Base, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Methylenchlorid, Trichlormethan, Tetrachlormethan oder 1,2-Dichlorethan, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist ein Gemisch aus Tetrahydrofuran und Wasser.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, bevorzugt ist Lithiumhydroxid.

Die Verbindungen der Formel (XIX) sind bekannt oder können hergestellt werden, indem Verbindungen der Formel 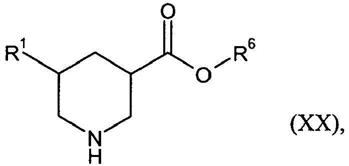 in welcher
R¹ und R⁶ die oben angegebene Bedeutung aufweisen,
mit Verbindungen der Formel (III) umgesetzt werden.

Die Umsetzung erfolgt wie unter Verfahren [A] beschrieben.

Die Verbindungen der Formel (XX) sind bekannt oder können hergestellt werden, indem Verbindungen der Formel 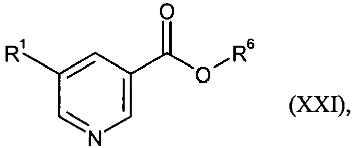 in welcher
R¹ und R⁶ die oben angegebene Bedeutung aufweisen,
hydriert werden.

Die Hydrierung erfolgt nach den unter der Hydrierung von Verbindungen der Formel (XIII) angegebenen Reaktionsbedingungen.

Die Verbindungen der Formel (XXI) sind bekannt oder können hergestellt werden, indem Verbindungen der Formel 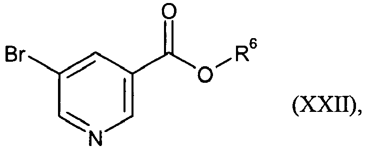 mit Verbindungen der Formel (XV) umgesetzt werden.

Die Umsetzung erfolgt nach den unter der Umsetzung von Verbindungen der Formel (XIV) mit Verbindungen der Formel (XV) angegebenen Reaktionsbedingungen.

Die Verbindungen der Formel (XXII) sind bekannt oder lassen sich nach bekannten Verfahren aus den entsprechenden Ausgangsverbindungen synthetisieren.

Die Verbindungen der Formel (XI) sind bekannt oder können hergestellt werden, indem Verbindungen der Formel 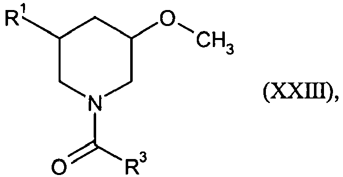 in welcher
R¹ und R³ die oben angegebene Bedeutung aufweisen,
mit Bortribromid umgesetzt werden.

Die Umsetzung erfolgt im Allgemeinen in inerten Lösungsmitteln, bevorzugt in einem Temperaturbereich von -20°C bis Raumtemperatur bei Normaldruck.

Inerte Lösungsmittel sind beispielsweise Halogenkohlenwasserstoffe wie Methylenchlorid, Trichlormethan, Tetrachlormethan oder 1,2-Dichlorethan, bevorzugt ist Methylenchlorid.

Die Verbindungen der Formel (XXIII) sind bekannt oder können hergestellt werden, indem Verbindungen der Formel 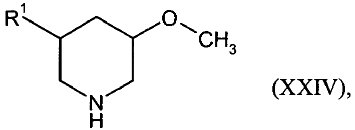 in welcher
R¹ die oben angegebene Bedeutung aufweist,
mit Verbindungen der Formel (III) umgesetzt werden.

Die Umsetzung erfolgt wie unter Verfahren [A] beschrieben.

Die Verbindungen der Formel (XXIV) sind bekannt oder können hergestellt werden, indem Verbindungen der Formel 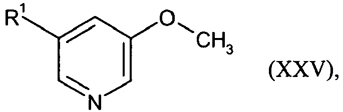 in welcher
R¹ die oben angegebene Bedeutung aufweist,
hydriert werden.

Die Hydrierung erfolgt nach den unter der Hydrierung von Verbindungen der Formel (XIII) angegebenen Reaktionsbedingungen.

Die Verbindungen der Formel (XXV) sind bekannt oder können hergestellt werden, indem die Verbindung der Formel 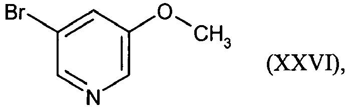 mit Verbindungen der Formel (XV) umgesetzt wird.

Die Umsetzung erfolgt nach den unter der Umsetzung von Verbindungen der Formel (XIV) mit Verbindungen der Formel (XV) angegebenen Reaktionsbedingungen.

Die Verbindung der Formel (XXVI) ist bekannt oder läßt sich nach bekannten Verfahren aus den entsprechenden Ausgangsverbindungen synthetisieren.

In den Verbindungen der oben genannten Verfahren sind freie Aminogruppen gegebenenfalls während der Umsetzung durch dem Fachmann bekannte Schutzgruppen geschützt, bevorzugt ist eine tert-Butoxycarbonyl-Schutzgruppe. Diese Schutzgruppen werden nach der Umsetzung durch dem Fachmann bekannte Reaktionen abgespalten, bevorzugt ist die Umsetzung mit Trifluoressigsäure oder konzentierter Salzsäure.

Die Herstellung der Verbindungen der Formel (I) kann durch folgende Syntheseschemata verdeutlicht werden.

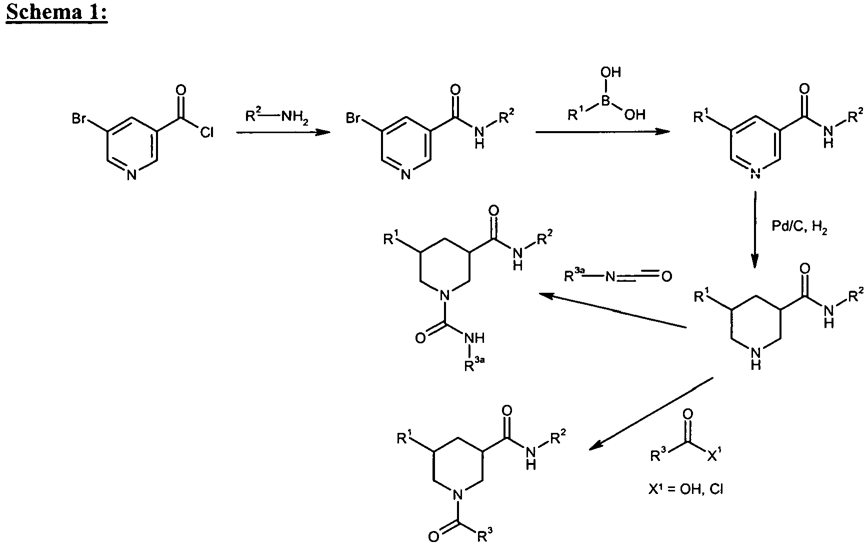

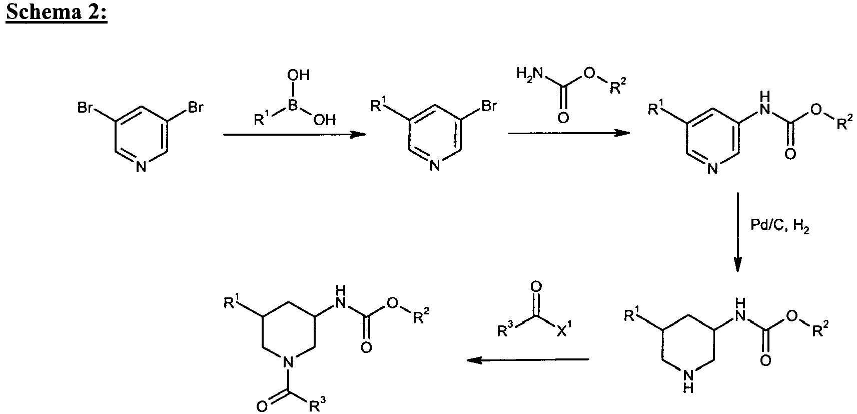

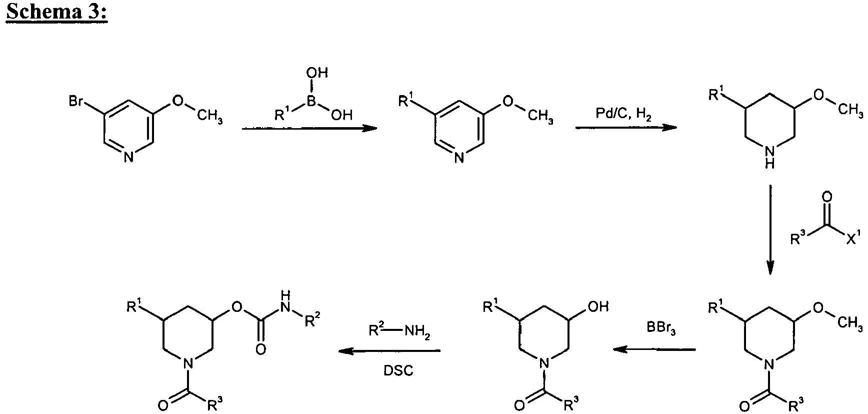

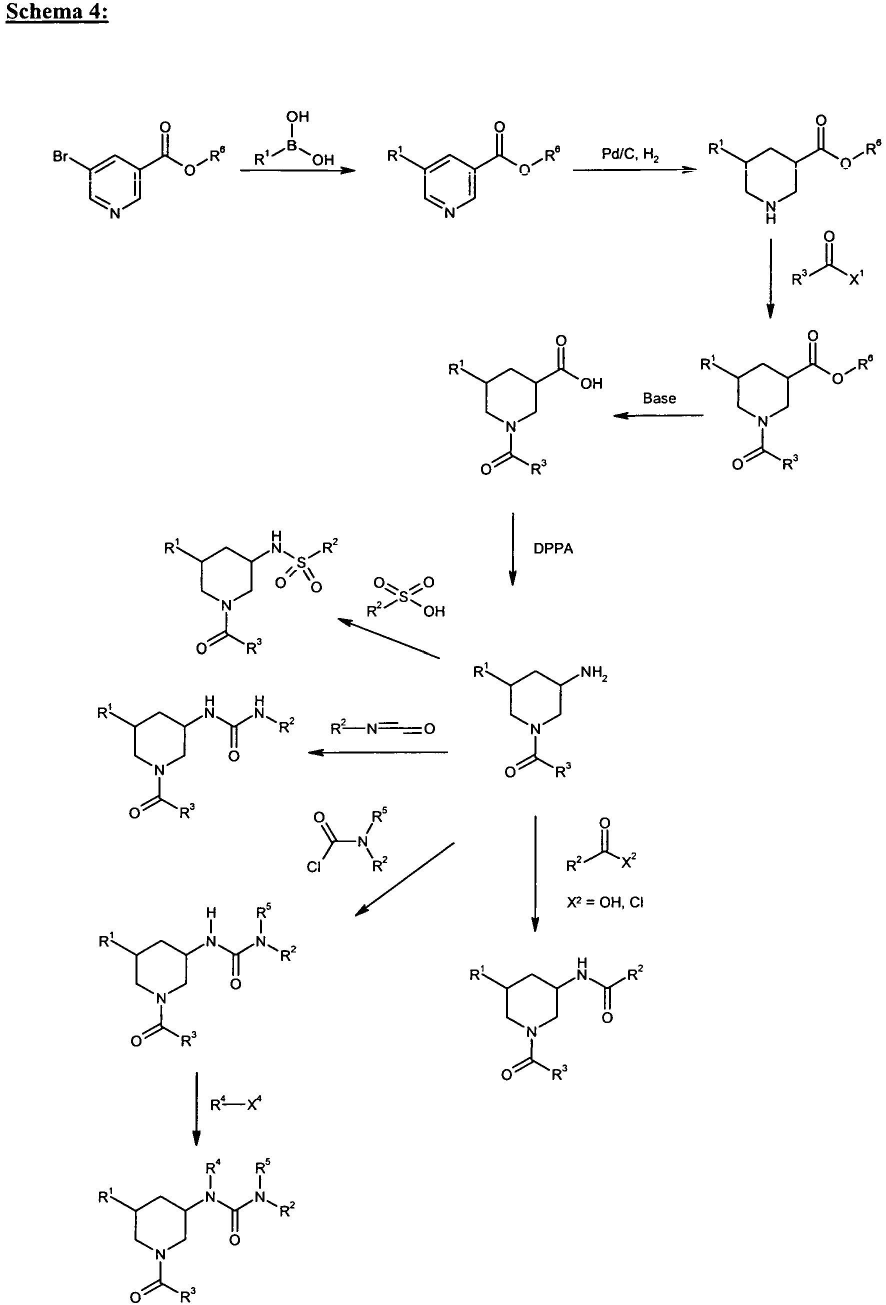

Die erfindungsgemäßen Verbindungen zeigen ein nicht vorhersehbares, wertvolles pharmakologisches und pharmakokinetisches Wirkspektrum. Es handelt sich dabei um selektive Antagonisten des PAR-1-Rezeptors, die insbesondere als Thrombozytenaggregationshemmer, als Hemmer der Endothelproliferation und als Hemmer des Tumorwachstums wirken.

Sie eigenen sich daher zur Verwendung als Arzneimittel zur Behandlung und/oder Prophylaxe von Krankheiten bei Menschen und Tieren.

Weiterer Gegenstand der vorliegenden Erfindung sind erfindungsgemäße Verbindungen zur Verwendung bei der Behandlung und/oder Prophylaxe von Erkrankungen, vorzugsweise von thromboembolischen Erkrankungen und/oder thromboembolischen Komplikationen.

Zu den "thromboembolischen Erkrankungen" im Sinne der vorliegenden Erfindung zählen insbesondere Erkrankungen wie Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie, Stentimplantation oder aortokoronarem Bypass, periphere arterielle Verschlusskrankheiten, Lungenembolien, tiefe venöse Thrombosen und Nierenvenenthrombosen, transitorische ischämische Attacken sowie thrombotischer und thromboembolischer Hirnschlag.

Die Substanzen eignen sich daher auch zur Prävention und Behandlung von kardiogenen Thromboembolien, wie beispielsweise Hirn-Ischämien, Schlaganfall (Stroke) und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit intravasalen Körpern, wie z. B. künstlichen Herzklappen, Kathetern, intraaortale Ballongegenpulsation und Schrittmachersonden.

Thromboembolische Komplikationen treten ferner auf bei mikroangiopathischen hämolytischen Anämien, extrakorporalen Blutkreisläufen, wie z. B. Hämodialyse, Hämofiltration, ventricular assist devices und Kunstherz, sowie Herzklappenprothesen.

Außerdem kommen die erfindungsgemäßen Verbindungen auch zur Beeinflussung der Wundheilung, für die Prophylaxe und/oder Behandlung von atherosklerotischen Gefäßerkrankungen und entzündlichen Erkrankungen wie rheumatische Erkrankungen des Bewegungsapparats, koronaren Herzkrankheiten, von Herzinsuffizienz, von Bluthochdruck, von entzündlichen Erkrankungen, wie z.B. Asthma, COPD, entzündlichen Lungenerkrankungen, Glomerulonephritis und entzündlichen Darmerkrankungen in Betracht, darüber hinaus ebenso für die Prophylaxe und/oder Behandlung der Alzheimer'schen Erkrankung, Autoimmunerkrankungen, Morbus Crohn und Kolitis Ulzerosa.

Außerdem können die erfindungsgemäßen Verbindungen zur Inhibition des Tumorwachstums und der Metastasenbildung, bei Mikroangiopathien, altersbedingter Makuladegeneration, diabetischer Retinopathie, diabetischer Nephropathie und anderen mikrovaskulären Erkrankungen sowie zur Prävention und Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Weiterhin eignen sich die erfindungsgemäßen Verbindungen zur Behandlung von Krebs. Krebserkrankungen schließen unter anderem ein: Karzinome (hierunter Brustkrebs, hepatozelluläre karzinome, Lunkenkrebs, kolorektaler Krebs, Kolonkrebs und Melanome), Lympohome (z.B. Non-Hodgkin-Lymphome und Mykosis fungoides), Leukämien, Sarkome, Mesotheliome, Hirnkrebs (z.B. Gliome), Germinome (z.B. Hodenkrebs und Ovarialkrebs), Choriokarzinome, Nierenkrebs, Bauchspeicheldrüsenkrebs, Schilddrüsenkrebs, Kopf- und Halskrebs, Endometrieum-Krebs, Zervix-Krebs, Blasenkrebs, Magenkrebs und multiples Myelom.

Außerdem vermittelt auf Endothelzellen exprimiertes PAR-1 Signale, die in Gefäßwachstum münden ("Angiogenese"), ein Vorgang, der zur Ermöglichung von Tumorwachstum über ca. 1 mm³ hinaus unerläßlich ist. Induktion der Angiogenese ist auch bei anderen Erkrankungen relevant, hierunter Erkrankungen des rheumatischen Formenkreises (z.B. rheumatoide Arthritis), bei Lungenerkrankungen (z.B. Lungenfibrose, pulmonale Hypertonie, insbesondere pulmonalarterielle Hypertonie, Erkrankungen, die durch Lungengefäßverschlüsse charakterisiert sind), Arteriosklerose, Plaqueruptur, diabetische Retinopathie und feuchte Makuladegeneration.

Darüber hinaus sind die erfindungsgemäßen Verbindungen zur Behandlung von Sepsis geeignet. Sepsis (oder Septikämie) ist eine häufige Erkrankung mit hoher Letalität. Anfängliche Symptome der Sepsis sind typischerweise unspezifisch (z.B. Fieber, reduziertes Allgemeinbefinden), im weiteren Verlauf kann es jedoch zur generalisierten Aktivierung des Gerinnungssystems kommen ("Disseminated Intravascular coagulation", oder "Verbrauchskoagulopathie", nachfolgend als "DIC" bezeichnet) mit Mikrothrombosierung in verschiedenen Organen und sekundärer Blutungskomplikationen. Außerdem kann es zur endothelialen Schädigung mit Erhöhung der Gefäßpermeabilität und Austritt von Flüssigkeit und Proteinen in den Extravasalraum kommen. Im weiteren Verlauf kann es zur Dysfunktion oder dem Versagen eines Organs (z.B. Nierenversagen, Leberversagen, Atemversagen, zentralnervöse Defizite und Herz-/Kreislaufversagen) bis hin zum Multiorganversagen kommen. Hiervon kann prinzipiell jedes Organ betroffen sein, am häufigsten tritt Organdysfunktion und -Versagen bei der Lunge, der Niere, dem Herz-Kreislaufsystem, dem Gerinnungssystem, dem zentralnervösen System, endokrinen Drüsen und der Leber auf. Eine Sepsis kann mit einem "Acute Respiratory Distress Syndrome" (nachfolgend als ARDS bezeichnet) einhergehen. Ein ARDS kann auch unabhängig von einer Sepsis auftreten. "Septischer Schock" bezeichnet das Auftreten einer behandlungspflichtigen Blutdruckerniedrigung, die eine weitere Organschädigung begünstigt und mit einer Verschlechterung der Prognose einhergeht.

Krankheitserreger können Bakterien (gram-negativ und gram-positiv), Pilze, Viren und/oder Eukaryonten sein. Eintrittspforte bzw. Primärinfektion können z.B. Pneumonie, Harnwegsinfekt, Peritonitis sein. Die Infektion kann, muß aber nicht zwingend, mit einer Bakteriämie einhergehen.

Sepsis wird definiert als das Vorliegen einer Infektion und eines "systemic inflammatory response syndrome" (nachfolgend mit "SIRS" bezeichnet). SIRS tritt im Rahmen von Infekten, aber auch von anderen Zuständen wie Verletzungen, Verbrennungen, Schock, Operationen, Ischämie, Pankreatitis, Reanimation oder Tumoren auf. Nach der Definition des ACCP/SCCM Consensus Conference Committee von 1992 (Crit. Care Med. 1992, 20, 864-874) werden die zur Diagnose "SIRS" erforderlichen Symptome zur Diagnose und Meßparameter beschrieben (u.a. veränderte Körpertemperatur, erhöhte Herzfrequenz, Atemschwierigkeiten und verändertes Blutbild). In der späteren (2001) SCCM/ESICM/ACCP/ATS/SIS International Sepsis Definitions Conference wurden die Kriterien im Wesentlichen beibehalten, in Details jedoch verfeinert (Levy et al., Crit. Care Med. 2003, 31, 1250-1256).

DIC und SIRS können im Rahmen einer Sepsis, aber auch infolge von Operationen, Tumorerkrankungen, Verbrennungen oder anderen Verletzungen auftreten. Bei der DIC kommt es an der Oberfläche von geschädigten Endothelzellen, Fremdkörperoberflächen oder verletztem extravaskulärem Gewebe zur massiven Aktivierung des Gerinnungssystems. Als Folge kommt es zur Gerinnung in kleinen Gefäßen verschiedener Organe mit Hypoxie und anschließender Organdysfunktion. Sekundär kommt es zum Verbrauch von Gerinnungsfaktoren (z.B. Faktor X, Prothrombin, Fibrinogen) und Plättchen, wodurch die Gerinnungsfähigkeit des Blutes herabgesetzt wird und schwere Blutungen auftreten können.

Die erfindungsgemäßen Verbindungen können darüber hinaus auch zur Verhinderung von Koagulation *ex vivo* eingesetzt werden, z.B. zur Konservierung von Blut- und Plasmaprodukten, zur Reinigung/Vorbehandlung von Kathetern und anderen medizinischen Hilfsmitteln und Geräten, einschließlich extrakorporaler Kreisläufe, zur Beschichtung künstlicher Oberflächen von *in vivo* oder *ex vivo* eingesetzten medizinischen Hilfsmitteln und Geräten oder bei biologischen Proben, die Blutplättchen enthalten.

Weiterer Gegenstand der vorliegenden Erfindung ist die Verwendung der erfindungsgemäßen Verbindungen zur Beschichtung von medizinischen Instrumenten und Implantaten, z.B. Kathetern, Prothesen, Stents oder künstlichen Herzklappen. Die erfindungsgemäßen Verbindungen können dabei fest an die Oberfläche gebunden sein oder über einen bestimmten Zeitraum aus einer Trägerbeschichtung in die unmittelbare Umgebung zur lokalen Wirkung freigesetzt werden.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
Kalziumkanalblocker, z.B. Amlodipin Besilat (z.B. Norvasc^{®}), Felodipin, Diltiazem, Verapamil, Nifedipin, Nicardipin, Nisoldipin und Bepridil;
Iomerizin;
Statine, z.B. Atorvastatin, Fluvastatin, Lovastatin, Pitavastatin, Pravastatin, Rosuvastatin, und Simvastatin;
Cholesterinabsorption-Inhibitoren, z.B. Ezetimibe und AZD4121;
Cholesteryl Ester Transfer Protein ("CETP") Inhibitoren, z.B. Torcetrapib;
Niedrigmolekualre Heparine, z.B. Dalteparin Natrium, Ardeparin, Certoparin, Enoxaparin, Parnaparin, Tinzaparin, Reviparin und Nadroparin;
Weitere Antikoagulanzien, z.B. Warfarin, Marcumar, Fondaparinux;
Antiarrhythmika, z.B. Dofetilid, Ibutilid, Metoprolol, Metoprololtartrat, Propranolol, Atenolol, Ajmalin, Disopyramid, Prajmalin, Procainamid, Quinidin, Spartein, Aprindine, Lidocain, Mexiletin, Tocamid, Encamid, Flecamid, Lorcamid, Moricizin, Propafenon, Acebutolol, Pindolol, Amiodaron, Bretylium-Tosylat, Bunaftin, Sotalol, Adenosin, Atropin und Digoxin;
Alpha-adrenerge Agonisten, z.B. Doxazosin-Mesylat, Terazoson und Prazosin;
Beta-adrenerge Blocker, z.B. Carvedilol, Propranolol, Timolol, Nadolol, Atenolol, Metoprolol, Bisoprolol, Nebivolol, Betaxolol, Acebutolol und Bisoprolol;
Aldosteron-Antagonisten, z.B. Eplerenon und Spironolacton;
Angiotensin-converting-enzyme Inhbitoren ("ACE-Inhibitoren"), z.B. Moexipril, Quinapril-Hydrochlorid, Ramipril, Lisinopril, Benazepril-Hydrochlorid, Enalapril, Captopril, Spirapril, Perindopril, Fosinopril und Trandolapril,;
Angiotensin II Receptorblockers ("ARBs"), z.B. Olmesartan-Medoxomil, Candesartan, Valsartan, Telmisartan, Irbesartan, Losartan und Eprosartan,;
Endothelinantagonisten, z.B. Tezosentan, Bosentan und Sitaxsentan-Natrium;
Neutral Endopeptidaseinhibitoren, z.B. Candoxatril und Ecadotril;
Phosphodiesteraseinhibitoren, z.B. Milrinoon, Theophyllin, Vinpocetin, EHNA (erythro-9-(2-hydroxy-3-nonyl)adenine), Sildenafil, Vardenafil und Tadalafil;
Fibrinolytika, z.B. Reteplase, Alteplase und Tenecteplase;
GP IIb/IIIa-Antagonisten, z.B. Integrillin, Abciximab und Tirofiban;
Direkte Thrombininhibitoren, z.B. AZD0837, Argatroban, Bivalirudin und Dabigatran;
Indirekte Thrombininhibitoren, z.B. Odiparcil;
Direkte und indirekte Faktor Xa Inhibitoren, z.B. Fondaparinux-Natrium, Apixaban, Razaxaban, Rivaroxaban (BAY 59-7939), KFA-1982, DX-9065a, AVE3247, Otamixaban (XRP0673), AVE6324, SAR377142, Idraparinux, SSR126517, DB-772d, DT-831j, YM-150, 813893, LY517717 und DU-1766.;
Direkte und indirekte Faktor Xa/IIa Inhibitoren, z.B. Enoxaparin-Natrium, AVE5026, SSR128428, SSR128429 und BIBT-986 (Tanogitran);
Lipoprotein-assoziierte Phospholipase A2 ("LpPLA2") Modulatoren;
Diuretika, z.B. Chlorthalidon, Ethacrynsäure, Furosemid, Amilorid, Chlorothiazid, Hydrochlorothiazid, Methylchtothiazid und Benzthiazid;
Nitrate, z.B. Isosorbide-5-Mononitrat;
Thromboxan-Antagonisten, z.B. Seratrodast, Picotamid und Ramatroban;
Plättchenaggregations-Inhibitoren, z.B. Clopidogrel, Tiklopidin, Cilostazol, Aspirin, Abciximab, Limaprost, Eptifibatide und CT-50547;
Cyklooxygenase-Inhibitoren, z.B. Meloxicam, Rofecoxib und Celecoxib;
B-Typ Natriuretic Peptide, z.B. Nesiritid und Ularitide;
NV1FGF-Modulatoren, z.B. XRP0038;
HT1B/5-HT2A-Antagonisten, z.B. SL65.0472;
Guanylatcyclase-Aktivatoren, z.B. Ataciguat (HMR1766) und HMR1069;
e-NOS Transkriptions-Enhancers, z.B. AVE9488 and AVE3085;
Anti-atherogene Substanzen, z.B. AGI-1067:
CPU-Inhibitoren, z.B. AZD9684;
Renininhibitoren, z.B. Aliskirin und VNP489;
Inhibitoren der Adenosindiphosphat-induzierten Plättchenaggregation, z.B. Clopidogrel, Tiklopidin, Prasugrel und AZD6140;
NHE-1 Inhibitoren, z.B. AVE4454 und AVE4890.

Antibiotische Therapie: Verschiedene Antibiotika oder antifungale Medikamenten-Kombinationen kommen in Frage, entweder als kalkulierte Therapie (vor Vorliegen des mikrobiellen Befundes) oder als spezifische Therapie; Flüssigkeitstherapie, z.B. Kristalloide oder kolloidale Flüssigkeiten; Vasopressoren, z.B. Norepinephrine, Dopamine oder Vasopressin; Inotrope Therapie, z.B. Dobutamin; Kortikosteroide, z.B. Hydrokortison, oder Fludrokortison; rekombinantes humanes aktivierte Protein C, Xigris; Blutprodukte, z.B. Erythrozytenkonzentrate, Thrombozytenkonzentrate, Erythropietin oder Fresh Frozen Plasma; Maschinelle Beatmung bei sepsisinduziertem Acute Lung Injury (ALI) bzw. Acute Respiratory Distress Syndrome (ARDS), z.B. Permissive Hyperkapnie, niedrigere Tidalvolumina; Sedierung: z.B. Diazepam, Lorazepam, Midazolam oder Propofol. Opioide: z.B. Fentanyl, Hydromorphon, Morphin, Meperidin oder Remifentanil. NSAIDs: z.B. Ketorolac, Ibuprofen oder Acetaminophen. Neuromuskuläre Blockade: z.B. Pancuronium; Glukose-Kontrolle, z.B. Insulin, Glukose; Nierenersatzverfahren, z.B. kontinuierliche veno-venöse-Hämofiltration oder intermittierende Hämodialyse. Dopamin niedrig-dosiert zur renalen Protektion; Antikoagulantien, z.B. zur Thromboseprophylaxe oder bei Nierenersatzverfahren, z.B. unfraktionierte Heparine, low molecular weight Heparine, Heparinoide, Hirudin, Bivalirudin oder Argatroban; Bikarbonat-Therapie; Streßulkusprophylaxe, z.B. H2-Rezeptorinhibitoren, Antazida

Medikamente bei proliferativen Erkrankungen: Urazil, Chlormethin, Cyklophosphamid, Ifosfamid, Melphalan, Chlorambucil, Pipobroman, Triethylenemelamin, Triethylenethiophosphoramin, Busulfan, Carmustine, Lomustine, Streptozocin, Dacarbazine, Methotrexate, 5- Fluorouracil, Floxuridine, Cytarabine, 6-Mercaptopurine, 6-Thioguanine, Fludarabine phosphate, Pentostatine, Vinblastine, Vincristine, Vindesine, Bleomycin, Dactinomycin, Daunorubicin, Doxorubicin, Epirubicin, Idarubicin, Paclitaxel, Mithramycin, Deoxycoformycin, Mitomycin-C, L-Asparaginase, Interferons, Etoposide, Teniposide 17.alpha.- Ethinylestradiol, Diethylstilbestrol, Testosterone, Prednisone, Fluoxymesterone, Dromostanolone propionate, Testolactone, Megestrolacetate, Tamoxifen, Methylprednisolone, Methyltestosterone, Prednisolone, Triamcinolone, Chlorotrianisene, Hydroxyprogesterone, Aminoglutethimide, Estranrustine, Medroxyprogesteroneacetate, Leuprolide, Flutamide, Toremifene, Goserelin, Cisplatin, Carboplatin, Hydroxyurea, Amsacrine, Procarbazine, Mitotane, Mitoxantrone, Levamisole, Navelbene, Anastrazole, Letrazole, Capecitabine, Reloxafine, Droloxafine, Hexamethylmelamine, Oxaliplatin (Eloxatin^{®}), Iressa (gefmitib, Zd1839), XELODA^{®} (capecitabine), Tarceva^{®} (erlotinib), Azacitidine (5-Azacytidine; 5-AzaC), Temozolomide (Temodar^{®}), Gemcitabine (e.g., GEMZAR^{®} (gemcitabine HCl)), Vasostatin oder eine Kombination zweier oder mehrerer der oben genannten.

Weiterer Gegenstand der vorliegenden Erfindung ist ein Verfahren zur Verhinderung der Blutkoagulation *in vitro,* insbesondere bei Blutkonserven oder biologischen Proben, die Blutplättchen enthalten, das dadurch gekennzeichnet ist, dass eine antikoagulatorisch wirksame Menge der erfindungsgemäßen Verbindung zugegeben wird.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Bevorzugt ist die orale Applikation.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutisch geeigneten Hilfsstoff enthalten, sowie deren Verwendung zu den zuvor genannten Zwecken.

Im Allgemeinen hat es sich als vorteilhaft erwiesen, bei parenteraler Applikation Mengen von etwa 5 bis 250 mg je 24 Stunden zur Erzielung wirksamer Ergebnisse zu verabreichen. Bei oraler Applikation beträgt die Menge etwa 5 bis 100 mg je 24 Stunden.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10% w/v": 100 ml Lösung oder Suspension enthalten 10 g Substanz.

### A) Beispiele

### Abkürzungen:

- ca.: circa
- CDI: Carbonyldiimidazol
- d: Tag(e), Dublett (bei NMR)
- DC: Dünnschicht-Chromatographie
- DCI: direkte chemische Ionisation (bei MS)
- dd: Doppeltes Dublett (bei NMR)
- DMAP: 4-Dimethylaminopyridin
- DMF: *N,N*-Dimethylformamid
- DMSO: Dimethylsulfoxid
- DPPA: Diphenylphosphorazidat
- DSC: Disuccinimidylcarbonat
- d. Th.: der Theorie (bei Ausbeute)
- eq.: Äquivalent(e)
- ESI: Elektrospray-Ionisation (bei MS)
- h: Stunde(n)
- HATU: *O*-(7-Azabenzotriazol-1-yl)-*N,N,N',N'*-tetramethyluronium-Hexafluorphosphat
- HPLC: Hochdruck-, Hochleistungsflüssigchromatographie
- LC-MS: Flüssigchromatographie-gekoppelte Massenspektroskopie
- LDA: Lithiumdiisopropylamid
- m: Multiplett (bei NMR)
- min: Minute(n)
- MS: Massenspektroskopie
- NMR: Kernresonanzspektroskopie
- PYBOP: Benzotriazol-1-yloxy-tris(pyrrolidino)phosphonium-Hexafluorophosphat
- q: Quartett (bei NMR)
- RP: reverse phase (bei HPLC)
- RT: Raumtemperatur
- Rₜ: Retentionszeit (bei HPLC)
- s: Singulett (bei NMR)
- t: Triplett (bei NMR)
- THF: Tetrahydrofuran

### HPLC-Methoden

Methode 1A: Instrument: HP 1100 mit DAD-Detektion; Säule: Kromasil 100 RP-18, 60 mm x 2.1 mm, 3.5 µm; Eluent A: 5 ml Perchlorsäure (70%-ig) / 1 Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

Methode 2A: Instrument: HP 1100 mit DAD-Detektion; Säule: Kromasil 100 RP-18, 60 mm x 2.1 mm, 3.5 µm; Eluent A: 5 ml Perchlorsäure (70%-ig) / 1 Wasser, Eluent B: Acetonitril; Gradient: 0 min 2% B → 0.5 min 2% B → 4.5 min 90% B → 9 min 0% B → 9.2 min 2% B → 10 min 2% B; Fluss: 0.75 ml/min; Säulentemperatur: 30°C; UV-Detektion: 210 nm.

Methode 3A: Phase: Kromasil 100, C18, 5 µm 250 mm x 4 mm; Eluent: Wasser/Acetonitril 50:50; Fluss: 1 ml/min; T: 40°C; UV: 210 nm.

### LC-MS-Methoden:

Methode 1B: Gerätetyp MS: Micromass ZQ; Gerätetyp HPLC: HP 1100 Series; UV DAD; Säule: Phenomenex Gemini 3 µ, 30 mm x 3.0 mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

Methode 2B: Instrument: Micromass QuattroPremier mit Waters UPLC Acquity; Säule: Thermo Hypersil GOLD 1.9 µ, 50 mm x 1 mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 0.1 min 90%A → 1.5 min 10%A → 2.2 min 10%A; Ofen: 50°C; Fluss: 0.33 ml/min; UV-Detektion: 210 nm.

Methode 3B: Gerätetyp MS: Micromass ZQ; Gerätetyp HPLC: Waters Alliance 2795; Säule: Phenomenex Synergi 2.5 µ MAX-RP 100A Mercury, 20 mm x 4 mm; Eluent A: 1 1 Wasser + 0.5 ml 50%ige Ameisensäure, Eluent B: 11 Acetonitril + 0.5 ml 50%ige Ameisensäure; Gradient: 0.0 min 90%A → 0.1 min 90%A → 3.0 min 5%A → 4.0 min 5%A → 4.01 min 90%A; Fluss: 2 ml/min; Ofen: 50°C; UV-Detektion: 210 nm.

Methode 4B: Gerätetyp MS: Waters ZQ; Gerätetyp HPLC: Waters Alliance 2795; Säule: Phenomenex Onyx Monolithic C18, 100 mm x 3 mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 210 nm.

Methode 5B: Instrument: Micromass Quattro Micro MS mit HPLC Agilent Serie 1100; Säule: Thermo Hypersil GOLD 3 µ 20 mm x 4 mm; Eluent A: 11 Wasser + 0.5 ml 50%ige Ameisensäure, Eluent B: 11 Acetonitril + 0.5 ml 50%ige Ameisensäure; Gradient: 0.0 min 100%A →3.0 min 10%A → 4.0 min 10%A → 4.01 min 100%A → 5.00 min 100%A; Ofen: 50°C; Fluss: 2 ml/min; UV-Detektion: 210 nm.

Methode 6B: Instrument: Micromass Quattro LCZ mit HPLC Agilent Serie 1100; Säule: Phenomenex Synergi 2.5 µ MAX-RP 100A Mercury 20 mm x 4 mm; Eluent A: 1 1 Wasser + 0.5 ml 50%ige Ameisensäure, Eluent B: 11 Acetonitril + 0.5 ml 50%ige Ameisensäure; Gradient: 0.0 min 90%A → 0.1 min 90%A → 3.0 min 5%A → 4.0 min 5%A → 4.1 min 90%A; Fluss: 2 ml/min; Ofen: 50°C; UV-Detektion: 208-400 nm.

Methode 7B: Instrument: Micromass Quattro LCZ mit HPLC Agilent Serie 1100; Säule: Phenomenex Onyx Monolithic C18, 100 mm x 3 mm. Eluent A: 11 Wasser + 0.5 ml 50%ige Ameisensäure, Eluent B: 11 Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 208-400 nm.

Methode 8B: Instrument: Micromass Platform LCZ mit HPLC Agilent Serie 1100; Säule: Thermo HyPURITY Aquastar 3µ 50 mm x 2.1 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

Methode 9B: Instrument MS: Waters ZQ 2000; Instrument HPLC: Agilent 1100, 2-Säulen-Schaltung, Autosampler: HTC PAL; Säule: YMC-ODS-AQ, 50 mm x 4.6 mm, 3.0 µm; Eluent A: Wasser + 0.1% Ameisensaeure, Eluent B: Acetonitril + 0.1% Ameisensaeure; Gradient: 0.0 min 100%A → 0.2 min 95%A → 1.8 min 25%A → 1.9 min 10%A → 2.0 min 5%A → 3.2 min 5%A → 3.21 min 100%A → 3.35 min 100%A; Ofen: 40°C; Fluss: 3.0 ml/min; UV-Detektion: 210 nm.

Methode 10B: Gerätetyp MS: Waters ZQ; Gerätetyp HPLC: Agilent 1100 Series; UV DAD; Säule: Thermo Hypersil GOLD 3µ 20 mm x 4 mm; Eluent A: 11 Wasser + 0.5 ml 50%ige Ameisensäure, Eluent B: 11 Acetonitril + 0.5 ml 50%ige Ameisensäure; Gradient: 0.0 min 100% A → 3.0 min 10% A → 4.0 min 10% A → 4.1 min 100% (Fluss: 2.5 ml/min); Ofen: 55°C; Fluss: 2 ml/min; UV-Detektion: 210 nm.

Methode 11B: Instrument: Waters ACQUITY SQD UPLC System; Säule: Waters Acquity UPLC HSS T3 1.8µ 50 mm x 1 mm; Eluent A: 11 Wasser + 0.25 ml 99%ige Ameisensäure, Eluent B: 11 Acetonitril + 0.25 ml 99%ige Ameisensäure; Gradient: 0.0 min 90%A → 1.2 min 5%A → 2.0 min 5%A; Ofen: 50°C; Fluss: 0.40 ml/min; UV-Detektion: 210 - 400 nm.

### Präparative Diastereomerentrennung:

Methode 1C: Phase: Kromasil 100 C18, 5 µm 250 mm x 20 mm, Eluent: 0.2%-ige wässrige Trifluoressigsäure/Acetonitril 47:53; Fluss: 25 ml/min, Temperatur: 23°C; UV-Detektion: 210 nm.

Methode 2C: Phase: Sunfire C18, 5 µm 150 mm x 19 mm, Eluent: Wasser/Acetonitril 50:50; Fluss: 25 ml/min, Temperatur: 24°C; UV-Detektion: 225 nm.

Methode 3C: Phase: Kromasil 100 C18, 5 µm 250 mm x 20 mm, Eluent: Wasser/Acetonitril 50:50; Fluss: 25 ml/min, Temperatur: 40°C; UV-Detektion: 210 nm.

Methode 4C: Phase: Kromasil 100 C18, 5 µm 250 mm x 20 mm, Eluent: Wasser/Acetonitril 35:65; Fluss: 25 ml/min, Temperatur: 30°C; UV-Detektion: 210 nm.

Methode 5C: Phase: Sunfire C18, 5 µm 150 mm x 30 mm, Eluent: Wasser/Acetonitril 50:50; Fluss: 56 ml/min, Temperatur: 30°C; UV-Detektion: 210 nm.

Methode 6C: Phase: XBridge C18, 5 µm OBD, 150 mm x 19 mm, Eluent: 0.1%-ige Diethylamin-Lösung/Acetonitril 30:70; Fluss: 25 ml/min, Temperatur: 24°C; UV-Detektion: 254 nm.

Methode 7C: Phase: Sunfire C18, 5 µm 250 mm x 20 mm, Eluent: Wasser/Acetonitril 55:45; Fluss: 25 ml/min, Temperatur: 30°C; UV-Detektion: 210 nm.

Methode 8C: Phase: Sunfire C180BD, 5 µm 150 mm x 19 mm, Eluent: Wasser/Acetonitril 62:38; Fluss: 25 ml/min, Temperatur: 40°C; UV-Detektion: 210 nm.

Methode 9C: Phase: Kromasil 100 C, 5 µm 150 mm x 19 mm, Eluent: 0.2%ige wässrige Trifluoressigsäure/Acetonitril 50:50; Fluss: 25 ml/min, Temperatur: 40°C; UV-Detektion: 210 nm.

Methode 10C: Phase: XBridge C18, 5 µm OBD, 19 mm x 150 mm, Eluent: 0.1%-ige wässrige Ammonik-Lösung/Acetonitril 50:50; Fluss: 25 ml/min, Temperatur: 30°C; UV-Detektion: 210 nm.

Methode 11C: Phase: XBridge C18, 5 µm OBD, 19 mm x 150 mm, Eluent: wässrige AmmoniakLösung/Acetonitril 70:30; Fluss: 25 ml/min, Temperatur: 40°C; UV-Detektion: 210 nm.

Methode 12C: Phase: XBridge C18, 5 µm OBD, 19 mm x 150 mm, Eluent: wässrige Ammonik-Lösung/Acetonitril 65:35; Fluss: 25 ml/min, Temperatur: 40°C; UV-Detektion: 210 nm.

Methode 13C: Phase: Daisogel SP120-, 10 µm -ODS Bio, 250 mm x 20 mm, Eluent: 0.1%-ige Diethylamin-Lösung/Acetonitril 50:50; Fluss: 25 ml/min, Temperatur: 30°C; UV-Detektion: 240 nm.

Methode 14C: Phase: XBridge C18, 5 µm OBD, 19 mm x 150 mm, Eluent: 0.1%-ige Diethylamin-Lösung/Acetonitril 55:45; Fluss: 25 ml/min, Temperatur: 20°C (RT); UV-Detektion: 254 nm.

Methode 15C: Phase: XBridge C18, 5 µm OBD, 19 mm x 150 mm, Eluent: 0.2%-ige Diethylamin-Lösung/Acetonitril 63:37; Fluss: 25 ml/min, Temperatur: 20°C (RT); UV-Detektion: 235 nm.

### Präparative Enantiomerentrennung:

Methode 1D: Phase: chirale Kieselgelphase basierend auf dem Selektor Poly(*N*-methacryloyl-L-leucin-I-menthylamid, 250 mm x 20 mm, Eluent: iso-Hexan/Essigsäureethylester 70:30; Fluss: 25 ml/min, Temperatur: 24°C; UV-Detektion: 260 nm.

Methode 2D: Phase: Daicel Chiralpak AD-H, 5 µm 250 mm x 20 mm; Eluent: Isopropanol/iso-Hexan 30:70; Fluss: 20 ml/min; Temperatur: 25°C; UV-Detektion: 260 nm.

Methode 3D: Phase: Daicel Chiralpak AD-H, 5 µm 250 mm x 20 mm; Eluent: Isopropanol/iso-Hexan 50:50; Fluss: 18 ml/min; Temperatur: 25°C; UV-Detektion: 260 nm.

Methode 4D: Phase: chirale Kieselgelphase basierend auf dem Selektor Poly(*N*- ethacryloyl-L-leucin-I-menthylamid, 670 mm x 40 mm, Eluent: iso-Hexan/Essigsäureethylester 50:50; Fluss: 80 ml/min, Temperatur: 24°C; UV-Detektion: 260 nm.

Methode 5D: Phase: Daicel Chiralcel OD-H, 5 µm 250 mm x 20 mm, Eluent: Isopropanol/iso-Hexan 40:60; Fluss: 15 ml/min, Temperatur: 24°C; UV-Detektion: 230 nm.

Methode 6D: Phase: Daicel Chiralpak AD-H, 5 µm 250 mm x 20 mm; Eluent: Isopropanol/iso-Hexan 40:60; Fluss: 20 ml/min; Temperatur: 24°C; UV-Detektion: 260 nm.

Methode 7D: Phase: Daicel Chiralcel OD-H, 5 µm 250 mm x 20 mm, Eluent: iso-Hexan/Ethanol 90:10; Fluss: 15 ml/min, Temperatur: 30°C; UV-Detektion: 220 nm.

Methode 8D: Phase: Daicel Chiralcel AD-H, 5 µm 250 mm x 20 mm, Eluent: iso-Hexan/Ethanol 55:45; Fluss: 15 ml/min, Temperatur: 40°C; UV-Detektion: 220 nm.

Methode 9D: Phase: Daicel Chiralcel AS-H, 5 µm 250 mm x 20 mm, Eluent: iso-Hexan/2-Propanol 75:25; Fluss: 15 ml/min, Temperatur: 40°C; UV-Detektion: 220 nm.

Methode 10D: Phase: Daicel Chiralcel AD-H, 5 µm 250 mm x 20 mm, Eluent: iso-Hexan/Ethanol 50:50; Fluss: 15 ml/min, Temperatur: 40°C; UV-Detektion: 220 nm.

Methode 11D: Phase: Daicel Chiralpak AD-H, 5 µm, 250 mm x 20 mm, Eluent: Isopropanol/Iso-Hexan 30:70; Fluss: 20 ml/min, Temperatur: 20°C (RT); UV-Detektion: 230 nm.

Methode 12D: Phase: Daicel Chiralpak AD-H, 5 µm, 250 mm x 20 mm, Eluent: Iso-Hexan/Ethanol 50:50; Fluss: 15 ml/min, Temperatur: 40°C; UV-Detektion: 220 nm.

Methode 13D: Phase: Waters Sunfire C18, 5 µm, 250 mm x 20 mm, Eluent A: Wasser, Eluent B: Acetonitril; Gradient: 0.0 min 70% A -> 15 min 10% A -> 15.1 min 70% A -> 20 min 70% A; Fluss: 25 ml/min, Temperatur: 30°C; UV-Detektion: 210 nm.

Methode 14D: Phase: Kromasil 100 C18, 5 µm, 250 mm x 20 mm, Eluent: Wasser/Acetonitril 62:38; Fluss: 25 ml/min, Temperatur: 40°C; UV-Detektion: 210 nm.

Methode 15D: Phase: Daicel Chiralcel OD-H, 5 µm, 250 mm x 20 mm, Eluent: Iso-Hexan/Ethanol 70:30; Fluss: 15 ml/min, Temperatur: 40°C; UV-Detektion: 220 nm.

Methode 16D: Phase: Daice1 Chiralpak AD-H, 5 µm, 250 mm x 20 mm, Eluent: Iso-Hexan/Ethanol 75:25; Fluss: 15 ml/min, Temperatur: 25°C; UV-Detektion: 220 nm.

Methode 17D: Phase: Daicel Chiralpak AD-H, 5 µm, 250 mm x 20 mm, Eluent: Iso-Hexan/Ethanol 80:20; Fluss: 15 ml/min, Temperatur: 38°C; UV-Detektion: 220 nm.

### Analytische Enantiomerentrennung:

Methode 1E: Phase: chirale Kieselgelphase basierend auf dem Selektor Poly(*N*-methacryloyl-L-leucin-1-menthylamid, 250 mm x 4.6 mm, Eluent: iso-Hexan/Essigsäureethylester 50:50; Fluss: 1 ml/min, Temperatur: 20°C; UV-Detektion: 265 nm.

Methode 2E: Phase: Daicel Chiralpak AD-H, 5 µm 250 mm x 4 mm; Eluent: Isopropanol/iso-Hexan: 50:50; Fluss: 1 ml/min; UV-Detektion: 230 nm.

Methode 3E: Phase: chirale Kieselgelphase basierend auf dem Selektor Poly(*N*-methacryloyl-L-leucin-I-menthylamid, 250 mm x 4.6 mm, Eluent: iso-Hexan/Essigsäureethylester 50:50; Fluss: 2 ml/min, Temperatur: 20°C; UV-Detektion: 265 nm.

Methode 4E: Phase: Daicel Chiralcel OD-H, 5 µm 250 mm x 4 mm, Eluent: Isopropanol/iso-Hexan 50:50; Fluss: 1 ml/min, Temperatur: 20°C; UV-Detektion: 230 nm.

Methode 5E: Phase: Daicel Chiralcel OD-H, 5 µm 250 mm x 4.6 mm, Eluent: iso-Hexan/Ethanol 85:15; Fluss: 1 ml/min, Temperatur: 40°C; UV-Detektion: 220 nm.

Methode 6E: Phase: Daicel Chiralcel AD-H, 5 µm 250 mm x 4.6 mm, Eluent: iso-Hexan/Ethanol 50:50; Fluss: 1 ml/min, Temperatur: 40°C; UV-Detektion: 220 nm.

Methode 7E: Phase: Daicel Chiralcel AS-H, 5 µm 250 mm x 20 mm, Eluent: iso-Hexan/2-Propanol 75:25; Fluss: 1 ml/min, Temperatur: 40°C; UV-Detektion: 220 nm.

Methode 8E: Phase: Daicel Chiralcel AD-H, 5 µm 250 mm x 4.6 mm, Eluent: iso-Hexan/Ethanol 70:30 + 0.2% Trifluoressigsäure + 1% Wasser; Fluss: 1 ml/min, Temperatur: 40°C; UV-Detektion: 220 nm.

Als Mikrowellenreaktor wurde ein "single mode" Gerät vom Typ Emrys^{™} Optimizer verwendet.

### Ausgangsverbindungen

### Allgemeine Methode 1A: Suzuki-Kupplung

Eine Mischung des entsprechenden Brompyridins in Toluol (1.8 ml/mmol) wird unter Argon bei RT mit Tetrakis-(triphenylphosphin)-palladium (0.02 eq.), mit einer Lösung der entsprechenden Arylboronsäure (1.2 eq.) in Ethanol (0.5 ml/mmol) und mit einer Lösung aus Kaliumfluorid (2.0 eq.) in Wasser (0.2 ml/mmol) versetzt. Das Reaktionsgemisch wird mehrere Stunden bis zur weitgehend vollständigen Umsetzung unter Rückfluß gerührt. Nach Zugabe von Essigsäureethylester und Phasentrennung wird die organische Phase einmal mit Wasser und einmal mit gesättigter, wässriger Natriumchlorid-Lösung gewaschen, getrocknet (Magnesiumsulfat), filtriert und im Vakuum eingeengt. Das Rohprodukt wird mittels Flash-Chromatographie (Kieselgel-60, Eluent: Dichlormethan-Methanol-Gemische) aufgereinigt.

### Allgemeine Methode 2A: Hydrierung des Pyridins

Eine Lösung des Pyridins in Ethanol (9 ml/mmol) wird unter Argon mit Palladium auf Aktivkohle (angefeuchtet mit ca. 50% Wasser, 0.3 g/mmol) versetzt und bei 60°C über Nacht in einer 50 bar Wasserstoff-Atmosphäre hydriert. Anschließend wird der Katalysator über eine Filterschicht abfiltriert und mehrmals mit Ethanol gewaschen. Die vereinigten Filtrate werden im Vakuum eingeengt.

### Allgemeine Methode 3A: Umsetzung mit Carbamoylchloriden oder Carbonylchloriden

Eine Lösung des Piperidins in Dichlormethan (2.5 ml/mmol) wird unter Argon bei 0°C tropfenweise mit *N*,*N*-Diisopropylethylamin (1.2 eq.) und dem entsprechenden Carbamoylchlorid oder Carbonylchlorid (1.2 eq.) versetzt. Das Reaktionsgemisch wird bei RT gerührt. Nach Zugabe von Wasser und Phasentrennung wird die organische Phase dreimal mit Wasser und einmal mit gesättigter, wässriger Natriumchlorid-Lösung gewaschen, getrocknet (Natriumsulfat), filtriert und im Vakuum eingeengt.

### Allgemeine Methode 4A: Verseifung

Eine Lösung des entsprechenden Esters in einem Gemisch aus Tetrahydrofuran/Wasser (3:1, 12.5 ml/mmol) wird bei RT mit Lithiumhydroxid (2 eq.) versetzt. Das Reaktionsgemisch wird bei 60°C gerührt und anschließend mit wässriger, 1 N Salzsäure-Lösung auf pH 1 gestellt. Nach Zugabe von Wasser/Essigsäureethylester wird die wässrige Phase dreimal mit Essigsäureethylester extrahiert. Die vereinigten organischen Phasen werden getrocknet (Natriumsulfat), filtriert und im Vakuum eingeengt.

### Allgemeine Methode 5A: Curtius-Abbau

Eine Lösung der entsprechenden Carbonsäure in *tert*.-Butanol/Toluol (1:1, 8 ml/mmol) wird unter Argon bei RT tropfenweise mit Triethylamin (1.7 eq.), Diphenylphosphorazidat (1.1 eq.) und Di-*tert*.-butyldicarbonat (1.1 eq.) versetzt. Das Reaktionsgemisch wird 30 min bei 65°C und anschließend über Nacht bei 95°C gerührt. Nach Zugabe von Wasser und Phasentrennung wird die organische Phase mit Wasser und mit gesättigter, wässriger Natriumchlorid-Lösung gewaschen, getrocknet (Natriumsulfat), filtriert und im Vakuum eingeengt. Die Aufreinigung des Rohproduktes erfolgt anschließend mittels Flash-Chromatographie (Kieselgel-60, Eluent: Dichlormethan-Methanol-Gemische). Im Folgenden wird eine Lösung dieses boc-geschützten Amins in Dichlormethan (10 ml/mmol) unter Argon bei RT mit Trifluoressigsäure (10 eq.) versetzt. Das Reaktionsgemisch wird bei RT gerührt und anschließend im Vakuum eingeengt. Der Rückstand wird dreimal mit Toluol und Dichlormethan coevaporiert und ohne weitere Aufreinigung in der nächsten Stufe eingesetzt.

### Allgemeine Methode 6A: Hydrierung des Pyridins mittels Durchfluss-Hydrierapparatur

Eine Lösung des Pyridins in konzentrierter Essigsäure (ca. 35 ml/mmol) wird in einer Durchfluss-Hydrierapparatur ("H-Cube" der Firma ThalesNano, Budapest, Ungarn) hydriert (Bedingungen: 10% Pd/C-Katalysator, "controlled"-Modus, 60 bar, 0.5 ml/min, 85°C). Nach Entfernen des Lösungsmittels am Rotationsverdampfer wird das entsprechende Rohprodukt erhalten, welches gegebenenfalls mittels präparativer HPLC gereinigt wird.

### Allgemeine Methode 7A: Methylesterverseifung/Epimerisierung

Zu einer Lösung des entsprechenden Methylesters (1.0 eq.) in Methanol (35-40 ml/mmol) wird bei RT Kalium-tert.-butylat (10 eq.) gegeben. Die Mischung wird über Nacht bei 60°C gerührt. Bei unvollständiger Umsetzung wird Wasser (1.0 eq.) zugesetzt und bis zum vollständigen Umsatz bei 60°C gerührt. Zur Aufarbeitung wird im Vakuum das Methanol entfernt, der Rückstand mit Wasser versetzt und mit wässriger 1 N Salzsäure-Lösung sauer (pH 1) gestellt. Die Mischung wird mit Essigsäureethylester extrahiert, die organische Phase mit Magnesiumsulfat getrocknet, filtriert und im Vakuum eingeengt.

### Allgemeine Methode 8A: Harnstoffbildung

Eine Lösung des Nitrophenylcarbamates (1.0 eq.) in Dimethylformamid (10 ml/mmol) wird bei RT mit dem entsprechenden Amin (2.0-3.0 eq.) und Kaliumcarbonat (1.0 eq.) versetzt und in 15 ml-Portionen in einer *Single Mode*-Mikrowelle (Emrys Optimizer) für 0.5-1 h bei 150°C gerührt. Die Reaktionslösung wird filtriert und das Filtrat mittels präparativer HPLC aufgereinigt.

### Allgemeine Methode 9A: Methylesterverseifung/Epimerisierung

Zu einer Lösung des entsprechenden Methylesters (1.0 eq.) in Methanol (35-40 ml/mmol) wird bei RT Kalium-*tert*.-butylat (10 eq.) gegeben. Die Mischung wird über Nacht bei 60°C gerührt. Bei unvollständiger Umsetzung wird Wasser (1.0 eq.) zugesetzt und bis zum vollständigen Umsatz bei 60°C gerührt. Zur Aufarbeitung wird im Vakuum das Methanol entfernt, der Rückstand mit Wasser versetzt und mit 1N Salzsäure auf pH=1 gestellt. Die Mischung wird mit Essigsäureethylester extrahiert, die organische Phase über Magnesiumsulfat getrocknet, filtriert und im Vakuum eingeengt.

### Beispiel 1A

### 5-(4-Ethylphenyl)pyridin-3-carbonsäuremethylester

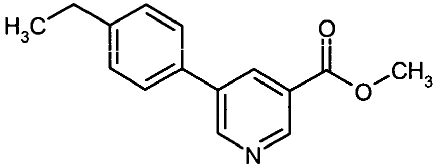

Nach der Allgemeinen Methode 1A wurden 32 g (148 mmol) 5-Bromnicotinsäuremethylester und 27 g (178 mmol, 1.2 eq.) 4-Ethylphenylboronsäure umgesetzt. Ausbeute: 24 g (64% d. Th.)

LC-MS (Methode 3B): Rₜ = 2.03 min; MS (ESIpos): m/z = 242 [M+H]⁺.

### Beispiel 2A

### 5-(4-Ethylphenyl)piperidin-3-carbonsäuremethylester [racemisches cis-/trans-Isomerengemisch]

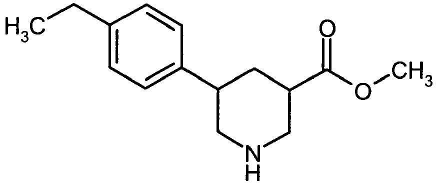

Nach der Allgemeinen Methode 2A wurden 24 g (94 mmol) 5-(4-Ethylphenyl)pyridin-3-carbonsäuremethylester hydriert. Ausbeute: 20 g (77% d. Th.)

LC-MS (Methode 5B): Rₜ = 1.43 min; MS (ESIpos): m/z = 248 [M+H]⁺.

### Beispiel 3A

### 5-(4-Ethylphenyl)pyridin-3-carbonsäureethylester

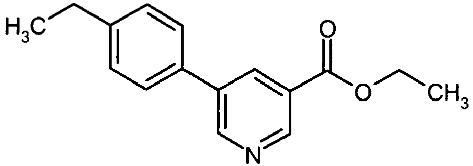

Nach der Allgemeinen Methode 1A wurden 29 g (126 mmol) 5-Bromnicotinsäureethylester und 23 g (152 mmol, 1.2 eq.) 4-Ethylphenylboronsäure umgesetzt. Ausbeute: 32 g (82% d. Th.)

LC-MS (Methode 4B): Rₜ = 3.80 min; MS (ESIpos): m/z = 256 [M+H]⁺.

### Beispiel 4A

### 5-(4-Ethylphenyl)piperidin-3-carbonsäureethylester [racemisches cis-/trans-Isomerengemisch]

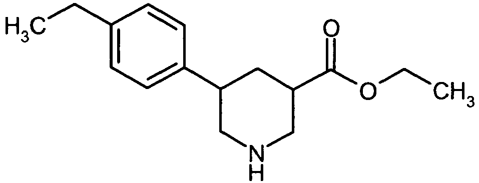

Nach der Allgemeinen Methode 2A wurden 24 g (71 mmol) 5-(4-Ethylphenyl)pyridin-3-carbonsäureethylester hydriert. Ausbeute: 15 g (81 % d. Th.)

LC-MS (Methode 5B): Rₜ = 1.78 min und 1.91 min (cis-/trans-Isomere); MS (ESIpos): m/z = 262 [M+H]⁺.

### Beispiel 5A

### 1-(Cyclopentylcarbonyl)-5-(4-ethylphenyl)piperidin-3-carbonsäureethylester [racemisches cis-/ trans-Isomerengemisch]

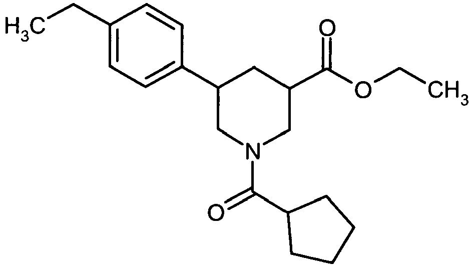

Nach der Allgemeinen Methode 3A wurden 5.2 g (14.0 mmol) 5-(4-Ethylphenyl)piperidin-3-carbonsäureethylester mit 2.1 g (2.1 mmol, 1.2 eq.) Cyclopentancarbonylchlorid umgesetzt. Ausbeute: 4.8 g (96% d. Th.)

LC-MS (Methode 4B): Rₜ = 4.04 min und 4.14 min (cis-/trans-Isomere); MS (ESIpos): m/z = 358 [M+H]⁺.

### Beispiel 6A

### 1-(Cyclopentylcarbonyl)-5-(4-ethylphenyl)piperidin-3-carbonsäure [racemisches cis-/trans-Isomerengemisch]

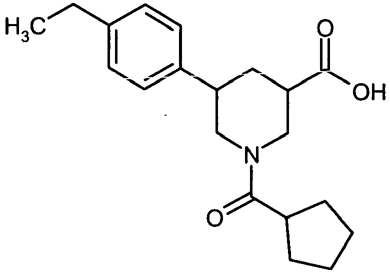

Nach der Allgemeinen Methode 4A wurden 13.8 g (38.6 mmol) 1-(Cyclopentylcarbonyl)-5-(4-ethylphenyl)piperidin-3-carbonsäureethylester verseift. Ausbeute: 11.5 g (87% d. Th.)

LC-MS (Methode 1B): Rₜ = 2.50 min und 2.57 min (cis-/trans-Isomere); MS (ESIpos): m/z = 330 [M+H]⁺.

### Beispiel 7A

### 1-(Cyclopentylcarbonyl)-5-(4-ethylphenyl)piperidin-3-carbonsäure [racemisches cis-Isomer]

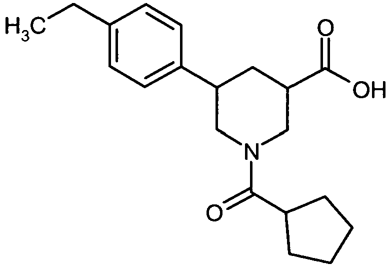

Diastereomerentrennung von 11.5 g des cis-/trans-Isomerengemisches des Beispiels 6A nach Methode 1C ergab 4.1 g der Titelverbindung 7A (cis-Isomer) und 4.1 g des trans-Isomers.

LC-MS (Methode 1B): Rₜ = 2.57 min; MS (ESIpos): m/z = 330 [M+H]⁺.

### Beispiel 8A

### 1-(Cyclopentylcarbonyl)-5-(4-ethylphenyl)piperidin-3-amin-Trifluoracetat [racemisches cis-Isomer]

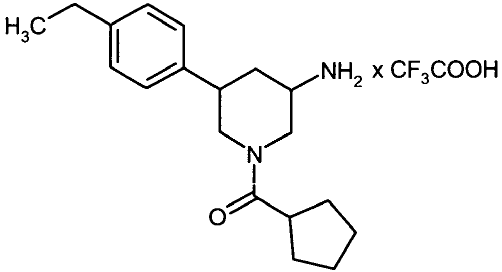

Nach der Allgemeinen Methode 5A wurden 816 mg (2.48 mmol) 1-(Cyclopentylcarbonyl)-5-(4-ethylphenyl)piperidin-3-carbonsäure in einem Curtius-Abbau umgesetzt. Ausbeute: 730 mg (71% d. Th.)

LC-MS (Methode 5B): Rₜ = 1.68 min; MS (ESIpos): m/z = 301 [M+H]⁺ (freie Base).

### Beispiel 9A

### 5-(4-Ethylphenyl)-1-(pyrrolidin-1-ylcarbonyl)piperidin-3-carbonsäuremethylester [racemisches cis-/trans-Isomerengemisch]

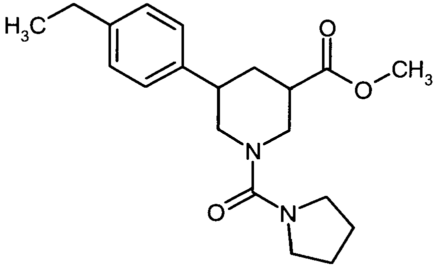

Nach der Allgemeinen Methode 3A wurden 6.7 g (24.1 mmol) 5-(4-Ethylphenyl)piperidin-3-carbonsäuremethylester mit 4.2 g (31.4 mmol, 1.3 eq.) Pyrrolidin-1-carbonylchlorid umgesetzt. Ausbeute: 7.6 g (91% d. Th.)

LC-MS (Methode 3B): Rₜ = 2.08 min und 2.16 min (cis-/trans-Isomere); MS (ESIpos): m/z = 345 [M+H]⁺.

### Beispiel 10A

### 5-(4-Ethylphenyl)-1-(pyrrolidin-1-ylcarbonyl)piperidin-3-carbonsäuremethylester[racemisches cis-Isomer]

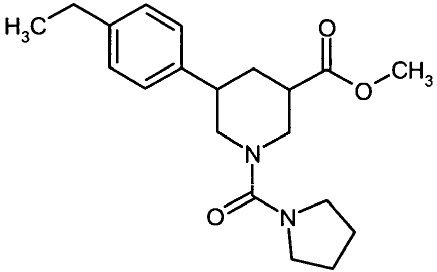

Diastereomerentrennung von 7.6 g des cis-/trans-Isomerengemisches des Beispiels 9A nach Methode 4C ergab 1.6 g des Beispiels 10A (cis-Isomer) und 4.1 g des trans-Isomers.

LC-MS (Methode 1B): Rₜ = 2.55 min; MS (ESIpos): m/z = 345 [M+H]⁺.

### Beispiel 11A

### 5-(4-Ethylphenyl)-1-(pyrrolidin-1-ylcarbonyl)piperidin-3-carbonsäure [racemisches cis-Isomer]

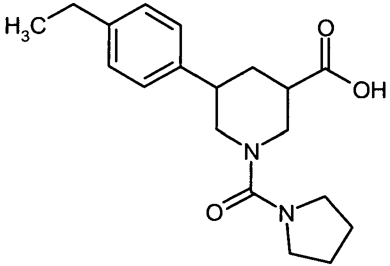

Nach der Allgemeinen Methode 4A wurden 1.4 g (3.9 mmol) 5-(4-Ethylphenyl)-1-(pyrrolidin-1-ylcarbonyl)piperidin-3-carbonsäuremethylester verseift. Ausbeute: 1.2 g (92% d. Th.)

LC-MS (Methode 2B): Rₜ = 1.18 min; MS (ESIpos): m/z = 331 [M+H]⁺.

### Beispiel 12A

### 5-(4-Ethylphenyl)-1-(pyrrolidin-1-ylcarbonyl)piperidin-3-amin-Trifluoracetat [racemisches cis-Isomer]

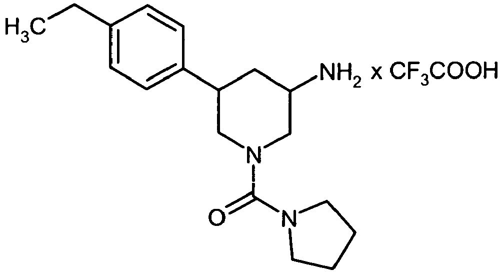

Nach der Allgemeinen Methode 5A wurden 1.2 g (3.6 mmol) 5-(4-Ethylphenyl)-1-(pyrrolidin-1-yl-carbonyl)piperidin-3-carbonsäure in einem Curtius-Abbau umgesetzt. Ausbeute: 1.2 g (78% d. Th.)

LC-MS (Methode 5B): Rₜ = 1.61 min; MS (ESIpos): m/z = 302 [M+H]⁺ (freie Base).

### Beispiel 13A

### 5-[4-(Trifluormethoxy)phenyl]pyridin-3-carbonsäuremethylester

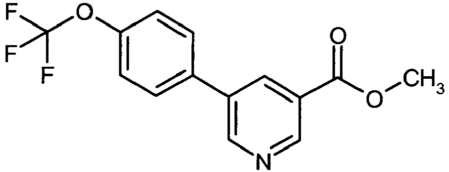

Nach der Allgemeinen Methode 1A wurden 23 g (105 mmol) 5-Bromnicotinsäuremethylester und 26 g (126 mmol, 1.2 eq.) 4-Trifluormethoxyphenylboronsäure umgesetzt. Ausbeute: 14 g (41% d. Th.)

LC-MS (Methode 1B): Rₜ = 2.44 min; MS (ESIpos): m/z = 298 [M+H]⁺.

### Beispiel 14A

### 5-Brom-N-phenylpyridin-3-carboxamid

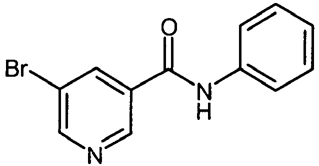

Eine Lösung aus 18.6 g (200 mmol) Anilin in 500 ml Tetrahydrofuran wurde unter Argon bei RT mit 32.4 ml (400 mmol, 2 eq.) Pyridin, 2.4 g (20 mmol, 0.1 eq.) 4-Dimethylaminopyridin und tropfenweise mit einer Lösung aus 44.1 g (200 mmol, 1.0 eq.) 5-Brompyridin-3-carbonylchlorid in 200 ml DMF versetzt. Nach 3 h wurde das Reaktionsgemisch im Vakuum eingeengt. Der Rückstand wurde in Wasser/Dichlormethan suspendiert, der Feststoff gut verrührt, abfiltriert, mehrmals mit Wasser und Dichlormethan gewaschen und im Vakuum getrocket. Ausbeute: 35.7 g (52% d. Th.). Nach Phasentrennung der Mutterlauge wurde die organische Phase zweimal mit Dichlormethan extrahiert. Die vereinigten, organischen Phasen wurden getrocknet (Natriumsulfat), filtriert und im Vakuum eingeengt. Der Rückstand wurde mittels Flash-Chromatographie (Kieselgel, Dichlormethan/Methanol 100:1) aufgereinigt, wobei 11.7 g (18% d. Th.) des Beispiels 14A erhalten wurden.

LC-MS (Methode 6B): Rₜ = 1.77 min; MS (ESIpos): m/z = 277 [M+H]⁺.

### Beispiel 15A

### 5-(4-Ethylphenyl)-N-phenylpyridin-3-carboxamid

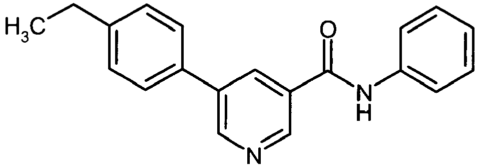

Nach der Allgemeinen Methode 1A wurden 74 g (222 mmol) 5-Brom-*N*-phenylpyridin-3-carboxamid und 40 g (266 mmol, 1.2 eq.) 4-Ethylphenylboronsäure umgesetzt. Ausbeute: 68 g (87% d. Th.)

LC-MS (Methode 6B): Rₜ = 2.26 min; MS (ESIpos): m/z = 303 [M+H]⁺.

### Beispiel 16A

### 5-(4-Ethylphenyl)-N-phenylpiperidin-3-carboxamid [racemisches cis-/trans-Isomerengemisch]

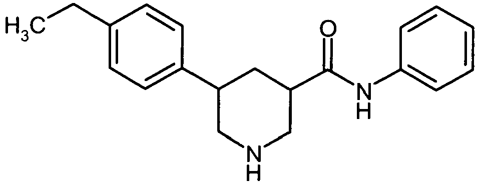

Nach der Allgemeinen Methode 2A wurden 67 g (218 mmol) 5-(4-Ethylphenyl)-*N*-phenylpyridin-3-carboxamid hydriert. Ausbeute: 63 g (88% d. Th.)

### Beispiel 17A

### 5-(4-Ethylphenyl)-N-phenylpiperidin-3-carboxamid [racemisches cis-Isomer]

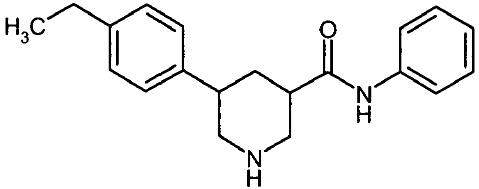

Die Diastereomerentrennung von 63 g des cis-/trans-Isomerengemisches (Beispiel 16A) nach Methode 6C ergab 25.9 g des Beispiels 17A (cis-Isomer) und 15.4 g des trans-Isomers.

LC-MS (Methode 6B): Rₜ = 1.35 min; MS (ESIpos): m/z = 309 [M+H]⁺.

### Beispiel 18A

### Methyl-1-[(4-cyanpiperidin-1-yl)carbonyl]-5-(4-ethylphenyl)piperidin-3-carboxylat [racemisches cis-/trans-Isomerengemisch]

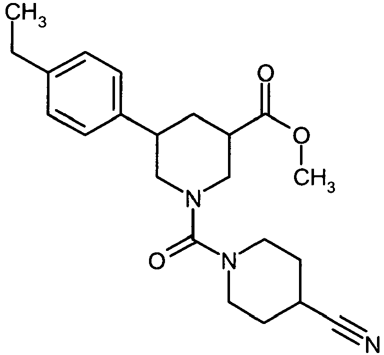

Nach der Allgemeinen Methode 8A wurden 3.0 g (6.1 mmol) der Verbindung aus Beispiel 65A umgesetzt. Ausbeute: 2.1 g (83% d. Th.)

LC-MS (Methode 11B): Rₜ = 1.12 min und 1.14 min (cis-/trans-Isomere); MS (ESIpos): m/z = 384 [M+H]⁺.

### Beispiel 19A

### 3-Brom-5-(4-ethylphenyl)pyridin

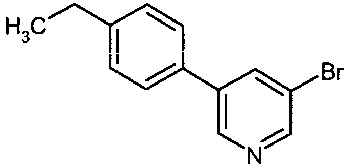

Nach der Allgemeinen Methode 1A wurden 23 g (97 mmol) 3,5-Dibrompyridin und 18 g (117 mmol, 1.2 eq.) 4-Ethylphenylboronsäure umgesetzt. Ausbeute: 17 g (66% d. Th.)

LC-MS (Methode 3B): Rₜ = 2.35 min; MS (ESIpos): m/z = 262 [M+H]⁺.

### Beispiel 20A

### [5-(4-Ethylphenyl)pyridin-3-yl]methylcarbamat

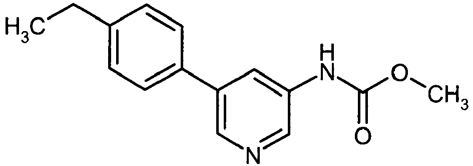

Eine Suspension von 555 mg (0.96 mmol, 0.015 eq.) Xantphos (4,5-Bis(diphenylphosphino)-9,9'-dimethylxanthen), 293 mg (0.32 mmol, 0.005 eq.) Tris(dibenzylidenaceton)dipalladium, 29.1 g (89 mmol, 1.4 eq.) Cäsiumcarbonat und 5.8 g (77 mmol, 1.2 eq.) Methylcarbamat in 100 ml Dioxan wurde unter Argon bei RT mit einer Lösung von 16.8 g (64 mmol) 3-Brom-5-(4-ethylphenyl)pyridin in 70 ml Dioxan versetzt. Das Reaktionsgemisch wurde 20 h bei 90°C gerührt. Da die Reaktion nur langsam fortschritt, wurde das Reaktionsgemisch portionsweise insgesamt dreimal mit 555 mg (0.96 mmol, 0.015 eq.) Xantphos und 293 mg (0.32 mmol, 0.005 eq.) Tris(dibenzyliden-aceton)dipalladium versetzt und anschließend jeweils über Nacht bei 90°C gerührt. Nach Zugabe von Dichlormethan wurde das Reaktionsgemisch über Celite filtriert und das Filtrat im Vakuum eingeengt. Das Rohprodukt wurde mit Acetonitril verrührt und der verbleibende Rückstand mehrmals mit kaltem Acetonitril gewaschen und im Vakuum getrocknet. Ausbeute: 15 g (90% d. Th.)

LC-MS (Methode 5B): Rₜ = 1.84 min; MS (ESIpos): m/z = 257 [M+H]⁺.

### Beispiel 21A

### [5-(4-Ethylphenyl)piperidin-3-yl]methylcarbamat [racemisches cis-/trans-Isomerengemisch)

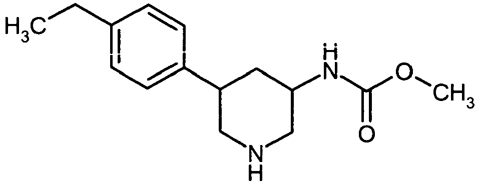

Eine Lösung von 1.0 g (3.7 mmol) [5-(4-Ethylphenyl)pyridin-3-yl]methylcarbamat in 100 ml Ethanol und 100 ml Essigsäure wurde nach der Allgemeinen Methode 5A umgesetzt. Die Lösung wurde im Vakuum eingeengt. Das Rohprodukt wurde mittels Flash-Chromatographie (Kieselgel, Dichlormethan/Methanol 4:1) aufgereinigt. Ausbeute: 171 mg (16% d. Th.)

LC-MS (Methode 2B): Rₜ = 0.81 min; MS (ESIpos): m/z = 263 [M+H]⁺.

### Beispiel 22A

### 3-(4-Ethylphenyl)-5-methoxypyridin

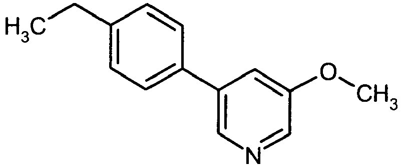

Nach der Allgemeinen Methode 1A wurden 5.0 g (27 mmol) 3-Brom-5-Methoxypyridin und 4.8 g (32 mmol, 1.2 eq.) 4-Ethylphenylboronsäure umgesetzt. Ausbeute: 3.0 g (53% d. Th.)

LC-MS (Methode 1 B): Rₜ = 2.10 min; MS (ESIpos): m/z = 214 [M+H]⁺.

### Beispiel 23A

### 3-(4-Ethylphenyl)-5-methoxypiperidin [racemisches cis-/trans-Isomerengemisch]

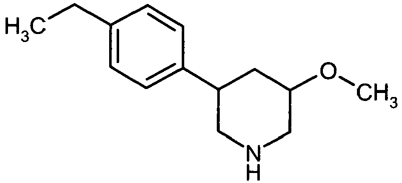

Eine Lösung von 1.0 g (4.7 mmol) 3-(4-Ethylphenyl)-5-methoxypyridin in 120 ml Essigsäure wurde nach der Allgemeinen Methode 5A umgesetzt. Die Lösung wurde im Vakuum eingeengt. Das Rohprodukt wurde mittels Flash-Chromatographie (Kieselgel, Dichlormethan/Methanol-Gradient) aufgereinigt. Ausbeute: 880 mg (86% d. Th.)

LC-MS (Methode 1B): Rₜ = 1.10 min; MS (ESIpos): m/z = 220 [M+H]⁺.

### Beispiel 24A

### 4-{[3-(4-Ethylphenyl)-5-methoxypiperidin-1-yl]carbonyl}morpholin [racemisches cis-/trans-Isomerengemisch]

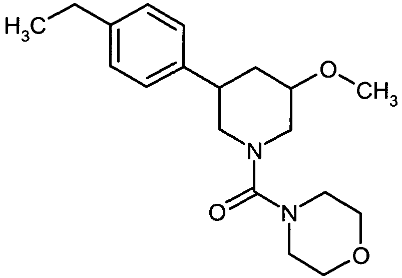

Nach der Allgemeinen Methode 3A wurden 880 mg (4.0 mmol) 3-(4-Ethylphenyl)-5-methoxypiperidin mit 780 mg (5.2 mmol, 1.3 eq.) Morpholin-4-carbonylchlorid umgesetzt. Ausbeute: 896 mg (58% d. Th.)

LC-MS (Methode 3B): Rₜ = 1.81 min und 1.84 min (cis-/trans-Isomere); MS (ESIpos): m/z = 333 [M+H]⁺.

### Beispiel 25A

### 5-(4-Ethylphenyl)-1-(morpholin-4-ylcarbonyl)piperidin-3-ol [racemisches cis-/trans-Isomerengemisch]

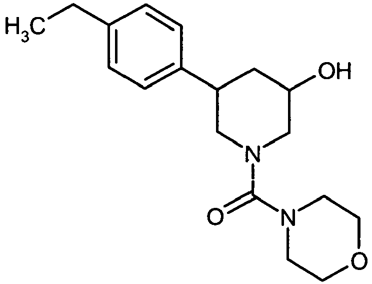

Eine Lösung von 890 mg (2.3 mmol) 4-{[3-(4-Ethylphenyl)-5-methoxypiperidin-1-yl]carbonyl}-morpholin in 22 ml Dichlormethan wurde unter Argon bei 0°C mit 4.6 ml einer Bortribromid-Lösung (1 molar in Dichlormethan, 4.6 mmol, 2 eq.) versetzt. Das Reaktionsgemisch wurde 2 h bei RT gerührt, anschließend auf Eis gegossen, mit festem Natriumhydrogencarbonat neutralisiert und nach Zugabe von Wasser und Phasentrennung dreimal mit Dichlormethan extrahiert. Die vereinigten, organischen Phasen wurden über Natriumsulfat getrocknet, filtriert und im Vakuum eingeengt. Die Aufreinigung des Rohproduktes erfolgte anschließend mittels präparativer HPLC (Reprosil C18, Wasser mit 0.1% Trifluoressigsäure-Acetonitril-Gradient). Ausbeute: 314 mg (43% d. Th.)

LC-MS (Methode 3B): Rₜ = 1.50 min; MS (ESIpos): m/z = 319 [M+H]⁺.

### Beispiel 26A

### N-{5-[4-(Trifluormethoxy)phenyl]pyridin-3-yl}benzolcarboxamid

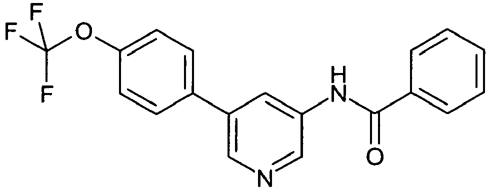

11.8 g (41.4 mmol) *N*-(5-Brompyridin-3-yl)benzamid, 12.8 (62.1 mmol) [4-(Trifluormethoxy)-phenyl]boronsäure und 11.4 g (82.7 mmol) Kaliumcarbonat wurden in 70 ml 1,2-Dimethoxyethan, 21 ml Wasser und 156 ml DMF bei 50°C gelöst. Es wurde mit Argon gespült und 0.24 g (0.2 mmol) Tetrakis(triphenylphosphin)palladium(0) wurden zugegeben und 12 h bei 85°C gerührt. Das Reaktionsgemisch wurde am Rotationsverdampfer etwas eingeengt, mit Wasser verdünnt und mit Dichlormethan extrahiert. Die organische Phase wurde im Vakuum eingeengt und der Rückstand mit Methy-*tert*.-butylether verrührt. Der Feststoff wurde abfiltriert und das Filtrat säulenchromatographisch an Kieselgel aufgereinigt (Cyclohexan/Essigsäureethylester 2:1 -> 1:5).

Der abgetrennte Festoff und die geeignete Fraktion aus der Chromatographie wurden vereingt. Ausbeute: 6.7 g (45% d. Th.)

LC-MS (Methode 1B): Rₜ = 2.47 min; m/z = 359 [M+H]⁺.

### Beispiel 27A

### N-{5-[4-(Trifluormethoxy)phenyl]piperidin-3-yl}benzolcarboxamid [racemisches cis-/trans-Isomerengemisch]

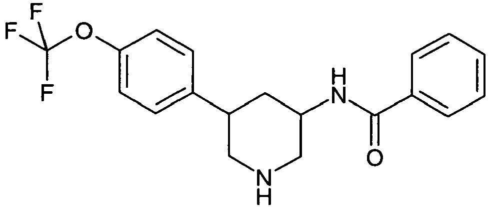

10.4 g (29.0 mmol) der Verbindung aus Beispiel 26A wurden nach der Allgemeinen Methode 2A umgesetzt. Das Reaktionsgemisch wurde säulenchromographisch an Kieselgel aufgereinigt (Dichlormethan/Methanol 10:1). Ausbeute: 4.1 g (29% d. Th.)

LC-MS (Methode 1B): Rₜ = 1.50 min; m/z = 365 [M+H]⁺.

### Beispiel 28A

### N-{5-[4-(Trifluormethoxy)phenyl]piperidin-3-yl}benzolcarboxamid [racemisches cis-Isomer]

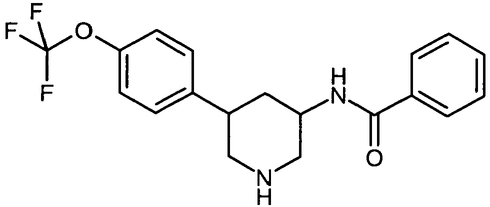

Die Diastereomerentrennung von 4.1 g des cis-/trans-Isomerengemisches (Beispiel 27A) nach Methode 10C ergab 1.1 g des Beispiels 28A (cis-Isomer).

LC-MS (Methode 1B): Rₜ = 1.56 min; m/z = 365 [M+H]⁺.

### Beispiel 29A

### 4-Nitrophenyl-3-[(phenylcarbonyl)amino]-5-[4-(trifluormethoxy)phenyl]piperidin-1-carboxylat [racemisches cis-Isomer]

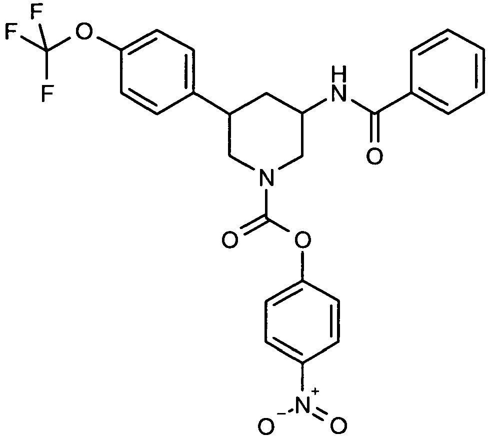

Bei 0°C wurden 200 mg (0.55 mmol) *N*-{5-[4-(Trifluormethoxy)phenyl]piperidin-3-yl}benzamid und 153 µl (1.1 mmol) Triethylamin in 17 ml Dichlormethan vorgelegt und langsam mit 111 mg (0.55 mmol) 4-Nitrophenylchlorcarbonat versetzt. Es wurde 2 h bei 0°C gerührt und anschließend auf RT erwärmt. Das Reaktionsgemisch wurde mit Wasser und gesättigter, wäßriger Natriumhydrogencarbonatlösung versetzt und extrahiert. Die organische Phase wurde über Natriumsulfat getrocknet und im Vakuum eingeengt. Die Aufreinigung des Rohproduktes erfolgt anschließend mittels präparativer HPLC (Reprosil C18, Wasser-Acetonitril-Gradient). Ausbeute: 254 mg (87% d. Th.)

LC-MS (Methode 1B): Rₜ = 2.88 min; m/z = 530 [M+H]⁺.

### Beispiel 30A

### 1-[(4-Cyanpiperidin-1-yl)carbonyl]-5-(4-ethylphenyl)piperidin-3-carboxylat [racemisches cis-Isomer]

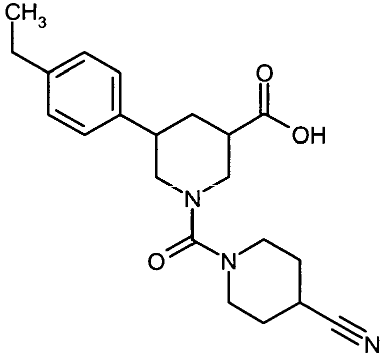

Nach der Allgemeinen Methode 7A wurden 2.0 g (5.2 mmol) der Verbindung aus Beispiel 18A umgesetzt. Ausbeute: 1.8 g (91% d. Th.)

LC-MS (Methode 2B): Rₜ = 1.13 min; MS (ESIpos): m/z = 370 [M+H]⁺.

### Beispiel 31A

### N-[5-(3,4-Dimethylphenyl)pyridin-3-yl]benzolcarboxamid

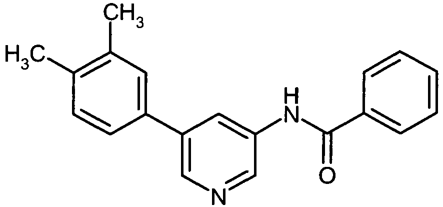

4.5 g (16.2 mmol) *N*-(5-Brompyridin-3-yl)benzamid, 3.0 g (19.5 mmol) (3,4-Dimethylphenyl)-boronsäure und 4.5 g (32.5 mmol) Kaliumcarbonat wurden in 59 ml 1,2-Dimethoxyethan, 19 ml Wasser und 117 ml DMF bei 50°C gelöst. Es wurde mit Argon gespült und 0.1 g (0.08 mmol) Tetrakis(triphenylphosphin)palladium(0) wurden zugegeben und 12 h bei 85°C gerührt. Das Reaktionsgemisch wurde am Rotationsverdampfer etwas eingeengt, mit Wasser verdünnt und mit Dichlormethan extrahiert. Die organische Phase wurde eingeengt und säulenchromatographisch an Kieselgel aufgereinigt (Dichlormethan/Methanol 100:1 -> 100:4). Ausbeute: 3.4 g (68% d. Th.)

LC-MS (Methode 2B): Rₜ = 1.17 min; m/z = 303 [M+H]⁺.

### Beispiel 32A

### N-[5-(3,4-Dimethylphenyl)piperidin-3-yl]benzolcarboxamid [racemisches cis-/trans-Isomerengemisch]

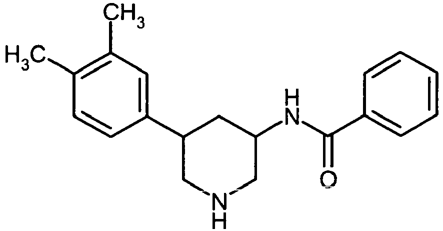

Eine Lösung von 1.0 g (3.7 mmol) *N*-[5-(3,4-Dimethylphenyl)pyridin-3-yl]benzamid in 120 ml Essigsäure wurde nach der Allgemeinen Methode 6A umgesetzt. Die Lösung wurde im Vakuum eingeengt. Ausbeute: 1.85 g

LC-MS (Methode 2B): Rₜ = 0.93 min; MS (ESIpos): m/z = 309 [M+H]⁺.

### Beispiel 33A

### N-[5-(3,4-Dimethylphenyl)piperidin-3-yl]benzolcarboxamid [racemisches cis-Isomer]

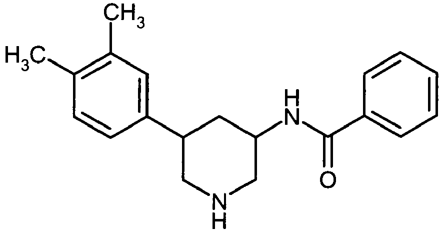

Die Diastereomerentrennung von 3.7 g des cis-/trans-Isomerengemisches (Beispiel 32A) nach Methode 11C ergab 0.8 g des Beispiels 33A (cis-Isomer).

LC-MS (Methode 2B): Rₜ = 0.93 min; m/z = 309 [M+H]⁺.

### Beispiel 34A

### tert.-Butyl-[4-({3-(3,4-dimethylphenyl)-5-[(phenylcarbonyl)amino]piperidin-1-yl}carbonyl)-tetrahydro-2H-pyran-4-yl]carbamat [racemisches cis-Isomer]

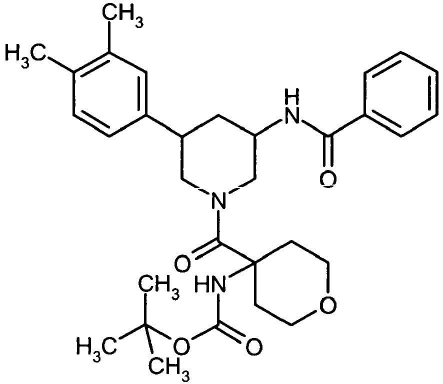

75 mg (0.3 mmol) 4-[(*tert*.-Butoxycarbonyl)amino]tetrahydro-2*H*-pyran-4-carbonsäure wurden zusammen mit 110 mg (0.3 mmol) HATU und 47 mg (0.4 mmol) 4-Dimethylaminopyridin in 2 ml DMF vorgelegt und mit 60 mg (0.2 mmol) *N*-[5-(3,4-Dimethylphenyl)piperidin-3-yl]benzamid versetzt. Es wurde über Nacht bei RT gerührt und anschließend das Reaktionsgemisch mittels präparativer HPLC (Reprosil C18, Wasser-Acetonitril-Gradient) gereinigt. Ausbeute: 46 mg (44% d. Th.)

LC-MS (Methode 1B): Rₜ = 2.45 min; m/z = 536 [M+H]⁺.

### Beispiel 35A

### tert.-Butyl-[1-({3-(3,4-dimethylphenyl)-5-[(phenylcarbonyl)amino]piperidin-1-yl}carbonyl)-cyclobutyl]carbamat [racemisches cis-Isomer]

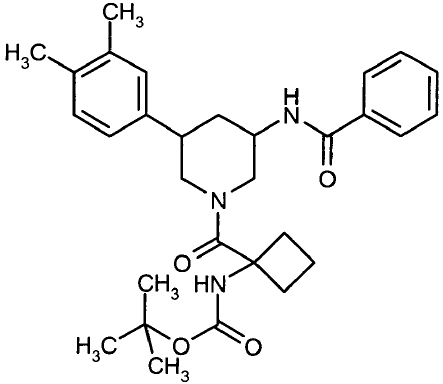

62 mg (0.3 mmol) 1-[(*tert*.-Butoxycarbonyl)amino]cyclobutancarbonsäure wurden zusammen mit 110 mg (0.3 mmol) HATU und 47 mg (0.4 mmol) 4-Dimethylaminopyridin in 2 ml DMF vorgelegt und mit 60 mg (0.2 mmol) *N*-[-5-(3,4-Dimethylphenyl)piperidin-3-yl]benzamid versetzt. Es wurde über Nacht bei RT gerührt und anschließend das Reaktionsgemisch mittels präparativer HPLC (Reprosil C18, Wasser-Acetonitril-Gradient) gereinigt. Ausbeute: 54 mg (55% d. Th.)

LC-MS (Methode 1B): Rₜ = 2.61 min; m/z = 505 [M+H]⁺.

### Beispiel 36A

### N-{5-[3-(Propan-2-yl)phenyl]pyridin-3-yl}benzolcarboxamid

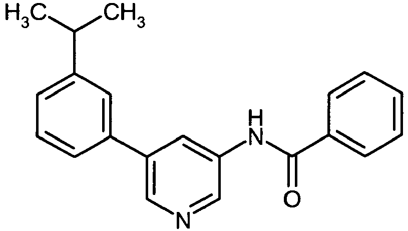

1.4 g (4.9 mmol) *N*-(5-Brompyridin-3-yl)benzamid, 1.0 g (5.9 mmol) [3-(1-Methylethyl)phenyl]-boronsäure und 1.4 g (9.9 mmol) Kaliumcarbonat wurden in 15 ml 1,2-Dimethoxyethan, 5 ml Wasser und 30 ml DMF bei 50°C gelöst. Es wurde mit Argon gespült und 29 mg (0.03 mmol) Tetrakis(triphenylphosphin)palladium(0) wurden zugegeben und 12 h bei 85°C gerührt. Das Reaktionsgemisch wurde am Rotationsverdampfer etwas eingeengt, mit Wasser verdünnt und mit Dichlormethan extrahiert. Die organische Phase wurde eingeengt und anschließend mittels präparativer HPLC (Reprosil C18, Wasser-Acetonitril-Gradient) gereinigt. Ausbeute: 1.4 g (92% d. Th.)

LC-MS (Methode 2B): Rₜ = 1.27 min; m/z = 317 [M+H]⁺.

### Beispiel 37A

### N-{5-[3-(Propan-2-yl)phenyl]piperidin-3-yl}benzolcarboxamid [racemisches cis-/trans-Isomerengemisch] Trifluoressigsäure

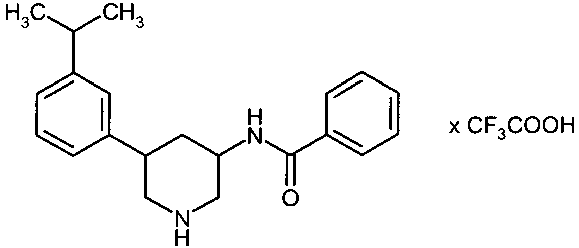

Eine Lösung von 1.43 g (4.52 mmol) *N*-{5-[3-(1-Methylethyl)phenyl]pyridin-3-yl}benzamid in 130 ml Ethanol und 30 ml Essigsäure wurde nach der Allgemeinen Methode 2A umgesetzt. Die Lösung wurde über Celite filtriert und das Filtrat im Vakuum eingeengt. Es wurde chromatographisch aufgereinigt. Ausbeute: 0.18 g (8.8% d. Th.)

LC-MS (Methode 3B): Rₜ = 1.20 min; MS (ESIpos): m/z = 323 [M+H]⁺.

### Beispiel 38A

### N-[5-(2,3-Dimethylphenyl)pyridin-3-yl]benzolcarboxamid

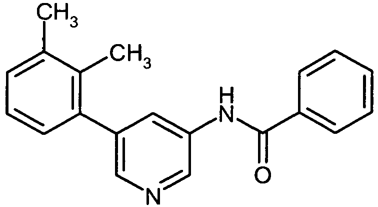

3.93 g (13.9 mmol) *N*-(5-Brompyridin-3-yl)benzamid, 2.50 g (16.7 mmol) (2,3-Dimethylphenyl)-boronsäure und 3.84 g (27.8 mmol) Kaliumcarbonat wurden in 50 ml 1,2-Dimethoxyethan, 17 ml Wasser und 100 ml DMF bei 50°C gelöst. Es wurde mit Argon gespült und 81 mg (0.07 mmol) Tetrakis(triphenylphosphin)palladium(0) wurden zugegeben und 12 h bei 85°C gerührt. Das Reaktionsgemisch wurde am Rotationsverdampfer etwas eingeengt, mit Wasser verdünnt und mit Dichlormethan extrahiert. Die organische Phase wurde im Vakuum eingeengt und der Rückstand mit Methyl-*tert*.-butylether verrührt, der Feststoff wurde abfiltriert und das Filtrat säulenchromatographisch an Kieselgel aufgereinigt (Cyclohexan/Essigsäureethylester 2:1 -> 1:1). Der abgetrennte Festoff und die geeinete Fraktion aus der Chromatographie wurden vereinigt. Ausbeute: 4.2 g (96% d. Th.)

LC-MS (Methode 1B): Rₜ = 2.24 min; m/z = 303 [M+H]⁺.

### Beispiel 39A

### N-[5-(2,3-Dimethylphenyl)piperidin-3-yl]benzolcarboxamid [racemisches cis-/trans-Isomerengemisch]

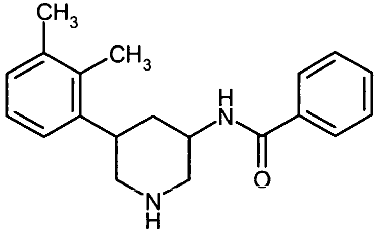

Eine Lösung von 550 mg (1.82 mmol) *N*-[5-(2,3-Dimethylphenyl)pyridin-3-yl]benzamid in 150 ml Ethanol wurden nach der Allgemeinen Methode 5A umgesetzt. Die Lösung wurde im Vakuum eingeengt. Es wurde mittels präparativer HPLC (Reprosil C18, Wasser-Acetonitril-Gradient) gereinigt. Ausbeute: 760 mg (77% d. Th., 57% Reinheit)

LC-MS (Methode 3B): Rₜ = 1.26 min; MS (ESIpos): m/z = 309 [M+H]⁺.

### Beispiel 40A

### N-[5-(2,3-Dimethylphenyl)piperidin-3-yl]benzolcarboxamid[racemisches cis-Isomer]

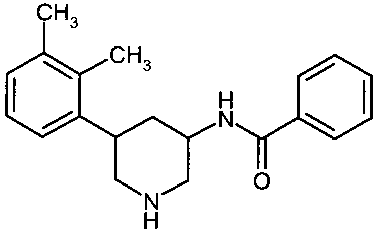

Die Diastereomerentrennung von 720 mg des cis-/trans-Isomerengemisches (Beispiel 39A) nach Methode 12C ergab 95 mg des Beispiels 40A (cis-Isomer).

LC-MS (Methode 1B): Rₜ = 1.41 min; m/z = 309 [M+H]⁺.

### Beispiel 41A

### 4-{5-[(Phenylcarbonyl)amino]pyridin-3-yl}benzolcarbonsäureethylester

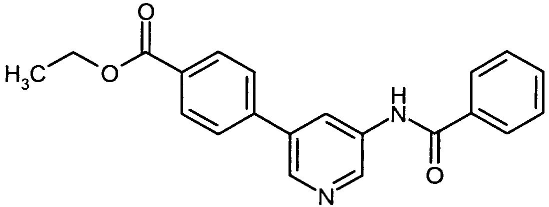

5.14 g (18.2 mmol) *N*-(5-Brompyridin-3-yl)benzamid, 5.40 g (27.8 mmol) 4-Ethoxycarbonylphenylboronsäure und 5.03 g (36.4 mmol) Kaliumcarbonat wurden in 30 ml 1,2-Dimethoxyethan, 9 ml Wasser und 65 ml DMF bei 50°C gelöst. Es wurde mit Argon gespült und 105 mg (0.09 mmol) Tetrakis(triphenylphosphin)palladium(0) wurden zugegeben und 12 h bei 85°C gerührt. Das Reaktionsgemisch wurde am Rotationsverdampfer etwas eingeengt, mit Wasser verdünnt und mit Dichlormethan extrahiert. Die organische Phase wurde im Vakuum eingeengt und der Rückstand mit Methyl-*tert*.-butylether verrührt, der Feststoff wurde abfiltriert und das Filtrat säulenchromatographisch aufgereinigt (Cyclohexan/Essigsäureethylester 2:1 -> 1:1). Der abgetrennte Festoff und die geeinete Fraktion aus der Chromatographie wurden vereinigt. Ausbeute: 4.6 g (60% d. Th.)

LC-MS (Methode 1B): Rₜ = 2.36 min; m/z = 347 [M+H]⁺.

### Beispiel 42A

### 4-{5-[(Phenylcarbonyl)amino]piperidin-3-yl}benzolcarbonsäureethylester [racemisches cis-/trans-Isomerengemisch]

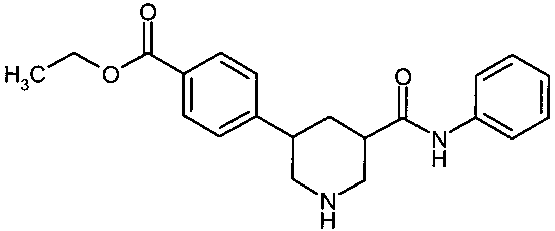

Nach der Allgemeinen Methode 2A wurden 17.45 g (50.37 mmol) 4-{5-[(Phenylcarbonyl)amino]pyridin-3-yl}benzoesäureethylester umgesetzt. Gereinigt wurde das Reaktionsgemisch säulenchromographisch (Dichlormethan/Methanol 10:1). Ausbeute: 10.4 g (ca. 75% Reinheit).

LC-MS (Methode 2B): Rₜ = 0.85 min; m/z = 353 [M+H]⁺.

### Beispiel 43A

### Ethyl-4-{5-[(phenylcarbonyl)amino]piperidin-3-yl}benzolcarboxylat [racemisches cis-Isomer]

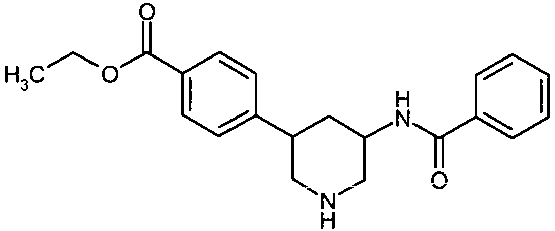

Die Diastereomerentrennung von 10.3 g des cis-/trans-Isomerengemisches (Beispiel 42A) nach Methode 13C ergab 4.2 g des Beispiels 43A (cis-Isomer).

LC-MS (Methode 2B): Rₜ = 0.87 min; m/z = 353 [M+H]⁺.

### Beispiel 44A

### N-{5-[3-(Trifluormethyl)phenyl]pyridin-3-yl}benzolcarboxamid

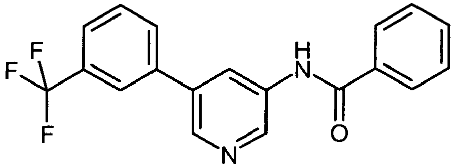

1.95 g (7.02 mmol) *N*-(5-Brompyridin-3-yl)benzamid, 2.00 g (10.5 mmol) [3-(Trifluormethyl)-phenyl]boronsäure und 1.49 g (14.04 mmol) Natriumcarbonat wurden in 12 ml 1,2-Dimethoxyethan, 3.5 ml Wasser und 261 ml DMF bei 50°C gelöst. Es wurde mit Argon gespült und 41 mg (0.04 mmol) Tetrakis(triphenylphosphin)palladium(0) wurden zugegeben und 12 h bei 85°C gerührt. Das Reaktionsgemisch wurde am Rotationsverdampfer etwas eingeengt, mit Wasser verdünnt und mit Dichlormethan extrahiert. Die organische Phase wurde im Vakuum eingeengt und der Rückstand säulenchromatographisch (Cyclohexan/Essigsäureethylester 2:1) gereingt. Ausbeute: 860 mg (35% d. Th.)

LC-MS (Methode 5B): Rₜ = 2.26 min; m/z = 343 [M+H]⁺.

### Beispiel 45A

### N-{5-[3-(Trifluormethyl)phenyl]piperidin-3-yl}benzolcarboxamid [racemisches cis-/trans-Isomerengemisch]

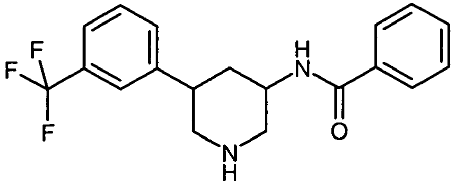

Eine Lösung von 840 mg (1.82 mmol) *N*-{5-[3-(Trifluormethyl)phenyl]pyridin-3-yl}benzamid in 100 ml Eisessig wurde nach der Allgemeinen Methode 6A umgesetzt. Die Lösung wurde im Vakuum eingeengt. Es wurde mittels präparativer HPLC (Reprosil C18, Wasser-Acetonitril-Gradient) gereinigt. Ausbeute: 700 mg

LC-MS (Methode 5B): Rₜ = 1.55 min; m/z = 349 [M+H]⁺.

### Beispiel 46A

### N-{5-[3-(Trifluormethyl)phenyl]piperidin-3-yl}benzolcarboxamid [racemisches cis-Isomer]

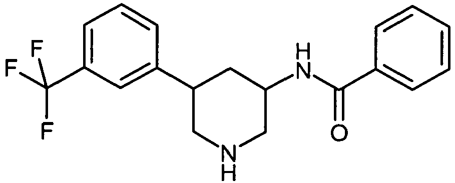

Die Diastereomerentrennung von 840 mg des cis-/trans-Isomerengemisches (Beispiel 45A) nach Methode 14C ergab 176 mg des Beispiels 46A (cis-Isomer).

¹H-NMR (400 MHz, DMSO-*d*₆): δ = 8.22 (d, 1H), 7.85 (d, 2H), 7.39 - 7.63 (m, 7H), 3.88 - 4.06 (m, 1H), 3.09 (dd, 1H), 2.99 (d, 1H), 2.85 - 2.95 (m, 1H), 2.37 - 2.47 (m, 2H), 2.02 - 2.14 (m, 1H), 1.74 (q, 1H).

### Beispiel 47A

### N-{5-[4-(Trifluormethyl)phenyl]pyridin-3-yl}benzolcarboxamid

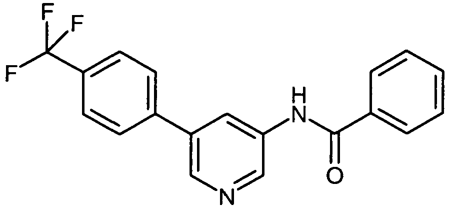

11.35 g (40.96 mmol) *N*-(5-Brompyridin-3-yl)benzamid, 11.67 g (61.43 mmol) [4-(Trifluormethyl)phenyl]boronsäure und 8.68 g (81.91 mmol) Natriumcarbonat wurden in 68 ml 1,2-Dimethoxyethan, 21 ml Wasser und 151 ml DMF bei 50°C gelöst. Es wurde mit Argon gespült und 237 mg (0.21 mmol) Tetrakis(triphenylphosphin)palladium(0) wurden zugegeben und 12 h bei 85°C gerührt. Das Reaktionsgemisch wurde am Rotationsverdampfer etwas eingeengt, mit Wasser verdünnt und mit Dichlormethan extrahiert. Die organische Phase wurde im Vakuum eingeengt und der Rückstand säulenchromatographisch (Cyclohexan/Essigsäureethylester 1:1) gereinigt. Ausbeute: 10.6 g (76% d. Th.)

LC-MS (Methode 5B): Rₜ = 2.31 min; m/z = 343 [M+H]⁺.

### Beispiel 48A

### N-{5-[4-(Trifluormethyl)phenyl]piperidin-3-yl}benzolcarboxamid [racemisches cis-/trans-Isomerengemisch]

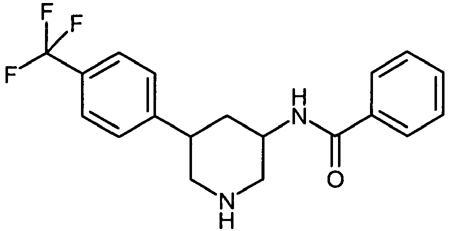

Eine Lösung von 1.0 g (2.9 mmol) *N*-{5-[4-(Trifluormethyl)phenyl]pyridin-3-yl}benzamid in 120 ml Eisessig wurde nach der Allgemeinen Methode 6A umgesetzt. Die Lösung wurde im Vakuum eingeengt. Es wurde mittels präparativer HPLC (Reprosil C18, Wasser-Acetonitril-Gradient) gereinigt. Ausbeute: 657 mg (50% Reinheit)

LC-MS (Methode 5B): Rₜ = 1.69 min; m/z = 349 [M+H]⁺.

### Beispiel 49A

### N-{5-[4-(Trifluormethyl)phenyl]piperidin-3-yl}benzolcarboxamid [racemisches cis-Isomer]

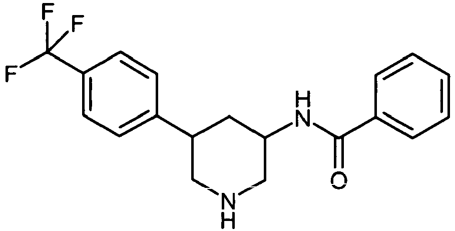

Die Diastereomerentrennung von 2.0 g des cis-/trans-Isomerengemisches (Beispiel 48A) nach Methode 15C ergab 708 mg des Beispiels 49A (cis-Isomer).

¹H NMR (400 MHz, DMSO-*d*₆): δ = 8.24 (d, 1H), 7.84 (d, 2H), 7.68 (d, 2H), 7.40 - 7.56 (m, 5H), 3.93 - 4.06 (m, 1H), 3.09 (dd, 1H), 2.99 (d, 1H), 2.83 - 2.94 (m, 1H), 2.37 - 2.47 (m, 2H), 2.08 (d, 1H), 1.72 (q, 1H).

### Beispiel 50A

### 4-Nitrophenyl-3-[(phenylcarbonyl)amino]-5-[4-(trifluormethyl)phenyl]piperidin-1-carboxylat [racemisches cis-Isomer]

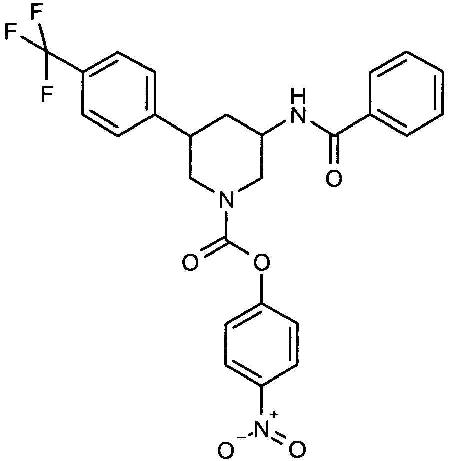

Bei 0°C wurden 1.5 g (4.3 mmol) *N*-{5-[4-(Trifluormethyl)phenyl]piperidin-3-yl}benzamid und 1.2 ml (1.1 mmol) Triethylamin in 131 ml Dichlormethan vorgelegt und langsam mit 868 mg (4.3 mmol) 4-Nitrophenylchlorocarbonat versetzt. Es wurde 2 h bei 0°C gerührt und anschließend auf RT erwärmt. Das Reaktionsgemisch wurde mit Wasser und gesättigter, wässriger Natriumhydrogencarbonat-Lösung versetzt und extrahiert. Die organische Phase wurde im Vakuum eingeengt und säulenchromatographisch (Cyclohexan/Essigsäureethylester 1:2) gereinigt. Ausbeute: 1.95 g (88% d. Th.)

LC-MS (Methode 1B): Rₜ = 2.82 min; m/z = 514 [M+H]⁺.

### Beispiel 51A

### tert.-Butyl-[1-({3-[(phenylcarbonyl)amino]-5-[4-(trifluormethyl)phenyl]piperidin-1-yl}carbonyl)-cyclopropyl]carbamat [racemisches cis-Isomer]

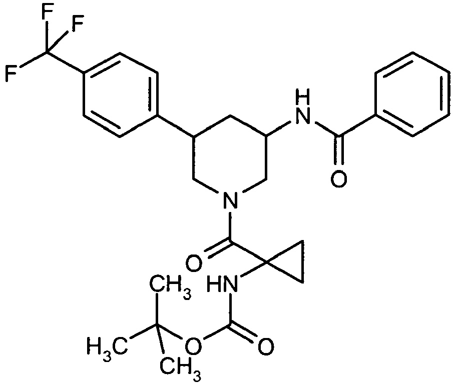

125 mg (0.62 mmol) 1-[(*tert*.-Butoxycarbonyl)amino]cyclopropancarbonsäure wurden zusammen mit 295 mg (0.78 mmol) HATU und 127 mg (1.03 mmol) 4-Dimethylaminopyridin in 8 ml DMF vorgelegt und mit 180 mg (0.52 mmol) *N*-{5-[4-(Trifluormethyl)phenyl]piperidin-3-yl}benzamid versetzt. Es wurde über Nacht bei RT gerührt und anschließend das Reaktionsgemisch mittels präparativer HPLC (Reprosil C18, Wasser-Acetonitril-Gradient) gereinigt. Ausbeute: 179 mg (65% d. Th.)

### Beispiel 52A

### 5-[4-(Trifluormethyl)phenyl]pyridin-3-carbonsäuremethylester

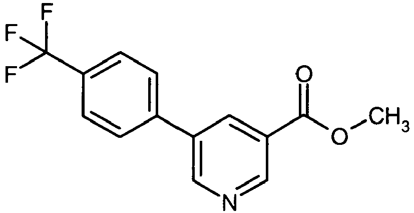

Nach der Allgemeinen Methode 1A wurden 28 g (132 mmol) 5-Bromnicotinsäuremethylester und 30 g (158 mmol, 1.2 eq.) 4-Trifluormethylphenylboronsäure umgesetzt. Ausbeute: 32 g (85% d. Th.)

LC-MS (Methode 8B): Rₜ = 2.27 min; MS (ESIpos): m/z = 282 [M+H]⁺.

### Beispiel 53A

### 5-[4-(Trifluormethyl)phenyl]piperidin-3-carbonsäuremethylester [racemisches cis-/trans-Isomerengemisch]

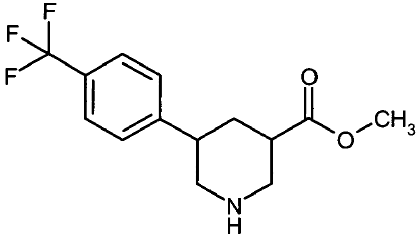

Nach der Allgemeinen Methode 2A wurden 32 g (112 mmol) 5-[4-(Trifluormethyl)phenyl]-pyridin-3-carbonsäuremethylester (Beispiel 52A) hydriert. Ausbeute: 26 g (82% d. Th.)

LC-MS (Methode 1B): Rₜ = 1.35 und 1.41 min (cis-/trans-Isomere); MS (ESIpos): m/z = 288 [M+H]⁺.

### Beispiel 54A

### 1-(Morpholin-4-ylcarbonyl)-5-[4-(trifluormethyl)phenyl]piperidin-3-carbonsäuremethylester [racemisches cis-/trans-Isomerengemisch]

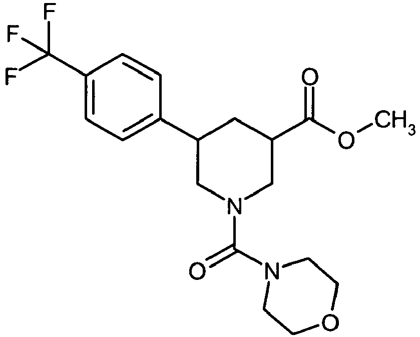

Nach der Allgemeinen Methode 3A wurden 9.25 g (32.2 mmol) 5-[4-(Trifluormethyl)phenyl]-piperidin-3-carbonsäuremethylester mit 9.63 g (64.7 mmol) Morpholin-4-carbonylchlorid umgesetzt. Man erhielt so 16.3 g Rohprodukt in 76%-iger Reinheit (LC-MS), welches ohne weitere Reinigungsoperationen umgesetzt wurde.

LC-MS (Methode 10B): Rₜ = 1.19 und 1.22 min (cis-/trans-Isomere); MS (ESIpos): m/z = 401 [M+H]⁺.

### Beispiel 55A

### 1-(Morpholin-4-ylcarbonyl)-5-[4-(trifluormethyl)phenyl]piperidin-3-carbonsäure [racemisches cis-Isomer]

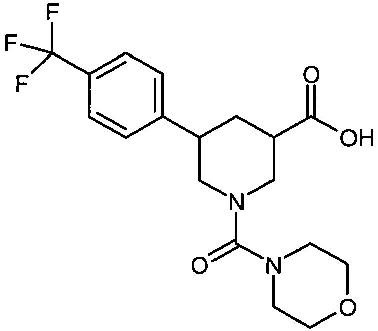

Nach der Allgemeinen Methode 7A wurden 22.19 g (39.90 mmol) der Verbindung aus Beispiel 54A und 44.78 g (399.0 mmol) Kalium-*tert*.-butylat umgesetzt. Ausbeute: 18.29 g (100% d. Th.)

LC-MS (Methode 5B): Rₜ = 1.95 min; MS (ESIpos): m/z = 387 [M+H]⁺.

### Beispiel 56A

### 1-{[3-Amino-5-(4-ethylphenyl)piperidin-1-yl]carbonyl}piperidin-4-carbonitril [racemisches cis-Isomer]

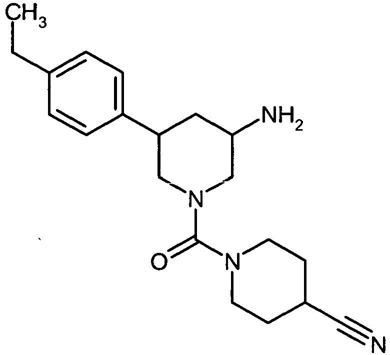

Nach der Allgemeinen Methode 5A wurden 1.9 g (5.2 mmol) der Verbindung aus Beispiel 30A umgesetzt. Ausbeute: 0.9 g (49% d. Th.)

LC-MS (Methode 11B): Rₜ = 0.72 min; MS (ESIpos): m/z = 341 [M+H]⁺.

### Beispiel 57A

### {3-Amino-5-[4-(trifluormethyl)phenyl]piperidin-1-yl}(morpholin-4-yl)methanonhydrochlorid [racemisches cis-Isomer]

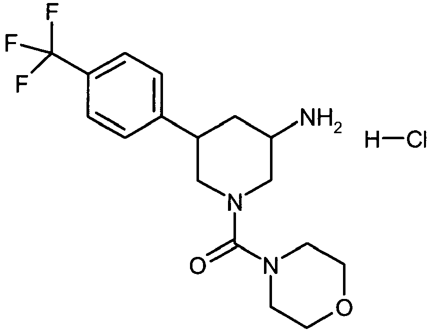

Das Carbamat aus Beispiel 172 (9.50 g, 20.1 mmol) wurde mit 101 ml einer 4 N Hydrogenchlorid-Lösung in Dioxan versetzt und anschließend für 1 h bei RT gerührt. Die Reaktionslösung wurde im Vakuum eingeengt und der Rückstand in 1 N wässriger Hydrogenchlorid-Lösung aufgenommen. Nach Waschen der wässrigen Phase mit Diethylether wurde die wässrige Phase im Vakuum eingeengt. Das so erhaltende Rohprodukt wurde ohne weitere Reinigung weiter eingesetzt. Ausbeute: 5.27 g (65% d. Th.)

LC-MS (Methode 11B): Rₜ = 0.71 min; MS (ESIpos): m/z = 358 [M+H]⁺.

### Beispiel 58A

### 3-Methyl-1-(4-nitrophenyl)-5-[4-(trifluormethyl)phenyl]piperidin-1,3-dicarboxylat [racemisches cis-/trans-Isomerengemisch]

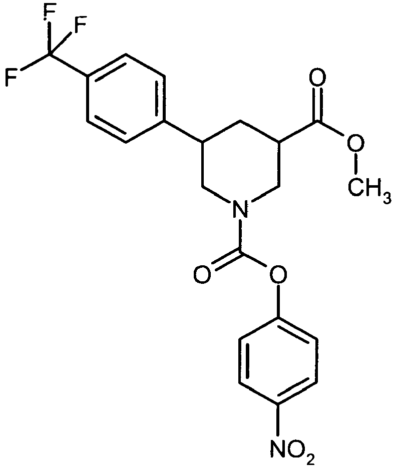

20.0 g (69.6 mmol) der Verbindung aus Beispiel 53A wurden in 1.01 Dichlormethan gelöst und bei 0°C mit 14.1 g (139 mmol) Triethylamin versetzt. Anschließend wurden 14.0 g (69.6 mmol) 4-Nitrophenylchlorocarbonat zugetropft. Die Reaktionsmischung wurde für 2 h bei 0°C und anschließend 16 h bei RT gerührt. Zur Aufarbeitung wurde mit gesättigter, wässriger Natriumhydrogencarbonat-Lösung gewaschen. Die organische Phase wurde über Magnesiumsulfat getrocknet, filtriert und im Vakuum eingeengt. Es wurden 31.3 g Rohprodukt erhalten, welches ohne weitere Reinigungsoperationen umgesetzt wurde.

LC-MS (Methode 1B): Rₜ = 2.44 min und 2.48 min (cis-/trans-Isomere); MS (ESIpos): m/z = 453 [M+H]⁺.

### Beispiel 59A

### Methyl-1-(1,4-dioxa-8-azaspiro[4.5]dec-8-ylcarbonyl)-5-[4-(trifluormethyl)phenyl]piperidin-3-carboxylat [racemisches cis/trans-Isomerengemisch]

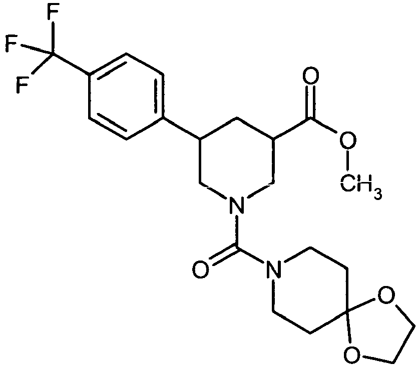

Nach der Allgemeinen Methode 8A wurden 6.00 g (13.3 mmol) der Verbindung aus Beispiel 58A, 4.75 g (33.2 mmol) 8-Aza-1,4-dioxa-spiro[4.5]decan und 1.83 g (13.3 mmol) Kaliumcarbonat umgesetzt. Ausbeute: 5.15 g (81% d. Th.)

LC-MS (Methode 11B): Rₜ = 1.12 und 1.14 min (cis-/trans-Isomere); MS (ESIpos): m/z = 457 [M+H]⁺.

### Beispiel 60A

### 1-(1,4-Dioxa-8-azaspiro[4.5]dec-8-ylcarbonyl)-5-[4-(trifluormethyl)phenyl]piperidin-3-carbonsäure [racemisches cis-Isomer]

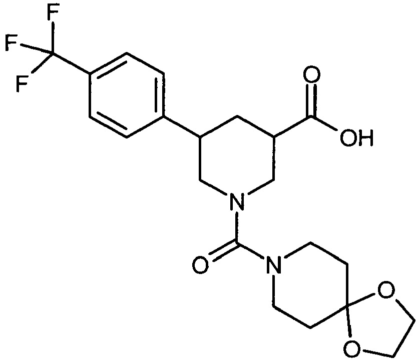

Nach der Allgemeinen Methode 7A wurden 5.15 g (11.3 mmol) der Verbindung aus Beispiel 59A und 12.7 g (113 mmol) Kalium-*tert*.-butylat umgesetzt. Ausbeute: 5.00 g (99% d. Th.)

LC-MS (Methode 11B): Rₜ = 0.99 min; MS (ESIpos): m/z = 443 [M+H]⁺.

### Beispiel 61A

### tert.-Butyl-{1-(1,4-dioxa-8-azaspiro[4.5]dec-8-ylcarbonyl)-5-[4-(trifluormethyl)phenyl]piperidin-3-yl}carbamat [racemisches cis-Isomer]

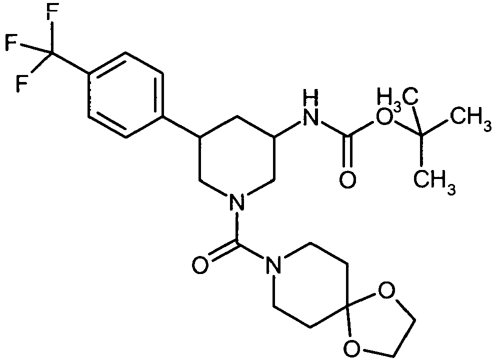

Die Carbonsäure aus Beispiel 60A (5.00 g, 10.4 mmol) in *tert*.-Butanol (189 ml) wurde mit aktiviertem Molsieb 4 Å (ca. 8.4 g), 1.74 ml Triethylamin (12.5 mmol) und 3.15 g (11.4 mmol) Diphenylphosphorazidat versetzt und unter Rückfluss über Nacht gerührt. Die Reaktionslösung wurde abgekühlt und anschließend das Molsieb abfiltriert und gründlich mit Essigsäureethylester gewaschen. Die Lösungsmittel wurden im Vakuum entfernt und der Rückstand in Essigsäureethylester aufgenommen. Nach Waschen mit 2 N wässriger Hydrogenchlorid-Lösung, gesättigter, wässriger Natriumhydrogencarbonat-Lösung und Wasser wurde die organische Phase über Magnesiumsulfat getrocknet, filtriert und im Vakuum eingeengt. Das Rohprodukt wurde ohne weitere Reinigung in der Folgestufe eingesetzt. Ausbeute: 5.32 g (70% d. Th., 71% Reinheit).

LC-MS (Methode 11B): Rₜ = 1.21 min; MS (ESIpos): m/z = 514 [M+H]⁺.

### Beispiel 62A

### {3-Amino-5-[4-(trifluormethyl)phenyl]piperidin-1-yl}(1,4-dioxa-8-azaspiro[4.5]dec-8-yl)-methanon-hydrochlorid [racemisches cis-Isomer]

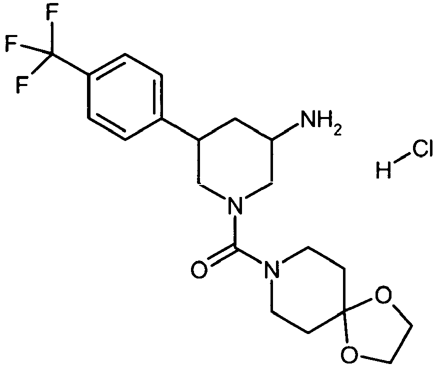

Das Carbamat aus Beispiel 61A (5.30 g, 7.33 mmol) wurde mit 37 ml einer 4 N Salzsäure-Lösung in Dioxan versetzt und anschließend für 1 h bei RT gerührt. Es wurden erneut 37 ml einer 4 N Salzsäure-Lösung in Dioxan zugegeben und 3 h bei 60°C gerührt. Die Reaktionslösung wurde im Vakuum eingeengt und der Rückstand in 1 N wässriger Hydrogenchlorid-Lösung aufgenommen. Nach Waschen der wässrigen Phase mit Diethylether wurde die wässrige Phase im Vakuum eingeengt. Das so erhaltende Rohprodukt wurde ohne weitere Reinigung weiter eingesetzt. Ausbeute: 3.20 g (92% d. Th.)

LC-MS (Methode 5B): Rₜ = 1.57 min; MS (ESIpos): m/z = 414 [M+H]⁺.

### Beispiel 63A

### N-{1-(1,4-Dioxa-8-azaspiro[4.5]dec-8-ylcarbonyl)-5-[4-(trifluormethyl)phenyl]piperidin-3-yl}-cyclo-pentancarboxamid [racemisches cis-Isomer]

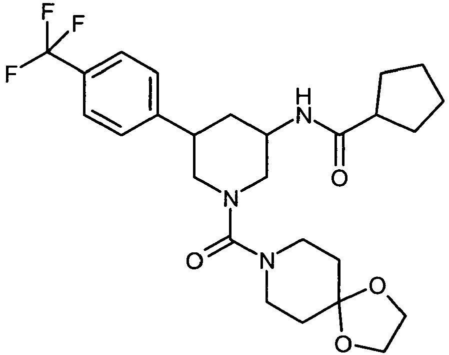

Eine Lösung des Amin-Hydrochlorids aus Beispiel 62A (200 mg, 0.422 mmol) in Dichlormethan (10 ml) wurde mit 177 µl Triethylamin (1.27 mmol) und 15.5 mg DMAP (0.127 mmol) versetzt und anschließend bei 0°C 84 mg Cyclopentancarbonsäurechlorid (0.633 mmol) zugegeben. Die Reaktionsmischung wurde auf RT erwärmt und über Nacht gerührt. Die Reaktionslösung wurde mit wässriger 1 N Hydrogenchlorid-Lösung gewaschen und die organische Phase über Magnesiumsulfat getrocknet, filtriert und im Vakuum eingeengt. Das Rohprodukt wurde über präparative HPLC (RP18-Säule; Acetonitril-Wasser-Gradient) gereinigt. Ausbeute: 112 mg (52% d. Th.)

LC-MS (Methode 2B): Rₜ = 1.28 min; MS (ESIpos): m/z = 510 [M+H]⁺.

### Beispiel 64A

### 3-Chlor-N-{1-(1,4-dioxa-8-azaspiro[4.5]dec-8-ylcarbonyl)-5-[4-(trifluormethyl)phenyl]piperidin-3-yl}benzolcarboxamid [racemisches cis-Isomer]

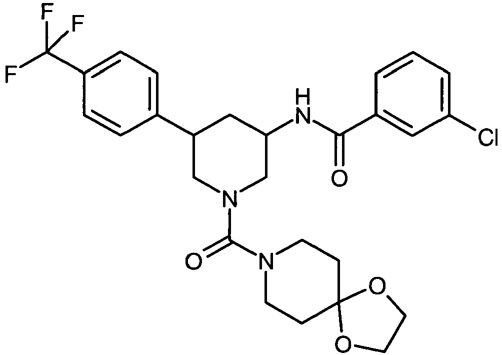

Eine Lösung der Verbindung aus Beispiel 62A (500 mg, 1.06 mmol) in *N*,*N*'-Dimethylformamid (18 ml) wurde mit 0.59 ml (3.38 mmol) *N*,*N*'-Diisopropylethylamin und 248 mg (1.58 mmol) 3-Chlorbenzoesäure versetzt. Nach Zugabe von 522 mg (1.37 mmol) HATU wurde über Nacht bei RT gerührt. Das Reaktionsgemisch wurde direkt über präparative HPLC (RP18-Säule; Acetonitril-Wasser-Gradient) gereinigt. Ausbeute: 414 mg (67% d. Th.)

LC-MS (Methode 2B): Rₜ = 1.36 min; MS (ESIpos): m/z = 552 [M+H]⁺.

### Beispiel 65A

### 3-Methyl-1-(4-nitrophenyl)-5-(4-ethylphenyl)piperidin-1,3-dicarboxylat [racemisches cis-/trans-Isomerengemisch]

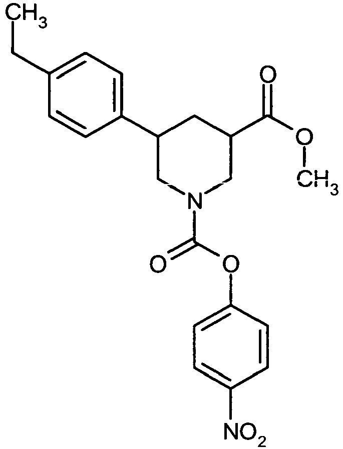

10.0 g (40.4 mmol) der Verbindung aus Beispiel 2A wurden in 135 ml Dichlormethan vorgelegt, auf 0°C abgekühlt und mit 11.2 ml (8.2 g, 80.9 mmol) Triethylamin sowie 8.5 g (40.4 mmol) Chlorameisensäure-4-nitrophenylester versetzt. Das Reaktionsgemisch ließ man innerhalb von 2 h auf RT erwärmen. Zur Aufarbeitung wurde zweimal mit Wasser gewaschen und die organische Phase über Natriumsulfat getrocknet. Das Lösungsmittel wurde im Vakuum entfernt und der Rückstand im Hochvakuum getrocknet. Ausbeute: 16.5 g (83% d. Th.).

LC-MS (Methode 11B): Rₜ = 1.31 min und 1.33 min (cis-/trans-Isomere); MS (ESIpos): m/z = 413 [M+H]⁺.

### Beispiel 66A

### 1-[(4-Cyanopiperidin-1-yl)carbonyl]-5-[4-(trifluormethyl)phenyl]piperidin-3-carbonsäure-methylester [racemisches cis-/trans-Isomerengemisch]

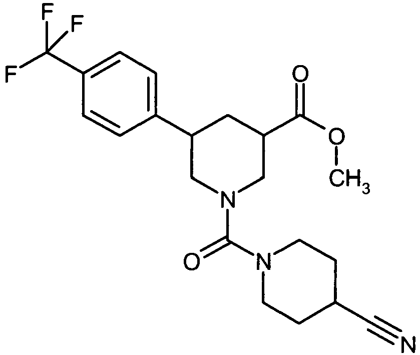

Nach der Allgemeinen Methode 8A wurden 4.00 g (8.84 mmol) 3-Methyl-1-(4-nitrophenyl)-5-[4-(trifluormethyl)phenyl]piperidin-1,3-dicarboxylat (Beispiel 58A) und 2.92 g (26.5 mmol) Piperidin-4-carbonitril umgesetzt. Ausbeute: 3.15 g (77% d. Th.)

LC-MS (Methode 1B): Rₜ = 2.35 min und 2.41 min (cis-/trans-Isomere); MS (ESIpos): m/z = 424 [M+H]⁺.

### Beispiel 67A

### 1-[(4-Cyanopiperidin-1-yl)carbonyl]-5-[4-(trifluormethyl)phenyl]piperidin-3-carbonsäure [racemisches cis-Isomer]

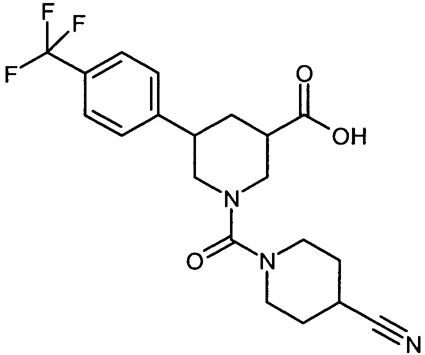

Nach der Allgemeinen Methode 9A (Reaktionszeit: 2 h) wurden 2.90 g (6.85 mmol) 1-[(4-Cyanopiperidin-1-yl)carbonyl]-5-[4-(trifluormethyl)phenyl]piperidin-3-carbonsäuremethylester (Beispiel 66A) umgesetzt. Ausbeute: 2.86 g (98% d. Th.)

LC-MS (Methode 1B): Rₜ = 2.15 min; MS (ESIpos): m/z = 410 [M+H]⁺.

### Beispiel 68A

### tert.-Butyl-{1-[(4-cyanpiperidin-1-yl)carbonyl]-5-[4-(trifluormethyl)phenyl]piperidin-3-yl}-carbamat [racemisches cis/trans-Isomerengemisch]

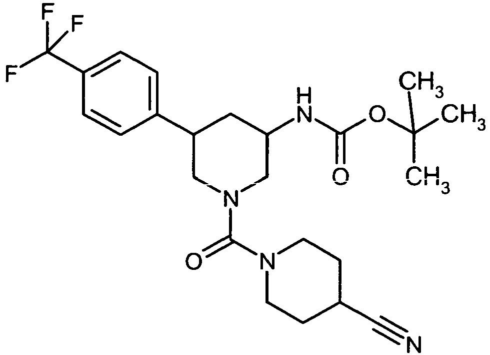

Eine Lösung von 7.90 g (19.3 mmol) der Carbonsäure aus Beispiel 67A in 350 ml *tert*.-Butanol wurde mit 18 g Molsieb 4Å, 3.2 ml (2.3 g, 23 mmol) Triethylamin und 4.6 ml (5.8 g, 21 mmol) Diphenylphosphorazidat versetzt und über Nacht unter Rückfluss gerührt. Anschließend wurde das Molsieb abfiltriert, mehrfach mit Essigsäureethylester gewaschen und das Filtrat im Vakuum eingeengt. Der Rückstand wurde in Essigsäureethylester aufgenommen und nachfolgend mit 2N Salzsäure, gesättigter wässriger Natriumhydrogencarbonat-Lösung und Wasser gewaschen. Die organische Phase wurde über Magnesiumsulfat getrocknet, filtriert und im Vakuum eingeengt. Ausbeute: 8.34 g mit 91%iger Reinheit (82% d. Th.)

LC-MS (Methode 11B): Rₜ = 1.16 und 1.19 min (cis-/trans-Isomere); MS (ESIpos): m/z = 481 [M+H]⁺.

### Beispiel 69A

### 1-({3-Amino-5-[4-(trifluormethyl)phenyl]piperidin-1-yl}carbonyl)piperidin-4-carbonitril-Hydrochlorid [racemisches cis-Isomer]

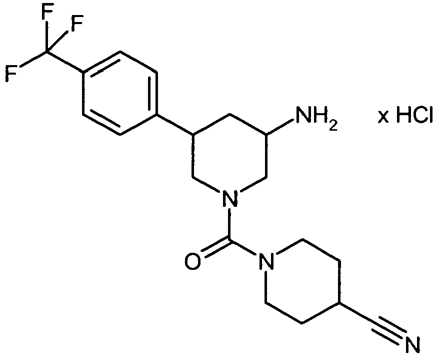

8.30 g (15.7 mmol) Carbamat aus Beispiel 68A mit 91%iger Reinheit wurden mit 78 ml einer 4N Chlorwasserstoff-Lösung in Dioxan versetzt und für 1 h bei Raumtemperatur gerührt. Die Reaktionslösung wurde im Vakuum eingeengt und der Rückstand in 1N Salzsäure aufgenommen. Nach Waschen mit Diethylether wurde die organische Phase im Vakuum eingeengt, der Rückstand in Dichlormethan aufgenommen und mehrfach mit 1N Salzsäure extrahiert. Die vereinigten wässrigen Phasen wurden im Vakuum eingeengt und der Rückstand im Hochvakuum getrocknet. Ausbeute: 4.93 g (71% d. Th.).

LC-MS (Methode 2B): Rₜ = 0.82 min; MS (ESIpos): m/z = 381 [M+H-HCl]⁺.

### Ausführungsbeispiele

### Allgemeine Methode 1: Amidkupplung mit Carbonsäuren

Eine Lösung der entsprechenden Carbonsäure (1.1 eq.) in Dimethylformamid (10 ml/mmol) wird unter Argon bei RT mit HATU (1.2 eq.) und *N*,*N*-Diisopropylethylamin (3.2 eq.) versetzt. Das Reaktionsgemisch wird 30 min bei RT gerührt und mit dem Amin (1.0 eq.) versetzt. Das Reaktionsgemisch wird bei RT gerührt. Nach Zugabe von Wasser und Phasentrennung wird die organische Phase mit Wasser und mit gesättigter, wässriger Natriumchlorid-Lösung gewaschen, getrocknet (Natriumsulfat), filtriert und im Vakuum eingeengt. Die Aufreinigung des Rohproduktes erfolgt anschließend mittels präparativer HPLC (Reprosil C18, Wasser-Acetonitril-Gradient).

### Allgemeine Methode 2: Amidkupplung mit Carbonsäurechloriden

Eine Lösung des Amins (1.0 eq.) in Tetrahydrofuran (10 ml/mmol) wird unter Argon bei RT tropfenweise mit dem entsprechenden Carbonsäurechlorid (1.1 eq.) und *N*,*N-*Diisopropylethylamin (3.2 eq.) versetzt. Das Reaktionsgemisch wird bei RT gerührt. Nach Zugabe von Wasser und Phasentrennung wird die organische Phase mit Wasser und mit gesättigter, wässriger Natriumchlorid-Lösung gewaschen, getrocknet (Natriumsulfat), filtriert und im Vakuum eingeengt. Die Aufreinigung des Rohproduktes erfolgt anschließend mittels präparativer HPLC (Reprosil C18, Wasser-Acetonitril-Gradient).

### Allgemeine Methode 3: Umsetzung mit Carbamoylchloriden

Eine Lösung des Amins (1.0 eq.) in Tetrahydrofuran (1.25 ml/mmol) wird unter Argon bei RT tropfenweise mit *N*,*N*-Diisopropylethylamin (2.5 eq.) und dem entsprechenden Carbamoylchlorid (1.3 eq.) versetzt. Das Reaktionsgemisch wird bei RT gerührt. Nach Zugabe von Wasser und Phasentrennung wird die organische Phase mit Wasser und mit gesättigter, wässriger Natriumchlorid-Lösung gewaschen, getrocknet (Natriumsulfat), filtriert und im Vakuum eingeengt. Die Aufreinigung des Rohproduktes erfolgt anschließend mittels präparativer HPLC (Reprosil C18, Wasser-Acetonitril-Gradient).

### Allgemeine Methode 4: Harnstoffbildung mit Isocyanat

Eine Lösung des Amins (1.0 eq.) in Tetrahydrofuran (10 ml/mmol) wird unter Argon bei RT tropfenweise mit *N*,*N-*Diisopropylethylamin (3.2 eq.) und dem entsprechenden Isocyanat (1.1 eq.) versetzt. Das Reaktionsgemisch wird bei RT gerührt. Nach Zugabe von Wasser und Phasentrennung wird die organische Phase mit Wasser und mit gesättigter, wässriger Natriumchlorid-Lösung gewaschen, getrocknet (Natriumsulfat), filtriert und im Vakuum eingeengt. Die Aufreinigung des Rohproduktes erfolgt anschließend mittels präparativer HPLC (Reprosil C18, Wasser-Acetonitril-Gradient).

### Allgemeine Methode 5: Umsetzung mit Chlorameisensäureester

Eine Lösung des Amins (1.0 eq.) in Tetrahydrofuran (1.25 ml/mmol) wird unter Argon bei RT tropfenweise mit *N*,*N*-Diisopropylethylamin (3 eq.) und dem entsprechenden Chlorameisensäureester (1.3 eq.) versetzt. Das Reaktionsgemisch wird bei RT gerührt. Nach Zugabe von Wasser und Phasentrennung wird die organische Phase mit Wasser und mit gesättigter, wässriger Natriumchlorid-Lösung gewaschen, getrocknet (Natriumsulfat), filtriert und im Vakuum eingeengt. Die Aufreinigung des Rohproduktes erfolgt anschließend mittels präparativer HPLC (Reprosil C18, Wasser-Acetonitril-Gradient).

### Allgemeine Methode 6: Sulfonamidbildung

Eine Lösung aus dem entsprechenden Amin (1 eq.) in Dichlormethan (21 ml/mmol) wird bei RT mit *N*,*N*'-Diisopropylethylamin (2.5 eq.) und dem entsprechenden Sulfonsäurechlorid (1.5 eq.) versetzt. Das Reaktionsgemisch wird über Nacht bei Raumtemperatur gerührt. Zur Aufarbeitung wird Dichlormethan im Vakuum entfernt, und der Rückstand wird mittels präparativer HPLC gereinigt.

### Allgemeine Methode 7: Suzuki-Reaktion

Eine Lösung des Bromides (1.0 eq.) in Toluol (5 ml/mmol) wird unter Argon bei RT mit Tetrakis-(triphenylphosphin)-palladium (0.02 eq.), mit einer Lösung der Boronsäure (1.2 eq.) in Ethanol (1 ml/mmol) und mit einer Lösung von Kaliumfluorid (2.0 eq.) in Wasser (1 ml/mmol) versetzt. Das Reaktionsgemisch wird am Rückfluss gerührt. Nach Zugabe von Essigsäureethylester und Phasentrennung wird die organische Phase mit Wasser gewaschen, getrocknet (Magnesiumsulfat), filtriert und im Vakuum eingeengt. Die Aufreinigung des Rohproduktes erfolgt anschließend mittels präparativer HPLC (Reprosil C18, Wasser-Acetonitril-Gradient).

### Allgemeine Methode 8: Amidbildung

Eine Lösung aus 1.0 Äquivalenten des entsprechenden Amin-Hydrochlorids in *N*,*N*'-Dimethyl-formamid wird bei RT mit HATU (1.3 eq.) und *N*,*N*'-Diisopropylethylamin (3.2 eq.) versetzt. Anschließend werden 1.5 Äquivalenten der entsprechenden Carbonsäure zugegeben. Das Reaktionsgemisch wird 16 h bei RT gerührt und das Reaktionsgemisch anschließend mittels präparativer HPLC aufgereinigt.

### Beispiel 1

### 1-(Cyclopropylcarbonyl)-5-(4-ethylphenyl)-N-phenylpiperidin-3-carboxamid [racemisches cis-Isomer]

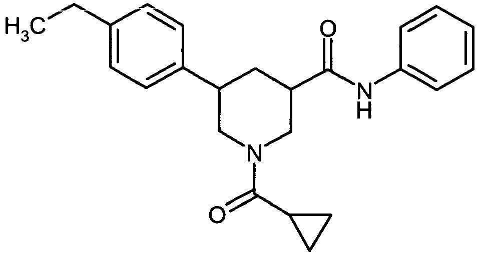

Nach der Allgemeinen Methode 1 wurden 68 mg (0.20 mmol) 5-(4-Ethylphenyl)-*N-*phenylpiperidin-3-carboxamid (Beispiel 17A) und 19 mg (0.22 mmol, 1.1 eq.) Cyclopropancarbonsäure umgesetzt. Ausbeute: 38 mg (50% d. Th.)

HPLC (Methode 1A): Rₜ = 4.81 min; MS (ESIpos): m/z = 377 [M+H]⁺;

¹H-NMR (400 MHz, DMSO-d₆): δ = 10.04 (s, 1H), 7.59 (d, 2H), 7.33-7.12 (m, 6H), 7.04 (t, 1H), 4.61 (br d, 0.5H), 4.52-4.41 (m, 1H), 4.28 (br d, 0.5H), 3.28-3.13 (m, 2H), 2.80-2.69 (m, 1H), 2.65-2.53 (m, 3H), 2.20-1.89 (m, 3H), 1.17 (t, 3H), 0.88-0.65 (m, 4H).

### Beispiel 2

### 1-(2,2-Dimethylpropanoyl)-5-(4-ethylphenyl)-N-phenylpiperidin-3-carbonsäureamid [racemisches cis-Isomer]

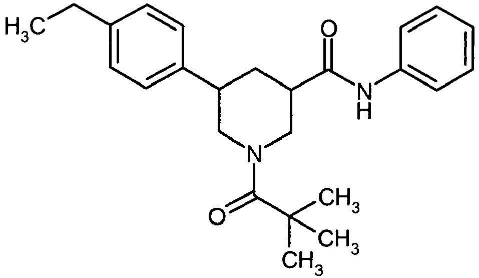

Nach der Allgemeinen Methode 1 wurden 68 mg (0.20 mmol) 5-(4-Ethylphenyl)-*N-*phenylpiperidin-3-carboxamid (Beispiel 17A) und 22 mg (0.22 mmol, 1.1 eq.) Pivalinsäure umgesetzt. Ausbeute: 37 mg (34% d. Th.)

HPLC (Methode 2A): Rₜ = 5.08 min; MS (ESIpos): m/z = 393 [M+H]⁺;

¹H-NMR (400 MHz, DMSO-d₆): δ = 10.03 (s, 1H), 7.59 (d, 2H), 7.29 (t, 2H), 7.23 (d, 2H), 7.18 (d, 2H), 7.04 (t, 1H), 4.51 (br d, 1H), 4.25 (br d, 1H), 3.03-2.88 (m, 2H), 2.71-2.58 (m, 2H), 2.58 (q, 2H), 2.13 (br d, 1H), 1.97 (q, 1H), 1.22 (s, 9H), 1.17 (t, 3H).

### Beispiel 3

### 1-(2,2-Dimethylpropanoyl)-5-(4-ethylphenyl)-N-phenylpiperidin-3-carbonsäureamid [enantiomerenreines cis-Isomer]

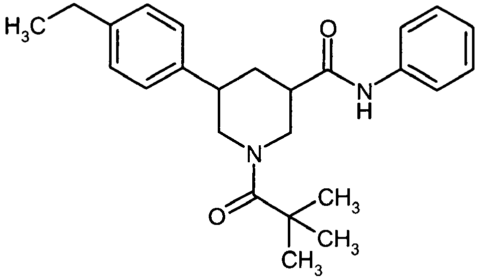

Die Enantiomerentrennung von 13 mg des Racemates aus Beispiel 2 nach Methode 1D ergab 5.9 mg der Verbindung aus Beispiel 3 (Enantiomer 1) und 5.3 mg der Verbindung aus Beispiel 4 (Enantiomer 2).

LC-MS (Methode 1E): Rₜ = 3.17 min; MS (ESIpos): m/z = 393 [M+H]⁺.

### Beispiel 4

### 1-(2,2-Dimethylpropanoyl)-5-(4-ethylphenyl)-N-phenylpiperidin-3-carbonsäureamid [enantiomerenreines cis-Isomer]

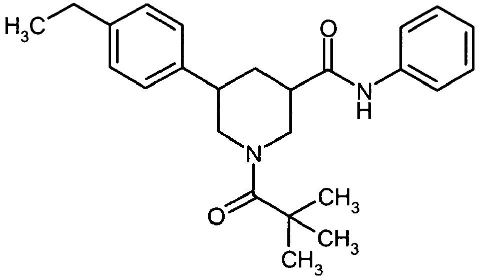

Die Enantiomerentrennung von 13 mg des Racemates aus Beispiel 2 nach Methode 1D ergab 5.9 mg der Verbindung aus Beispiel 3 (Enantiomer 1) und 5.3 mg der Verbindung aus Beispiel 4 (Enantiomer 2).

LC-MS (Methode 1E): Rₜ = 4.65 min; MS (ESIpos): m/z = 393 [M+H]⁺.

### Beispiel 5

### 1-(Cyclopropylacetyl)-5-(4-ethylphenyl)-N-phenylpiperidin-3-carbonsäureamid [racemisches cis-Isomer]

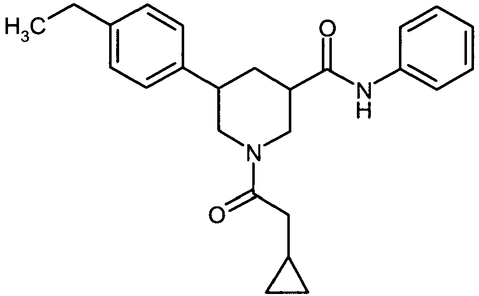

Nach der Allgemeinen Methode 1 wurden 68 mg (0.20 mmol) 5-(4-Ethylphenyl)-*N-*phenylpiperidin-3-carboxamid (Beispiel 17A) und 22 mg (0.22 mmol, 1.1 eq.) Cyclopropylessigsäure umgesetzt. Ausbeute: 65 mg (79% d. Th.)

HPLC (Methode 1 A): Rₜ = 4.83 min; MS (ESIpos): m/z = 391 [M+H]⁺;

¹H-NMR (500 MHz, DMSO-d₆): δ = 10.02 (s, 0.5H), 9.95 (s, 0.5H), 7.59 (d, 2H), 7.29 (t, 2H), 7.25 (d, 1H), 7.22 (d, 1H), 7.17 (d, 2H), 7.04 (t, 1H), 4.65 (br d, 0.5H), 4.49 (br d, 0.5H), 4.03 (br d, 0.5H), 3.84 (br d, 0.5H), 3.19 (t, 0.5H), 3.10 (t, 0.5H), 2.75-2.53 (m, 3H), 2.58 (q, 2H), 2.40-2.35 (m, 1H), 2.36 (dd, 0.5H), 2.28 (dd, 0.5H), 2.18-2.05 (m, 1H), 2.00-1.86 (m, 1H), 1.17 (t, 3H), 1.03-0.93 (m, 1H), 0.51-0.43 (m, 2H), 0.17-0.09 (m, 2H).

### Beispiel 6

### 1-(Cyclopropylacetyl)-5-(4-ethylphenyl)-N-phenylpiperidin-3-carbonsäureamid [enantiomerenreines cis-Isomer]

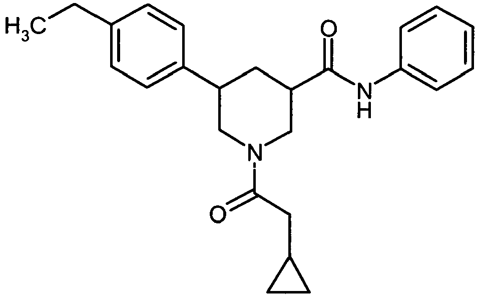

Enantiomerentrennung von 50 mg des Racemates aus Beispiel 5 nach Methode 2D ergab 18 mg der Verbindung aus Beispiel 6 (Enantiomer 1) und 18 mg der Verbindung aus Beispiel 7 (Enantiomer 2).

LC-MS (Methode 2E): Rₜ = 6.40 min; MS (ESIpos): m/z = 391 [M+H]⁺.

### Beispiel 7

### 5-(4-Ethylphenyl)-1-(3-fluor-2,2-dimethylpropanoyl)-N-phenylpiperidin-3-carboxamid [racemisches cis-Isomer]

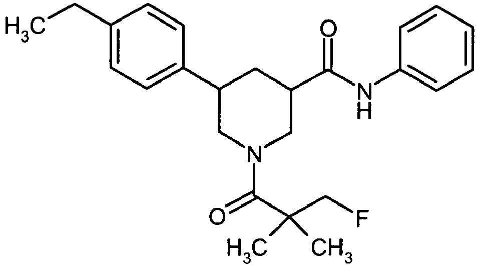

Nach der Allgemeinen Methode 2 wurden 62 mg (0.20 mmol) 5-(4-Ethylphenyl)-*N-*phenylpiperidin-3-carboxamid (Beispiel 17A) und 42 mg (0.3 mmol, 1.5 eq.) 3-Fluor-2,2-dimethylpropanoylchlorid umgesetzt. Ausbeute: 68 mg (83% d. Th.)

HPLC (Methode 1A): Rₜ = 4.97 min; MS (ESIpos): m/z = 411 [M+H]⁺;

¹H-NMR (400 MHz, DMSO-d₆): δ = 10.03 (s, 1H), 7.60 (d, 2H), 7.29 (t, 2H), 7.24 (d, 2H), 7.19 (d, 2H), 7.04 (t, 1H), 4.54-4.43 (m, 2H), 4.40-4.31 (m, 1H), 4.23 (br d, 1H), 3.06-2.90 (m, 2H), 2.73-2.59 (m, 2H), 2.58 (q, 2H), 2.14 (br d, 1H), 1.98 (q, 1H), 1.28 (d, 3H), 1.26 (d, 3H), 1.17 (t, 3H).

### Beispiel 8

### 5-(4-Ethylphenyl)-1-(3-methoxypropanoyl)-N-phenylpiperidin-3-carboxamid [racemisches cis-Isomer]

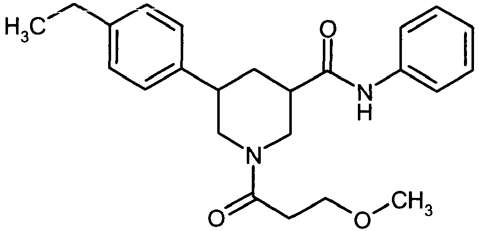

Nach der Allgemeinen Methode 2 wurden 62 mg (0.18 mmol) 5-(4-Ethylphenyl)-*N-*phenylpiperidin-3-carboxamid (Beispiel 17A) und 33 mg (0.27 mmol, 1.5 eq.) Methoxypropionsäurechlorid umgesetzt. Ausbeute: 65 mg (91% d. Th.)

HPLC (Methode 1A): Rₜ = 4.64 min; MS (ESIpos): m/z = 395 [M+H]⁺;

¹H-NMR (400 MHz, DMSO-d₆): δ = 10.04 (s, 0.5H), 10.00 (s, 0.5H), 7.59 (d, 2H), 7.35-7.12 (m, 6H), 7.04 (t, 1H), 4.64 (br d, 0.5H), 4.47 (br d, 0.5H), 4.10 (br d, 0.5H), 3.89 (br d, 0.5H), 3.65-3.50 (m, 2H), 3.27 (s, 3H), 3.18 (t, 0.5H), 3.09 (t, 0.5H), 2.80-2.60 (m, 3H), 2.60-2.50 (m, 3H), 2.19-2.02 (m, 1H), 2.01-1.85 (m, 1H), 1.17 (t, 3H).

### Beispiel 9

### 5-(4-Ethylphenyl)-N-phenyl-1-(tetrahydro-2H-pyran-4-ylcarbonyl)piperidin-3-carboxamid [racemisches cis-Isomer]

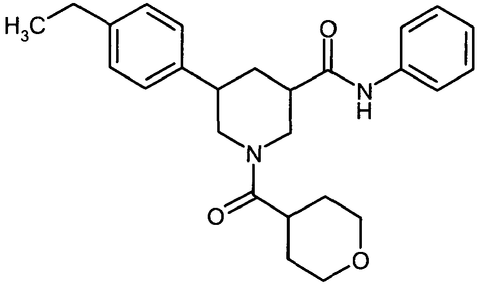

Nach der Allgemeinen Methode 2 wurden 62 mg (0.18 mmol) 5-(4-Ethylphenyl)-*N-*phenylpiperidin-3-carboxamid (Beispiel 17A) und 41 mg (0.27 mmol, 1.5 eq.) Tetrahydro-2*H-*pyran-4-carbonylchlorid umgesetzt. Ausbeute: 67 mg (88% d. Th.)

HPLC (Methode 1A): Rₜ = 4.64 min; MS (ESIpos): m/z = 421 [M+H]⁺;

¹H-NMR (400 MHz, DMSO-d₆): δ = 10.05 (s, 0.5H), 10.01 (s, 0.5H), 7.65-7.55 (m, 2H), 7.32-7.23 (m, 3H), 7.23-7.13 (m, 3H), 7.04 (t, 1H), 4.66 (br d, 0.5H), 4.49 (br d, 0.5H), 4.20 (br d, 0.5H), 3.99 (br d, 0.5H), 3.91-3.77 (m, 2H), 3.49-3.33 (m, 2H), 3.24 (t, 0.5H), 3.16 (t, 0.5H), 3.10-2.92 (m, 1H), 2.75-2.53 (m, 5H), 2.20-2.03 (m, 1H), 2.01-1.83 (m, 1H), 1.75-1.45 (m, 4H), 1.17 (t, 3H).

### Beispiel 10

### 1-[(1-Aminocyclopropyl)carbonyl]-5-(4-ethylphenyl)-N-phenylpiperidin-3-carboxamid [racemisches cis-Isomer]

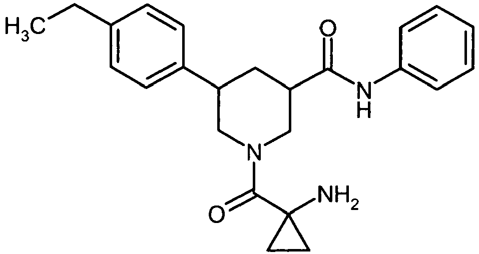

### Stufe a): (1-{[3-(4-Ethylphenyl)-5-(phenylcarbamoyl)piperidin-1-yl]carbonyl}cyclopropyl)-tert.-butylcarbamat [racemisches cis-Isomer]

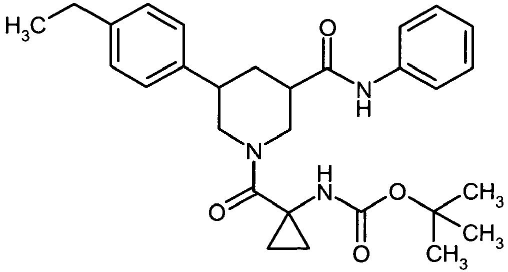

Nach der Allgemeinen Methode 1 wurden 169 mg (0.50 mmol) 5-(4-Ethylphenyl)-*N-*phenylpiperidin-3-carboxamid (Beispiel 17A) und 111 mg (0.55 mmol, 1.1 eq.) 1-[(*tert*.-Butoxycarbonyl)amino]cyclopropancarbonsäure umgesetzt. Ausbeute: 119 mg (48% d. Th.)

HPLC (Methode 2A): Rₜ = 4.90 min; MS (ESIpos): m/z = 492 [M+H]⁺.

### Stufe b): 1-[(1-Aminocyclopropyl)carbonyl]-5-(4-ethylphenyl)-N-phenylpiperidin-3-carboxamid [racemisches cis-Isomer]

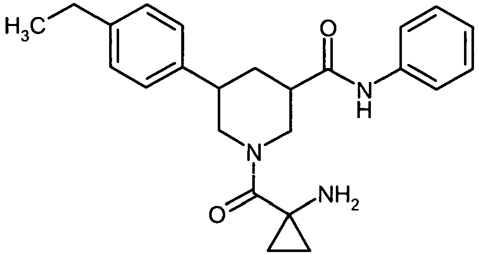

Eine Lösung von 81 mg (0.16 mmol) (1-{[3-(4-Ethylphenyl)-5-(phenylcarbamoyl)piperidin-1-yl]carbonyl}cyclopropyl)-*tert*.-butylcarbamat in 2 ml Dichlormethan wurde unter Argon bei RT mit 190 µl (2.5 mmol, 15 eq.) Trifluoressigsäure versetzt. Das Reaktionsgemisch wurde 20 h bei RT gerührt, anschließend im Vakuum eingeengt und mehrmals mit Toluol coevaporiert. Das Rohprodukt wurde mittels präparativer HPLC (Reprosil C18, Wasser-Acetonitril-Gradient) aufgereinigt. Ausbeute: 29 mg (45% d. Th.)

HPLC (Methode 1A): Rₜ = 4.20 min; MS (ESIpos): m/z = 392 [M+H]⁺;

¹H-NMR (500 MHz, DMSO-d₆): δ = 9.99 (s, 1H), 7.60 (d, 2H), 7.29 (t, 2H), 7.24 (d, 2H), 7.18 (d, 2H), 7.04 (t, 1H), 4.54 (br d, 1H), 4.42 (br d, 1H), 3.09-2.79 (m, 2H), 2.80-2.61 (m, 2H), 2.57 (q, 2H), 2.33-2.20 (m, 2H), 2.13 (br d, 1H), 1.94 (q, 1H), 1.17 (t, 3H), 0.94-0.82 (m, 2H), 0.72-0.61 (m, 2H).

### Beispiel 11

### 5-(4-Ethylphenyl)-N-phenyl-1-(tetrahydrofuran-2-ylcarbonyl)piperidin-3-carboxamid [racemisches cis-Isomer]

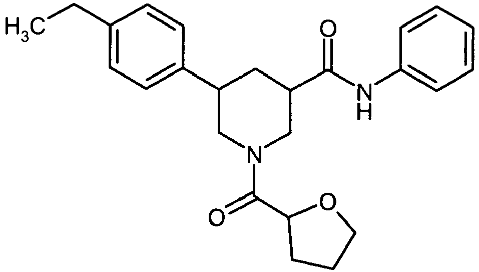

Nach der Allgemeinen Methode 1 wurden 68 mg (0.20 mmol) 5-(4-Ethylphenyl)-*N-*phenylpiperidin-3-carboxamid (Beispiel 17A) und 26 mg (0.22 mmol, 1.1 eq.) Tetrahydrofuran-2-carbonsäure umgesetzt. Ausbeute: 67 mg (81% d. Th.)

HPLC (Methode 1A): Rₜ = 4.66 min; MS (ESIpos): m/z = 407 [M+H]⁺;

¹H-NMR (400 MHz, DMSO-d₆): δ = 10.06-9.06 (m, 1H), 7.59 (d, 2H), 7.29 (t, 2H), 7.26-7.20 (m, 2H), 7.20-7.13 (m, 2H), 7.04 (t, 1H), 4.85-4.77 (m, 0.5H), 4.77-4.69 (m, 0.5H), 4.62-4.54 (m, 0.5H), 4.42 (br d, 0.5H), 4.29-4.12 (m, 0.5H), 4.09-3.96 (m, 0.5H), 3.86-3.69 (m, 2H), 3.21 (t, 0.5H), 3.07 (t, 0.5H), 2.82-2.53 (m, 5H), 2.20-1.78 (m, 6H), 1.17 (t, 3H).

Analog wurden folgende Beispiele [racemische cis-Isomere] dargestellt:

| **Beispiel** | **Struktur** | **LC-MS Methode** | **Rₜ [min]** | **MS (ESIpos) m/z** |
|---|---|---|---|---|
| **12** | 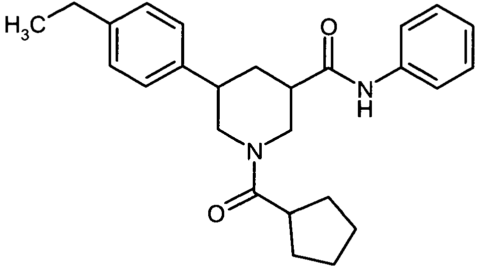 | 1B | 2.98 | 405 |
| **13** | 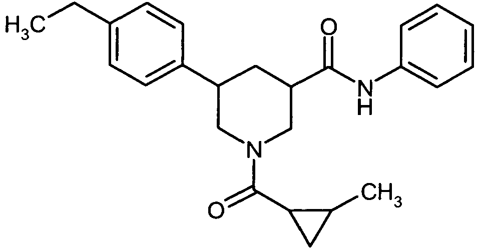 | 2B | 1.41 | 391 |
| **14** | 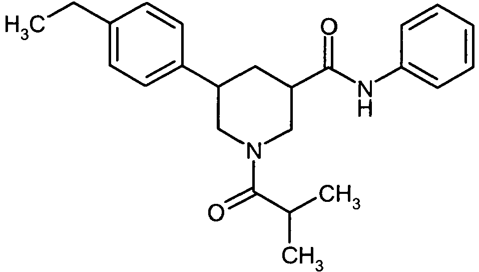 | 2B | 1.36 | 379 |
| **15** | 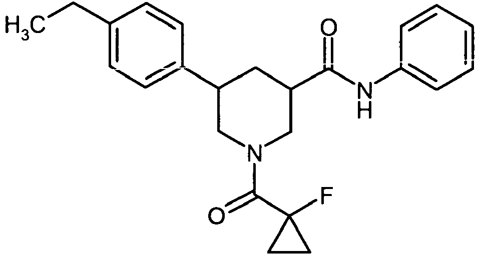 | 1B | 2.86 | 395 |
| **16** | 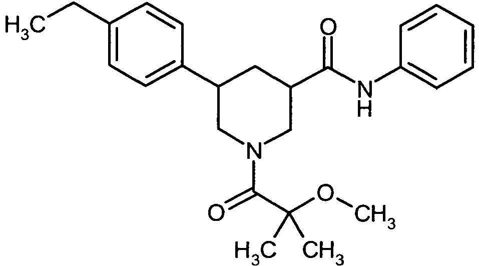 | 1B | 2.87 | 409 |
| **17** | 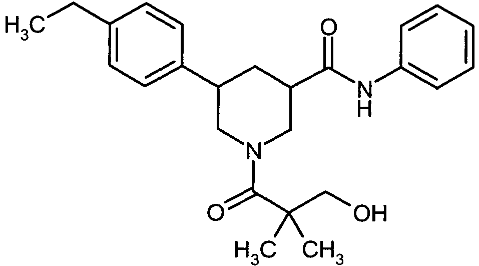 | 1B | 2.09 | 409 |
| **18** | 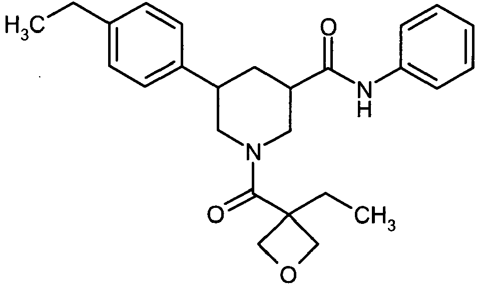 | 3B | 2.13 | 421 |
| **19** | 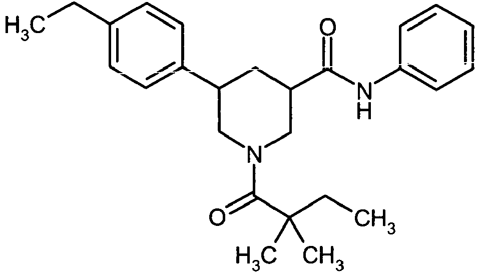 | 2B | 1.49 | 407 |
| **20** | 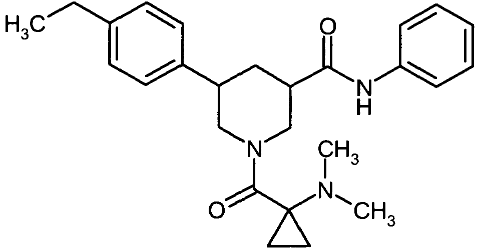 | 5B | 1.93 | 420 |
| **21** | 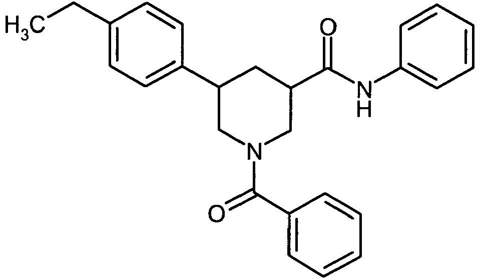 | 1B | 2.86 | 413 |
| **22** | 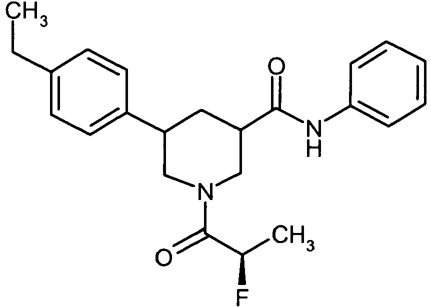 | 9B | 2.27 | 383 |
| **23** | 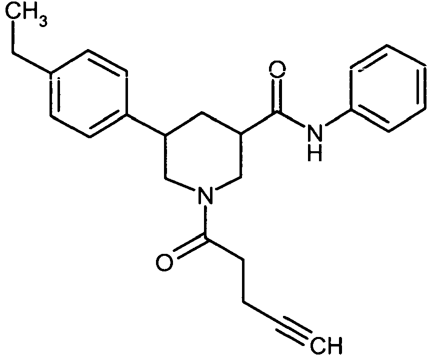 | 9B | 2.29 | 389 |
| **24** | 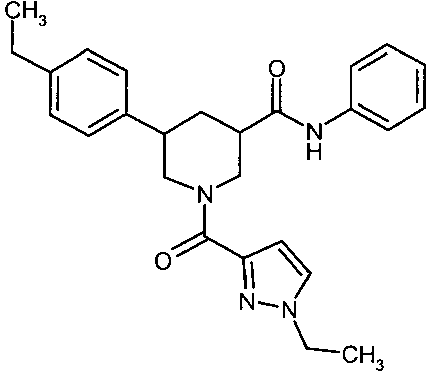 | 9B | 2.29 | 431 |
| **25** | 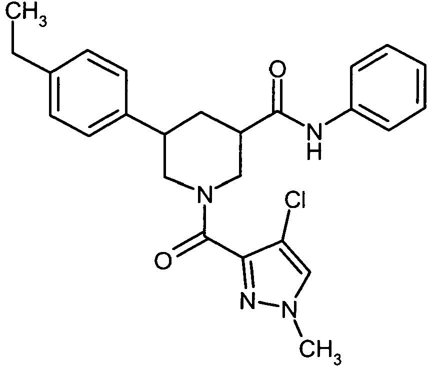 | 9B | 2.31 | 451 |
| **26** | 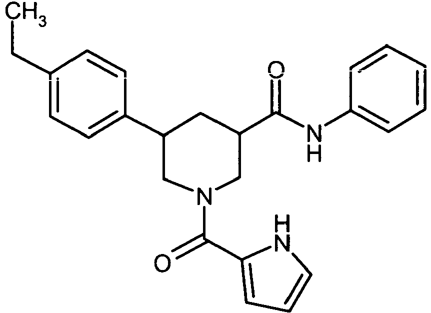 | 9B | 2.31 | 402 |
| **27** | 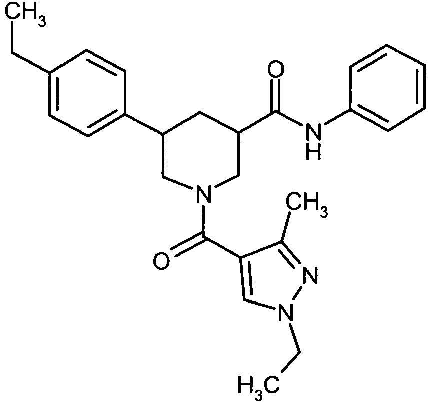 | 9B | 2.26 | 445 |
| **28** | 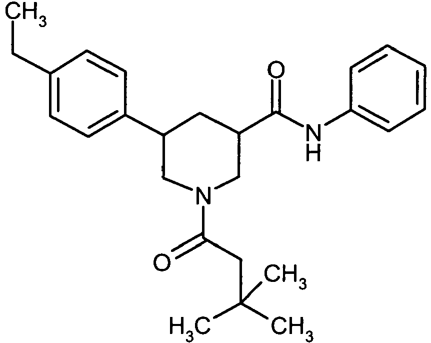 | 9B | 2.42 | 407 |
| **29** | 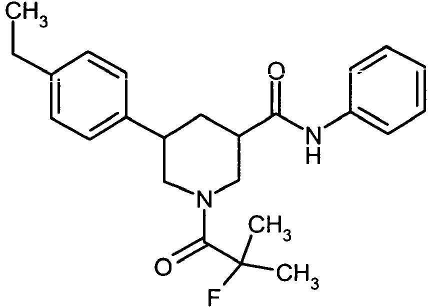 | 9B | 2.36 | 397 |
| **30** | 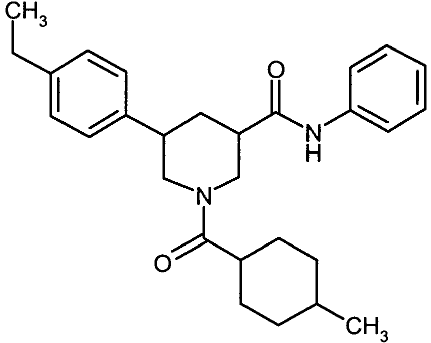 | 9B | 2.48 | 433 |
| **31** | 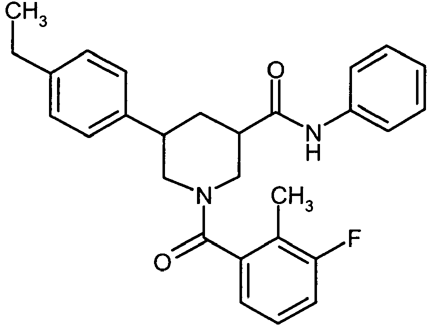 | 9B | 2.4 | 445 |
| **32** | 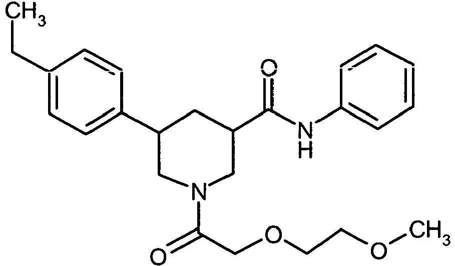 | 9B | 2.23 | 425 |
| **33** | 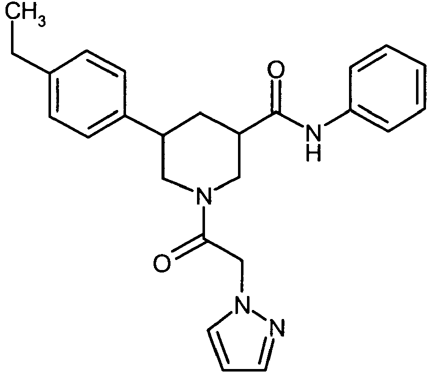 | 9B | 2.2 | 417 |
| **34** | 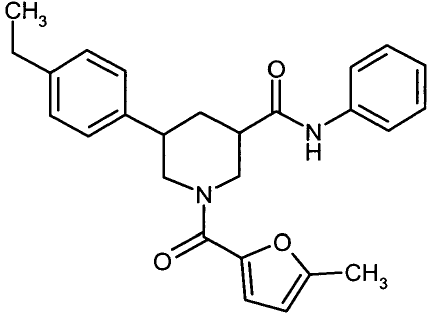 | 9B | 2.36 | 417 |
| **35** | 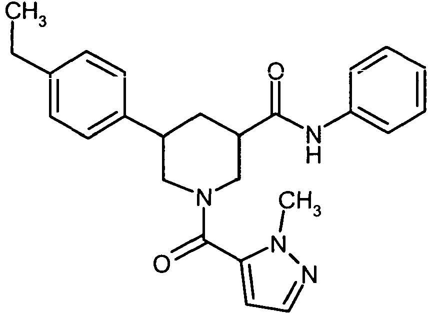 | 9B | 2.26 | 417 |
| **36** | 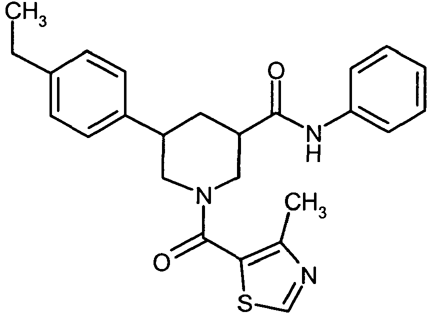 | 9B | 2.25 | 434 |
| **37** | 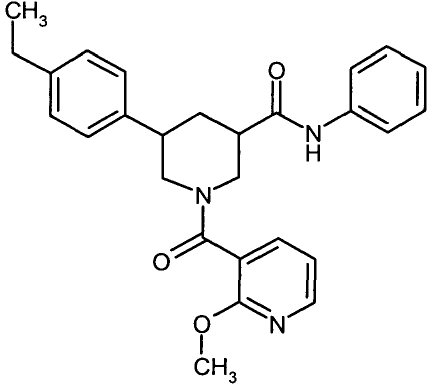 | 9B | 2.31 | 444 |

### Beispiel 38

### N¹-Cyclopentyl-5-(4-ethylphenyl)-N³-phenylpiperidin-1,3-dicarboxamid [racemisches cis-Isomer]

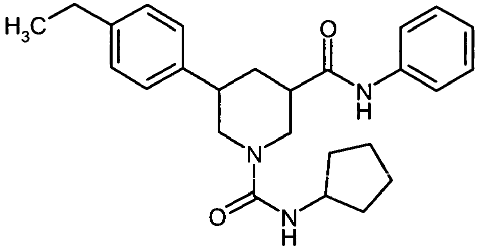

Nach der Allgemeinen Methode 4 wurden 62 mg (0.20 mmol) 5-(4-Ethylphenyl)-*N-*phenylpiperidin-3-carboxamid (Beispiel 17A) und 22 mg (0.20 mmol, 1.0 eq.) Cyclopentylisocyanat umgesetzt. Ausbeute: 70 mg (83% d. Th.)

HPLC (Methode 1A): Rₜ = 4.94 min; MS (ESIpos): m/z = 420 [M+H]⁺;

¹H-NMR (400 MHz, DMSO-d₆): δ = 10.00 (s, 1H), 7.59 (d, 2H), 7.29 (t, 2H), 7.21 (d, 2H), 7.16 (d, 2H), 7.03 (t, 1H), 6.36 (d, 1H), 4.22 (br d, 1H), 4.08 (br d, 1H), 3.99-3.88 (m, 1H), 2.80 (t, 1H), 2.67 (q, 1H), 2.64-2.58 (m, 2H), 2.57 (q, 2H), 2.07 (br d, 1H), 1.88-1.72 (m, 3H), 1.68-1.55 (m, 2H), 1.52-1.33 (m, 4H), 1.16 (t, 3H).

### Beispiel 39

### N¹-Cyclopropyl-5-(4-ethylphenyl)-N³-phenylpiperidin-1,3-dicarboxamid [racemisches cis-Isomer]

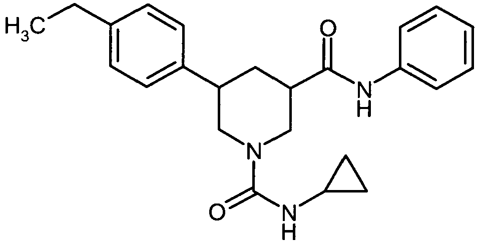

Nach der Allgemeinen Methode 4 wurden 62 mg (0.20 mmol) 5-(4-Ethylphenyl)-*N-*phenylpiperidin-3-carboxamid (Beispiel 17A) und 17 mg (0.20 mmol, 1.0 eq.) Cyclopropylisocyanat umgesetzt. Ausbeute: 63 mg (80% d. Th.)

HPLC (Methode 1A): Rₜ = 4.63 min; MS (ESIpos): m/z = 392 [M+H]⁺;

¹H-NMR (400 MHz, DMSO-d₆): δ = 10.00 (s, 1H), 7.59 (d, 2H), 7.29 (t, 2H), 7.21 (d, 2H), 7.16 (d, 2H), 7.03 (t, 1H), 6.68 (d, 1H), 4.16 (br d, 1H), 4.02 (br d, 1H), 2.81 (t, 1H), 2.69 (q, 1H), 2.64-2.52 (m, 3H), 2.57 (q, 2H), 2.07 (br d, 1H), 1.83 (q, 1H), 1.16 (t, 3H), 0.57-0.52 (m, 2H), 0.42-0.35 (m, 2H).

### Beispiel 40

### N¹-tert.-Butyl-5-(4-ethylphenyl)-N³-phenylpiperidin-1,3-dicarboxamid [racemisches cis-Isomer]

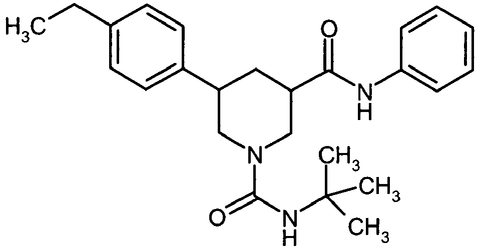

Nach der Allgemeinen Methode 4 wurden 62 mg (0.20 mmol) 5-(4-Ethylphenyl)-*N-*phenylpiperidin-3-carboxamid (Beispiel 17A) und 20 mg (0.20 mmol, 1.0 eq.) *tert*.-Butylisocyanat umgesetzt. Ausbeute: 34 mg (42% d. Th.)

HPLC (Methode 1A): Rₜ = 4.95 min; MS (ESIpos): m/z = 408 [M+H]⁺;

¹H-NMR (400 MHz, DMSO-d₆): δ = 10.00 (s, 1H), 7.60 (d, 2H), 7.29 (t, 2H), 7.21 (d, 2H), 7.16 (d, 2H), 7.03 (t, 1H), 5.93 (s, 1H), 4.19 (br d, 1H), 4.05 (br d, 1H), 2.77 (t, 1H), 2.70-2.53 (m, 3H), 2.57 (q, 2H), 2.06 (br d, 1H), 1.81 (q, 1H), 1.27 (s, 9H), 1.16 (t, 3H).

### Beispiel 41

### 5-(4-Ethylphenyl)-N-phenyl-1-(pyrrolidin-1-ylcarbonyl)piperidin-3-carboxamid [racemisches cis-Isomer]

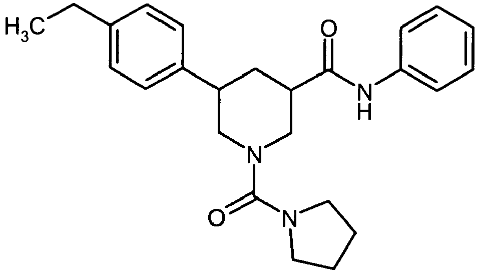

Nach der Allgemeinen Methode 1 wurden 33 mg (0.10 mmol) 5-(4-Ethylphenyl)-1-(pyrrolidin-1-ylcarbonyl)piperidin-3-carbonsäure (Beispiel 11A) und 10 mg (0.11 mmol, 1.1 eq.) Anilin umgesetzt. Ausbeute: 26 mg (64% d. Th.)

HPLC (Methode 1A): Rₜ = 4.89 min; MS (ESIpos): m/z = 406 [M+H]⁺;

¹H-NMR (400 MHz, DMSO-d₆): δ = 10.00 (s, 1H), 7.59 (d, 2H), 7.29 (t, 2H), 7.21 (d, 2H), 7.16 (d, 2H), 7.03 (t, 1H), 3.87 (br d, 1H), 3.68 (br d, 1H), 3.31-3.22 (m, 4H), 2.88 (t, 1H), 2.81-2.65 (m, 3H), 2.57 (q, 2H), 2.10 (br d, 1H), 1.87 (q, 1H), 1.81-1.68 (m, 4H), 1.16 (t, 3H).

### Beispiel 42

### 5-(4-Ethylphenyl)-1-(morpholin-4-ylcarbonyl)-N-phenylpiperidin-3-carboxamid [racemisches cis-Isomer]

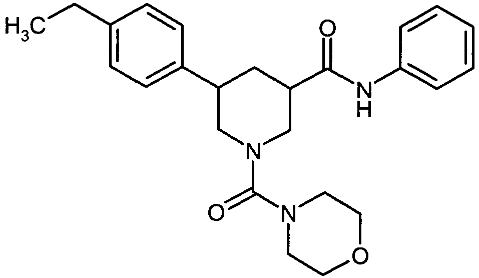

Nach der Allgemeinen Methode 3 wurden 250 mg (0.74 mmol) 5-(4-Ethylphenyl)-*N-*phenylpiperidin-3-carboxamid (Beispiel 17A) und 143 mg (0.96 mmol, 1.3 eq.) Morpholin-4-carbonylchlorid umgesetzt. Ausbeute: 276 mg (88% d. Th.)

HPLC (Methode 1A): Rₜ = 4.55 min; MS (ESIpos): m/z = 422 [M+H]⁺;

¹H-NMR (400 MHz, DMSO-d₆): δ = 10.00 (s, 1H), 7.59 (d, 2H), 7.29 (t, 2H), 7.21 (d, 2H), 7.16 (d, 2H), 7.03 (t, 1H), 3.80 (br d, 1H), 3.61 (br d, 1H), 3.60-3.51 (m, 4H), 3.22-3.12 (m, 4H), 2.94 (t, 1H), 2.84 (q, 1H), 2.80-2.66 (m, 2H), 2.57 (q, 2H), 2.10 (br d, 1H), 1.87 (q, 1H), 1.17 (t, 3H).

### Beispiel 43

### 5-(4-Ethylphenyl)-1-(morpholin-4-ylcarbonyl)-N-phenylpiperidin-3-carboxamid [enantiomerenreines cis-Isomer]

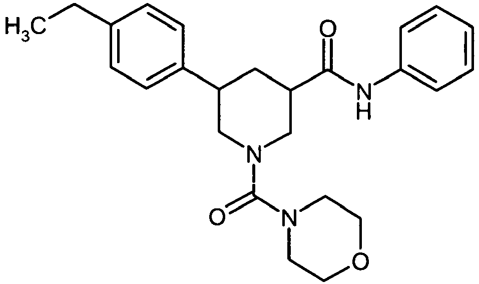

Enantiomerentrennung von 280 mg des Racemates aus Beispiel 42 nach Methode 3D ergab 131 mg der Verbindung aus Beispiel 43 (Enantiomer 1) und 145 mg der Verbindung aus Beispiel 44 (Enantiomer 2).

LC-MS (Methode 2E): Rₜ = 4.67 min; MS (ESIpos): m/z = 422 [M+H]⁺.

### Beispiel 44

### 5-(4-Ethylphenyl)-1-(morpholin-4-ylcarbonyl)-N-phenylpiperidin-3-carboxamid [enantiomerenreines cis-Isomer]

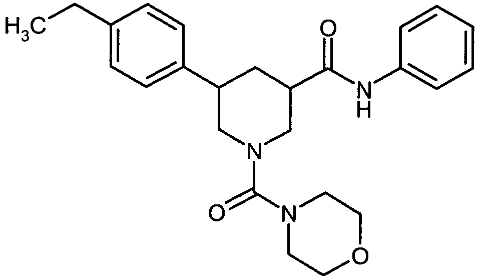

Enantiomerentrennung von 280 mg des Racemates aus Beispiel 42 nach Methode 3D ergab 131 mg der Verbindung aus Beispiel 43 (Enantiomer 1) und 145 mg der Verbindung aus Beispiel 44 (Enantiomer 2).

LC-MS (Methode 2E): Rₜ = 6.54 min; MS (ESIpos): m/z = 422 [M+H]⁺.

### Beispiel 45

### N¹-Ethyl-5-(4-ethylphenyl)-N¹-methyl-N³-phenylpiperidin-1,3-dicarboxamid [racemisches cis-Isomer]

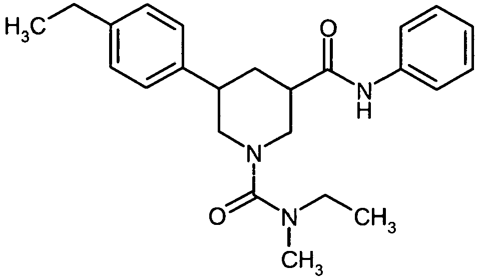

Nach der Allgemeinen Methode 3 wurden 250 mg (0.74 mmol) 5-(4-Ethylphenyl)-*N-*phenylpiperidin-3-carboxamid (Beispiel 17A) und 117 mg (0.96 mmol, 1.3 eq.) Ethyl(methyl)carbaminicchlorid umgesetzt. Ausbeute: 215 mg (74% d. Th.)

HPLC (Methode 1A): Rₜ = 4.74 min; MS (ESIpos): m/z = 394 [M+H]⁺;

¹H-NMR (400 MHz, DMSO-d₆): δ = 10.00 (s, 1H), 7.59 (d, 2H), 7.29 (t, 2H), 7.21 (d, 2H), 7.16 (d, 2H), 7.03 (t, 1H), 3.72 (br d, 1H), 3.53 (br d, 1H), 3.21-3.05 (m, 2H), 2.88 (t, 1H), 2.82-2.66 (m, 6H), 2.57 (q, 2H), 2.09 (br d, 1H), 1.86 (q, 1H), 1.16 (t, 3H), 1.06 (t, 3H).

### Beispiel 46

### 5-(4-Ethylphenyl)-1-[(4-methylpiperazin-1-yl)carbonyl]-N-phenylpiperidin-3-carboxamid [racemisches cis-Isomer]

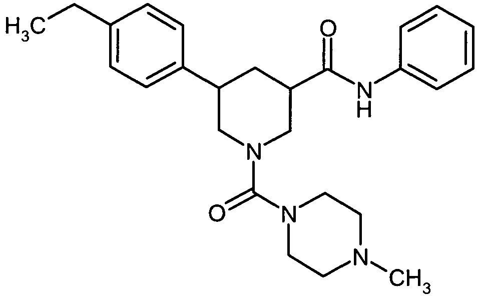

Nach der Allgemeinen Methode 3 wurden 250 mg (0.74 mmol) 5-(4-Ethylphenyl)-*N-*phenylpiperidin-3-carboxamid (Beispiel 17A) und 156 mg (0.96 mmol, 1.3 eq.) 4-Methylpiperazin-1-carbonylchlorid umgesetzt. Ausbeute: 213 mg (66% d. Th.)

HPLC (Methode 1A): Rₜ = 3.45 min; MS (ESIpos): m/z = 435 [M+H]⁺;

¹H-NMR (400 MHz, DMSO-d₆): δ = 10.00 (s, 1H), 7.59 (d, 2H), 7.29 (t, 2H), 7.21 (d, 2H), 7.16 (d, 2H), 7.03 (t, 1H), 3.77 (br d, 1H), 3.58 (br d, 1H), 3.23-3.10 (m, 4H), 2.92 (t, 1H), 2.81 (q, 1H), 2.79-2.66 (m, 2H), 2.57 (q, 2H), 2.09 (br d, 1H), 1.86 (q, 1H), 1.17 (t, 3H).

### Beispiel 47

### 5-(4-Ethylphenyl)-N-phenyl-1-(piperazin-1-ylcarbonyl)piperidin-3-carboxamid [racemisches cis-Isomer]

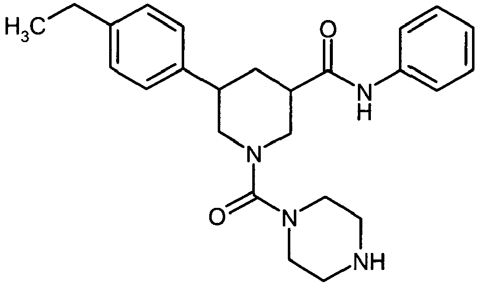

Eine Lösung aus 3.5 g (6.7 mmol) 4-{[3-(4-Ethylphenyl)-5-(phenylcarbamoyl)piperidin-1-yl]carbonyl}piperazin-1-carbonsäure-*tert*.-butylester (Beispiel 50) in 60 ml Dioxan wurde bei RT tropfenweise mit 13.4 ml (53.5 mmol, 8 eq.) einer 4-molaren Lösung von Chlorwasserstoff in Dioxan versetzt. Das Reaktionsgemisch wurde 20 h bei RT gerührt, anschließend im Vakuum eingeengt und zweimal mit Dioxan koevaporiert, wobei 3.37 g der Verbindung aus Beispiel 47 als Hydrochlorid erhalten wurden. Mittels präparativer HPLC (Reprosil C18, Wasser-Acetonitril-Gradient mit 0.1% Triethylamin) wurden 180 mg des Rohproduktes zur freien Base (136 mg) weiter aufgereinigt.

HPLC (Methode 1A): Rₜ = 4.18 min; MS (ESIpos): m/z = 421 [M+H]⁺;

¹H-NMR (400 MHz, DMSO-d₆): δ = 10.00 (s, 1H), 7.60 (d, 2H), 7.29 (t, 2H), 7.21 (d, 2H), 7.17 (d, 2H), 7.03 (t, 1H), 3.77 (br d, 1H), 3.57 (br d, 1H), 3.16-3.03 (m, 4H), 2.90 (t, 1H), 2.85-2.69 (m, 3H), 2.69-2.60 (m, 4H), 2.57 (q, 2H), 2.40-2.30 (m, 1H), 2.10 (br d, 1H), 1.86 (q, 1H), 1.17 (t, 3H).

### Beispiel 48

### 1-[(4-Cyclopropylpiperazin-1-yl)carbonyl]-5-(4-ethylphenyl)-N-phenylpiperidin-3-carboxamid [racemisches cis-Isomer]

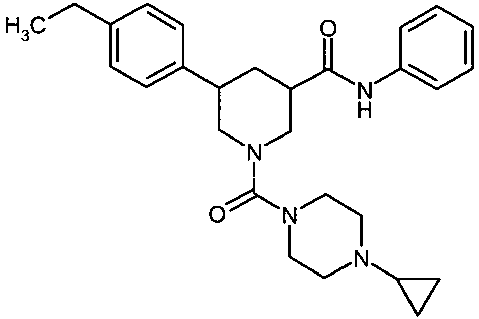

Eine Lösung von 94 mg (0.20 mmol) 5-(4-Ethylphenyl)-*N*-phenyl-1-(piperazin-1-ylcarbonyl)-piperidin-3-carboxamid, 241 µl (1.20 mmol, 6 eq.) [(1-Ethoxycyclopropyl)oxy]-(trimethyl)silan, 35 µl (0.62 mmol, 3.1 eq.) Essigsäure und 115 mg Molsieb (4Ǻ) in 1 ml Methanol wurde bei RT mit 898 µl einer 1-molaren Lösung von Natriumcyanoborhydrid in Tetrahydrofuran versetzt. Das Reaktionsgemisch wurde 5 h am Rückfluss gerührt, anschließend filtriert und mit 0.2-molarer Natriumhydroxid-Lösung auf pH 8 gestellt. Nach Zugabe von Wasser-Dichlormethan und Phasentrennung wurde die wässrige Phase zweimal mit Dichlormethan extrahiert. Die vereinigten, organischen Phasen wurden getrocknet (Natriumsulfat), filtriert und im Vakuum eingeengt. Das Rohprodukt wurde mittels präparativer HPLC (Reprosil C18, Wasser-Acetonitril-Gradient) aufgereinigt. Ausbeute: 60 mg (65% d. Th.)

HPLC (Methode 2A): Rₜ = 4.30 min; MS (ESIpos): m/z = 461 [M+H]⁺;

¹H-NMR (400 MHz, DMSO-d₆): δ = 10.01 (s, 1H), 7.59 (d, 2H), 7.29 (t, 2H), 7.21 (d, 2H), 7.16 (d, 2H), 7.03 (t, 1H), 3.77 (br d, 1H), 3.58 (br d, 1H), 3.19-3.07 (m, 4H), 2.92 (t, 1H), 2.81 (q, 1H), 2.78-2.65 (m, 2H), 2.57 (q, 2H), 2.09 (br d, 1H), 1.87 (q, 1H), 1.66-1.58 (m, 1H), 1.17 (t, 3H), 0.44-0.38 (m, 2H), 0.33-0.27 (m, 2H).

Analog wurde durch reduktive Aminierung folgendes Beispiel [racemische cis-Isomer] dargestellt:

| **Beispiel** | **Struktur** | **LC-MS Methode** | **Rₜ [min]** | **MS (ESIpos) m/z** |
|---|---|---|---|---|
| **49** | 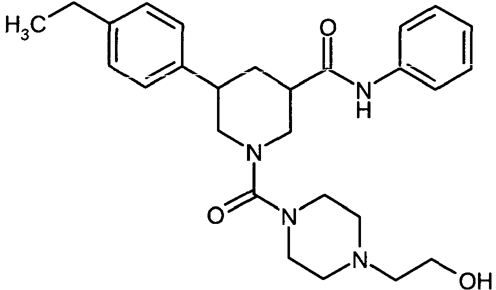 | 1B | 1.61 | 465 |

### Beispiel 50

### 4- {[3-(4-Ethylphenyl)-5-(phenylcarbamoyl)piperidin-1-yl]carbonyl}piperazin-1-carbonsäure-tert.-butylester [racemisches cis-Isomer]

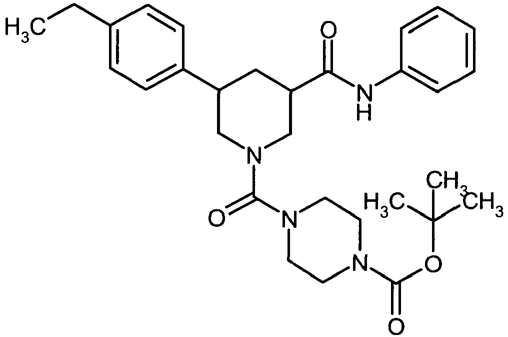

Eine Lösung aus 2.0 g (5.9 mmol) 5-(4-Ethylphenyl)-*N*-phenylpiperidin-3-carboxamid, 144 mg (1.2 mmol, 0.2 eq.) 4-Dimethylaminopyridin und 2.1 ml (14.6 mmol, 2.5 eq.) Triethylamin in 64 ml Tetrahydrofuran wurde unter Argon bei RT mit 1.9 g (7.7 mmol) 4-(Chlorcarbonyl)piperazin-1-carbonsäure-*tert*.-butylester versetzt. Das Reaktionsgemisch wurde 17 h bei RT gerührt und anschließend mit Wasser und Dichlormethan versetzt. Nach Phasentrennung wurde die wässrige Phase zweimal mit Dichlormethan extrahiert. Die vereinigten, organischen Phasen wurden getrocknet (Natriumsulfat), filtriert und im Vakuum eingeengt. Das Rohprodukt wurde mittels Flash-Chromatographie (Kieselgel 60, Dichlormethan[Methanol 300:1 → 100:1) gereinigt, wobei 3.5 g des Beispiels 50 erhalten wurden.

LC-MS (Methode 1B): Rₜ = 2.94 min; MS (ESIpos): m/z = 521 [M+H]⁺.

Nach der Allgemeinen Methode 3 wurden folgende Beispiele [racemische cis-Isomere] dargestellt:

| **Beispiel** | **Struktur** | **LC-MS Methode** | **Rₜ [min]** | **MS (ESIpos) m/z** |
|---|---|---|---|---|
| **51** | 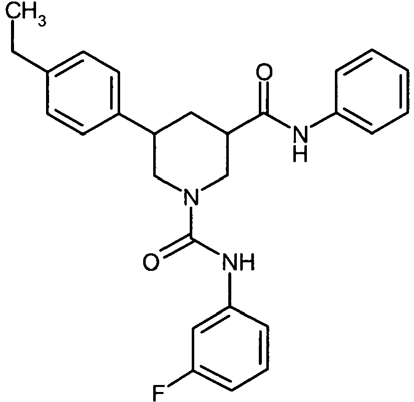 | 9B | 2.4 | 446 |
| **52** | 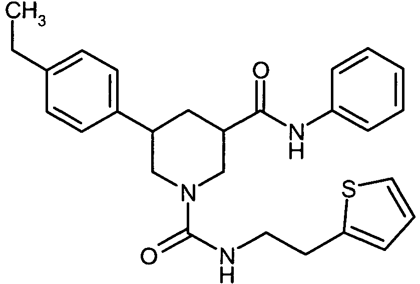 | 9B | 2.36 | 462 |
| **53** | 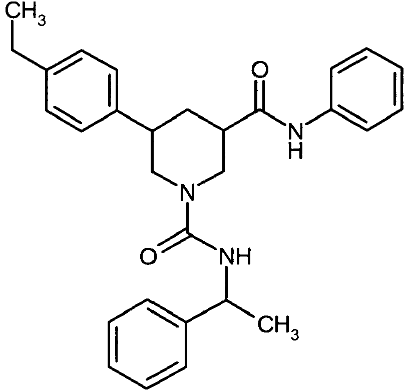 | 9B | 2.38 | 456 |
| **54** | 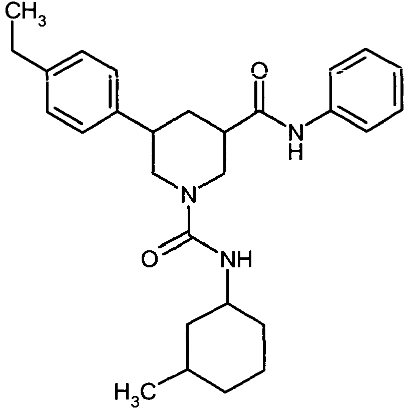 | 9B | 2.43 | 448 |
| **55** | 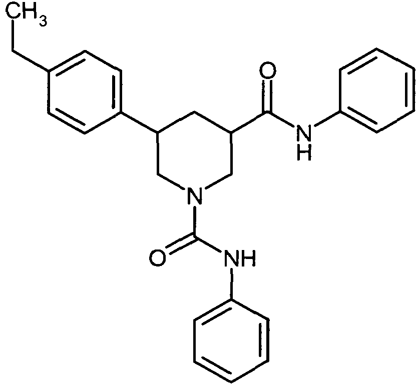 | 9B | 2.35 | 428 |
| **56** | 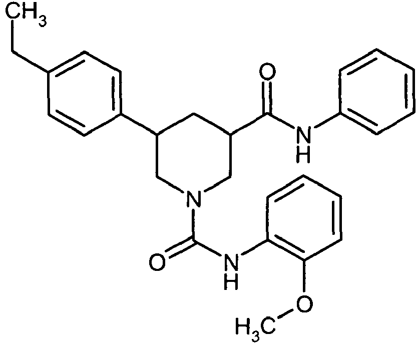 | 9B | 2.39 | 458 |
| **57** | 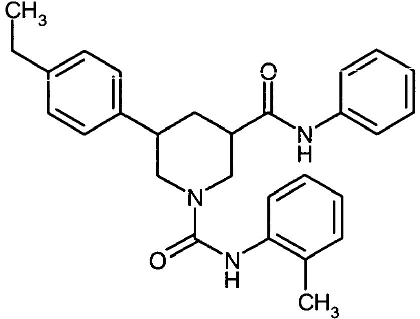 | 9B | 2.36 | 442 |
| **58** | 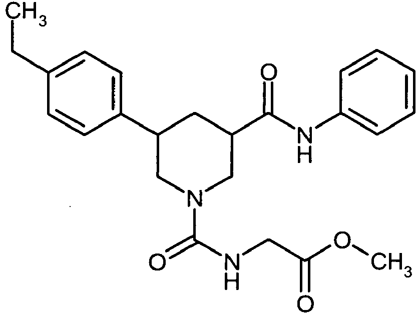 | 9B | 2.2 | 424 |
| **59** | 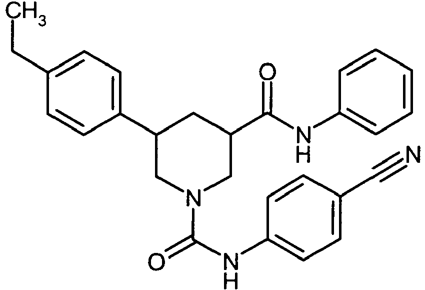 | 9B | 2.36 | 453 |
| **60** | 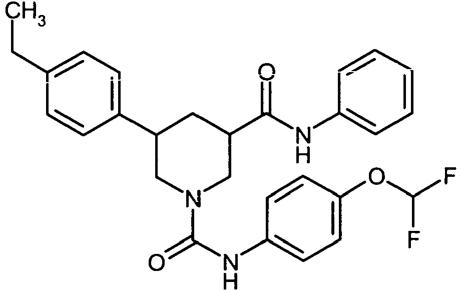 | 9B | 2.38 | 494 |
| **61** | 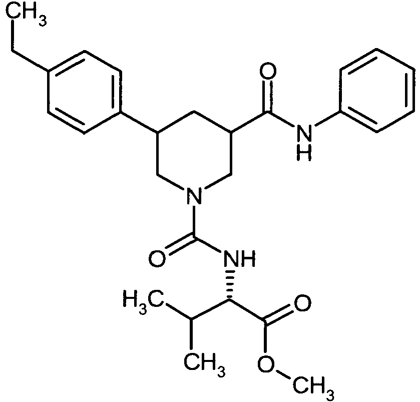 | 9B | 2.35 | 466 |
| **62** | 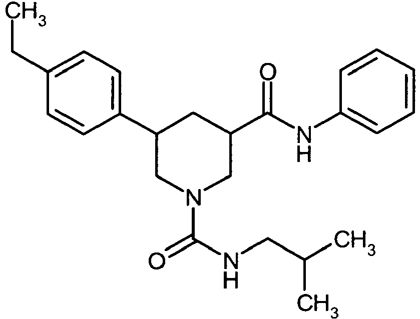 | 9B | 2.35 | 408 |
| **63** | 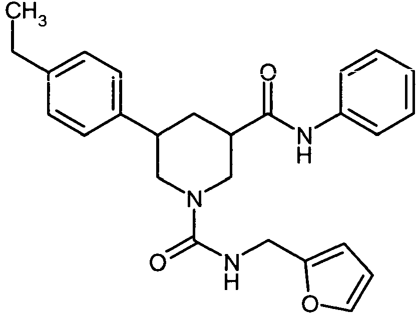 | 9B | 2.30 | 432 |
| **64** | 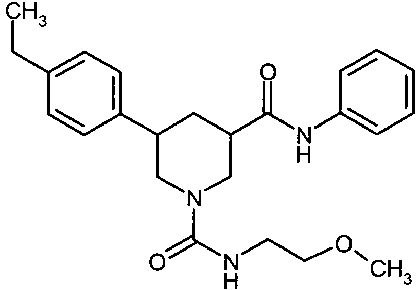 | 9B | 2.29 | 410 |
| **65** | 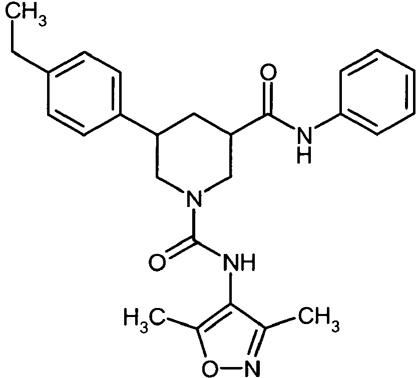 | 9B | 2.24 | 447 |
| **66** | 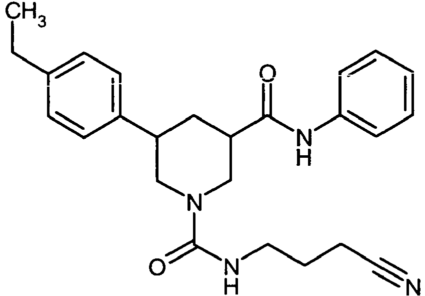 | 9B | 2.22 | 419 |
| **67** | 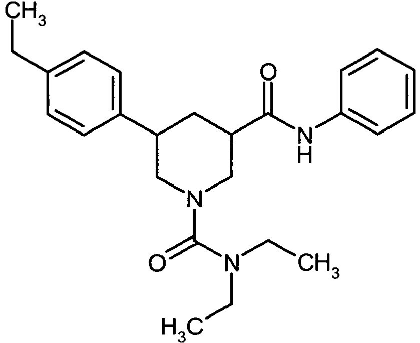 | 9B | 2.42 | 408 |
| **68** | 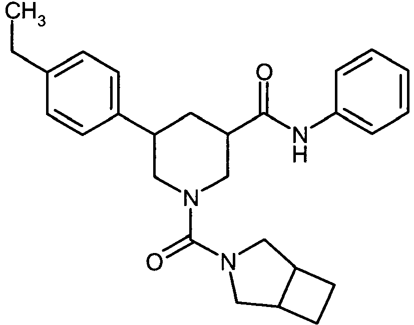 | 9B | 2.43 | 432 |

### Beispiel 69

### tert.-Butyl-4-({3-[(phenylcarbonyl)amino]-5-[4-(trifluormethoxy)phenyl]piperidin-1-yl}carbonyl)-piperazin-1-carboxylat [racemisches cis-Isomer]

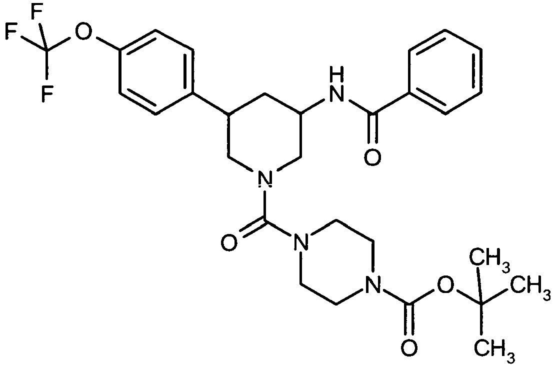

220 mg (0.55 mmol) *N*-{5-[4-(Trifluormethoxy)phenyl]piperidin-3-yl}benzamid wurden in 3 ml Dichlormethan bei 0°C vorgelegt und mit 273 mg (1.10 mmol) *tert*.-Butyl-4-(chlorcarbonyl)-piperazin-1-carboxylat und 116 µl (0.82 mmol) Triethylamin versetzt. Die Mischung wurde langsam über Nacht auf RT erwärmt. Es wurde Wasser zu dem Reaktionsgemisch gegeben und extrahiert. Die organische Phase wurde über Magnesiumsulfat getrocknet und im Vakuum eingeengt. Der Rückstand wurde anschließend mittels präparativer HPLC (Reprosil C18, Wasser-Acetonitril-Gradient) gereinigt. Ausbeute: 88 mg (28% d. Th.)

LC-MS (Methode 1B): Rₜ = 2.75 min; m/z = 577 [M+H]⁺.

### Beispiel 70

### 3-(4-Ethylphenyl)-5-(phenylcarbamoyl)piperidin-1-carbonsäure-2-methoxyethylester [racemisches cis-Isomer]

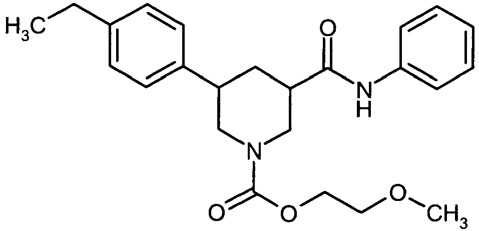

Nach der Allgemeinen Methode 5 wurden 68 mg (0.20 mmol) 5-(4-Ethylphenyl)-*N-*phenylpiperidin-3-carboxamid (Beispiel 17A) und 38 mg (0.22 mmol, 1.1 eq.) Chlorameisensäure-2-methoxyethylester umgesetzt. Ausbeute: 73 mg (89% d. Th.)

HPLC (Methode 2A): Rₜ = 4.67 min; MS (ESIpos): m/z = 411 [M+H]⁺;

¹H-NMR (400 MHz, DMSO-d₆): δ = 10.04 (s, 1H), 7.59 (d, 2H), 7.29 (t, 2H), 7.22 (d, 2H), 7.17 (d, 2H), 7.04 (t, 1H); 4.27-4.09 (m, 3H), 4.09-3.95 (m, 1H), 3.58-3.49 (m, 2H), 3.27 (s, 3H), 3.05-2.80 (m, 2H), 2.72-2.60 (m, 2H), 2.58 (q, 2H), 2.18-2.06 (m, 1H), 1.88 (q, 1H), 1.17 (t, 3H).

Analog wurden folgende Beispiele [racemische cis-Isomere] dargestellt:

| **Beispiel** | **Struktur** | **LC-MS Methode** | **Rₜ [min]** | **MS (ESIpos) m/z** |
|---|---|---|---|---|
| **71** | 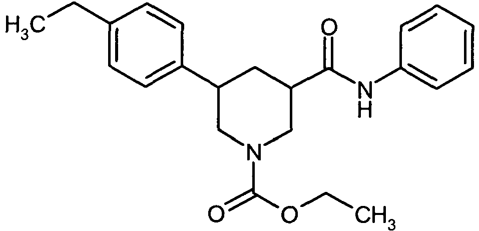 | 2B | 1.41 | 381 |
| **72** | 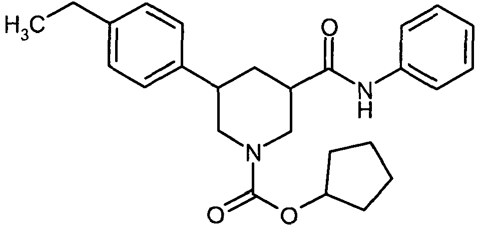 | 2B | 1.54 | 421 |

### Beispiel 73

### 1-(Cyclopentylcarbonyl)-5-(4-ethylphenyl)-N-(3-fluorphenyl)piperidin-3-carboxamid [racemisches cis-Isomer]

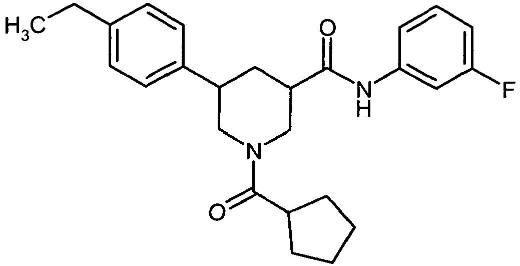

Nach der Allgemeinen Methode 1 wurden 66 mg (0.20 mmol) 1-(Cyclopentylcarbonyl)-5-(4-ethylphenyl)piperidin-3-carbonsäure (Beispiel 7A) und 24 mg (0.22 mmol, 1.1 eq.) 3-Fluoranilin umgesetzt. Ausbeute: 38 mg (45% d. Th.)

HPLC (Methode 1A): Rₜ = 5.31 min; MS (ESIpos): m/z = 423 [M+H]⁺;

¹H-NMR (500 MHz, DMSO-d₆): δ = 10.23 (s, 0.5H), 10.19 (s, 0.5H), 7.60 (d, 1H), 7.37-7.28 (m, 2H), 7.25 (d, 1H), 7.24-7.14 (m, 3H), 6.91-6.83 (m, 1H), 4.66 (br d, 0.5H), 4.50 (br d, 0.5H), 4.19 (br d, 0.5H), 3.97 (br d, 0.5H), 3.21 (t, 0.5H), 3.18-3.00 (m, 1.5H), 2.72-2.53 (m, 3H), 2.57 (q, 2H), 2.19-2.08 (m, 1H), 2.02-1.86 (m, 1H), 1.85-1.46 (m, 8H), 1.17 (t, 3H).

### Beispiel 74

### 1-(Cyclopentylcarbonyl)-5-(4-ethylphenyl)-N-(3-fluorphenyl)piperidin-3-carboxamid [enantiomerenreines cis-Isomer]

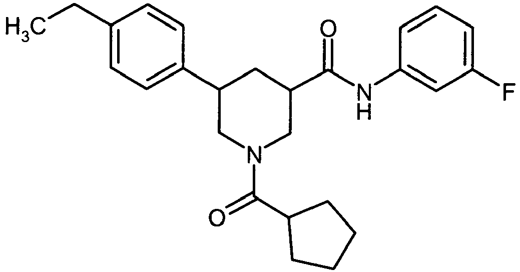

Enantiomerentrennung von 36 mg des Racemates aus Beispiel 73 nach Methode 4D ergab 14 mg der Verbindung aus Beispiel 74 (Enantiomer 1) und 13 mg des Enantiomers 2.

HPLC (Methode 3E): Rₜ = 2.26 min; MS (ESIpos): m/z = 423 [M+H]⁺.

### Beispiel 75

### 1-(Cyclopentylcarbonyl)-5-(4-ethylphenyl)-N-(3-methoxyphenyl)piperidin-3-carboxamid [racemisches cis-Isomer]

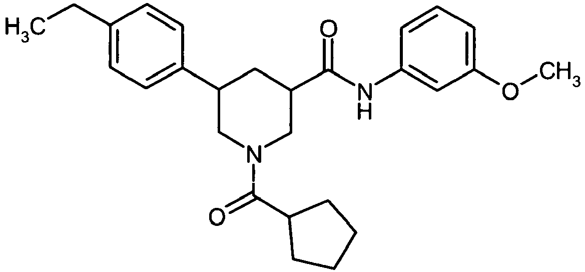

Nach der Allgemeinen Methode 1 wurden 66 mg (0.20 mmol) 1-(Cyclopentylcarbonyl)-5-(4-ethylphenyl)piperidin-3-carbonsäure (Beispiel 7A) und 27 mg (0.22 mmol, 1.1 eq.) 3-Methoxyanilin umgesetzt. Ausbeute: 52 mg (60% d. Th.)

HPLC (Methode 1A): Rₜ = 5.19 min; MS (ESIpos): m/z = 435 [M+H]⁺;

¹H-NMR (500 MHz, DMSO-d₆): δ = 10.00 (s, 0.5H), 9.96 (s, 0.5H), 7.31 (s, 1H), 7.28-7.11 (m, 6H), 6.62 (d, 1H), 4.65 (br d, 0.5H), 4.50 (br d, 0.5H), 4.17 (br d, 0.5H), 3.97 (br d, 0.5H), 3.21 (t, 0.5H), 3.15-3.00 (m, 1.5H), 2.72-2.53 (m, 3H), 2.57 (q, 2H), 2.17-2.06 (m, 1H), 2.01-1.86 (m, 1H), 1.86-1.46 (m, 8H), 1.17 (t, 3H).

### Beispiel 76

### N-[1-(Cyclopentylcarbonyl)-5-(4-ethylphenyl)piperidin-3-yl]benzamid [racemisches cis-Isomer]

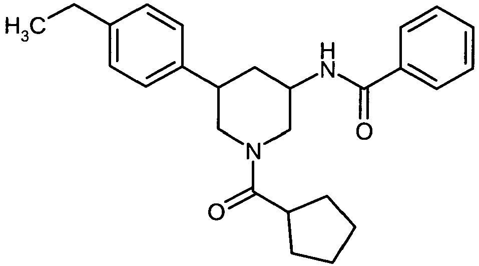

Nach der Allgemeinen Methode 1 wurden 170 mg (0.41 mmol) 1-(Cyclopentylcarbonyl)-5-(4-ethylphenyl)piperidin-3-amin-Trifluoracetat (Beispiel 8A) und 50 mg (0.41 mmol, 1.0 eq.) Benzoesäure umgesetzt. Ausbeute: 58 mg (35% d. Th.)

HPLC (Methode 1A): Rₜ = 4.89 min; MS (ESIpos): m/z = 405 [M+H]⁺;

¹H-NMR (500 MHz, DMSO-d₆): δ = 8.40 (d, 0.6H), 8.36 (d, 0.4H), 7.86 (dd, 2H), 7.53 (t, 1H), 7.47 (t, 2H), 7.27-7.16 (m, 4H), 4.67 (br d, 0.4H), 4.50 (br d, 0.6H), 4.23 (br d, 0.6H), 3.98 (br d, 0.4H), 3.97-3.83 (m, 1H), 3.08-2.99 (m, 1.4H), 2.89 (t, 0.6H), 2.83-2.75 (m, 0.4H), 2.72-2.63 (m, 0.6H), 2.58 (q, 2H), 2.12-2.01 (m, 1.6H), 1.95-1.78 (m, 1.4H), 1.78-1.46 (m, 8H), 1.17 (t, 3H).

### Beispiel 77

### N-[5-(4-Ethylphenyl)-1-(pyrrolidin-1-ylcarbonyl)piperidin-3-yl]benzamid [racemisches cis-Isomer]

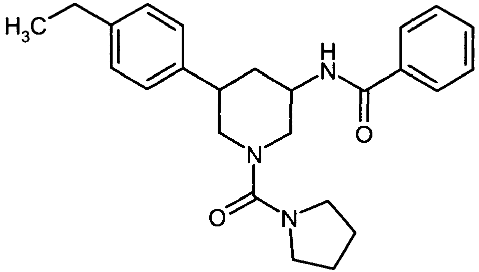

Nach der Allgemeinen Methode 1 wurden 125 mg (50%ig, 0.15 mmol) 5-(4-Ethylphenyl)-1-(pyrrolidin-1-ylcarbonyl)piperidin-3-amin-Trifluoracetat (Beispiel 12A) und 20 mg (0.17 mmol, 1.1 eq.) Benzoesäure umgesetzt. Ausbeute: 35 mg (56% d. Th.)

HPLC (Methode 1A): Rₜ = 4.72 min; MS (ESIpos): m/z = 406 [M+H]⁺;

¹H-NMR (400 MHz, DMSO-d₆): δ = 8.35 (d, 1H), 7.85 (d, 2H), 7.53 (t, 1H), 7.46 (t, 2H), 7.24-7.14 (m, 4H), 4.07-3.95 (m, 1H), 3.91 (br d, 1H), 3.71 (br d, 1H), 3.33-2.24 (m, 4H), 2.90-2.80 (m, 1H), 2.75-2.60 (m, 2H), 2.57 (q, 2H), 2.08 (br d, 1H), 1.85-1.72 (m, 5H), 1.17 (t, 3H).

### Beispiel 78

### N-[5-(4-Ethylphenyl)-1-(pyrrolidin-1-ylcarbonyl)piperidin-3-yl]-3-fluorbenzamid [racemisches cis-Isomer]

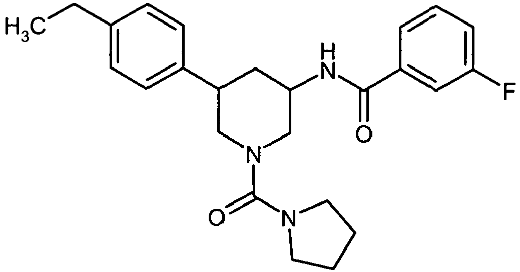

Nach der Allgemeinen Methode 1 wurden 83 mg (75%ig, 0.15 mmol) 5-(4-Ethylphenyl)-1-(pyrrolidin-1-ylcarbonyl)piperidin-3-amin-Trifluoracetat (Beispiel 12A) und 23 mg (0.17 mmol, 1.1 eq.) 3-Fluorbenzoesäure umgesetzt. Ausbeute: 41 mg (65% d. Th.)

HPLC (Methode 1A): Rₜ = 4.79 min; MS (ESIpos): m/z = 424 [M+H]⁺;

¹H-NMR (400 MHz, DMSO-d₆): δ = 8.45 (d, 1H), 7.71 (d, 1H), 7.66 (dd, 1H), 7.52 (dt, 1H), 7.38 (dt, 1H), 7.23-7.14 (m, 4H), 4.06-3.94 (m, 1H), 3.91 (br d, 1H), 3.70 (br d, 1H), 3.32-2.25 (m, 4H), 2.90-2.80 (m, 1H), 2.73-2.60 (m, 2H), 2.57 (q, 2H), 2.08 (br d, 1H), 1.84-1.71 (m, 5H), 1.17 (t, 3H).

### Beispiel 79

### N-[5-(4-Ethylphenyl)-1-(pyrrolidin-1-ylcarbonyl)piperidin-3-yl]-3-methoxybenzamid [racemisches cis-Isomer]

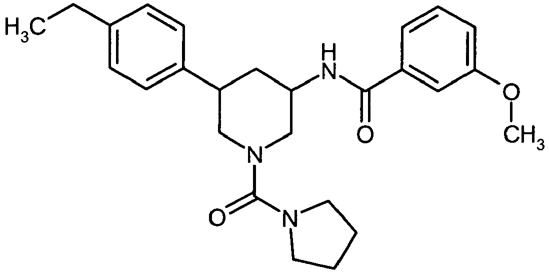

Nach der Allgemeinen Methode 1 wurden 83 mg (75%ig, 0.15 mmol) 5-(4-Ethylphenyl)-1-(pyrrolidin-1-ylcarbonyl)piperidin-3-amin-Trifluoracetat (Beispiel 12A) und 25 mg (0.17 mmol, 1.1 eq.) 3-Methoxybenzoesäure umgesetzt. Ausbeute: 47 mg (72% d. Th.)

HPLC (Methode 2A): Rₜ = 4.75 min; MS (ESIpos): m/z = 436 [M+H]⁺;

¹H-NMR (400 MHz, DMSO-d₆): δ = 8.33 (d, 1H), 7.47-7.34 (m, 3H), 7.23-7.14 (m, 4H), 7.09 (dd, 1H), 4.07-3.94 (m, 1H), 3.91 (br d, 1H), 3.80 (s, 3H), 3.71 (br d, 1H), 3.36-2.26 (m, 4H), 2.91-2.80 (m, 1H), 2.65 (t, 2H), 2.57 (q, 2H), 2.09 (br d, 1H), 1.86-1.70 (m, 5H), 1.17 (t, 3H).

### Beispiel 80

### N-[5-(4-Ethylphenyl)-1-(pyrrolidin-1-ylcarbonyl)piperidin-3-yl]-4-fluorbenzamid [racemisches cis-Isomer]

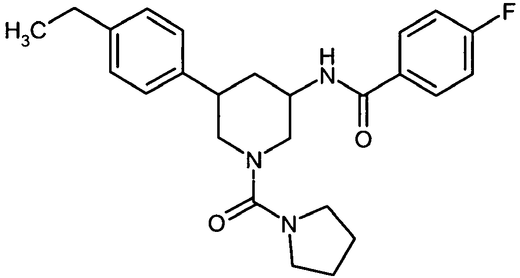

Nach der Allgemeinen Methode 1 wurden 125 mg (50%ig, 0.15 mmol) 5-(4-Ethylphenyl)-1-(pyrrolidin-1-ylcarbonyl)piperidin-3-amin-Trifluoracetat (Beispiel 12A) und 23 mg (0.17 mmol, 1.1 eq.) 4-Fluorbenzoesäure umgesetzt. Ausbeute: 53 mg (83% d. Th.)

HPLC (Methode 1A): Rₜ = 4.78 min; MS (ESIpos): m/z = 424 [M+H]⁺;

¹H-NMR (400 MHz, DMSO-d₆): δ = 8.37 (d, 1H), 7.93 (dd, 2H), 7.30 (dd, 2H), 7.24-7.14 (m, 4H), 4.06-3.93 (m, 1H), 3.90 (br d, 1H), 3.70 (br d, 1H), 3.32-2.22 (m, 4H), 2.90-2.80 (m, 1H), 2.73-2.60 (m, 2H), 2.57 (q, 2H), 2.08 (br d, 1H), 1.84-1.71 (m, 5H), 1.17 (t, 3H).

### Beispiel 81

### 5-Chlor-N-[1-(cyclopentylcarbonyl)-5-(4-ethylphenyl)piperidin-3-yl]pyridin-3-carbonsäureamid [racemisches cis-Isomer]

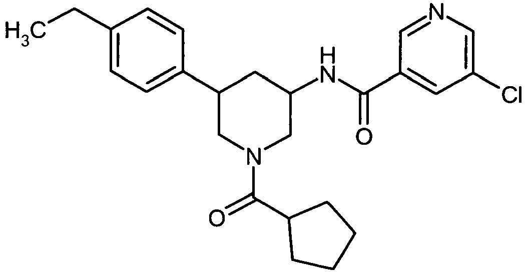

Nach der Allgemeinen Methode 1 wurden 65 mg (0.15 mmol) 1-(Cyclopentylcarbonyl)-5-(4-ethylphenyl)piperidin-3-amin-Trifluoracetat (Beispiel 8A) und 21 mg (0.13 mmol, 0.9 eq.) 5-Chlorpyridin-3-carbonsäure umgesetzt. Ausbeute: 39 mg (62% d. Th.)

HPLC (Methode 1A): Rₜ = 4.72 min; MS (ESIpos): m/z = 440 [M+H]⁺;

¹H-NMR (400 MHz, DMSO-d₆): δ = 8.96 (d, 1H), 8.79 (s, 1H), 8.74 (d, 0.5H), 8.70 (d, 0.5H), 8.33 (d, 1H), 7.27-7.13 (m, 4H), 4.69 (br d, 0.5H), 4.49 (br d, 0.5H), 4.26 (br d, 0.5H), 3.97 (br d, 0.5H), 3.95-3.81 (m, 1H), 3.10-2.97 (m, 1.5H), 2.90 (t, 0.5H), 2.85-2.75 (m, 0.5H), 2.73-2.62 (m, 0.5H), 2.58 (q, 2H), 2.16-1.98 (m, 1.5H), 1.92-1.45 (m, 9.5H), 1.17 (t, 3H).

### Beispiel 82

### 5-Brom-N-[1-(cyclopentylcarbonyl)-5-(4-ethylphenyl)piperidin-3-yl]pyridin-2-carbonsäureamid [racemisches cis-Isomer]

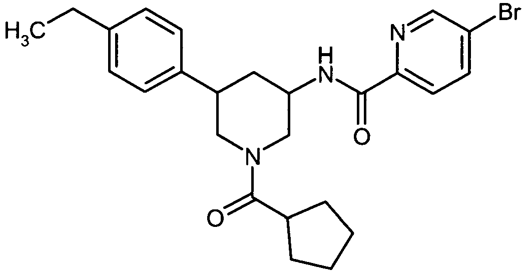

Nach der Allgemeinen Methode 1 wurden 139 mg (0.34 mmol) 1-(Cyclopentylcarbonyl)-5-(4-ethylphenyl)piperidin-3-amin-Trifluoracetat (Beispiel 8A) und 68 mg (0.34 mmol, 1.0 eq.) 5-Brompyridin-2-carbonsäure umgesetzt. Ausbeute: 75 mg (44% d. Th.)

HPLC (Methode 2A): Rₜ = 5.06 min; MS (ESIpos): m/z = 484 [M+H]⁺;

¹H-NMR (400 MHz, DMSO-d₆): δ = 8.83-8.75 (m, 2H), 8.34-8.23 (m, 1H), 8.02-7.96 (m, 1H), 7.26-7.15 (m, 4H), 4.58 (br d, 0.5H), 4.48 (br d, 0.5H), 4.14 (br d, 0.5H), 4.02-3.85 (m, 1.5H), 3.08-2.93 (m, 2H), 2.84-2.73 (m, 0.5H), 2.71-2.62 (m, 0.5H), 2.57 (q, 2H), 2.13-1.98 (m, 2.5H), 1.87-1.46 (m, 8.5H), 1.17 (t, 3H).

### Beispiel 83

### N-[1-(Cyclopentylcarbonyl)-5-(4-ethylphenyl)piperidin-3-yl]-5-[3-(trifluormethyl)phenyl]pyridin-2-carbonsäureamid [racemisches cis-Isomer]

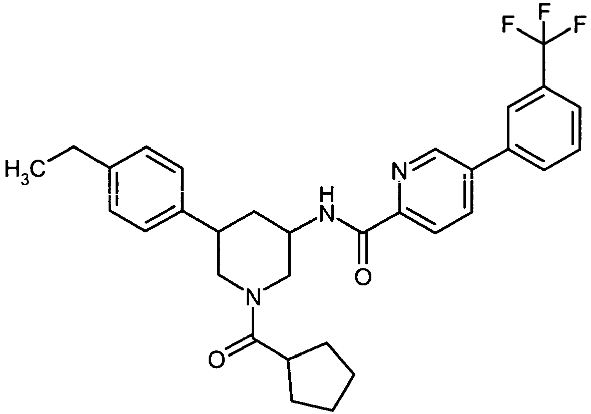

Nach der Allgemeinen Methode 7 wurden 59 mg (0.12 mmol) 5-Brom-*N*-[1-(cyclopentylcarbonyl)-5-(4-ethylphenyl)piperidin-3-yl]pyridin-2-carbonsäureamid (Beispiel 82) und 27 mg (0.14 mmol, 1.2 eq.) [3-(Trifluormethyl)phenyl]boronsäure umgesetzt. Ausbeute: 46 mg (71% d. Th.)

HPLC (Methode 2A): Rₜ = 5.40 min; MS (ESIpos): m/z = 550 [M+H]⁺;

¹H-NMR (400 MHz, DMSO-d₆): δ = 9.05-9.00 (m, 1H), 8.80 (t, 1H), 8.44-8.37 (m, 1H), 8.19-8.10 (m, 3H), 7.85 (d, 1H), 7.79 (t, 1H), 7.24 (t, 1H), 7.22-7.15 (m, 3H), 4.61 (br d, 0.5H), 4.49 (br d, 0.5H), 4.18 (br d, 0.5H), 4.07-3.91 (m, 1.5H), 3.12-2.96 (m, 2H), 2.87-2.77 (m, 0.5H), 2.72-2.63 (m, 1H), 2.58 (q, 2H), 2.18-2.01 (m, 2.5H), 1.87-1.47 (m, 8H), 1.17 (t, 3H).

### Beispiel 84

### N-[1-(Cyclopentylcarbonyl)-5-(4-ethylphenyl)piperidin-3-yl]-5-[3-(trifluormethyl)phenyl]pyridin-2-carbonsäureamid [enantiomerenreines cis-Isomer]

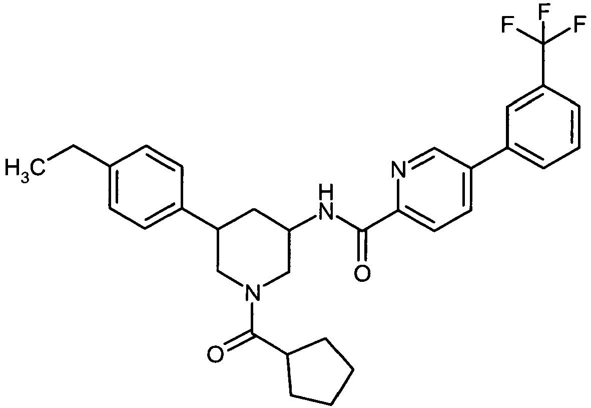

Enantiomerentrennung von 36 mg des Racemates aus Beispiel 83 nach Methode 6D ergab 17 mg der Verbindung aus Beispiel 84 (Enantiomer 1) und 15 mg der Verbindung aus Beispiel 85 (Enantiomer 2).

HPLC (Methode 2E): Rₜ = 6.39 min; MS (ESIpos): m/z = 550 [M+H]⁺.

### Beispiel 85

### N-[1-(Cyclopentylcarbonyl)-5-(4-ethylphenyl)piperidin-3-yl]-5-[3-(trifluormethyl)phenyl]pyridin-2-carbonsäureamid [enantiomerenreines cis-Isomer]

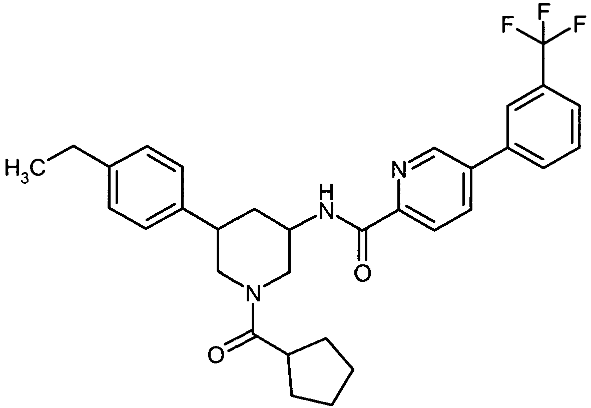

Enantiomerentrennung von 36 mg des Racemates aus Beispiel 83 nach Methode 6D ergab 17 mg der Verbindung aus Beispiel 84 (Enantiomer 1) und 15 mg der Verbindung aus Beispiel 85 (Enantiomer 2).

HPLC (Methode 2E): Rₜ = 7.07 min; MS (ESIpos): m/z = 550 [M+H]⁺.

### Beispiel 86

### N-[5-(4-Ethylphenyl)-1-(pyrrolidin-1-ylcarbonyl)piperidin-3-yl]-5-(3-fluorphenyl)pyridin-2-carboxamid [racemisches cis-Isomer]

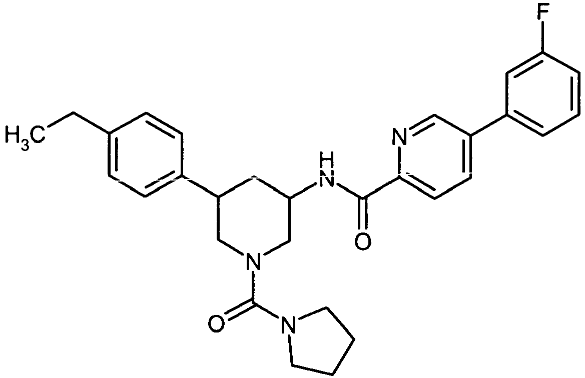

Nach der Allgemeinen Methode 1 wurden 62 mg (0.11 mmol) 5-(4-Ethylphenyl)-1-(pyrrolidin-1-ylcarbonyl)piperidin-3-amin-Trifluoracetat (Beispiel 12A) und 27 mg (0.12 mmol, 1.1 eq.) 5-(3-Fluorphenyl)pyridin-2-carbonsäure umgesetzt. Ausbeute: 31 mg (56% d. Th.)

HPLC (Methode 2A): Rₜ = 5.18 min; MS (ESIpos): m/z = 501 [M+H]⁺;

¹H-NMR (400 MHz, DMSO-d₆): δ = 8.98 (d, 1H), 8.73 (d, 1H), 8.34 (dd, 1H), 8.12 (d, 1H), 7.76-7.65 (m, 2H), 7.63-7.55 (m, 1H), 7.37-7.28 (m, 1H), 7.21 (d, 2H), 7.17 (d, 2H), 4.13-4.02 (br m, 1H), 3.85 (br d, 1H), 3.70 (br d, 1H), 3.32-3.25 (m, 4H), 2.91-2.81 (m, 1H), 2.77 (t, 1H), 2.72-2.63 (m, 1H), 2.57 (q, 2H), 2.10-1.93 (m, 2H), 1.83-1.71 (m, 4H), 1.17 (t, 3H).

### Beispiel 87

### 5-Chlor-N-[1-(cyclopentylcarbonyl)-5-(4-ethylphenyl)piperidin-3-yl]thiophen-2-carbonsäureamid [racemisches cis-Isomer]

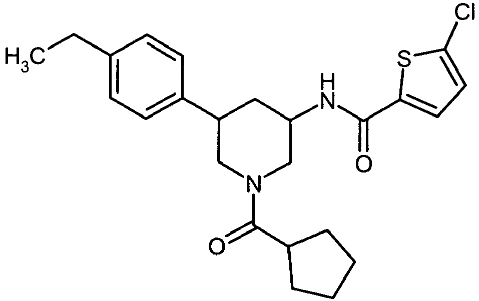

Nach der Allgemeinen Methode 1 wurden 65 mg (0.15 mmol) 1-(Cyclopentylcarbonyl)-5-(4-ethylphenyl)piperidin-3-amin-Trifluoracetat (Beispiel 8A) und 22 mg (0.13 mmol, 0.9 eq.) 5-Chlorthiophen-2-carbonsäure umgesetzt. Ausbeute: 43 mg (72% d. Th.)

HPLC (Methode 2A): Rₜ = 5.02 min; MS (ESIpos): m/z = 445 [M+H]⁺;

¹H-NMR (400 MHz, DMSO-d₆): δ = 8.55 (d, 0.6H), 8.50 (d, 0.4H), 7.71 (d, 0.6H), 7.68 (d, 0.4H), 7.26-7.13 (m, 5H), 4.64 (br d, 0.4H), 4.47 (br d, 0.6H), 4.21 (br d, 0.6H), 3.96 (br d, 0.4H), 3.90-3.73 (m, 1H), 3.07-2.97 (m, 1.4H), 2.87 (t, 0.6H), 2.82-2.73 (m, 0.4H), 2.71-2.61 (m, 0.6H), 2.58 (q, 2H), 2.43 (t, 0.6H), 2.11-1.96 (m, 1.4H), 1.91-1.44 (m, 9H), 1.17 (t, 3H).

### Beispiel 88

### N-[1-(Cyclopentylcarbonyl)-5-(4-ethylphenyl)piperidin-3-yl]-2,4-dimethyl-1,3-thiazol-5-carbonsäureamid [racemisches cis-Isomer]

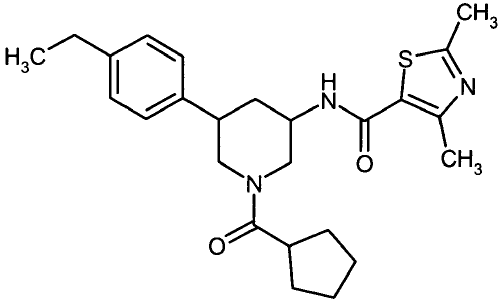

Nach der Allgemeinen Methode 1 wurden 65 mg (0.15 mmol) 1-(Cyclopentylcarbonyl)-5-(4-ethylphenyl)piperidin-3-amin-Trifluoracetat (Beispiel 8A) und 21 mg (0.13 mmol, 0.9 eq.) 2,4-Dimethyl-1,3-thiazol-5-carbonsäure umgesetzt. Ausbeute: 43 mg (73% d. Th.)

HPLC (Methode 1A): Rₜ = 4.50 min; MS (ESIpos): m/z = 440 [M+H]⁺;

¹H-NMR (400 MHz, DMSO-d₆): δ = 8.10 (t, 1H), 7.24-7.13 (m, 4H), 4.62 (br d, 0.4H), 4.47 (br d, 0.6H), 4.18 (br d, 0.6H), 3.96 (br d, 0.4H), 3.90-3.74 (m, 1H), 3.08-2.96 (m, 1.4H), 2.89 (t, 0.6H), 2.82-2.73 (m, 0.4H), 2.71-2.62 (m, 0.6H), 2.61 (s, 3H), 2.57 (q, 2H), 2.54 (s, 3H), 2.44 (t, 0.6H), 2.08-1.98 (m, 1.4H), 1.90-1.46 (m, 9H), 1.17 (t, 3H).

### Beispiel 89

### N-[1-(Cyclopentylcarbonyl)-5-(4-ethylphenyl)piperidin-3-yl]-2-phenylacetamid [racemisches cis-Isomer]

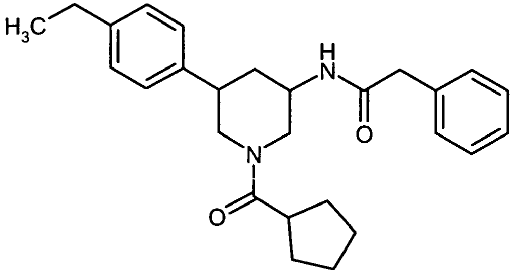

Nach der Allgemeinen Methode 1 wurden 57 mg (0.12 mmol) 1-(Cyclopentylcarbonyl)-5-(4-ethylphenyl)piperidin-3-amin-Trifluoracetat (Beispiel 8A) und 16 mg (0.12 mmol, 1.0 eq.) Phenylessigsäure umgesetzt. Ausbeute: 28 mg (58% d. Th.)

HPLC (Methode 2A): Rₜ = 4.79 min; MS (ESIpos): m/z = 419 [M+H]⁺;

¹H-NMR (400 MHz, DMSO-d₆): δ = 8.19 (d, 0.6H), 8.12 (d, 0.4H), 7.32-7.12 (m, 9H), 4.56 (br d, 0.5H), 4.44 (br d, 0.5H), 4.15 (br d, 0.5H), 3.92 (br d, 0.5H), 3.68-3.53 (m, 1H), 3.42 (s, 1.2H), 3.40 (s, 0.8H), 3.07-2.88 (m, 2H), 2.75 (t, 1H), 2.57 (q, 2H), 2.32 (t, 0.6H), 2.08-1.96 (m, 1.4H), 1.86-1.44 (m, 8H), 1.17 (t, 3H).

### Beispiel 90

### N-[1-(Cyclopentylcarbonyl)-5-(4-ethylphenyl)piperidin-3-yl]-2,2-dimethylpropanamid [racemisches cis-Isomer]

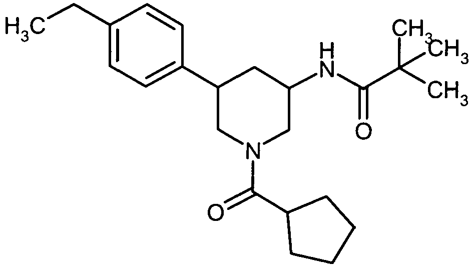

Nach der Allgemeinen Methode 1 wurden 99 mg (0.20 mmol) 1-(Cyclopentylcarbonyl)-5-(4-ethylphenyl)piperidin-3-amin-Trifluoracetat (Beispiel 8A) und 20 mg (0.20 mmol, 1.0 eq.) Pivalinsäure umgesetzt. Ausbeute: 56 mg (73% d. Th.)

HPLC (Methode 1A): Rₜ = 4.81 min; MS (ESIpos): m/z = 385 [M+H]⁺;

¹H-NMR (400 MHz, DMSO-d₆): δ = 7.33 (t, 1H), 7.23-7.14 (m, 4H), 4.52-4.42 (m, 1H), 4.06 (br d, 0.6H), 3.93 (br d, 0.4H), 3.72-3.60 (m, 1H), 3.05-2.92 (m, 1.4H), 2.77 (t, 0.6H), 2.74-2.65 (m, 0.4H), 2.63-2.53 (m, 2.6H), 2.37 (t, 0.6H), 2.08-1.97 (m, 0.4H), 1.96-1.88 (m, 1H), 1.87-1.45 (m, 9H), 1.16 (t, 3H), 1.10 (s, 5.4H), 1.09 (3.6H).

### Beispiel 91

### 1-[5-(4-Ethylphenyl)-1-(pyrrolidin-1-ylcarbonyl)piperidin-3-yl]-3-phenylharnstoff [racemisches cis-Isomer]

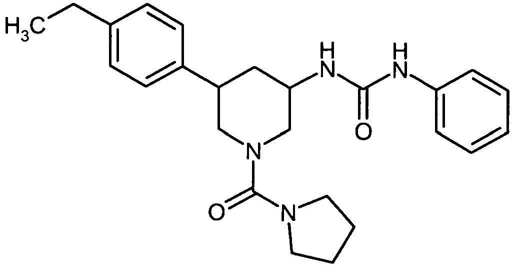

Nach der Allgemeinen Methode 4 wurden 125 mg (50%ig, 0.15 mmol) 5-(4-Ethylphenyl)-1-(pyrrolidin-1-ylcarbonyl)piperidin-3-amin-Trifluoracetat (Beispiel 12A) und 21 mg (0.18 mmol, 1.2 eq.) Phenylisocyanat umgesetzt. Ausbeute: 58 mg (92% d. Th.)

HPLC (Methode 1A): Rₜ = 4.72 min; MS (ESIpos): m/z = 421 [M+H]⁺;

¹H-NMR (400 MHz, DMSO-d₆): δ = 8.39 (s, 1H), 7.37 (d, 2H), 7.26-7.13 (m, 6H), 6.89 (t, 1H), 6.17 (d, 1H), 3.92 (br d, 1H), 3.72-3.60 (m, 2H), 3.30-3.23 (m, 4H), 2.88-2.77 (m, 1H), 2.66 (q, 1H), 2.57 (q, 2H), 2.52-2.40 (m, 1H), 2.06 (br d, 1H), 1.82-1.70 (m, 4H), 1.56 (q, 1H), 1.17 (t, 3H).

### Beispiel 92

### 1-[5-(4-Ethylphenyl)-1-(pyrrolidin-1-ylcarbonyl)piperidin-3-yl]-3-(3-methoxyphenyl)harnstoff [racemisches cis-Isomer]

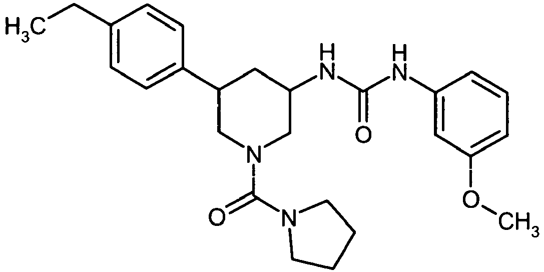

Nach der Allgemeinen Methode 4 wurden 83 mg (50%ig, 0.15 mmol) 5-(4-Ethylphenyl)-1-(pyrrolidin-1-ylcarbonyl)piperidin-3-amin-Trifluoracetat (Beispiel 12A) und 25 mg (0.17 mmol, 1.1 eq.) 3-Methoxyphenylisocyanat umgesetzt. Ausbeute: 46 mg (67% d. Th.)

HPLC (Methode 2A): Rₜ = 4.71 min; MS (ESIpos): m/z = 451 [M+H]⁺;

¹H-NMR (400 MHz, DMSO-d₆): δ = 8.41 (s, 1H), 7.20 (d, 2H), 7.16 (d, 2H), 7.14-7.08 (m, 2H), 6.85 (d, 1H), 6.48 (dd, 1H), 6.16 (d, 1H), 3.91 (br d, 1H), 3.70 (s, 3H), 3.70-3.60 (m, 2H), 3.32-3.25 (m, 4H), 2.87-2.77 (m, 1H), 2.65 (t, 1H), 2.58 (q, 2H), 2.50-2.44 (m, 1H), 2.05 (br d, 1H), 1.80-1.70 (m, 4H), 1.56 (q, 1H), 1.17 (t, 3H).

### Beispiel 93

### 1-[5-(4-Ethylphenyl)-1-(pyrrolidin-1-ylcarbonyl)piperidin-3-yl]-3-(4-fluorphenyl)harnstoff [racemisches cis-Isomer]

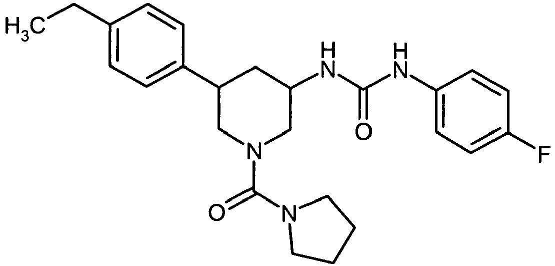

Nach der Allgemeinen Methode 4 wurden 83 mg (50%ig, 0.15 mmol) 5-(4-Ethylphenyl)-1-(pyrrolidin-1-ylcarbonyl)piperidin-3-amin-Trifluoracetat (Beispiel 12A) und 23 mg (0.17 mmol, 1.2 eq.) 4-Fluorphenylisocyanat umgesetzt. Ausbeute: 44 mg (67% d. Th.)

HPLC (Methode 2A): Rₜ = 4.73 min; MS (ESIpos): m/z = 439 [M+H]⁺;

¹H-NMR (400 MHz, DMSO-d₆): δ = 8.43 (s, 1H), 7.42-7.34 (m, 2H), 7.20 (d, 2H), 7.16 (d, 2H), 7.06 (t, 2H), 6.16 (d, 1H), 3.91 (br d, 1H), 3.71-3.60 (m, 2H), 3.32-3.25 (m, 4H), 2.83-2.76 (m, 1H), 2.66 (t, 1H), 2.58 (q, 2H), 2.46 (t, 1H), 2.05 (br d, 1H), 1.81-1.70 (m, 4H), 1.57 (q, 1H), 1.17 (t, 3H).

Analog wurde folgende Verbindung [racemische cis-Isomere] dargestellt:

| **Beispiel** | **Struktur** | **LC-MS Methode** | **Rₜ [min]** | **MS (ESIpos) m/z** |
|---|---|---|---|---|
| **94** | 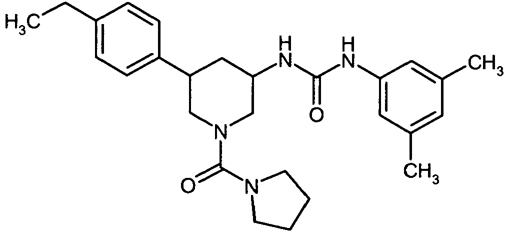 | 2B | 1.39 | 449 |

### Beispiel 95

### 3-[1-(Cyclopentylcarbonyl)-5-(4-ethylphenyl)piperidin-3-yl]-1-methyl-1-phenylharnstoff [racemisches cis-Isomer]

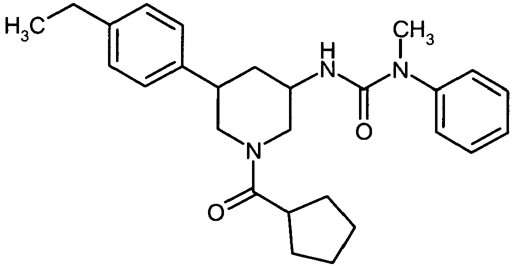

Nach der Allgemeinen Methode 3 wurden 60 mg (0.14 mmol) 1-(Cyclopentylcarbonyl)-5-(4-ethylphenyl)piperidin-3-amin-Trifluoracetat (Beispiel 8A) und 22 mg (0.14 mmol, 1.0 eq.) Methyl(phenyl)carbamoylchlorid umgesetzt. Ausbeute: 37 mg (69% d. Th.)

HPLC (Methode 1A): Rₜ = 4.87 min; MS (ESIpos): m/z = 434 [M+H]⁺;

¹H-NMR (400 MHz, DMSO-d₆): δ = 7.38 (t, 2H), 7.30-7.24 (m, 2H), 7.21 (t, 1H), 7.18-7.12 (m, 4H), 5.89 (d, 0.5H), 5.85 (d, 0.5H), 4.54 (br d, 0.5H), 4.42 (br d, 0.5H), 4.11 (br d, 0.5H), 3.89 (br d, 0.5H), 3.68-3.53 (m, 1H), 3.16 (s, 1.5H), 3.15 (s, 1.5H), 3.04-2.94 (m, 1H), 2.90 (t, 0.5H), 2.77 (t, 0.5H), 2.73-2.64 (m, 0.5H), 2.56 (q, 2H), 2.43 (t, 0.5H), 2.33 (t, 0.5H), 2.06-1.88 (m, 1.5H), 1.83-1.44 (m, 9H), 1.16 (t, 3H).

### Beispiel 96

### 1-[1-(Cyclopentylcarbonyl)-5-(4-ethylphenyl)piperidin-3-yl]-1,3-dimethyl-3-phenylharnstoff [racemisches cis-Isomer]

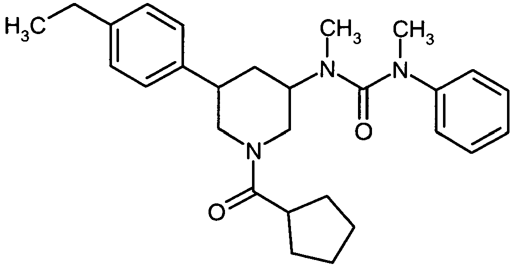

Eine Lösung aus 40 mg (0.10 mmol) 1-[1-(Cyclopentylcarbonyl)-5-(4-ethylphenyl)piperidin-3-yl]-3-phenylharnstoff (Beispiel 171) in 1 ml Dimethylformamid wurde unter Argon bei RT mit 8 mg Natriumhydrid (60%-ig in Mineralöl, 0.19 mmol, 2 eq.) versetzt und 30 min bei RT gerührt. Das Reaktionsgemisch wurde mit 13 µl (0.21 mmol, 2.2 eq.) Iodmethan versetzt und weitere 1.5 h bei RT gerührt. Nach Zugabe von Wasser-Dichlormethan und Phasentrennung wurde die wässrige Phase dreimal mit Dichlormethan extrahiert. Die vereinigten, organischen Phasen wurden getrocknet (Natriumsulfat), filtriert und im Vakuum eingeengt. Das Rohprodukt wurde mittels präparativer HPLC (Reprosil C18, Wasser-Acetonitril-Gradient) gereinigt. Ausbeute: 26 mg (61% d. Th.)

HPLC (Methode 2A): Rₜ = 5.03 min; MS (ESIpos): m/z = 448 [M+H]⁺;

¹H-NMR (400 MHz, DMSO-d₆): δ = 7.34 (d, 2H), 7.28-7.13 (m, 7H), 4.46-4.33 (m, 1H), 3.91-3.78 (m, 2H), 3.16 (t, 1H), 3.08 (s, 3H), 3.05-2.95 (m, 1H), 2.80-2.70 (m, 1H), 2.58 (q, 2H), 1.98-1.84 (m, 2H), 1.83-1.44 (m, 9H), 1.17 (t, 3H).

### Beispiel 97

### 1-tert.-Butyl-3-[1-(cyclopentylcarbonyl)-5-(4-ethylphenyl)piperidin-3-yl]harnstoff [racemisches cis-Isomer]

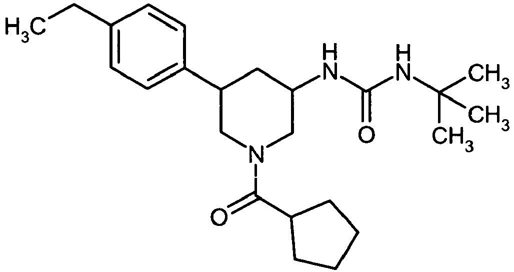

Nach der Allgemeinen Methode 4 wurden 108 mg (0.22 mmol) 1-(Cyclopentylcarbonyl)-5-(4-ethylphenyl)piperidin-3-amin-Trifluoracetat (Beispiel 7A) und 26 mg (0.26 mmol, 1.2 eq.) *tert.-*Butylisocyanat umgesetzt. Ausbeute: 69 mg (78% d. Th.)

HPLC (Methode 2A): Rₜ = 4.74 min; MS (ESIpos): m/z = 400 [M+H]⁺;

¹H-NMR (400 MHz, DMSO-d₆): δ = 7.24-7.14 (m, 4H), 5.73 (d, 0.5H), 5.67 (d, 0.5H), 5.66 (s, 0.5H), 5.61 (s, 0.5H), 4.57 (br d, 0.5H), 4.42 (br d, 0.5H), 4.14 (br d, 0.5H), 3.89 (br d, 0.5H), 3.50-3.36 (m, 1H), 3.05-2.94 (m, 1.5H), 2.73-2.61 (m, 1.5H), 2.57 (q, 2H), 2.18 (t, 0.5H), 2.07-1.92 (m, 1.5H), 1.83-1.43 (m, 9H), 1.22 (s, 4.5H), 1.21 (s, 4.5H), 1.16 (t, 3H).

### Beispiel 98

### 1-[1-(Cyclopentylcarbonyl)-5-(4-ethylphenyl)piperidin-3-yl]-3-cyclopropylharnstoff [racemisches cis-Isomer]

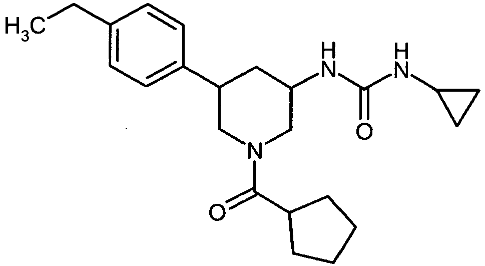

Nach der Allgemeinen Methode 4 wurden 108 mg (0.22 mmol) 1-(Cyclopentylcarbonyl)-5-(4-ethylphenyl)piperidin-3-amin-Trifluoracetat (Beispiel 7A) und 22 mg (0.26 mmol, 1.2 eq.) Cyclopropylisocyanat umgesetzt. Ausbeute: 64 mg (76% d. Th.)

HPLC (Methode 2A): Rₜ = 4.50 min; MS (ESIpos): m/z = 384 [M+H]⁺;

¹H-NMR (400 MHz, DMSO-d₆): δ = 7.24-7.14 (m, 4H), 6.14 (s, 0.6H), 6.12 (s, 0.4H), 5.86 (d, 0.6H), 5.81 (d, 0.4H), 4.56 (br d, 0.4H), 4.42 (br d, 0.6H), 4.15 (br d, 0.6H), 3.90 (br d, 0.4H), 3.56-3.41 (m, 1H), 3.04-2.92 (m, 1.4H), 2.77-2.65 (m, 1H), 2.64-2.52 (m, 0.6H und q, 2H), 2.45-2.35 (m, 1H), 2.26 (t, 0.4H), 2.08-1.91 (m, 1.6H), 1.83-1.43 (m, 9H), 1.16 (t, 3H), 0.60-0.52 (m, 2H), 0.36-0.28 (m, 2H).

### Beispiel 99

### N-[1-(Cyclopentylcarbonyl)-5-(4-ethylphenyl)piperidin-3-yl]pyrrolidin-1-carbonsäureamid [racemisches cis-Isomer]

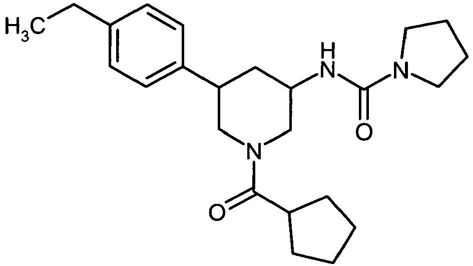

Nach der Allgemeinen Methode 3 wurden 57 mg (0.12 mmol) 1-(Cyclopentylcarbonyl)-5-(4-ethylphenyl)piperidin-3-amin-Trifluoracetat (Beispiel 7A) und 20 mg (0.15 mmol, 1.3 eq.) Pyrrolidin-*N*-carbonsäurechlorid umgesetzt. Ausbeute: 24 mg (52% d. Th.)

HPLC (Methode 2A): Rₜ = 4.60 min; MS (ESIpos): m/z = 398 [M+H]⁺;

¹H-NMR (400 MHz, DMSO-d₆): δ = 7.23-7.11 (m, 4H), 5.95 (d, 0.6H), 5.91 (d, 0.4H), 4.55 (br d, 0.4H), 4.45 (br d, 0.6H), 4.13 (br d, 0.6H), 3.92 (br d, 0.4H), 3.62-3.49 (m, 1H), 3.26-3.14 (m, 4H), 3.04-2.88 (m, 1.4H), 2.79-2.63 (m, 1H), 2.57 (q, 2H), 2.49-2.40 (m, 0.6H), 2.36-2.28 (m, 0.4H), 2.05-1.92 (m, 1.6H), 1.85-1.73 (m, 5H), 1.73-1.43 (m, 8H), 1.17 (t, 3H).

### Beispiel 100

### 1-Benzyl-3-[1-(cyclopentylcarbonyl)-5-(4-ethylphenyl)piperidin-3-yl] [racemisches cis-Isomer]

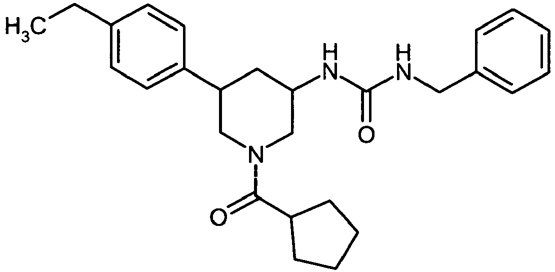

Nach der Allgemeinen Methode 4 wurden 108 mg (0.22 mmol) 1-(Cyclopentylcarbonyl)-5-(4-ethylphenyl)piperidin-3-amin-Trifluoracetat (Beispiel 7A) und 35 mg (0.26 mmol, 1.2 eq.) Benzylisocyanat umgesetzt. Ausbeute: 74 mg (77% d. Th.)

HPLC (Methode 1A): Rₜ = 4.74 min; MS (ESIpos): m/z = 434 [M+H]⁺;

¹H-NMR (400 MHz, DMSO-d₆): δ = 7.35-7.28 (m, 2H), 7.27-7.19 (m, 3H), 7.19-7.14 (m, 4H), 6.34 (t, 0.6H), 6.29 (t, 0.4H), 6.04 (d, 0.6H), 5.98 (d, 0.4H), 4.60 (br d, 0.4H), 4.43 (br d, 0.6H), 4.26 (dd, 0.6H), 4.23-4.14 (m, 2H), 3.90 (br d, 0.4H), 3.56-3.43 (m, 1H), 3.03-2.94 (m, 1.4H), 2.71 (t, 1H), 2.62-2.55 (m, 0.6H und q, 2H), 2.26 (t, 0.4H), 2.06-1.96 (m, 1.6H), 1.83-1.45 (m, 9H), 1.17 (t,3H).

### Beispiel 101

### N-[5-(4-Ethylphenyl)-1-(pyrrolidin-1-ylcarbonyl)piperidin-3-yl]-4-phenylpiperazin-1-carboxamid [racemisches cis-Isomer]

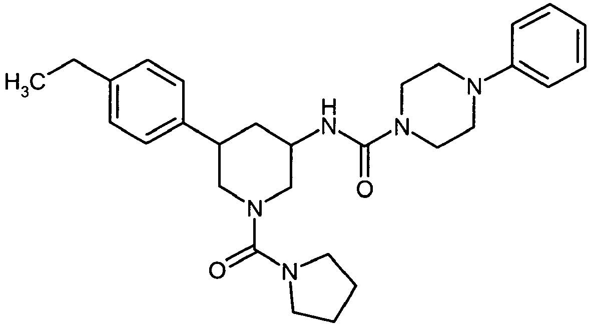

Nach der Allgemeinen Methode 3 wurden 125 mg (50%ig, 0.15 mmol) 5-(4-Ethylphenyl)-1-(pyrrolidin-1-ylcarbonyl)piperidin-3-amin-Trifluoracetat (Beispiel 12A) und 40 mg (0.18 mmol, 1.2 eq.) 4-Phenylpiperazin-1-carbonylchlorid umgesetzt. Ausbeute: 13 mg (18% d. Th.)

HPLC (Methode 2A): Rₜ = 4.37 min; MS (ESIpos): m/z = 490 [M+H]⁺;

¹H-NMR (400 MHz, DMSO-d₆): δ = 7.22 (t, 2H), 7.19-7.13 (m, 4H), 6.96 (d, 2H), 6.79 (t, 1H), 6.45 (d, 1H), 3.84 (br d, 1H), 3.73-3.61 (m, 2H), 3.48-3.41 (m, 4H), 3.30-3.23 (m, 4H), 3.12-3.04 (m, 4H), 2.83-2.73 (m, 1H), 2.57 (q, 2H), 2.48-2.40 (m, 2H), 1.99 (br d, 1H), 1.80-1.70 (m, 4H), 1.66 (q, 1H), 1.16 (t, 3H).

### Beispiel 102

### [5-(4-Ethylphenyl)-1-(morpholin-4-ylcarbonyl)piperidin-3-yl]methylcarbamat [racemisches cis-Isomer]

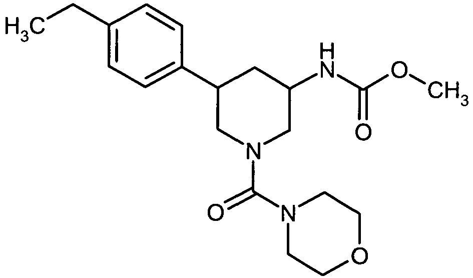

Nach der Allgemeinen Methode 3 wurden 170 mg (0.58 mmol) Methyl-[5-(4-ethylphenyl)piperidin-3-yl]carbamat (Beispiel 21A) und 113 mg (0.76 mmol, 1.3 eq.) Morpholin-4-carbonylchlorid umgesetzt. Die Diastereomerentrennung des cis-/trans-Isomerengemisches nach Methode 7C ergab 70 mg der Verbindung aus Beispiel 102.

LC-MS (Methode 1B): Rₜ = 2.13 min; MS (ESIpos): m/z = 376 [M+H]⁺;

¹H-NMR (400 MHz, DMSO-d₆): δ = 7.27 (d, 1H), 7.16 (s, 4H), 3.77 (br d, 1H), 3.62-3.51 (br d, 1H und s, 3H und m, 4H), 3.53-3.22 (m, 1H), 3.19-3.11 (m, 4H), 2.83-2.73 (m, 1H), 2.68 (t, 1H), 2.57 (q, 2H), 2.48 (t, 1H), 1.99 (br d, 1H), 1.54 (q, 1H), 1.16 (t, 3H).

### Beispiel 103

### [5-(4-Ethylphenyl)-1-(pyrrolidin-1-ylcarbonyl)piperidin-3-yl]phenylcarbamat [racemisches cis-Isomer]

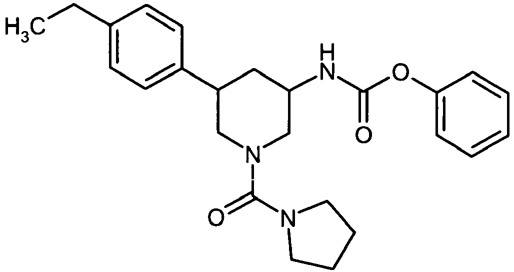

Nach der Allgemeinen Methode 5 wurden 83 mg (0.15 mmol) 5-(4-Ethylphenyl)-1-(pyrrolidin-1-ylcarbonyl)piperidin-3-amin-Trifluoracetat (Beispiel 12A) und 26 mg (0.17 mmol, 1.1 eq.) Chlorameisensäurephenylester umgesetzt. Ausbeute: 25 mg (39% d. Th.)

HPLC (Methode 2A): Rₜ = 4.90 min; MS (ESIpos): m/z = 422 [M+H]⁺;

¹H-NMR (400 MHz, DMSO-d₆): δ = 7.90 (d, 1H), 7.38 (d, 2H), 7.24-7.14 (m, 5H), 7.11 (d, 2H), 3.92 (br d, 1H), 3.67 (br d, 1H), 3.62-3.32 (m, 1H), 3.30-3.21 (m, 4H), 2.87-2.77 (m, 1H), 2.72-2.61 (m, 2H), 2.57 (q, 2H), 2.09 (br d, 1H), 1.80-1.70 (m, 4H), 1.63 (q, 1H), 1.17 (t, 3H).

### Beispiel 104

### N-[1-(Cyclopentylcarbonyl)-5-(4-ethylphenyl)piperidin-3-yl]-1-phenylmethansulfonamid [racemisches cis-Isomer]

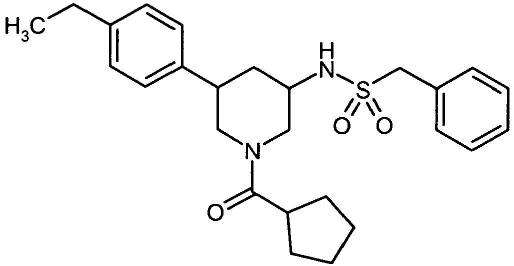

Nach der Allgemeinen Methode 6 wurden 55 mg (0.12 mmol) 1-(Cyclopentylcarbonyl)-5-(4-ethylphenyl)piperidin-3-amin-Trifluoracetat (Beispiel 7A) und 21mg (0.11 mmol, 0.9 eq.) Phenylmethansulfonsäure umgesetzt. Ausbeute: 14 mg (24% d. Th.)

HPLC (Methode 2A): Rₜ = 4.81 min; MS (ESIpos): m/z = 455 [M+H]⁺;

¹H-NMR (400 MHz, DMSO-d₆): δ = 7.43-7.33 (m, 5H), 7.21-7.12 (m, 4H), 4.68 (br d, 0.5H), 4.43-4.32 (br d, 0.5H und d, 2H), 4.03 (br d, 0.5H), 3.88 (br d, 0.5H), 3.20-3.02 (m, 1H), 3.01-2.87 (m, 1.5H), 2.76 (t, 0.5H), 2.58 (q, 2H), 2.11-1.99 (m, 1.5H), 1.92-1.82 (m, 0.5H), 1.77-1.47 (m, 9H), 1.17 (t, 3H).

### Beispiel 105

### 5-(4-Ethylphenyl)-1-(morpholin-4-ylcarbonyl)piperidin-3-yl-phenylcarbamat [racemisches cis-Isomer]

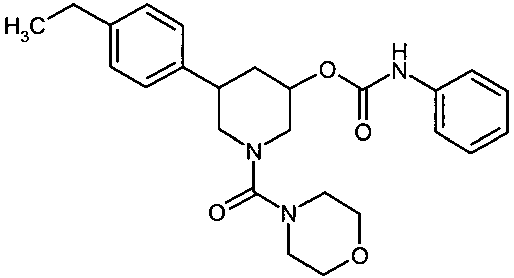

Eine Lösung von 112 mg (0.35 mmol) 5-(4-Ethylphenyl)-1-(morpholin-4-ylcarbonyl)piperidin-3-ol (Beispiel 25A) in 1.7 ml Acetonitril wurde unter Argon bei RT mit 184 µl (1.06 mmol, 3 eq.) *N,N-*Diisopropylethylamin und 135 mg (0.53 mmol, 1.5 eq.) *N,N'*-Disuccinimidylcarbonat versetzt. Das Reaktionsgemisch wurde 3 h bei RT gerührt, anschließend bei RT mit 96 µl (1.06 mmol, 3 eq.) Anilin und 61 µl (0.35 mmol, 1.0 eq.) *N,N*-Diisopropylethylamin versetzt und 1 h bei RT gerührt. Die Aufreinigung des Rohproduktes erfolgte anschließend mittels präparativer HPLC (Reprosil C18, Wasser-Acetonitril-Gradient). Ausbeute: 18 mg (12% d. Th.)

HPLC (Methode 2A): Rₜ = 4.75 min; MS (ESIpos): m/z = 438 [M+H]⁺;

¹H-NMR (400 MHz, DMSO-d₆): δ = 9.71 (s, 1H), 7.47 (d, 2H), 7.28 (t, 2H), 7.23 (d, 2H), 7.17 (d, 2H), 6.99 (t, 1H), 4.78-4.68 (m, 1H), 3.94 (br d, 1H), 3.62-3.53 (m, 5H), 3.23-3.15 (m, 4H), 2.94-2.83 (m, 1H), 2.83-2.70 (m, 2H), 2.58 (q, 2H), 2.25 (br d, 1H), 1.77 (q, 1H), 1.17 (t, 3H).

### Beispiel 106

### N-{1-[(4-Cyanpiperidin-1-yl)carbonyl]-5-[4-(trifluormethoxy)phenyl]piperidin-3-yl}benzol-carboxamid [racemisches cis-Isomer]

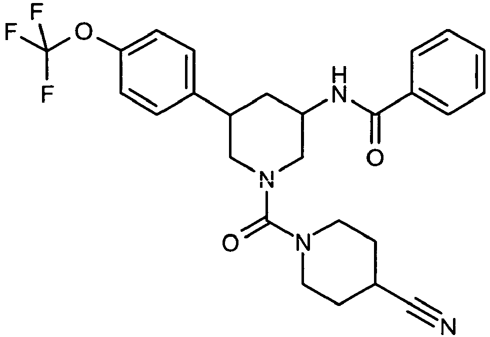

115 mg (0.22 mmol) 4-Nitrophenyl-3-[(phenylcarbonyl)amino]-5-[4-(trifluormethoxy)phenyl]-piperidin-1-carboxylat wurden in 3 ml DMF vorgelegt und mit 72 mg (0.65 mmol) Piperidin-4-carbonitril und 30 mg (0.22 mmol) Kaliumcarbonat versetzt. Die Mischung wurde bei 150°C 30 min in der Mikrowelle (Emrys Optimizer) umgesetzt. Die Aufreinigung des Rohproduktes erfolgte anschließend mittels präparativer HPLC (Reprosil C18, Wasser-Acetonitril-Gradient). Ausbeute: 53 mg (48% d. Th.)

LC-MS (Methode 1B): Rₜ = 2.43 min; m/z = 501 [M+H]⁺;

¹H-NMR (400 MHz, DMSO-d₆): δ = 8.39 (d, 1H), 7.89 - 7.83 (m, 2H), 7.50 - 7.55 (m, 1H), 7.49-7.41 (m, 4H), 7.35 (d, 2H), 4.07-3.96 (m, 1H), 3.81 (dd, 1H), 3.64 (d, 1H), 3.43 - 3.35 (m, 2H), 3.13-2.94 (m, 4H), 2.80-2.61 (m, 2H), 2.12 (d, 1H), 1.93-1.64 (m, 5H).

### Beispiel 107

### N-{1-[(4-Hydroxypiperidin-1-yl)carbonyl]-5-[4-(trifluormethoxy)phenyl]piperidin-3-yl}benzol-carboxamid [racemisches cis-Isomer]

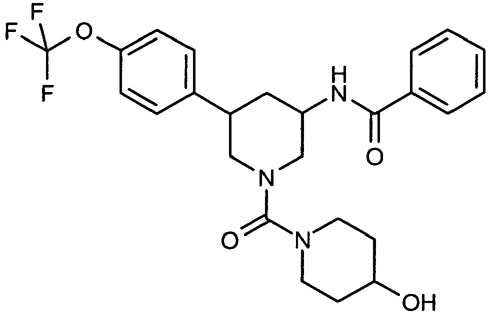

550 mg (1.04 mmol) 4-Nitrophenyl-3-[(phenylcarbonyl)amino]-5-[4-(trifluormethoxy)phenyl] piperidin-1-carboxylat wurden in 14 ml DMF vorgelegt und mit 315 mg (3.12 mmol) Piperidin-4-ol und 144 mg (1.04 mmol) Kaliumcarbonat versetzt. Die Mischung wurde bei 150°C 15 min in der Mikrowelle (Emrys Optimizer) umgesetzt. Die Aufreinigung des Rohproduktes erfolgte anschließend mittels präparativer HPLC (Reprosil C18, Wasser-Acetonitril-Gradient). Ausbeute: 430 mg (84% d. Th.)

LC-MS (Methode 1B): Rₜ = 2.19 min; m/z = 492 [M+H]⁺;

¹H-NMR (400 MHz, DMSO-*d*₆): δ = 8.38 (d, 1H), 7.86 (d, 2H), 7.52 (d, 1H), 7.47 (d, 2H), 7.44 (d, 2H), 7.34 (d, 2H), 4.68 (d, 1H), 4.09-3.93 (m, 1H), 3.83-3.74 (m, 1H), 3.66-3.57 (m, 2H), 3.49 (d, 2H), 2.90 (d, 3H), 2.76 (d, 1H), 2.65 (s, 1H), 2.15-2.06 (m, 1H), 1.82-1.69 (m, 3H), 1.33 (d, 2H).

### Beispiel 108

### N-{1-(Morpholin-4-ylcarbonyl)-5-[4-(trifluormethoxy)phenyl]piperidin-3-yl}benzolcarboxamid [racemisches cis-Isomer]

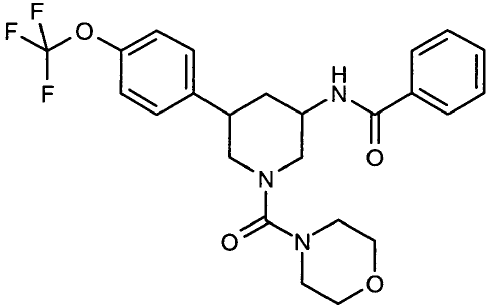

100 mg (0.27 mmol) *N*-{5-[4-(Trifluormethoxy)phenyl]piperidin-3-yl}benzamid wurden in 1.5 ml Dichlormethan bei 0°C vorgelegt und mit 82 mg (0.55 mmol) Morpholin-4-carbonylchlorid und 58 µl (0.412 mmol) Triethylamin versetzt. Die Mischung wurde langsam über Nacht auf RT erwärmt. Es wurde Wasser zu dem Reaktionsgemisch gegeben und extrahiert. Die organische Phase wurde über Magnesiumsulfat getrocknet und im Vakuum eingeengt. Der Rückstand wurde anschließend mittels präparativer HPLC (Reprosil C18, Wasser-Acetonitril-Gradient) gereinigt. Ausbeute: 118 mg (90% d. Th.)

LC-MS (Methode 3B): Rₜ = 1.95 min; m/z = 478 [M+H]⁺;

¹H-NMR (400 MHz, DMSO-*d*₆): δ = 8.39 (d, 1H), 7.86 (d, 2H), 7.52 (d, 1H), 7.50-7.41 (m, 4H), 7.35 (d, 2H), 4.08-3.93 (m, 1H), 3.85 (d, 1H), 3.67 (d, 1H), 3.58 (t, 4H), 3.20 (br. s., 4H), 3.05-2.93 (m, 1H), 2.89 (s, 1H), 2.83-2.63 (m, 2H), 2.11 (d, 1H), 1.86-1.74 (m, 1H).

### Beispiel 109

### N-{1-(Morpholin4-ylcarbonyl)-5-[4-(trifluormethoxy)phenyl]piperidin-3-yl}benzolcarboxamid [enantiomerenreines cis-Isomer]

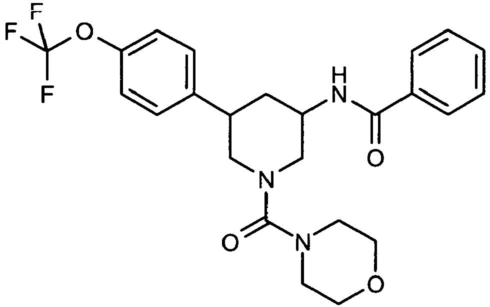

Die Enatiomerentrennung von 117 mg des racemischen cis-Isomerengemisches (Beispiel 108) nach Methode 11D ergab 34.8 mg der Verbindung aus Beispiel 109 (Enantiomer 1).

LC-MS (Methode 2B): Rₜ = 1.23 min; m/z = 478 [M+H]⁺.

### Beispiel 110

### N-{1-(Morpholin-4-ylcarbonyl)-5-[4-(trifluormethoxy)phenyl]piperidin-3-yl}benzolcarboxamid [enantiomerenreines cis-Isomer]

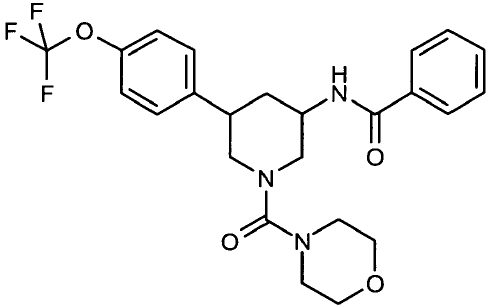

Die Enatiomerentrennung von 117 mg des racemischen cis-Isomerengemisches (Beispiel 108) nach Methode 11D ergab 29.7 mg der Verbindung aus Beispiel 110 (Enantiomer 2).

LC-MS (Methode 2B): Rₜ = 1.23 min; m/z = 478 [M+H]⁺.

### Beispiel 111

### N-(2-Methoxyethyl)-3-[(phenylcarbonyl)amino]-5-[4-(trifluormethoxy)phenyl]piperidin-1-carboxamid [racemisches cis-Isomer]

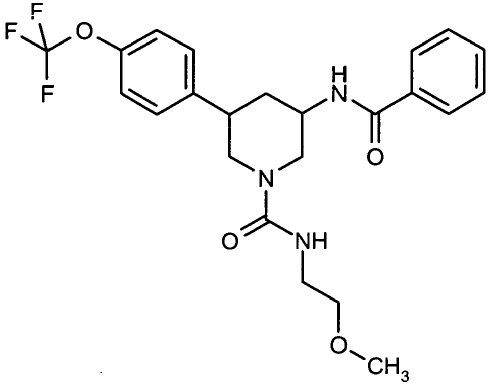

70 mg (0.19 mmol) *N*-{5-[4-(Trifluormethoxy)phenyl]piperidin-3-yl}benzamid wurden in 2.5 ml THF vorgelegt und mit 19 mg (0.19 mmol) 1-Isocyanato-2-methoxyethan und 29 µl (0.21 mmol) Triethylamin versetzt. Die Mischung wurde über Nacht bei RT gerührt. Das Reaktionsgemisch wurde anschließend mittels präparativer HPLC (Reprosil C18, Wasser-Acetonitril-Gradient) gereinigt. Ausbeute: 32 mg (35% d. Th.)

LC-MS (Methode 1B): Rₜ = 2.32 min; m/z = 466 [M+H]⁺;

¹H-NMR (400 MHz, DMSO-*d*₆): δ = 8.38 (d, 1H), 7.85 (d, 2H), 7.52 (d, 1H), 7.49-7.41 (m, 4H), 7.34 (d, 2H), 6.74-6.66 (m, 1H), 4.21 (dd, 1H), 4.05 (d, 1H), 4.00-3.89 (m, 1H), 3.34 (t, 2H), 3.24 (s, 3H), 3.19 (d, 2H), 2.82 (d, 1H), 2.74-2.56 (m, 2H), 2.10 (d, 1H), 1.73 (q, 1H).

### Beispiel 112

### Methyl-N-({3-[(phenylcarbonyl)amino]-5-[4-(trifluormethoxy)phenyl]piperidin-1-yl}carbonyl)-glycinat [racemisches cis-Isomer]

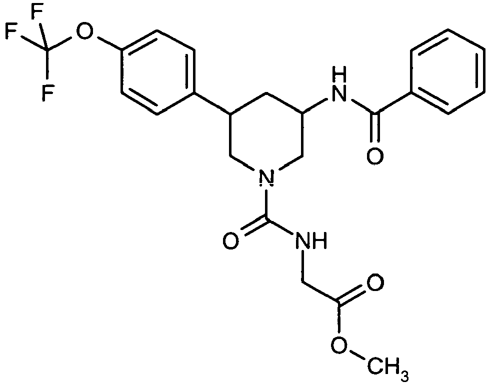

70 mg (0.19 mmol) *N*-{5-[4-(Trifluormethoxy)phenyl]piperidin-3-yl}benzamid wurden in 2.5 ml THF vorgelegt und mit 22 mg (0.19 mmol) Methyl-*N*-(oxomethyliden)glycinat und 29 µl (0.21 mmol) Triethylamin versetzt. Die Mischung wurde über Nacht bei RT gerührt. Das Reaktionsgemisch wurde anschließend mittels präparativer HPLC (Reprosil C18, Wasser-Acetonitril-Gradient) gereinigt. Ausbeute: 76 mg (82% d. Th.)

LC-MS (Methode 2B): Rₜ = 1.20 min; m/z = 480 [M+H]⁺;

¹H-NMR (400 MHz, DMSO-*d*₆): δ = 8.39 (d, 1H), 7.85 (d, 2H), 7.53 (d, 1H), 7.49-7.41 (m, 4H), 7.35 (d, 2H), 7.16 (t, 1H), 4.19 (dd, 1H), 4.07 (d, 1H), 4.02-3.91 (m, 1H), 3.75 (d, 2H), 3.63 (s, 3H), 2.93-2.82 (m, 1H), 2.76 (t, 1H), 2.67 (t, 1H), 2.12 (d, 1H), 1.75 (q, 1H).

### Beispiel 113

### N-{1-[(4-Methylpiperazin-1-yl)carbonyl]-5-[4-(trifluormethoxy)phenyl]piperidin-3-yl}benzol-carboxamid [racemisches cis-Isomer]

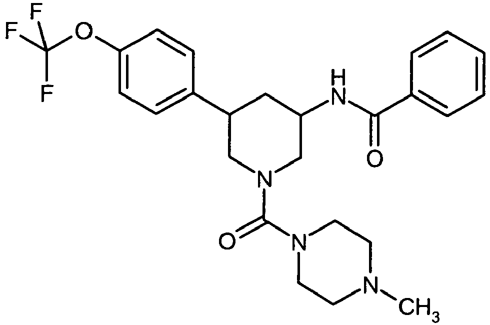

100 mg (0.27 mmol) *N*-{5-[4-(Trifluormethoxy)phenyl]piperidin-3-yl}benzamid wurden in 1.5 ml Dichlormethan bei 0°C vorgelegt und mit 89 mg (0.55 mmol) 4-Methylpiperazin-1-carbonylchlorid und 58 µl (0.412 mmol) Triethylamin versetzt. Die Mischung wurde langsam über Nacht auf RT erwärmt. Es wurde Wasser zu dem Reaktionsgemisch gegeben und extrahiert. Die organische Phase wurde über Magnesiumsulfat getrocknet und im Vakuum eingeengt. Der Rückstand wurde anschließend mittels präparativer HPLC (Reprosil C18, Wasser-Acetonitril-Gradient) gereinigt. Ausbeute: 104 mg (77% d. Th.)

LC-MS (Methode 2B): Rₜ = 1.01 min; m/z = 491 [M+H]⁺;

¹H-NMR (400 MHz, CD₃OD): δ = 7.83 (d, 2H), 7.54 (d, 1H), 7.50-7.40 (m, 4H), 7.26 (d, 2H), 4.21-4.08 (m, 1H), 4.02 (br. s., 1H), 3.87 (br. s., 1H), 3.50 (br. s., 4H), 3.05 (br. s., 4H), 2.91-2.76 (m, 2H), 2.72 (s, 3H), 2.25 (br. s., 1H), 2.01-1.84 (m, 2H), 1.35-1.25 (m, 1H).

### Beispiel 114

### N-{1-(Pyridin-4-ylcarbonyl)-5-[4-(trifluormethoxy)phenyl]piperidin-3-yl}benzolcarboxamid [racemisches cis-Isomer]

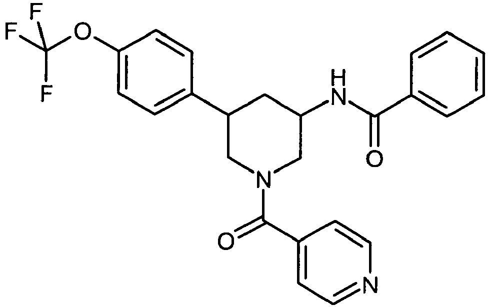

51 mg (0.41 mmol) Pyridin-4-carbonsäure wurden zusammen mit 157 mg (0.41 mmol) HATU und 67 mg (0.55 mmol) 4-Dimethylaminopyridin in 2 ml DMF vorgelegt und mit 100 mg (0.27 mmol) *N*-{5-[4-(Trifluormethoxy)phenyl]piperidin-3-yl}benzamid versetzt. Es wurde über Nacht bei RT gerührt und anschließend das Reaktionsgemisch mittels präparativer HPLC (Reprosil C18, Wasser-Acetonitril-Gradient) gereinigt. Ausbeute: 112 mg (87% d. Th.)

LC-MS (Methode 2B): Rₜ = 1.17 min; m/z = 470 [M+H]⁺;

¹H-NMR (400 MHz, DMSO-*d*₆): δ = 8.69 (dd, 2H), 8.43 (dd, 1H), 7.83 (dd, 2H), 7.60-7.24 (m, 9H), 4.67 (dd, 1H), 4.10 (d, 1H), 3.58 (dd, 1H), 3.19-2.97 (m, 2H), 2.81 (dt, 1H), 2.27-2.11 (m, 1H), 1.92 (q, 1H).

### Beispiel 115

### N-{1-(Pyridin-4-ylcarbonyl)-5-[4-(trifluormethoxy)phenyl]piperidin-3-yl}benzolcarboxamid [enantiomerenreines cis-Isomer]

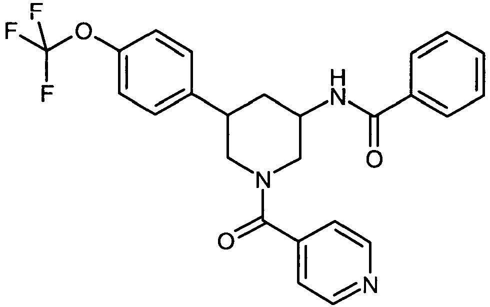

Die Enatiomerentrennung von 112 mg des racemischen cis-Isomerengemisches (Beispiel 114) nach Methode 12D ergab 37.6 mg der Verbindung aus Beispiel 115 (Enantiomer 1).

LC-MS (Methode 3B): Rₜ = 1.82 min; m/z = 470 [M+H]⁺.

### Beispiel 116

### N-{1-(Pyridin-4-ylcarbonyl)-5-[4-(trifluormethoxy)phenyl]piperidin-3-yl}benzolcarboxamid [enantiomerenreines cis-Isomer]

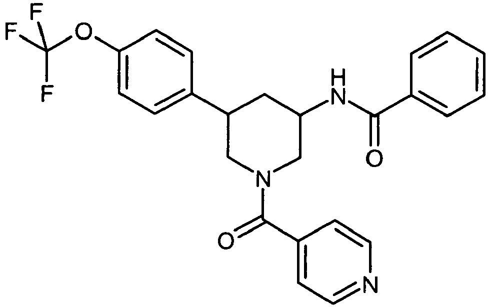

Die Enatiomerentrennung von 112 mg des racemischen cis-Isomerengemisches (Beispiel 114) nach Methode 12D ergab 33.4 mg der Verbindung aus Beispiel 116 (Enantiomer 2).

LC-MS (Methode 3B): Rₜ = 1.82 min; m/z = 470 [M+H]⁺.

### Beispiel 117

### N-{1-[(2R)-2-Methoxypropanoyl]-5-[4-(trifluormethoxy)phenyl]piperidin-3-yl}benzolcarboxamid [racemisches cis-Isomer]

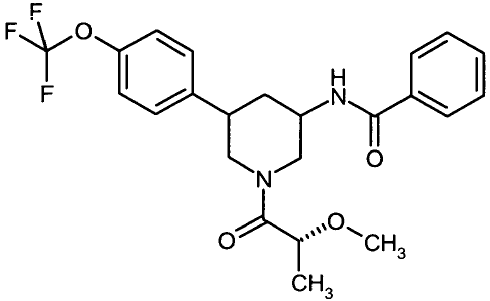

44 mg (0.41 mmol) (*2R*)-2-Methoxypropansäure wurden zusammen mit 157 mg (0.41 mmol) HATU und 67 mg (0.55 mmol) 4-Dimethylaminopyridin in 2 ml DMF vorgelegt und mit 100 mg (0.27 mmol) *N*-{5-[4-(Trifluormethoxy)phenyl]piperidin-3-yl}benzamid versetzt. Es wurde über Nacht bei RT gerührt und anschließend das Reaktionsgemisch mittels präparativer HPLC (Reprosil C18, Wasser-Acetonitril-Gradient) gereinigt. Ausbeute: 116 mg (94% d. Th.)

LC-MS (Methode 2B): Rₜ = 1.24 min; m/z = 451 [M+H]⁺;

¹H-NMR (400 MHz, DMSO-*d*₆): δ = 8.63-8.27 (m, 1H), 7.87 (t, 2H), 7.65-7.22 (m, 7H), 4.70-4.43 (m, 1H), 4.40-4.10 (m, 2H), 4.03-3.85 (m, 1H), 3.29-3.20 (m, 3H), 3.12-2.62 (m, 3H), 2.13 (d, 1H), 1.89 (t, 1H), 1.34 (t, 2H), 1.25 (dd, 1H).

### Beispiel 118

### N-[5-(3,4-Dimethylphenyl)-1-(morpholin-4-ylcarbonyl)piperidin-3-yl]benzolcarboxamid [racemisches cis-Isomer]

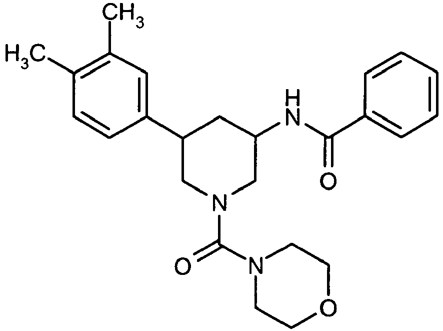

60 mg (0.19 mmol) *N*-5-(3,4-Dimethylphenyl)piperidin-3-yl]benzamid wurden in 1 ml Dichlormethan bei 0°C vorgelegt und mit 58 mg (0.39 mmol) Morpholin-4-carbonylchlorid und 41 µl (0.29 mmol) Triethylamin versetzt. Die Mischung wurde langsam über Nacht auf RT erwärmt. Es wurde Wasser zu dem Reaktionsgemisch gegeben und extrahiert. Die organische Phase wurde über Magnesiumsulfat getrocknet und im Vakuum eingeengt. Der Rückstand wurde anschließend mittels präparativer HPLC (Reprosil C18, Wasser-Acetonitril-Gradient) gereinigt. Ausbeute: 58 mg (71% d. Th.)

LC-MS (Methode 3B): Rₜ = 1.88 min; m/z = 422 [M+H]⁺;

¹H-NMR (400 MHz, DMSO-*d*₆): δ = 8.37 (d, 1H), 7.86 (d, 2H), 7.52 (d, 1H), 7.50-7.42 (m, 2H), 7.09 (d, 1H), 7.06 (s, 1H), 6.99 (d, 1H), 4.05-3.90 (m, 1H), 3.85 (d, 1H), 3.63 (d, 1H), 3.58 (s, 4H), 3.19 (br. s., 4H), 2.79 (d, 1H), 2.75-2.61 (m, 2H), 2.20 (d, 6H), 2.06 (d, 1H), 1.80 (d, 1H).

### Beispiel 119

### N-{1-[(1-Aminocyclobutyl)carbonyl]-5-(3,4-dimethylphenyl)piperidin-3-yl}benzolcarboxamid-Hydrochlorid [racemisches cis-Isomer]

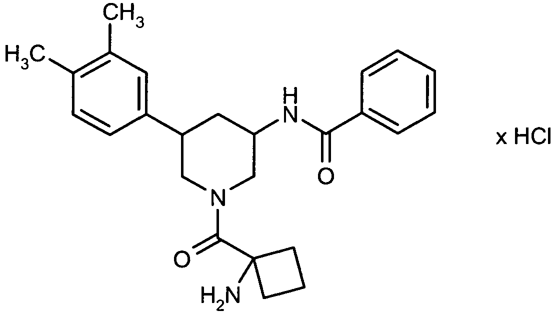

55 mg (0.14 mmol) *tert*.-Butyl-[1-({3-(3,4-dimethylphenyl)-5-[(phenylcarbonyl)amino]piperidin-1-yl}carbonyl)cyclobutyl]carbamat wurden in 7 ml Dioxan gelöst, mit 1.5 ml konzentrierter Salzsäure versetzt und sofort am Rotationsverdampfer eingeengt. Der Rückstand wurde anschließend mittels präparativer HPLC (Reprosil C18, Wasser-Acetonitril-Gradient) gereinigt. Ausbeute: 19 mg (39% d. Th.)

LC-MS (Methode 2B): Rₜ = 0.98 min; m/z = 406 [M+H]⁺.

### Beispiel 120

### N-{1-[(4-Aminotetrahydro-2H-pyran-4-yl)carbonyl]-5-(3,4-dimethylphenyl)piperidin-3-yl}benzolcarboxamid-Hydrochlorid [racemisches cis-Isomer]

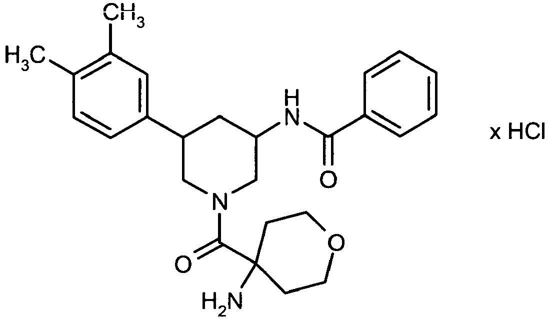

33 mg (0.06 mmol) *tert*.-Butyl-[4-({3-(3,4-dimethylphenyl)-5-[(phenylcarbonyl)amino]piperidin-1-yl}carbonyl)tetrahydro-2*H-*pyran-4-yl]carbamat wurden in 4 ml Dioxan gelöst, mit 1 ml konzentrierter Salzsäure versetzt und sofort am Rotationsverdampfer eingeengt. Der Rückstand wurde anschließend mittels präparativer HPLC (Reprosil C18, Wasser-Acetonitril-Gradient) gereinigt. Ausbeute: 15 mg (57% d. Th.)

LC-MS (Methode 2B): Rₜ = 0.97 min; m/z = 436 [M+H]⁺;

¹H-NMR (400 MHz, DMSO-*d*₆): δ = 8.38 (d, 1H), 7.86 (d, 2H), 7.52 (d, 1H), 7.50-7.43 (m, 2H), 7.09 (d, 1H), 7.05 (s, 1H), 6.99 (d, 1H), 4.02-3.86 (m, 1H), 3.77-3.66 (m, 2H), 3.63-3.46 (m, 2H), 2.81-2.58 (m, 3H), 2.20 (d, 7H), 2.08 (d, 6H), 1.85 (q, 1H), 1.61 (br. s., 1H), 1.46 (d, 1H).

### Beispiel 121

### N-{1-(Morpholin-4-ylcarbonyl)-5-[3-(propan-2-yl)phenyl]piperidin-3-yl}benzolcarboxamid [racemisches cis-Isomer]

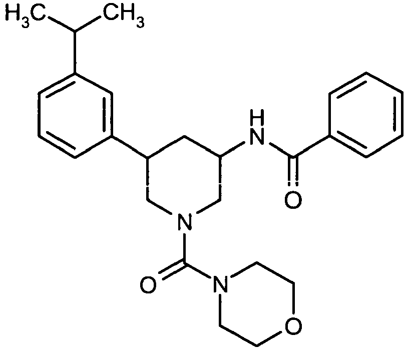

180 mg (0.54 mmol) *N*-{5-[3-(1-Methylethyl)phenyl]piperidin-3-yl}benzamid wurden in 4 ml Dichlormethan bei 0°C vorgelegt und mit 162 mg (1.08 mmol) Morpholin-4-carbonylchlorid und 114 µl (0.81 mmol) Triethylamin versetzt. Die Mischung wurde langsam über Nacht auf RT erwärmt. Es wurde Wasser zu dem Reaktionsgemisch gegeben und extrahiert. Die organische Phase wurde über Magnesiumsulfat getrocknet und im Vakuum eingeengt. Der Rückstand wurde anschließend mittels präparativer HPLC (Reprosil C18, Wasser-Acetonitril-Gradient) gereinigt. Ausbeute: 121 mg (51% d. Th.)

LC-MS (Methode 3B): Rₜ = 2.02 min; m/z = 436 [M+H]⁺;

¹H-NMR (500 MHz, DMSO-*d*₆): δ = 8.36 (d, 1H), 7.87 (d, 2H), 7.52 (d, 1H), 7.49 - 7.43 (m, 2H), 7.29-7.23 (m, 1H), 7.16 (s, 1H), 7.11 (dd, 2H), 4.06-3.94 (m, 1H), 3.86 (d, 1H), 3.66 (d, 1H), 3.58 (d, 4H), 3.20 (br. s., 4H), 2.92-2.83 (m, 2H), 2.76 (t, 1H), 2.67 (t, 1H), 2.10 (d, 1H), 1.83 (q, 1H), 1.21 (d, 6H).

### Beispiel 122

### N-[5-(2,3-Dimethylphenyl)-1-(morpholin-4-ylcarbonyl)piperidin-3-yl]benzolcarboxamid [racemisches cis-Isomer]

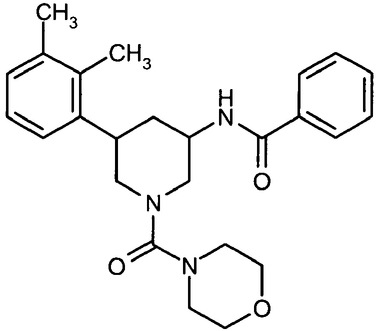

80 mg (0.26 mmol) *N*-[5-(2,3-Dimethylphenyl)piperidin-3-yl]benzamid wurden in 1.5 ml Dichlormethan bei 0°C vorgelegt und mit 78 mg (0.52 mmol) Morpholin-4-carbonylchlorid und 55 µl (0.39 mmol) Triethylamin versetzt. Die Mischung wurde langsam über Nacht auf RT erwärmt. Es wurde Wasser zu dem Reaktionsgemisch gegeben und extrahiert. Die organische Phase wurde über Magnesiumsulfat getrocknet und im Vakuum eingeengt. Der Rückstand wurde anschließend mittels präparativer HPLC (Reprosil C18, Wasser-Acetonitril-Gradient) gereinigt. Ausbeute: 75 mg (69% d. Th.)

LC-MS (Methode 2B): Rₜ = 1.17 min; m/z = 422 [M+H]⁺;

¹H-NMR (400 MHz, DMSO-*d*₆): δ = 8.38 (d, 1H), 7.87 (d, 2H), 7.55-7.51 (m, 1H), 7.47 (t, 2H), 7.12-6.98 (m, 3H), 4.12-3.96 (m, 1H), 3.85 (dd, 1H), 3.63 (d, 1H), 3.60-3.54 (m, 4H), 3.23-3.16 (m, 4H), 3.17-3.08 (m, 1H), 2.78-2.60 (m, 2H), 2.26 (s, 6H), 2.06-1.95 (m, 1H), 1.88 (q, 1H).

### Beispiel 123

### Ethyl-4-{1-(morpholin-4-ylcarbonyl)-5-[(phenylcarbonyl)amino]piperidin-3-yl}benzolcarboxylat [racemisches cis-Isomer]

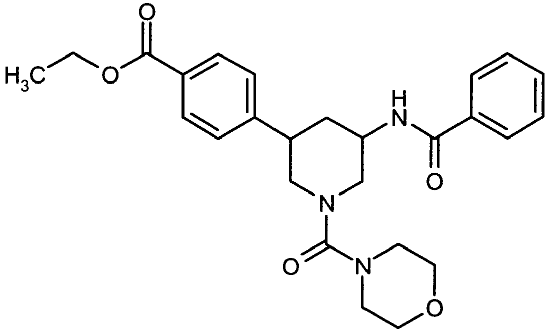

500 mg (1.42 mmol) Ethyl-4-{5-[(phenylcarbonyl)amino]piperidin-3-yl}benzoat wurden in 8 ml Dichlormethan bei 0°C vorgelegt und mit 424 mg (2.84 mmol) Morpholin-4-carbonylchlorid und 300 µl (2.13 mmol) Triethylamin versetzt. Die Mischung wurde langsam über Nacht auf RT erwärmt. Es wurde Wasser zu dem Reaktionsgemisch gegeben und extrahiert. Die organische Phase wurde über Magnesiumsulfat getrocknet und im Vakuum eingeengt. Der Rückstand wurde anschließend mittels präparativer HPLC (Reprosil C18, Wasser-Acetonitril-Gradient) gereinigt. Ausbeute: 551 mg (83% d. Th.)

LC-MS (Methode 2B): Rₜ = 1.12 min; m/z = 466 [M+H]⁺;

¹H-NMR (400 MHz, DMSO-*d*₆): δ = 8.41 (d, 1H), 7.94 (d, 2H), 7.86 (d, 2H), 7.57-7.50 (m, 1H), 7.47 (dd, 4H), 4.31 (q, 2H), 4.09-3.96 (m, 1H), 3.86 (d, 1H), 3.68 (d, 1H), 3.58 (d, 4H), 3.21 (br. s., 4H), 3.08-2.96 (m, 1H), 2.86-2.76 (m, 1H), 2.74-2.64 (m, 1H), 2.13 (d, 1H), 1.83 (q, 1H), 1.32 (t, 3H).

### Beispiel 124

### N-{1-(Morpholin-4-ylcarbonyl)-5-[3-(trifluormethyl)phenyl]piperidin-3-yl}benzolcarboxamid [racemisches cis-Isomer]

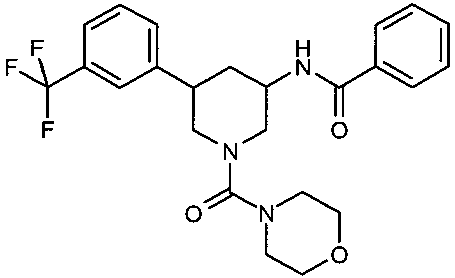

165 mg (0.47 mmol) *N*-{5-[3-(Trifluormethyl)phenyl]piperidin-3-yl}benzamid wurden in 17 ml Dichlormethan bei 0°C vorgelegt und mit 71 mg (0.47 mmol) Morpholin-4-carbonylchlorid und 132 µl (0.95 mmol) Triethylamin versetzt. Die Mischung wurde langsam über Nacht auf RT erwärmt. Es wurde Wasser zu dem Reaktionsgemisch gegeben und extrahiert. Die organische Phase wurde über Magnesiumsulfat getrocknet und im Vakuum eingeengt. Der Rückstand wurde anschließend mittels präparativer HPLC (Reprosil C18, Wasser-Acetonitril-Gradient) gereinigt. Ausbeute: 202 mg (92% d. Th.)

LC-MS (Methode 5B): Rₜ = 2.19 min; m/z = 462 [M+H]⁺;

¹H-NMR (400 MHz, DMSO-*d*₆): δ = 8.38 (d, 1H), 7.86 (d, 2H), 7.70-7.59 (m, 4H), 7.56-7.50 (m, 1H), 7.50-7.44 (m, 2H), 4.01 (dd, 1H), 3.86 (d, 1H), 3.74-3.52 (m, 5H), 3.21 (br. s., 4H), 3.11-3.02 (m, 1H), 2.84 (t, 1H), 2.75-2.63 (m, 1H), 2.13 (d, 1H), 1.86 (q, 1H).

### Beispiel 125

### N-{1-(Morpholin-4-ylcarbonyl)-5-[3-(trifluormethyl)phenyl]piperidin-3-yl}benzolcarboxamid [enantiomerenreines cis-Isomer]

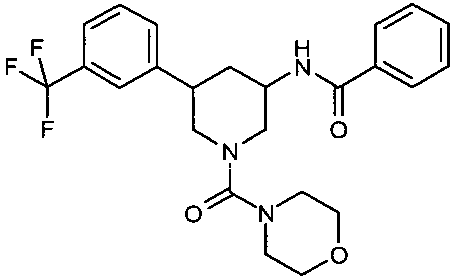

Die Enatiomerentrennung von 202 mg des racemischen cis-Isomerengemisches (Beispiel 124) nach Methode 13D ergab 66 mg der Verbindung aus Beispiel 125 (Enantiomer1).

LC-MS (Methode 1B): Rₜ = 2.31 min; m/z = 462 [M+H]⁺.

### Beispiel 126

### N-{1-(Morpholin-4-ylcarbonyl)-5-[4-(trifluormethyl)phenyl]piperidin-3-yl}benzolcarboxamid [racemisches cis-Isomer]

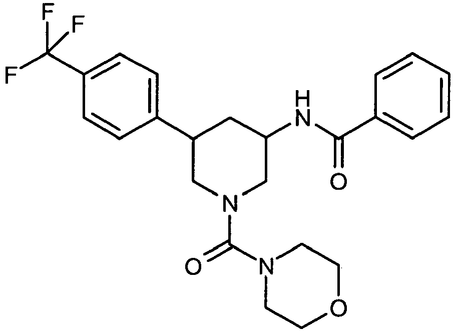

705 mg (2.02 mmol) *N*-{5-[4-(Trifluormethyl)phenyl]piperidin-3-yl}benzamid wurden in 70 ml Dichlormethan bei 0°C vorgelegt und mit 321 mg (2.02 mmol) Morpholin-4-carbonylchlorid und 564 µl (4.04 mmol) Triethylamin versetzt. Die Mischung wurde langsam über Nacht auf RT erwärmt. Es wurde Wasser zu dem Reaktionsgemisch gegeben und extrahiert. Die organische Phase wurde über Magnesiumsulfat getrocknet und im Vakuum eingeengt. Der Rückstand wurde anschließend mittels präparativer HPLC (Reprosil C18, Wasser-Acetonitril-Gradient) gereinigt. Ausbeute: 960 mg (100% d. Th.)

LC-MS (Methode 1B): Rₜ = 2.32 min; m/z = 462 [M+H]⁺;

¹H-NMR (400 MHz, DMSO-*d*₆): δ = 8.41 (d, 1H), 7.86 (d, 2H), 7.72 (d, 2H), 7.59-7.50 (m, 3H), 7.47 (t, 2H), 4.10-3.93 (m, 1H), 3.86 (d, 1H), 3.69 (d, 1H), 3.59 (t, 4H), 3.21 (br. s., 4H), 3.10-2.99 (m, 1H), 2.86-2.77 (m, 1H), 2.74-2.65 (m, 1H), 2.13 (d, 1H), 1.83 (q, 1H).

### Beispiel 127

### N-{1-(Morpholin-4-ylcarbonyl)-5-[4-(trifluormethyl)phenyl]piperidin-3-yl}benzolcarboxamid [enantiomerenreines cis-Isomer]

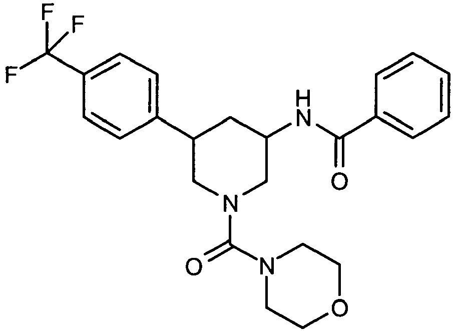

Die Enatiomerentrennung von 900 mg des racemischen cis-Isomerengemisches (Beispiel 126) nach Methode 14D ergab 388 mg der Verbindung aus Beispiel 127 (Enantiomer 1).

### Beispiel 128

### N-{1-(Morpholin-4-ylcarbonyl)-5-[4-(trifluormethyl)phenyl]piperidin-3-yl}benzolcarboxamid [enantiomerenreines cis-Isomer]

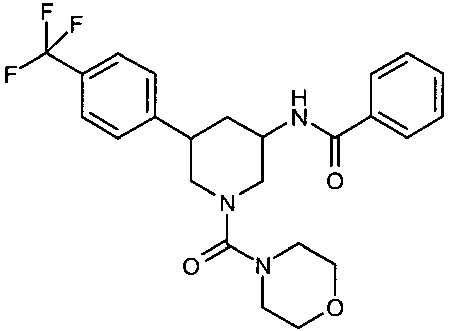

Die Enatiomerentrennung von 900 mg des racemischen cis-Isomerengemisches (Beispiel 126) nach Methode 14D ergab 361 mg der Verbindung aus Beispiel 128 (Enantiomer 2).

LC-MS (Methode 1B): Rₜ = 2.31 min; m/z = 462 [M+H]⁺.

### Beispiel 129

### N-{1-[(4-Hydroxypiperidin-1-yl)carbonyl]-5-[4-(trifluormethyl)phenyl]piperidin-3-yl}benzol-carboxamid [racemisches cis-Isomer]

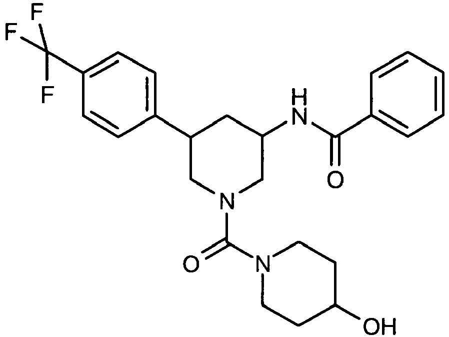

80 mg (0.16 mmol) 4-Nitrophenyl-3-[(phenylcarbonyl)amino]-5-[4-(trifluormethyl)phenyl]-piperidin-1-carboxylat wurden in 1.7 ml DMF vorgelegt und mit 47 mg (0.47 mmol) Piperidin-4-ol und 22 mg (0.16 mmol) Kaliumcarbonat versetzt. Die Mischung wurde bei 150°C 15 min in der Mikrowelle (Emrys Optimizer) umgesetzt. Die Aufreinigung des Rohproduktes erfolgt anschließend mittels präparativer HPLC (Reprosil C18, Wasser-Acetonitril-Gradient). Ausbeute: 62 mg (83% d. Th.)

LC-MS (Methode 3B): Rₜ = 1.79 min; m/z = 476 [M+H]⁺;

¹H-NMR (400 MHz, DMSO-*d*₆): δ = 8.39 (d, 1H), 7.86 (d, 2H), 7.72 (d, 2H), 7.58 - 7.50 (m, 3H), 7.50 - 7.40 (m, 2H), 4.68 (d, 1H), 4.11-3.95 (m, 1H), 3.79 (d, 1H), 3.63 (d, 2H), 3.49 (d, 2H), 3.05 (t, 1H), 2.97-2.85 (m, 2H), 2.78 (t, 1H), 2.73-2.61 (m, 1H), 2.13 (d, 1H), 1.82 (q, 1H), 1.73 (d, 2H), 1.38-1.27 (m, 2H).

### Beispiel 130

### N-{1-[(4-Hydroxypiperidin-1-yl)carbonyl]-5-[4-(trifluormethyl)phenyl]piperidin-3-yl}benzolcarboxamid [enantiomerenreines cis-Isomer]

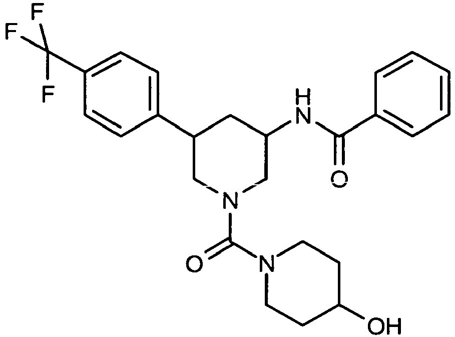

Die Enatiomerentrennung von 52 mg des racemischen cis-Isomerengemisches (Beispiel 129) nach Methode 15D ergab 19 mg der Verbindung aus Beispiel 130 (Enantiomer 1).

¹H-NMR (400 MHz, DMSO-*d*₆): δ = 8.39 (d, 1H), 7.85 (d, 2H), 7.72 (d, 2H), 7.58-7.50 (m, 3H), 7.49-7.35 (m, 2H), 4.68 (d, 1H), 4.11-3.96 (m, 1H), 3.77 (dd, 1H), 3.68-3.58 (m, 2H), 3.54-3.43 (m, 2H), 3.04 (t, 1H), 2.91 (dt, 2H), 2.78 (t, 1H), 2.72-2.62 (m, 1H), 2.21-2.05 (m, 1H), 1.82 (q, 1H), 1.76-1.67 (m, 2H), 1.39-1.26 (m, 2H).

### Beispiel 131

### N-{1-[(4-Hydroxypiperidin-1-yl)carbonyl]-5-[4-(trifluormethyl)phenyl]piperidin-3-yl}benzolcarboxamid [enantiomerenreines cis-Isomer]

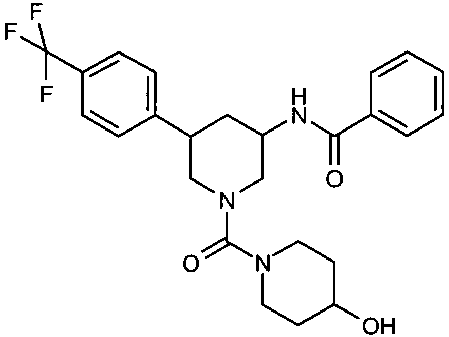

Die Enatiomerentrennung von 52 mg des racemischen cis-Isomerengemisches (Beispiel 129) nach Methode 15D ergab 20 mg der Verbindung aus Beispiel 131 (Enantiomer 2).

¹H-NMR (400 MHz, DMSO-*d*₆): δ = 8.39 (d, 1H), 7.85 (d, 2H), 7.72 (d, 2H), 7.58-7.50 (m, 3H), 7.49-7.35 (m, 2H), 4.68 (d, 1H), 4.11-3.96 (m, 1H), 3.77 (dd, 1H), 3.68-3.58 (m, 2H), 3.54-3.43 (m, 2H), 3.04 (t, 1H), 2.91 (dt, 2H), 2.78 (t, 1H), 2.72-2.62 (m, 1H), 2.21-2.05 (m, 1H), 1.82 (q, 1H), 1.76-1.67 (m, 2H), 1.39-1.26 (m, 2H).

### Beispiel 132

### N-{1-(Thiomorpholin-4-ylcarbonyl)-5-[4-(trifluormethyl)phenyl]piperidin-3-yl}benzolcarboxamid [racemisches cis-Isomer]

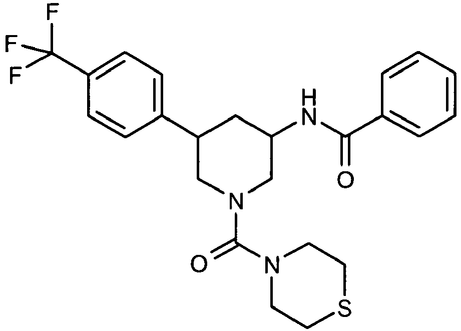

80 mg (0.16 mmol) 4-Nitrophenyl-3-[(phenylcarbonyl)amino]-5-[4-(trifluormethyl)phenyl]-piperidin-1-carboxylat wurden in 1.7 ml DMF vorgelegt und mit 48 mg (0.47 mmol) Thiomorpholin und 22 mg (0.16 mmol) Kaliumcarbonat versetzt. Die Mischung wurde bei 150°C 15 min in der Mikrowelle (Emrys Optimizer) umgesetzt. Die Aufreinigung des Rohproduktes erfolgt anschließend mittels präparativer HPLC (Reprosil C18, Wasser-Acetonitril-Gradient). Ausbeute: 45 mg (60% d. Th.)

LC-MS (Methode 3B): Rₜ = 2.10 min; m/z = 478 [M+H]⁺;

¹H-NMR (400 MHz, DMSO-*d*₆): δ = 8.40 (d, 1H), 7.86 (d, 2H), 7.72 (d, 2H), 7.59-7.50 (m, 3H), 7.50-7.43 (m, 2H), 4.09-3.95 (m, 1H), 3.79 (d, 1H), 3.64 (d, 1H), 3.46 (dt, 4H), 3.11-3.00 (m, 1H), 2.80 (t, 1H), 2.74-2.65 (m, 1H), 2.64-2.58 (m, 4H), 2.13 (d, 1H), 1.83 (q, 1H).

### Beispiel 133

### N-(1-{[(2R,5R)-2,5-Dimethylpyrrolidin-1-yl]carbonyl}-5-[4-(trifluormethyl)phenyl]piperidin-3-yl)benzolcarboxamid [racemisches cis-Isomer]

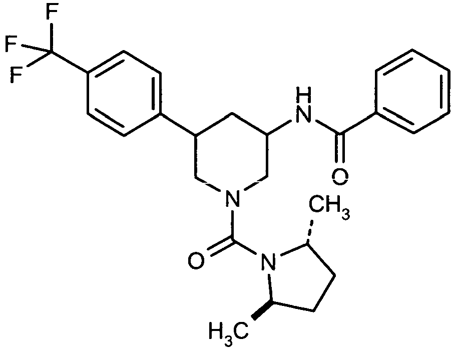

80 mg (0.16 mmol) 4-Nitrophenyl-3-[(phenylcarbonyl)amino]-5-[4-(trifluormethyl)phenyl]-piperidin-1-carboxylat wurden in 1.7 ml DMF vorgelegt und mit 46 mg (0.47 mmol) (*2R,5R*)-2,5-Dimethylpyrrolidin und 22 mg (0.16 mmol) Kaliumcarbonat versetzt. Die Mischung wurde bei 150°C 15 min in der Mikrowelle (Emrys Optimizer) umgesetzt. Die Aufreinigung des Rohproduktes erfolgte anschließend mittels präparativer HPLC (Reprosil C18, Wasser-Acetonitril-Gradient). Ausbeute: 12 mg (15% d. Th.)

LC-MS (Methode 2B): Rₜ = 0.73 min; m/z = 474 [M+H]⁺;

¹H-NMR (400 MHz, DMSO-*d*₆): δ = 8.38 (d, 1H), 7.89-7.81 (m, 2H), 7.71 (d, 2H), 7.59-7.51 (m, 3H), 7.49-7.43 (m, 2H), 4.16-3.93 (m, 3H), 3.92-3.61 (m, 2H), 3.13-2.57 (m, 3H), 2.19-1.99 (m, 2H), 1.92-1.76 (m, 2H), 1.64-1.38 (m, 2H), 1.20 (d, 3H), 1.07 (d, 3H).

### Beispiel 134

### N-{1-[(1,1-Dioxidothiomorpholin-4-yl)carbonyl]-5-[4-(trifluormethyl)phenyl]piperidin-3-yl}-benzolcarboxamid [racemisches cis-Isomer]

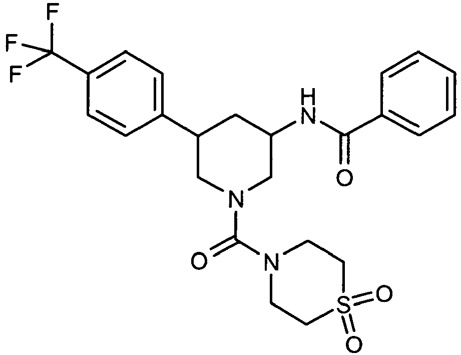

200 mg (0.39 mmol) 4-Nitrophenyl-3-[(phenylcarbonyl)amino]-5-[4-(trifluormethyl)phenyl]-piperidin-1-carboxylat wurden in 4.3 ml DMF vorgelegt und mit 158 mg (1.17 mmol) Thiomorpholin-1,1-dioxid und 54 mg (0.39 mmol) Kaliumcarbonat versetzt. Die Mischung wurde bei 150°C 15 min in der Mikrowelle (Emrys Optimizer) umgesetzt. Die Aufreinigung des Rohproduktes erfolgte anschließend mittels präparativer HPLC (Reprosil C18, Wasser-Acetonitril-Gradient). Ausbeute: 27 mg (14% d. Th.)

LC-MS (Methode 2B): Rₜ = 1.18 min; m/z = 510 [M+H]⁺;

¹H-NMR (400 MHz, DMSO-d₆): δ = 8.42 (d, 1H), 7.86 (d, 2H), 7.73 (d, 2H), 7.58-7.51 (m, 3H), 7.47 (t, 2H), 4.11-3.98 (m, 1H), 3.88 (d, 1H), 3.72 (d, 1H), 3.64 (br. s., 4H), 3.19 (br. s., 4H), 3.08 (t, 1H), 2.91-2.81 (m, 1H), 2.77-2.68 (m, 1H), 2.15 (d, 1H), 1.84 (q, 1H).

### Beispiel 135

### N-(2-Hydroxyethyl)-3-[(phenylcarbonyl)amino]-5-[4-(trifluormethyl)phenyl]piperidin-1-carboxamid [racemisches cis-Isomer]

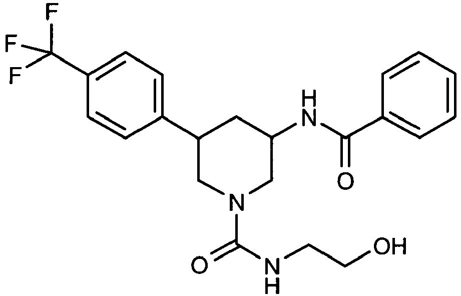

80 mg (0.16 mmol) 4-Nitrophenyl-3-[(phenylcarbonyl)amino]-5-[4-(trifluormethyl)phenyl]-piperidin-1-carboxylat wurden in 1.7 ml DMF vorgelegt und mit 29 mg (0.47 mmol) 2-Aminoethanol und 22 mg (0.16 mmol) Kaliumcarbonat versetzt. Die Mischung wurde bei 150°C 15 min in der Mikrowelle (Emrys Optimizer) umgesetzt. Die Aufreinigung des Rohproduktes erfolgte anschließend mittels präparativer HPLC (Reprosil C18, Wasser-Acetonitril-Gradient). Ausbeute: 10 mg (15% d. Th.)

LC-MS (Methode 1B): Rₜ = 2.12 min; m/z = 436 [M+H]⁺;

¹H-NMR (400 MHz, DMSO-*d*₆): δ = 8.39 (d, 1H), 7.85 (d, 2H), 7.72 (d, 2H), 7.59-7.50 (m, 3H), 7.47 (t, 2H), 6.64 (t, 1H), 4.60 (t, 1H), 4.20 (d, 1H), 4.08 (d, 1H), 4.03-3.91 (m, 1H), 3.40 (q, 2H), 3.11 (q, 2H), 2.96-2.84 (m, 1H), 2.77-2.68 (m, 1H), 2.68-2.58 (m, 1H), 2.10 (br. s., 1H), 1.76 (q, 1H).

### Beispiel 136

### N-{1-[(3-Hydroxyazetidin-1-yl)carbonyl]-5-[4-(trifluormethyl)phenyl]piperidin-3-yl}benzolcarboxamid [racemisches cis-Isomer]

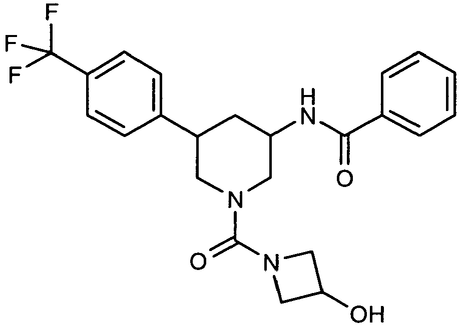

80 mg (0.16 mmol) 4-Nitrophenyl-3-[(phenylcarbonyl)amino]-5-[4-(trifluormethyl)phenyl]-piperidin-1-carboxylat wurden in 1.7 ml DMF vorgelegt und mit 51 mg (0.47 mmol) Azetidin-3-ol Hydrochlorid und 22 mg (0.16 mmol) Kaliumcarbonat versetzt. Die Mischung wurde bei 150°C 15 min in der Mikrowelle (Emrys Optimizer) umgesetzt. Die Aufreinigung des Rohproduktes erfolgte anschließend mittels präparativer HPLC (Reprosil C18, Wasser-Acetonitril-Gradient). Ausbeute: 44 mg (62% d. Th.)

LC-MS (Methode 3B): Rₜ = 1.74 min; m/z = 448 [M+H]⁺;

¹H-NMR (400 MHz, DMSO-*d*₆): δ = 8.38 (d, 1H), 7.86 (d, 2H), 7.72 (d, 2H), 7.59-7.50 (m, 3H), 7.47 (t, 2H), 5.58 (d, 1H), 4.45-4.35 (m, 1H), 4.18-4.05 (m, 2H), 3.97 (d, 2H), 3.87 (d, 1H), 3.77-3.62 (m, 2H), 2.93 (t, 1H), 2.83-2.62 (m, 2H), 2.10 (d, 1H), 1.84 (q, 1H).

### Beispiel 137

### N-{1-[(4-Cyanpiperidin-1-yl)carbonyl]-5-[4-(trifluormethyl)phenyl]piperidin-3-yl}benzol-carboxamid [racemisches cis-Isomer]

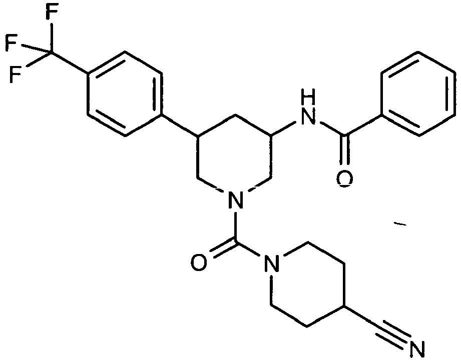

115 mg (0.22 mmol) 4-Nitrophenyl-3-[(phenylcarbonyl)amino]-5-[4-(trifluormethyl)phenyl]-piperidin-1-carboxylat wurden in 2.5 ml DMF vorgelegt und mit 74 mg (0.67 mmol) Piperidin-4-carbonitril und 31 mg (0.22 mmol) Kaliumcarbonat versetzt. Die Mischung wurde bei 150°C 15 min in der Mikrowelle (Emrys Optimizer) umgesetzt. Die Aufreinigung des Rohproduktes erfolgte anschließend mittels präparativer HPLC (Reprosil C18, Wasser-Acetonitril-Gradient). Ausbeute: 71 mg (65% d. Th.)

LC-MS (Methode 1B): Rₜ = 2.43 min; m/z = 485 [M+H]⁺;

¹H-NMR (400 MHz, DMSO-*d*₆): δ = 8.40 (d, 1H), 7.86 (d, 2H), 7.72 (d, 2H), 7.57-7.51 (m, 3H), 7.47 (t, 2H), 4.09-3.96 (m, 1H), 3.81 (d, 1H), 3.64 (d, 1H), 3.44-3.35 (m, 2H), 3.07 (m, 4H), 2.80 (t, 1H), 2.74-2.62 (m, 1H), 2.12 (br. s., 1H), 1.95-1.63 (m, 5H).

### Beispiel 138

### 3-[(Phenylcarbonyl)amino]-N-(pyridin-4-yl)-5-[4-(trifluormethyl)phenyl]piperidin-1-carboxamid [racemisches cis-Isomer]

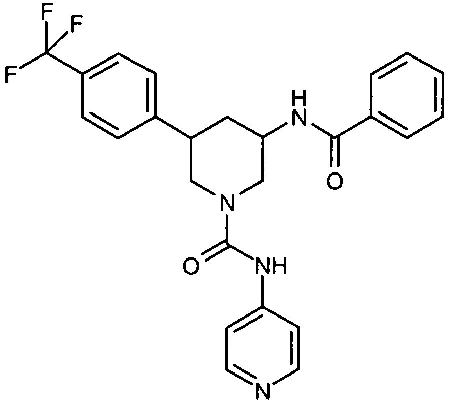

80 mg (0.23 mmol) Ethyl-*N-*{5-[4-(trifluormethyl)phenyl]piperidin-3-yl}benzamid wurden in 2 ml THF vorgelegt und mit 28 mg (0.23 mmol) 4-Isocyanatopyridin und 35 µl (0.25 mmol) Triethylamin versetzt. Die Mischung wurde über Nacht bei RT gerührt. Das Reaktionsgemisch wurde anschließend mittels präparativer HPLC (Reprosil C18, Wasser-Acetonitril-Gradient) gereinigt. Ausbeute: 75 mg (70% d. Th.)

LC-MS (Methode 1B): Rₜ = 1.64 min; m/z = 469 [M+H]⁺;

¹H-NMR (400 MHz, DMSO-*d*₆): δ = 9.07 (s, 1H), 8.47 (d, 1H), 8.32 (d, 2H), 7.87 (d, 2H), 7.74 (d, 2H), 7.59 (d, 2H), 7.54-7.42 (m, 5H), 4.47-4.22 (m, 2H), 4.16-3.98 (m, 1H), 3.11-2.90 (m, 2H), 2.80 (t, 1H), 2.16 (d, 1H), 1.84 (q, 1H).

### Beispiel 139

### N-(4-Hydroxycyclohexyl)-3-[(phenylcarbonyl)amino]-5-[4-(trifluomethyl)phenyl]piperidin-1-carboxamid [racemisches cis-Isomer]

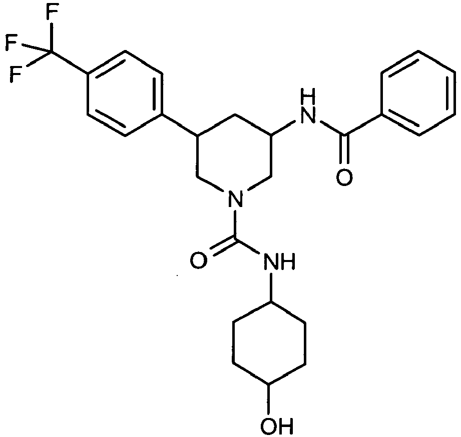

80 mg (0.16 mmol) 4-Nitrophenyl-3-[(phenylcarbonyl)amino]-5-[4-(trifluormethyl)phenyl]-piperidin-1-carboxylat wurden in 1.7 ml DMF vorgelegt und mit 71 mg (0.47 mmol) cis-4-Aminocyclohexanol Hydrochlorid und 129 mg (0.94 mmol) Kaliumcarbonat versetzt. Die Mischung wurde bei 150°C 15 min in der Mikrowelle (Emrys Optimizer) umgesetzt. Die Aufreinigung des Rohproduktes erfolgte anschließend mittels präparativer HPLC (Reprosil C18, Wasser-Acetonitril-Gradient). Ausbeute: 3 mg (4% d. Th.)

LC-MS (Methode 1B): Rₜ = 2.24 min; m/z = 490 [M+H]⁺;

¹H-NMR (500 MHz, CD₃OD): δ = 7.83 (d, 2H), 7.64 (d, 2H), 7.57-7.50 (m, 3H), 7.50-7.44 (m, 2H), 6.39 (d, 1H), 4.25 (dd, 4H), 4.17-4.08 (m, 2H), 3.87 (br. s., 2H), 3.65-3.58 (m, 2H), 3.02-2.94 (m, 2H), 2.84-2.74 (m, 4H), 2.28 (d, 2H), 1.87 (q, 2H).

### Beispiel 140

### N-{1-[(2,6-Dimethylmorpholin-4-yl)carbonyl]-5-[4-(trifluormethyl)phenyl]piperidin-3-yl}benzol-carboxamid[racemisches cis-Isomer]

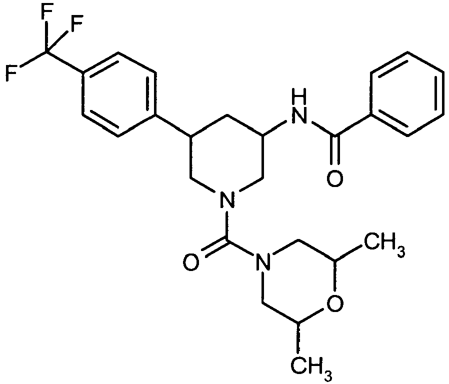

80 mg (0.16 mmol) 4-Nitrophenyl-3-[(phenylcarbonyl)amino]-5-[4-(trifluormethyl)phenyl]-piperidin-1-carboxylat wurden in 1.7 ml DMF vorgelegt und mit 53 mg (0.47 mmol) (*2R,6S*)-2,6-Dimethylmorpholin und 22 mg (0.16 mmol) Kaliumcarbonat versetzt. Die Mischung wurde bei 150°C 15 min in der Mikrowelle (Emrys Optimizer) umgesetzt. Die Aufreinigung des Rohproduktes erfolgte anschließend mittels präparativer HPLC (Reprosil C18, Wasser-Acetonitril-Gradient). Ausbeute: 49 mg (64% d. Th.)

LC-MS (Methode 3B): Rₜ = 1.80 min; m/z = 490 [M+H]⁺;

¹H-NMR (400 MHz, DMSO-*d*₆): δ = 8.41 (d, 1H), 7.86 (d, 2H), 7.72 (d, 2H), 7.58-7.50 (m, 3H), 7.47 (t, 2H), 4.13-3.94 (m, 1H), 3.83 (d, 1H), 3.67 (d, 1H), 3.59-3.44 (m, 4H), 3.11-3.00 (m, 1H), 2.80 (t, 1H), 2.69 (t, 1H), 2.48-2.42 (m, 2H), 2.13 (d, 1H), 1.86 (q, 1H), 1.12 (d, 3H), 1.08 (d, 3H).

### Beispiel 141

### N-{1-[(3-Oxopiperazin-1-yl)carbonyl]-5-[4-(trifluormethyl)phenyl]piperidin-3-yl}benzol-carboxamid [racemisches cis-Isomer]

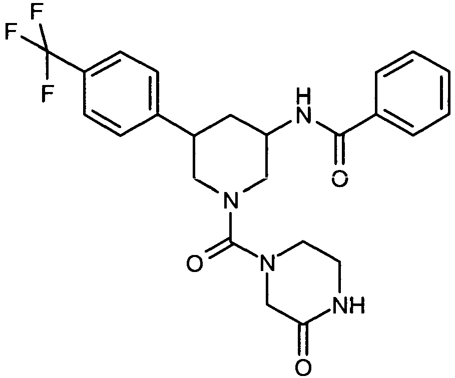

80 mg (0.16 mmol) 4-Nitrophenyl-3-[(phenylcarbonyl)amino]-5-[4-(trifluormethyl)phenyl]-piperidin-1-carboxylat wurden in 1.7 ml DMF vorgelegt und mit 47 mg (0.47 mmol) Piperazin-2-on und 22 mg (0.16 mmol) Kaliumcarbonat versetzt. Die Mischung wurde bei 150°C 15 min in der Mikrowelle (Emrys Optimizer) umgesetzt. Die Aufreinigung des Rohproduktes erfolgt anschließend mittels präparativer HPLC (Reprosil C18, Wasser-Acetonitril-Gradient). Ausbeute: 39 mg (52% d. Th.)

LC-MS (Methode 3B): Rₜ = 1.69 min; m/z = 475 [M+H]⁺;

¹H-NMR (400 MHz, DMSO-*d*₆): δ = 8.41 (d, 1H), 7.96 (br. s., 1H), 7.86 (d, 2H), 7.72 (d, 2H), 7.58-7.42 (m, 5H), 4.11-3.98 (m, 1H), 3.85 (d, 1H), 3.76 (s, 2H), 3.70 (d, 1H), 3.46-3.38 (m, 2H), 3.23 (br. s., 2H), 3.07 (t, 1H), 2.85 (t, 1H), 2.78-2.67 (m, 1H), 2.14 (d, 1H), 1.85 (q, 1H).

### Beispiel 142

### 3-[(Phenylcarbonyl)amino]-N-(tetrahydrofuran-2-ylmethyl)-5-[4-(trifluormethyl)phenyl]piperidin-1-carboxamid [racemisches cis-Isomer]

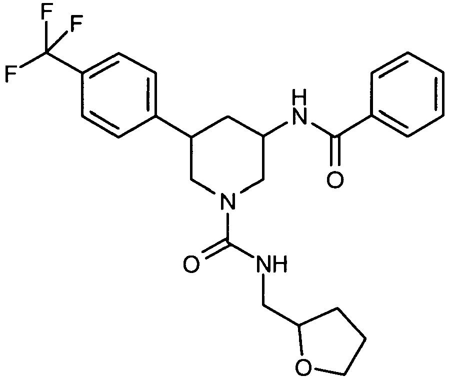

80 mg (0.23 mmol) Ethyl-*N*-{5-[4-(trifluormethyl)phenyl]piperidin-3-yl}benzamid wurden in 2 ml THF vorgelegt und mit 29 mg (0.23 mmol) 2-(Isocyanatomethyl)tetrahydrofuran und 35 µl (0.25 mmol) Triethylamin versetzt. Die Mischung wurde über Nacht bei RT gerührt. Das Reaktionsgemisch wurde anschließend mittels präparativer HPLC (Reprosil C18, Wasser-Acetonitril-Gradient) gereinigt. Ausbeute: 88 mg (81% d. Th.)

LC-MS (Methode 1B): Rₜ = 2.34 min; m/z = 476 [M+H]⁺;

¹H-NMR (400 MHz, DMSO-*d*₆): δ = 8.39 (d, 1H), 7.85 (d, 2H), 7.72 (d, 2H), 7.59-7.44 (m, 5H), 6.72 (q, 1H), 4.20 (d, 1H), 4.11 (d, 1H), 3.96 (br. s., 1H), 3.90-3.81 (m, 1H), 3.79-3.70 (m, 1H), 3.60 (q, 1H), 3.19-3.00 (m, 2H), 2.93-2.83 (m, 1H), 2.79-2.69 (m, 1H), 2.63 (t, 1H), 2.12 (d, 1H), 1.91-1.69 (m, 4H), 1.62-1.48 (m, 1H).

Die folgenden Beispiele wurden gemäß der Synthese von Beispiel 141 hergestellt:

| **Beispiel** | **Struktur** | **LC-MS Methode** | **Rₜ [min]** | **MS (ESIpos) m/z** |
|---|---|---|---|---|
| **143** | 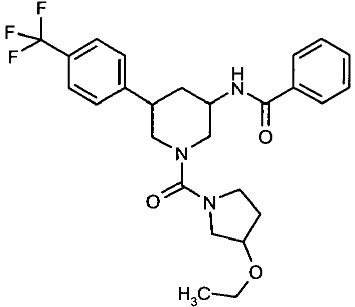 | 1B | 2.50 | 490 |
| **144** | 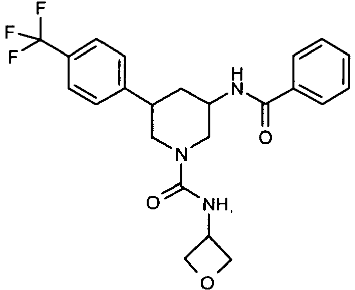 | 1B | 2.25 | 448 |
| **145** | 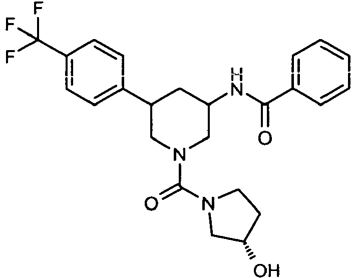 | 2B | 1.14 | 462 |
| **146** | 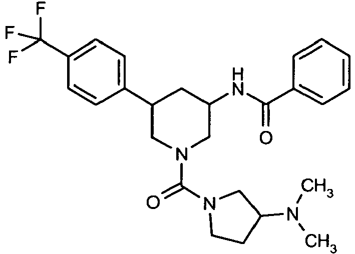 | 1B | 1.57 | 489 |
| **147** | 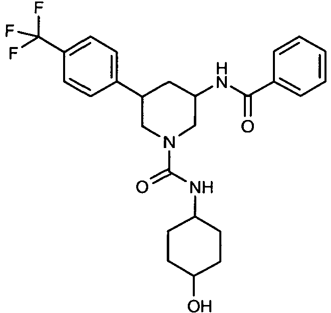 | 3B | 1.79 | 490 |
| **148** | 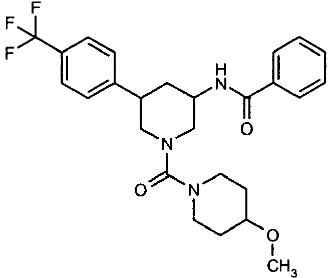 | 2B | 1.27 | 490 |
| **149** | 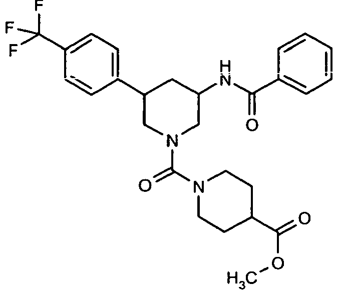 | 1B | 2.51 | 518 |

### Beispiel 150

### 1-({3-[(Phenylcarbonyl)amino]-5-[4-(trifluormethyl)phenyl]piperidin-1-yl}carbonyl)piperidin-4-carbonsäure [racemisches cis-Isomer]

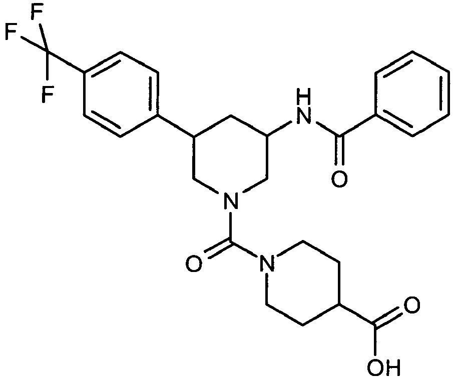

27 mg (0.05 mmol) Methyl-1-({3-[(phenylcarbonyl)amino]-5-[4-(trifluormethyl)phenyl]piperidin-1-yl}carbonyl)piperidin-4-carboxylat wurden in je 0.8 ml THF und Wasser vorgelegt und mit 4 mg (0.15 mmol) Lithiumhydroxid versetzt. Es wurde über Nacht bei RT gerührt. Die Reaktionslösung wurde im Vakuum eingeengt, angesäuert und der ausfallende Niederschlag abfiltriert und getrocknet. Ausbeute: 16 mg (62% d. Th.)

LC-MS (Methode 1B): Rₜ = 2.29 min; m/z = 504 [M+H]⁺;

¹H-NMR (400 MHz, DMSO-*d*₆): δ = 12.23 (s, 1H), 8.39 (d, 1H), 7.86 (d, 2H), 7.72 (d, 2H), 7.57-7.50 (m, 3H), 7.49-7.40 (m, 2H), 4.03 (dd, 1H), 3.80 (d, 1H), 3.71-3.52 (m, 3H), 3.10-2.98 (m, 1H), 2.94-2.62 (m, 4H), 2.47-2.35 (m, 1H), 2.11 (br. s., 1H), 1.86-1.77 (m, 3H), 1.49 (d, 2H).

### Beispiel 151

### 1-({3-[(Phenylcarbonyl)amino]-5-[4-(trifluormethyl)phenyl]piperidin-1-yl}carbonyl)piperidin-4-carboxamid [racemisches cis-Isomer]

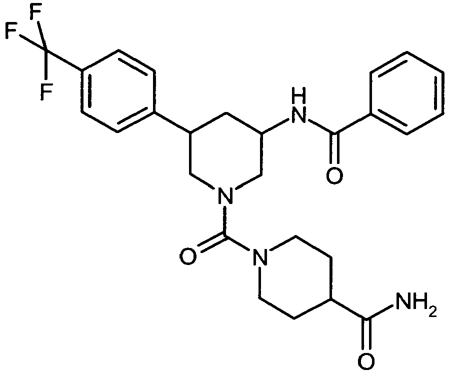

85 mg (0.17 mmol) 1-({3-[(Phenylcarbonyl)amino]-5-[4-(trifluormethyl)phenyl]piperidin-1-yl}carbonyl)piperidin-4-carbonsäure wurden zusammen mit 80 mg (0.21 mmol) HATU und 34 mg (0.28 mmol) 4-Dimethylaminopyridin in 1.3 ml DMF vorgelegt und mit 11 mg (0.14 mmol) Ammoniumacetat versetzt. Es wurde über Nacht bei RT gerührt und anschließend das Reaktionsgemisch mittels präparativer HPLC (Reprosil C18, Wasser-Acetonitril-Gradient) gereinigt. Ausbeute: 46 mg (53% d. Th.)

LC-MS (Methode 3B): Rₜ = 1.72 min; m/z = 503 [M+H]⁺;

¹H-NMR (400 MHz, DMSO-*d*₆): δ = 8.40 (d, 1H), 7.86 (d, 2H), 7.72 (d, 2H), 7.59-7.51 (m, 3H), 7.51-7.41 (m, 2H), 7.27 (s, 1H), 6.77 (s, 1H), 4.02 (d, 1H), 3.80 (d, 1H), 3.65 (br. s., 3H), 3.11-2.97 (m, 1H), 2.84-2.64 (m, 4H), 2.32-2.21 (m, 1H), 2.12 (br. s., 1H), 1.82 (q, 1H), 1.68 (br. s., 2H), 1.49 (br. s., 2H).

### Beispiel 152

### N,N-Dimethyl-1-({3-[(phenylcarbonyl)amino]-5-[4-(trifluormethyl)phenyl]piperidin-1-yl}-carbonyl)piperidin-4-carboxamid [racemisches cis-Isomer]

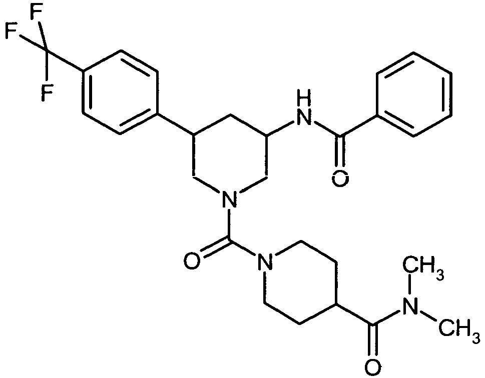

85 mg (0.17 mmol) 1-({3-[(Phenylcarbonyl)amino]-5-[4-(trifluormethyl)phenyl]piperidin-1-yl}carbonyl)piperidin-4-carbonsäure wurden zusammen mit 80 mg (0.21 mmol) HATU und 34 mg (0.28 mmol) 4-Dimethylaminopyridin in 1.3 ml DMF vorgelegt und mit 6 mg (0.14 mmol) *N-*Methylmethanamin versetzt. Es wurde über Nacht bei RT gerührt und anschließend das Reaktionsgemisch mittels präparativer HPLC (Reprosil C18, Wasser-Acetonitril-Gradient) gereinigt. Ausbeute: 62 mg (69% d. Th.)

LC-MS (Methode 3B): Rₜ = 1.86 min; m/z = 531 [M+H]⁺;

¹H-NMR (400 MHz, DMSO-*d*₆): δ = 8.40 (d, 1H), 7.85 (d, 2H), 7.72 (d, 2H), 7.58-7.42 (m, 5H), 4.10-3.99 (m, 1H), 3.78 (br. s., 1H), 3.72-3.59 (m, 3H), 3.09-2.98 (m, 4H), 2.90-2.75 (m, 7H), 2.73-2.63 (m, 1H), 2.12 (d, 1H), 1.82 (q, 1H), 1.61 (br. s., 2H), 1.48 (d, 2H).

### Beispiel 153

### N-{1-[(1-Cyancyclopropyl)carbonyl]-5-[4-(trifluormethyl)phenyl]piperidin-3-yl}benzol-carboxamid [racemisches cis-Isomer]

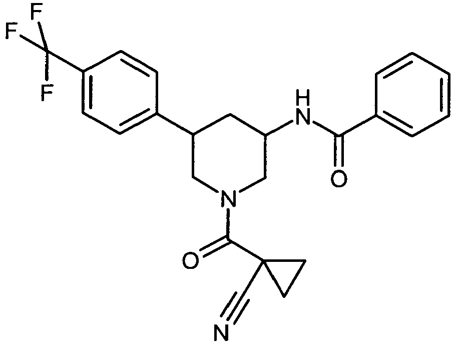

31 mg (0.28 mmol) 1-Cyanocyclopropancarbonsäure wurden zusammen mit 131 mg (0.34 mmol) HATU und 56 mg (0.46 mmol) 4-Dimethylaminopyridin in 2 ml DMF vorgelegt und mit 80 mg (0.23 mmol) *N*-{5-[4-(Trifluormethyl)phenyl]piperidin-3-yl}benzamid versetzt. Es wurde über Nacht bei RT gerührt und anschließend das Reaktionsgemisch mittels präparativer HPLC (Reprosil C18, Wasser-Acetonitril-Gradient) gereinigt. Ausbeute: 73 mg (72% d. Th.)

LC-MS (Methode 2B): Rₜ = 1.25 min; m/z = 442 [M+H]⁺;

¹H-NMR (400 MHz, DMSO-*d*₆): δ = 8.50 (d, 1H), 7.88 (d, 2H), 7.76 (d, 2H), 7.65-7.43 (m, 5H), 4.53 (d, 1H), 4.31 (d, 1H), 4.05 (br. s., 1H), 3.26-3.09 (m, 2H), 2.18 (d, 1H), 1.97 (q, 1H), 1.64 (d, 5H).

### Beispiel 154

### N-{1-[(3-Methylpyridin-4-yl)carbonyl]-5-[4-(trifluormethyl)phenyl]piperidin-3-yl}benzol-carboxamid [racemisches cis-Isomer]

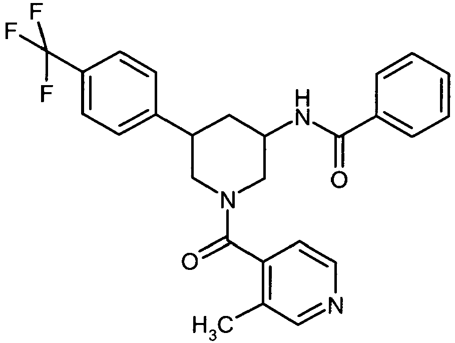

38 mg (0.28 mmol) 3-Methylpyridin-4-carbonsäure wurden zusammen mit 131 mg (0.34 mmol) HATU und 56 mg (0.46 mmol) 4-Dimethylaminopyridin in 2 ml DMF vorgelegt und mit 80 mg (0.23 mmol) *N*-{5-[4-(Trifluormethyl)phenyl]piperidin-3-yl}benzamid versetzt. Es wurde über Nacht bei RT gerührt und anschließend das Reaktionsgemisch mittels präparativer HPLC (Reprosil C18, Wasser-Acetonitril-Gradient) gereinigt. Ausbeute: 65 mg (59% d. Th.)

LC-MS (Methode 1B): Rₜ = 2.14 min; m/z = 468 [M+H]⁺.

### Beispiel 155

### N-{1-[(4-Cyanpiperidin-1-yl)carbonyl]-5-(4-ethylphenyl)piperidin-3-yl}pyridin-4-carboxamid [racemisches cis-Isomer]

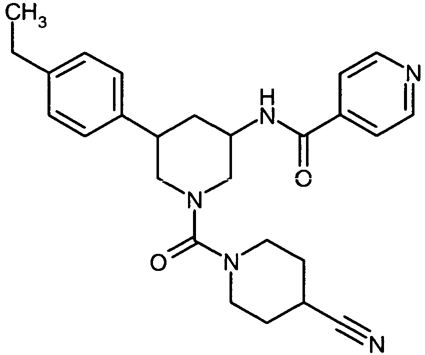

Nach der Allgemeinen Methode 1 wurden 70 mg (0.21 mmol) der Verbindung aus Beispiel 56A mit 28 mg (0.23 mmol) Isonicotinsäure umgesetzt. Abweichend von der Allgemeinen Methode 1 wurden nochmal Isonicotinsäure, HATU sowie *N,N*-Diisopropylamin zugegeben. Nach insgesamt zwei Tagen Reaktionszeit wurde, wie in der Allgemeinen Methode 1 beschrieben, aufgearbeitet. Ausbeute: 42 mg (46% d. Th.)

HPLC (Methode 2A): Rₜ = 3.94 min; m/z = 446 [M+H]⁺;

¹H-NMR (400 MHz, DMSO-d₆): δ = 8.72 (d, 2H), 8.67 (d, 1H), 7.76 (d, 2H), 7.21-7.19 (m, 3H), 4.05-3.95 (m, 1H), 3.80 (br d, 1H), 3.60 (br d, 1H), 3.37 (q, 2H), 3.12-3.02 (m, 3H), 2.90-2.82 (m, 1H), 2.77-2.60 (m, 3H), 2.37 (q, 2H), 2.08 (br d, 1H), 1.92-1.85 (m, 2H), 1.77 (q, 1H), 1.72-1.62 (m, 2H), 1.17 (t, 3H).

### Beispiel 156

### N-{1-[(4-Cyanpiperidin-1-yl)carbonyl]-5-(4-ethylphenyl)piperidin-3-yl}-3-methoxybenzol-carboxamid [racemisches cis-Isomer]

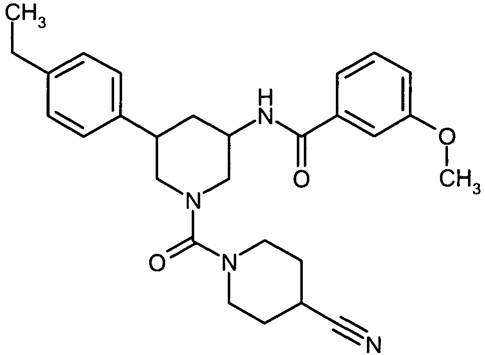

Nach der Allgemeinen Methode 2 wurden 70 mg (0.21 mmol) der Verbindung aus Beispiel 56A mit 39 mg (0.23 mmol) 3-Methoxybenzoylchlorid in Dichlormethan umgesetzt. Ausbeute: 87 mg (89% d. Th.)

HPLC (Methode 2A): Rₜ = 4.48 min; m/z = 475 [M+H]⁺;

¹H-NMR (400 MHz, DMSO-*d*₆): δ = 8.34 (d, 1H), 7.47 - 7.42 (m, 1H), 7.42 - 7.34 (m, 2H), 7.23 - 7.15 (m, 4H), 7.09 (dd, 1H), 4.05 - 3.92 (m, 1H), 3.83 - 3.75 (m, 1H), 3.80 (s, 3H), 3.62 (br d, 1H), 3.38 (br d, 2H), 3.13 - 3.01 (m, 3H), 2.91 - 2.81 (m, 1H), 2.71 (s, 2H), 2.58 (q, 2H), 2.07 (br d, 1H), 1.93 - 1.84 (m, 2H), 1.78 (q, 1H), 1.74 - 1.63 (m, 2H), 1.17 (t, 3H).

### Beispiel 157

### N-{1-[(4-Cyanpiperidin-1-yl)carbonyl]-5-(4-ethylphenyl)piperidin-3-yl}-3-fluorbenzolcarboxamid [racemisches cis-Isomer]

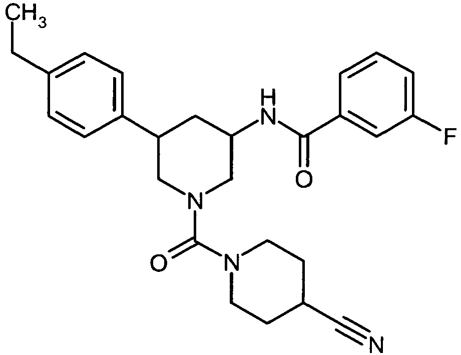

Nach der Allgemeinen Methode 2 wurden 70 mg (0.21 mmol) der Verbindung aus Beispiel 56A mit 36 mg (0.23 mmol) 3-Fluorbenzoylchlorid in Dichlormethan umgesetzt. Ausbeute: 62 mg (66% d. Th.)

HPLC (Methode 2A): Rₜ = 4.49 min; m/z = 463 [M+H]⁺;

¹H-NMR (400 MHz, DMSO-*d*₆): δ = 8.46 (d, 1H), 7.74 - 7.64 (m, 2H), 7.53 (td, 1H), 7.42 - 7.36 (m, 1H), 7.22 - 7.15 (m, 4H), 3.99 (td, 1H), 3.83 - 3.76 (m, 1H), 3.60 (br d, 1H), 3.42 - 3.33 (m, 2H), 3.12 - 3.01 (m, 3H), 2.91 - 2.82 (m, 1H), 2.72-2.69 (m, 2H), 2.56 (q, 2H), 2.07 (br d, 1H), 1.92 - 1.63 (m, 5H), 1.17 (t, 3H).

### Beispiel 158

### N-{1-[(4-Cyanpiperidin-1-yl)carbonyl]-5-(4-ethylphenyl)piperidin-3-yl}-3-methylbenzol-carboxamid [racemisches cis-Isomer]

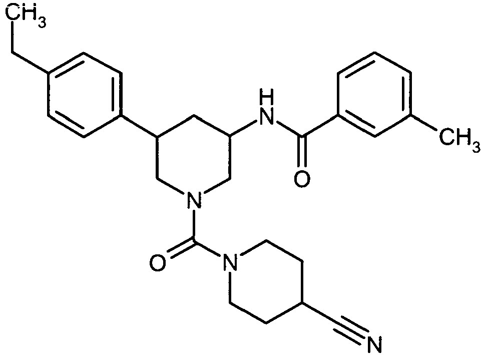

Nach der Allgemeinen Methode 2 wurden 70 mg (0.21 mmol) der Verbindung aus Beispiel 56A mit 35 mg (0.23 mmol) 3-Methylbenzolcarbonylchlorid in Dichlormethan umgesetzt. Ausbeute: 85 mg (91% d. Th.)

HPLC (Methode 2A): Rₜ = 4.56 min; m/z = 459 [M+H]⁺;

¹H-NMR (400 MHz, DMSO-*d*₆): δ = 8.32 (d, 1H), 7.79 - 7.50 (m, 2H), 7.37 - 7.32 (m, 2H), 7.23 - 7.14 (m, 4H), 3.98 (dd, 1H), 3.83 - 3.76 (m, 1H), 3.61 (br d, 1H), 3.42 - 3.34 (m, 2H), 3.12 - 3.01 (m, 3H), 2.90 - 2.82 (m, 1H), 2.71-2.68 (m, 2H), 2.58 (q, 2H), 2.07 (br d, 1H), 1.92 - 1.63 (m, 5H), 1.17 (t, 3H).

### Beispiel 159

### 3-Cyan-N-{1-(morpholin-4-ylcarbonyl)-5-[4-(trifluormethyl)phenyl]piperidin-3-yl}benzolcarboxamid [racemisches cis-Isomer]

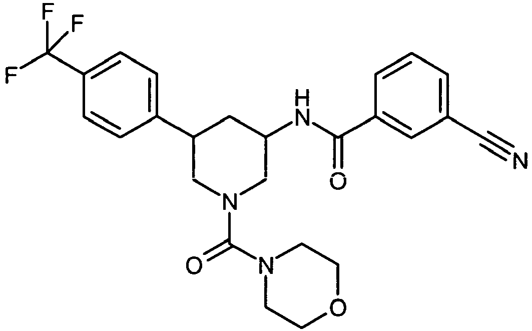

Nach der Allgemeinen Methode 8 wurden 200 mg (0.508 mmol) Amin aus Beispiel 57A [racemisches cis-Isomer] und 112 mg (0.762 mmol) 3-Cyanbenzoesäure umgesetzt. Ausbeute: 206 mg (80% d. Th.)

LC-MS (Methode 2B): Rₜ = 1.18 min; MS (ESIpos): m/z = 487 [M+H]⁺;

¹H NMR (400 MHz, DMSO-d₆): δ = 8.63 (d, 1H), 8.30 (s, 1H), 8.17 (d, 1H), 8.02 (d, 1H), 7.63-7.79 (m, 3H), 7.55 (d, 2H), 3.94-4.10 (m, 1H), 3.87 (d, 1H), 3.68 (d, 1H), 3.59 (br. s., 4H), 3.21 (br. s., 4H), 3.06 (t, 1H), 2.79-2.89 (m, 1H), 2.70 (t, 1H), 2.16 (d, 1H), 1.81 (q, 1H).

Analog wurden folgende Verbindungen [racemische cis-Isomere] hergestellt:

| **Beispiel** | **Struktur** | **LC-MS Methode** | **Rₜ [min]** | **MS (ESIpos) m/z** |
|---|---|---|---|---|
| **160** | 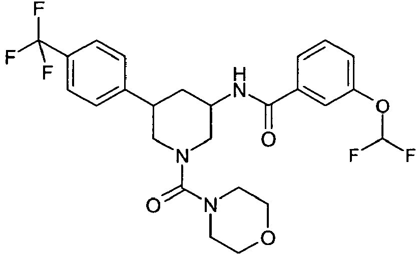 | 2B | 1.29 | 528 |
| **161** | 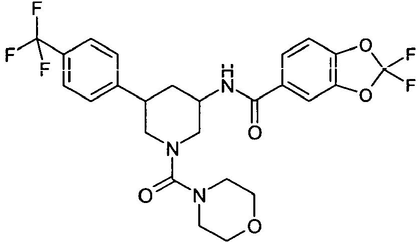 | 2B | 1.35 | 542 |
| **162** | 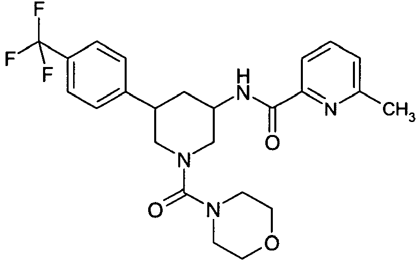 | 11B | 1.10 | 477 |
| **163** | 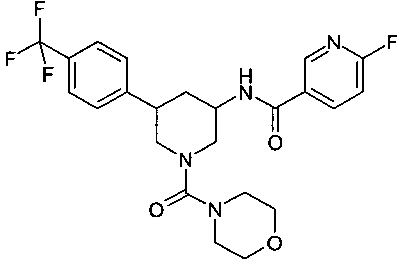 | 2B | 1.13 | 481 |
| **164** | 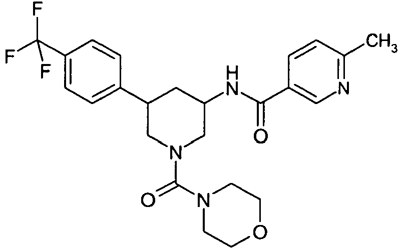 | 2B | 1.03 | 477 |
| **165** | 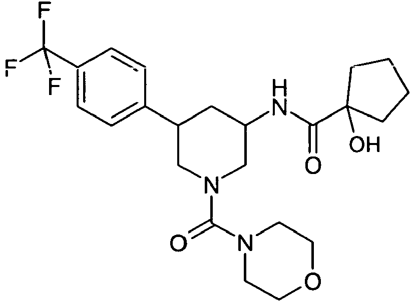 | 2B | 1.11 | 470 |

### Beispiel 166

### N-{1-(Morpholin-4-ylcarbonyl)-5-[4-(trifluormethyl)phenyl]piperidin-3-yl}cyclopentan-carboxamid [racemisches cis-Isomer]

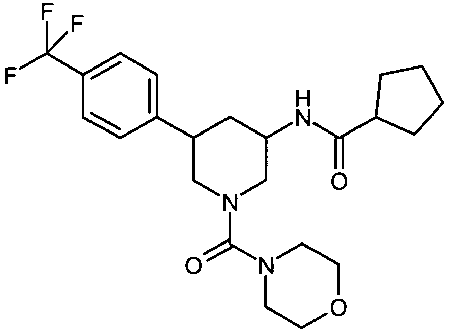

Eine Lösung der Verbindung aus Beispiel 57A (200 mg, 0.508 mmol) in Dichlormethan (12 ml) wurde mit 212 µl Triethylamin (1.52 mmol) und 18.6 mg DMAP (0.152 mmol) versetzt und anschließend bei 0°C 93 µl Cyclopentancarbonsäurechlorid (0.762 mmol) zugegeben. Die Reaktionsmischung wurde auf RT erwärmt und über Nacht gerührt. Die Reaktionslösung wurde mit wässriger 1 N Salzsäure gewaschen und die organische Phase über Magnesiumsulfat getrocknet, filtriert und im Vakuum eingeengt. Das Rohprodukt wurde über präparative HPLC (RP18-Säule; Acetonitril-Wasser-Gradient) gereinigt. Ausbeute: 133 mg (57% d. Th.)

LC-MS (Methode 5B): Rₜ = 2.17 min; MS (ESIpos): m/z = 454 [M+H]⁺;

¹H-NMR (400 MHz, DMSO-d₆): δ = 7.80 (d, 1H), 7.70 (d, 2H), 7.52 (d, 2H), 3.74 (d, 2H), 3.63 (d, 1H), 3.56 (br. s., 4H), 3.17 (br. s., 4H), 2.97 (br. s., 1H), 2.84-2.73 (m, 1H), 2.02 (d, 1H), 1.78-1.66 (m, 2H), 1.54-1.66 (m, 5H), 1.48 (br. s., 2H).

### Beispiel 167

### 2,2-Dimethyl-N-{1-(morpholin-4-ylcarbonyl)-5-[4-(trifluormethyl)phenyl]piperidin-3-yl}propanamid [racemisches cis-Isomer]

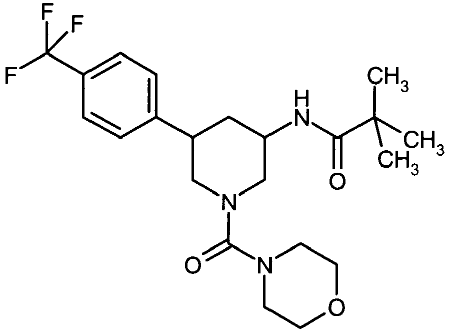

Eine Lösung der Verbindung aus Beispiel 57A (200 mg, 0.508 mmol) in Dichlormethan (12 ml) wurde mit 212 µl Triethylamin (1.52 mmol) und 18.6 mg DMAP (0.152 mmol) versetzt und anschließend bei 0°C 94 µl Pivalinsäurechlorid (0.76 mmol) zugegeben. Die Reaktionsmischung wurde auf RT erwärmt und über Nacht gerührt. Die Reaktionslösung wurde mit wässriger 1 N Salzsäure gewaschen und die organische Phase über Magnesiumsulfat getrocknet, filtriert und im Vakuum eingeengt. Das Rohprodukt wurde über präparative HPLC (RP18-Säule; Acetonitril-Wasser-Gradient) gereinigt. Ausbeute: 154 mg (68% d. Th.)

LC-MS (Methode 5B): Rₜ = 2.17 min; MS (ESIpos): m/z = 442 [M+H]⁺;

¹H-NMR (400 MHz, DMSO-d₆): δ = 7.70 (d, 2H), 7.52 (d, 2H), 7.31 (d, 1H), 3.85-3.72 (m, 1H), 3.71-3.61 (m, 2H), 3.56 (d, 4H), 3.18 (br. s., 4H), 3.04-2.92 (m, 1H), 2.76 (t, 1H), 1.97 (d, 1H), 1.73 (q, 1H), 1.09 (s, 9H).

### Beispiel 168

### 2-Methyl-N-{1-(morpholin-4-ylcarbonyl)-5-[4-(trifluormethyl)phenyl]piperidin-3-yl}propanamid [racemisches cis-Isomer]

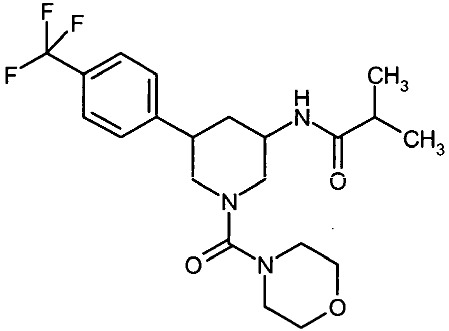

Eine Lösung der Verbindung aus Beispiel 57A (200 mg, 0.508 mmol) in Dichlormethan (12 ml) wurde mit 212 µl Triethylamin (1.52 mmol) und 18.6 mg DMAP (0.152 mmol) versetzt und anschließend bei 0°C 80 µl Isobuttersäurechlorid (0.76 mmol) zugegeben. Die Reaktionsmischung wurde auf RT erwärmt und über Nacht gerührt. Die Reaktionslösung wurde mit wässriger 1 N Salzsäure gewaschen und die organische Phase über Magnesiumsulfat getrocknet, filtriert und im Vakuum eingeengt. Das Rohprodukt wurde über präparative HPLC (RP18-Säule; Acetonitril-Wasser-Gradient) gereinigt. Ausbeute: 142 mg (65% d. Th.)

LC-MS (Methode 5B): Rₜ = 2.03 min; MS (ESIpos): m/z = 428 [M+H]⁺;

¹H-NMR (400 MHz, DMSO-d₆): δ = 7.76 (d, 1H), 7.70 (d, 2H), 7.52 (d, 2H), 3.74 (d, 2H), 3.63 (d, 1H), 3.56 (t, 4H), 3.18 (d, 4H), 2.92-3.03 (m, 1H), 2.74-2.84 (m, 1H), 2.29-2.39 (m, 1H), 2.01 (d, 1H), 1.60 (q, 1H), 1.00 (t, 7H).

### Beispiel 169

### 3-Chlor-N-{1-[(4-hydroxypiperidin-1-yl)carbonyl]-5-[4-(trifluormethyl)phenyl]piperidin-3-yl}benzol-carboxamid [racemisches cis-Isomer]

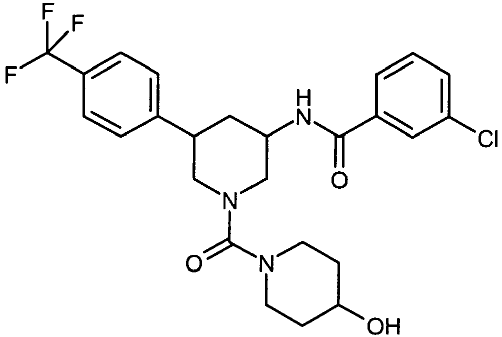

Eine Lösung des Ketons aus Beispiel 174 (60 mg, 0.118 mmol) in Methanol (2.4 ml) wurde bei 0°C mit 7.2 mg Natriumborhydrid (0.19 mmol) versetzt. Die Reaktionsmischung wurde auf RT erwärmt und für 1 h gerührt. Die Reaktionslösung wurde im Vakuum eingeengt, in Wasser aufgenommen und mit Dichlormethan extrahiert. Die organische Phase wurde über Magnesiumsulfat getrocknet, filtriert und im Vakuum eingeengt. Ausbeute: 52 mg (85% d. Th.)

LC-MS (Methode 2B): Rₜ = 1.24 min; MS (ESIpos): m/z = 510 [M+H]⁺;

¹H-NMR (400 MHz, DMSO-d₆) δ = 8.52 (d, 1H), 7.91 (s, 1H), 7.82 (d, 1H), 7.72 (d, 2H), 7.58-7.64 (m, 1H), 7.48-7.57 (m, 3H), 4.68 (d, 1H), 3.96-4.09 (m, 1H), 3.78 (d, 1H), 3.62 (d, 2H), 3.44-3.54 (m, 2H), 2.97-3.09 (m, 1H), 2.86-2.96 (m, 2H), 2.74-2.84 (m, 1H), 2.63-2.73 (m, 1H), 2.13 (d, 1H), 1.66-1.88 (m, 3H), 1.32 (q, 2H).

### Beispiel 170

### N-{1-[(4-Hydroxypiperidin-1-yl)carbonyl]-5-[4-(trifluormethyl)phenyl]piperidin-3-yl}cyclopentan-carboxamid [racemisches cis-Isomer]

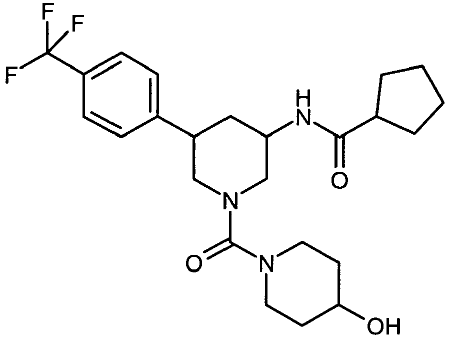

Eine Lösung des Ketons aus Beispiel 173 (52.6 mg, 0.113 mmol) in Methanol (3.0 ml) wurde bei 0°C mit 6.8 mg Natriumborhydrid (0.18 mmol) versetzt. Die Reaktionsmischung wurde auf RT erwärmt und für 2.5 h gerührt. Die Reaktionslösung wurde im Vakuum eingeengt, in Wasser aufgenommen und mit Dichlormethan extrahiert. Die organische Phase wurde über Magnesiumsulfat getrocknet, filtriert und im Vakuum eingeengt. Ausbeute: 46.6 mg (91% d. Th.)

LC-MS (Methode 2B): Rₜ = 1.16 min; MS (ESIpos): m/z = 468 [M+H]⁺;

¹H-NMR (400 MHz, DMSO-d₆): δ = 7.78 (d, 1H), 7.70 (d, 2H), 7.51 (d, 2H), 4.67 (d, 1H), 3.64-3.81 (m, 2H), 3.53-3.64 (m, 2H), 3.46 (d, 2H), 2.81-3.04 (m, 3H), 2.69-2.79 (m, 1H), 2.01 (d, 1H), 1.71 (d, 4H), 1.55-1.66 (m, 5H), 1.43-1.54 (m, 2H), 1.23-1.35 (m, 2H).

### Beispiel 171

### 1-[1-(Cyclopentylcarbonyl)-5-(4-ethylphenyl)piperidin-3-yl]-3-phenylharnstoff [racemisches cis-Isomer]

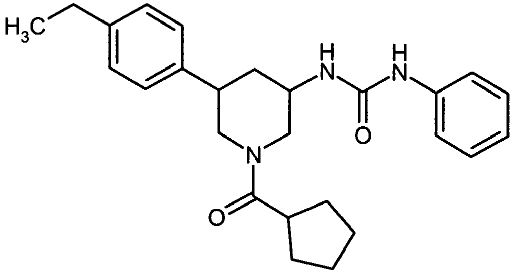

Nach der Allgemeinen Methode 4 wurden 108 mg (0.22 mmol) 1-(Cyclopentylcarbonyl)-5-(4-ethylphenyl)piperidin-3-amin-Trifluoracetat (Beispiel 8A) und 31 mg (0.26 mmol, 1.2 eq.) Phenylisocyanat umgesetzt. Ausbeute: 72 mg (79% d. Th.)

HPLC (Methode 1): Rₜ = 4.79 min; MS (ESIpos): m/z = 420 [M+H]⁺;

¹H-NMR (500 MHz, DMSO-d₆): δ = 8.45 (s, 0.6H), 8.41 (s, 0.4H), 7.38 (d, 2H), 7.28-7.13 (m, 6H), 6.95-6.87 (m, 1H), 6.25 (d, 0.6H), 6.19 (d, 0.4H), 4.66 (br d, 0.4H), 4.45 (br d, 0.6H), 4.22 (br d, 0.6H), 3.93 (br d, 0.4H), 3.66-3.49 (m, 1H), 3.08-2.97 (m, 1.4H), 2.83-2.72 (m, 1H), 2.69-2.61 (m, 0.6H), 2.58 (q, 2H), 2.33 (t, 0.6H), 2.10-2.00 (m, 1.4H), 1.85-1.44 (m, 9H), 1.17 (t, 3H).

### Beispiel 172

### tert.-Butyl-{1-(morpholin-4-ylcarbonyl)-5-[4-(trifluormethyl)phenyl]piperidin-3-yl}carbamat [racemisches cis-Isomer]

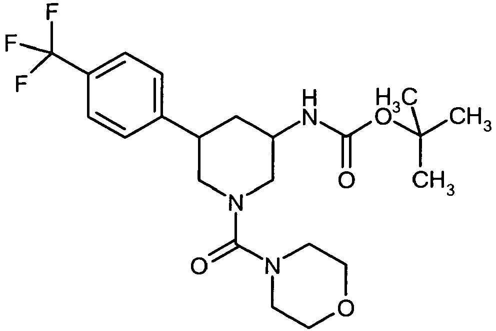

Die Carbonsäure aus Beispiel 55A (5.00 g, 12.9 mmol) in *tert*.-Butanol (235 ml) wurde mit aktiviertem Molsieb 4 Å (ca. 10 g), 2.16 ml (15.5 mmol) Triethylamin und 3.92 g (14.2 mmol) Diphenylphosphorazidat versetzt und unter Rückfluss über Nacht gerührt. Die Reaktionslösung wurde abgekühlt und anschließend das Molsieb abfiltriert und gründlich mit Essigsäureethylester gewaschen. Die Lösungsmittel wurden im Vakuum entfernt und der Rückstand in Essigsäureethylester aufgenommen. Nach Waschen mit 2 N wässriger Hydrogenchlorid-Lösung, gesättigter, wässriger Natriumhydrogencarbonat-Lösung und Wasser wurde die organische Phase über Magnesiumsulfat getrocknet, filtriert und im Vakuum eingeengt. Das Rohprodukt wurde ohne weitere Reinigung in der Folgestufe eingesetzt. Ausbeute: 5.53 g (89% d. Th.)

LC-MS (Methode 11B): Rₜ = 1.15 min; MS (ESIpos): m/z = 458 [M+H]⁺.

### Beispiel 173

### N-{1-[(4-Oxopiperidin-1-yl)carbonyl]-5-[4-(trifluormethyl)phenyl]piperidin-3-yl}cyclopentan-carboxamid [racemisches cis-Isomer]

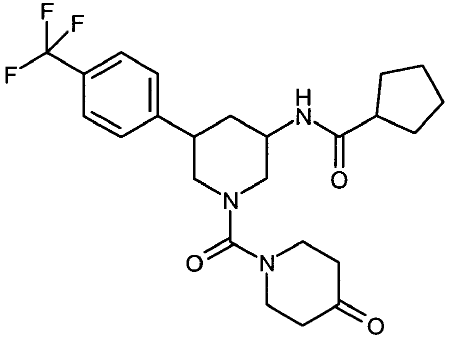

Eine Lösung des Acetals aus Beispiel 63A (97.2 mg, 0.191 mmol) in 5.5 ml Aceton/Wasser (10:1) wurde mit 14.3 mg Pyridinium-*p*-toluolsulfonat (0.057 mmol) versetzt und über Nacht unter Rückfluss gerührt. Es wurden erneut 14.3 mg Pyridinium-*p*-toluolsulfonat (0.057 mmol) zugegeben und eine weitere Nacht unter Rückfluss gerührt. Anschließend wurde der Ansatz abgekühlt und der entstandene Niederschlag abfiltriert. Ausbeute: 66.2 mg (74% d. Th.)

LC-MS (Methode 11B): Rₜ = 1.05 min; MS (ESIpos): m/z = 466 [M+H]⁺.

### Beispiel 174

### 3-Chlor-N-{1-[(4-oxopiperidin-1-yl)carbonyl]-5-[4-(trifluormethyl)phenyl]piperidin-3-yl}benzol-carboxamid [racemisches cis-Isomer]

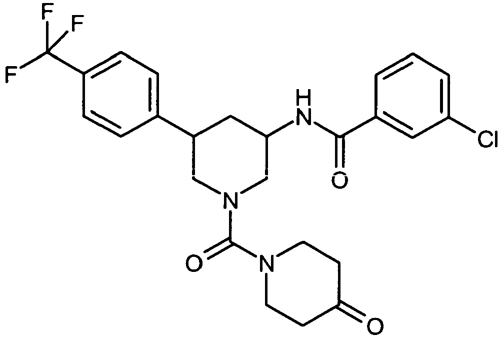

Eine Lösung des Acetals aus Beispiel 64A (400 mg, 0.725 mmol) in 15 ml Aceton/Wasser (10:1) wurde mit 54.6 mg Pyridinium-*p*-toluolsulfonat (0.217 mmol) versetzt und 3 d unter Rückfluss gerührt, wobei nach 24 h und 48 h erneut 54.6 mg Pyridinium-*p*-toluolsulfonat (0.217 mmol) zugegeben wurde. Anschließend wurde das Lösungsmittel im Vakuum entfernt, der Rückstand in Wasser aufgenommen und mit Dichlormethan extrahiert. Die organische Phase wurde über Magnesiumsulfat getrocknet, filtriert und im Vakuum eingeengt. Ausbeute: 363 mg (92% d. Th.)

LC-MS (Methode 11B): Rₜ = 1.12 min; MS (ESIpos): m/z = 508 [M+H]⁺.

### Beispiel 175

### N-{1-[(4-Cyanpiperidin-1-yl)carbonyl]-5-(4-ethylphenyl)piperidin-3-yl}cyclopentancarboxamid [racemisches cis-Isomer]

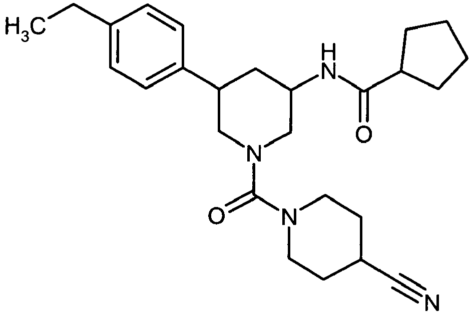

80 mg (0.24 mmol) der Verbindung aus Beispiel 56A wurden bei 0°C in 2.5 ml Dichlormethan vorgelegt, mit 49 µl (36 mg, 1.5 eq.) Triethylamin und 58 µl (64 mg, 0.47 mmol) Cyclopentancarbonsäurechlorid versetzt. Man ließ das Reaktionsgemisch auf RT erwärmen und rührte 16 h bei RT. Anschließend wurde zweimal mit Wasser gewaschen, die organische Phase über Natriumsulfat getrocknet, filtriert und im Vakuum eingeengt. Das Rohprodukt wurde in Methanol/DMF aufgenommen, der Rückstand abgetrennt und das Filtrat über präparative HPLC (RP18-Säule; Acetonitril-Wasser-Gradient) gereinigt. Der Rückstand und die produkthaltigen Fraktionen wurden vereinigt. Ausbeute: 94 mg (91% d. Th.).

LC-MS (Methode 5B): Rₜ = 2.31 min; MS (ESIpos): m/z = 437 [M+H]⁺;

¹H-NMR (400 MHz, DMSO-d₆): δ = 7.51 (d, NH), 7.36-6.98 (m, 4H), 3.70 (d, 2H), 3.56 (d, 1H), 3.04 (t, 4H), 2.86-2.72 (m, 1H), 2.72-2.60 (m, 2H), 2.55 (q, 2H), 1.97 (d, 1H), 1.86 (d, 2H), 1.79-1.43 (m, 12H), 1.16 (t, 3H).

### Beispiel 176

### N-{1-[(4-Hydroxypiperidin-1-yl)carbonyl]-5-[4-(trifluormethyl)phenyl]piperidin-3-yl}cyclopentan-carboxamid [enantiomerenreines cis-Isomer]

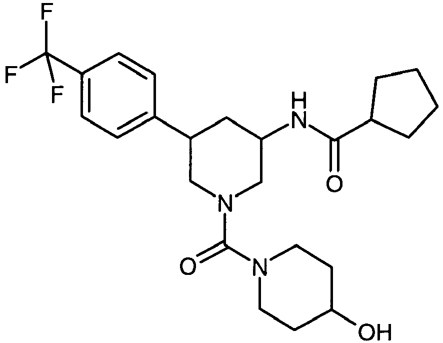

Die Enantiomerentrennung von 48.6 mg des Racemates aus Beispiel 170 nach Methode 16D ergab 14 mg der Verbindung aus Beispiel 176 (Enantiomer 1) und 16 mg der Verbindung aus Beispiel 177 (Enantiomer 2).

HPLC (Methode 8E): Rₜ = 4.50 min, >99.0% ee;

LC-MS (Methode 11B): Rₜ = 1.00 min; MS (ESIpos): m/z = 468 [M+H]⁺;

¹H-NMR (400 MHz, DMSO-d₆): δ = 7.78 (d, 1H), 7.70 (d, 2H), 7.51 (d, 2H), 4.67 (d, 1H), 3.82-3.53 (m, 4H), 3.46 (d, 2H), 3.05-2.81 (m, 3H), 2.80-2.69 (m, 1H), 2.01 (d, 1H), 1.81-1.41 (m, 12H), 1.37-1.19 (m, 2H).

### Beispiel 177

### N-{1-[(4-Hydroxypiperidin-1-yl)carbonyl]-5-[4-(trifluormethyl)phenyl]piperidin-3-yl}cyclopentan-carboxamid [enantiomerenreines cis-Isomer]

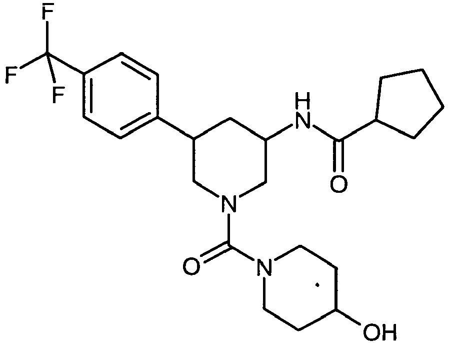

Die Enantiomerentrennung von 48.6 mg des Racemates aus Beispiel 170 nach Methode 16D ergab 14 mg der Verbindung aus Beispiel 176 (Enantiomer 1) und 16 mg der Verbindung aus Beispiel 177 (Enantiomer 2).

HPLC (Methode 8E): Rₜ = 5.14 min, >95.0% ee;

LC-MS (Methode 11B): Rₜ = 1.00 min; MS (ESIpos): m/z = 468 [M+H]⁺;

¹H-NMR (400 MHz, DMSO-d₆): δ = 7.78 (d, 1H), 7.70 (d, 2H), 7.51 (d, 2H), 4.67 (d, 1H), 3.82-3.53 (m, 4H), 3.46 (d, 2H), 3.05-2.81 (m, 3H), 2.80-2.69 (m, 1H), 2.01 (d, 1H), 1.81-1.41 (m, 12H), 1.37-1.19 (m, 2H).

### Beispiel 178

### N-{1-[(4-Cyanpiperidin-1-yl)carbonyl]-5-[4-(trifluormethyl)phenyl]piperidin-3-yl}cyclopentan-carboxamid [enantiomerenreines cis-Isomer]

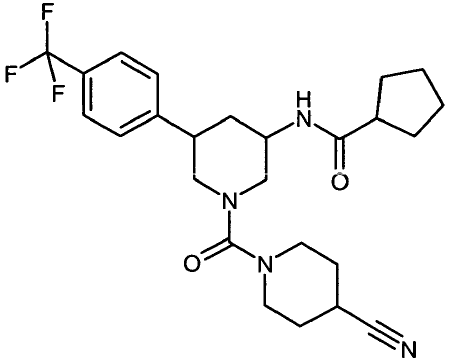

90 mg (0.21 mmol) der Verbindung aus Beispiel 69A wurden bei 0°C in 5.0 ml Dichlormethan vorgelegt, mit 86 µl (62 mg, 0.62 mmol.) Triethylamin, 37 µl (41 mg, 0.31 mmol) Cyclopentancarbonsäurechlorid und 7.5 mg (0.062 mmol) 4-(Dimethylamino)-pyridin versetzt. Man ließ das Reaktionsgemisch auf RT erwärmen und rührte 16 h bei RT. Anschließend wurde mit 1N Salzsäure gewaschen, die organische Phase über Magnesiumsulfat getrocknet, filtriert und im Vakuum eingeengt. Das Rohprodukt wurde durch präparative HPLC (RP18-Säule; Acetonitril-Wasser-Gradient) gereinigt. Ausbeute: 74.8 mg (77% d. Th.). Die Enantiomerentrennung von 74.8 mg des Racemates nach Methode 17D ergab 27 mg der Verbindung aus Beispiel 178 (Enantiomer 1) und 28 mg der Verbindung aus Beispiel 179 (Enantiomer 2).

HPLC (Methode 8E): Rₜ = 5.96 min, >99.0% ee;

LC-MS (Methode 11 B): Rₜ = 1.11 min; MS (ESIpos): m/z = 477 [M+H]⁺;

¹H-NMR (400 MHz, DMSO-d₆): δ = 7.80 (d, 1H), 7.70 (d, 2H), 7.52 (d, 2H), 3.80-3.65 (m, 2H), 3.60 (d, 1H), 3.14-2.90 (m, 4H), 2.85-2.72 (m, 1H), 2.01 (d, 1H), 1.85 (br. s, 2H), 1.77-1.39 (m, 16H), 4H verdeckt.

### Beispiel 179

### N-{1-[(4-Cyanpiperidin-1-yl)carbonyl]-5-[4-(trifluormethyl)phenyl]piperidin-3-yl}cyclopentan-carboxamid [enantiomerenreines cis-Isomer]

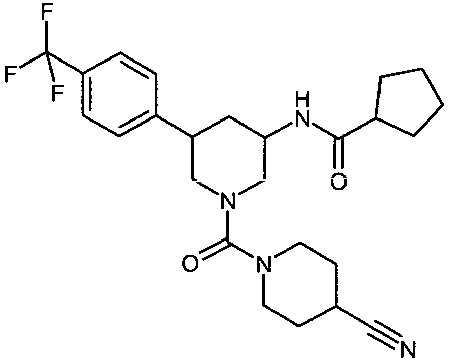

Die Enantiomerentrennung von 74.8 mg des Racemates aus Beispiel 178 nach Methode 17D ergab 27 mg der Verbindung aus Beispiel 178 (Enantiomer 1) und 28 mg der Verbindung aus Beispiel 179 (Enantiomer 2).

HPLC (Methode 8E): Rₜ = 6.68 min, >98.0% ee;

LC-MS (Methode 11 B): Rₜ = 1.11 min; MS (ESIpos): m/z = 477 [M+H]⁺;

¹H-NMR (400 MHz, DMSO-d₆): δ = 7.80 (d, 1H), 7.70 (d, 2H), 7.52 (d, 2H), 3.80-3.65 (m, 2H), 3.60 (d, 1H), 3.14-2.90 (m, 4H), 2.85-2.72 (m, 1H), 2.01 (d, 1H), 1.85 (br. s, 2H), 1.77-1.39 (m, 16H), 4H verdeckt.

### B) Bewertung der physiologischen Wirksamkeit

### Abkürzungen:

- BSA: Rinderserum-Albumin
- DMEM: Dulbecco's Modified Eagle Medium
- EGTA: Ethylenglykol-glykol-bis-(2-aminoäthyl)-*N,N,N',N'*-tetra-essigsäure
- FCS: Fetal Calf Serum
- HEPES: 4-(2-Hydroxyethyl)-1-piperazinethansulfonsäure
- [3H]haTRAP: tritiated high affinity thrombin receptor activating peptide
- PRP: Plättchenreiches Plasma

Die Eignung der erfindungsgemäßen Verbindungen zur Behandlung von thromboembolischen Erkrankungen kann in folgenden Assaysystemen gezeigt werden:

### 1.) In vitro Assays

### 1.a) Zellulärer, funktioneller in vitro-Test

Die Identifizierung von Antagonisten des humanen Protease Aktivierten Rezeptors 1 (PAR-1) sowie die Quantifizierung der Wirksamkeit der hier beschriebenen Substanzen erfolgt mit Hilfe einer rekombinanten Zelllinie. Die Zelle leitet sich ursprünglich von einer embryonalen Nierenzelle des Menschen (HEK293; ATCC: American Type Culture Collection, Manassas, VA 20108, USA) ab. Die Testzelllinie exprimiert konstitutiv eine modifizierte Form des calciumsensitiven Photoproteins Aequorin, das nach Rekonstitution mit dem Co-Faktor Coelenterazin bei Erhöhungen der freien Calcium-Konzentration im inneren mitochondrialen Kompartiment Licht emittiert (Rizzuto R, Simpson AW, Brini M, Pozzan T.; Nature 1992, 358, 325-327). Zusätzlich exprimiert die Zelle stabil den endogenen humanen PAR-1-Rezeptor sowie den endogenen purinergen Rezeptor P2Y2. Die resultierende PAR-1-Testzelle reagiert auf Stimulation des endogenen PAR-1 oder P2Y2-Rezeptors mit einer intrazellulären Freisetzung von Calcium-Ionen, die durch die resultierende Aequorin-Lumineszens mit einem geeigneten Luminometer quantifiziert werden kann (Milligan G, Marshall F, Rees S, *Trends in Pharmacological Sciences* 1996, *17*, 235-237).

Für die Prüfung der Substanz-Spezifität wird deren Wirkung nach Aktivierung des endogenen PAR-1-Rezeptors mit der Wirkung nach Aktivierung des endogenen purinergen P2Y2-Rezeptors verglichen, der den gleichen intrazellulären Signalweg nutzt.

Testablauf: Die Zellen werden zwei Tage (48 Std.) vor dem Test in Kulturmedium (DMEM F12, ergänzt mit 10% FCS, 2 mM Glutamine, 20 mM HEPES, 1.4 mM Pyruvat, 0.1 mg/ml Gentamycin, 0.15% Na-Bicarbonat; BioWhittaker Cat.# BE04-687Q; B-4800 Verviers, Belgien) in 384-Loch-Mikrotiterplatten ausplattiert und in einem Zellinkubator (96% Luftfeuchtigkeit, 5% v/v CO₂, 37°C) gehalten. Am Testtag wird das Kulturmedium durch eine Tyrodelösung (in mM: 140 Natriumchlorid, 5 Kaliumchlorid, 1 Magnesiumchlorid, 2 Calciumchlorid, 20 Glucose, 20 HEPES), das zusätzlich den Co-Faktor Coelenterazin (25 µM) und Glutathion (4 mM) enthält, ausgetauscht und die Mikrotiterplatte anschließend für weitere 3-4 Stunden inkubiert. Dann werden die Testsubstanzen auf die Mikrotiterplatte pipettiert und 5 Minuten nach Übertragung der Testsubstanzen in die Wells der Mikrotiterplatte wird die Platte in das Luminometer transferiert, eine PAR-1-Agonist-Konzentration, die EC₅₀ entspricht, zugeschossen und sofort das resultierende Lichtsignal im Luminometer gemessen. Zur Unterscheidung einer Antagonist-Substanzwirkung von einer toxischen Wirkung wird unmittelbar anschließend der endogene purinerge Rezeptor mit Agonist aktiviert (ATP, 10 µM Endkonzentration) und das resultierende Lichtsignal gemessen. Die Ergebnisse sind in Tabelle A gezeigt:

**Tabelle A:**

| **Beispiel-Nr.** | **IC₅₀ [nM]** | | **Beispiel-Nr.** | **IC₅₀ [nM]** |
|---|---|---|---|---|
| 7 | 29 | | 115 | 11.1 |
| 8 | 109 | | 119 | 15.7 |
| 18 | 483 | | 121 | 79.9 |
| 36 | 98.2 | | 128 | 17.04 |
| 83 | 19.7 | | 129 | 33.6 |
| 90 | 359 | | 133 | 14.6 |
| 91 | 17.4 | | 134 | 15.4 |
| 95 | 237 | | 141 | 53.7 |
| 97 | 109 | | 156 | 11.1 |
| 102 | 165 | | 162 | 25 |
| 104 | 248 | | 170 | 21.3 |
| 105 | 18.4 | | 177 | 81 |
| 106 | 7.05 | | 178 | 63 |

### 1.b) PAR-1 Rezeptor Bindungsassay

Thrombozytenmembranen werden mit 12 nM [3H]haTRAP und Testsubstanz in verschiedenen Konzentrationen in einem Puffer (50 mM Tris pH 7.5, 10 mM Magnesiumchlorid, 1 mM EGTA, 0.1% BSA) bei Raumtemperatur für 80 min inkubiert. Danach wird der Ansatz auf eine Filterplatte übertragen und zweimal mit Puffer gewaschen. Nach Zugabe von Scintillationsflüssigkeit wird die Radioaktivität auf dem Filter in einem beta-Counter vermessen.

### 1.c) Thrombozytenaggregation in Plasma

Zur Bestimmung der Thrombozytenaggregation wird Blut von gesunden Probanden beiderlei Geschlechts, die innerhalb der letzten zehn Tage keine die Thrombozytenaggregation beeinflussende Medikation erhalten hatten, verwendet. Das Blut wird in Monovetten (Sarstedt, Nümbrecht, Deutschland) die als Antikoagulans Natrium Citrat 3.8% (1 Teil Citrat + 9 Teile Blut) enthalten, aufgenommen. Zur Gewinnung von plättchenreichem Plasma wird das Citrat-Vollblut bei 140g für 20 min zentrifugiert.

Für die Aggregationsmessungen werden Aliquots des plättchenreichen Plasmas mit aufsteigenden Konzentrationen an Prüfsubstanz 10 min bei 37°C inkubiert. Anschließend wird die Aggregation durch Zugabe eines Thrombin-Rezeptor Agonisten (TRAP6, SFLLRN) in einem Aggregometer ausgelöst und mittels der turbidimetrischen Methode nach Born (Born, G.V.R., Cross M.J., The Aggregation of Blood Platelets; J. Physiol. 1963, 168, 178-195) bei 37°C bestimmt. Die SFLLRN-Konzentration, die zur maximalen Aggregation führt, wird gegebenenfalls jeweils für jeden Spender individuell ermittelt.

Zur Berechnung der inhibitorischen Wirkung wird die maximale Zunahme der Lichttransmission (Amplitude der Aggregationskurve in %) innerhalb 5 Minuten nach Zugabe des Agonisten in Gegenwart und Abwesenheit von Prüfsubstanz ermittelt und die Inhibition berechnet. Aus den Inhibitionskurven wird die Konzentration berechnet, die die Aggregation zu 50% hemmt.

### 1.d) Thrombozytenaggregation in Puffer

Zur Bestimmung der Thrombozytenaggregation wird Blut von gesunden Probanden beiderlei Geschlechts, die innerhalb der letzten zehn Tage keine die Thrombozytenaggregation beeinflussende Medikation erhalten hatten, verwendet. Das Blut wird in Monovetten (Sarstedt, Nümbrecht, Deutschland) die als Antikoagulans Natriumcitrat 3.8% (1 Teil Citrat + 9 Teile Blut) enthalten, aufgenommen. Zur Gewinnung von plättchenreichem Plasma wird das Citrat-Vollblut bei 140g für 20 min zentrifugiert. Zu dem PRP wird ein Viertel des Volumens ACD-Puffer (44.8 mM Natriumcitrat, 20.9 mM Citronensäure, 74.1 mM Glucose und 4 mM Kaliumchlorid) zugegeben und 10 Minuten bei 1000g zentrifugiert. Das Thrombozyten-Pellet wird mit WaschPuffer resuspendiert und 10 Minuten bei 1000g zentrifugiert. Die Thrombozyten werden in Inkubations-Puffer resuspendiert und auf 200000 Z/µl eingestellt. Vor Versuchsbeginn wird Calciumchlorid und Magnesiumchlorid, Endkonzentration je 2mM (Stammlösung 2M, 1:1000 Verdünnung) zugegeben. Besonderheit: bei ADP-induzierter Aggregation wird nur Calciumchlorid zugegeben. Folgende Agonisten können eingesetzt werden: TRAP6-Trifluoracetat- Salz, Kollagen, Humanes α-Thrombin und U-46619. Die Konzentration des Agonisten wird zu jedem Spender ausgetestet.

Testdurchführung: Es werden 96er-Loch Mikrotiterplatten verwendet. Die Prüfsubstanz wird in DMSO verdünnt und 2 µl je Loch vorgelegt. 178 µl Thrombozyten-Suspension werden zugegeben und 10 Minuten bei Raumtemperatur vorinkubiert. 20 µl Agonist werden zugegeben und die Messung im Spectramax, OD 405 nm, sofort gestartet. Die Kinetik wird in 11 Messungen von je 1 Minute bestimmt. Zwischen den Messungen wird 55 Sekunden geschüttelt.

### 1.e) Thrombozytenaggregation in fibrinogendepletiertem Plasma

Zur Bestimmung der Thrombozytenaggregation wird Blut von gesunden Probanden beiderlei Geschlechts, die innerhalb der letzten zehn Tage keine die Thrombozytenaggregation beeinflussende Medikation erhalten hatten, verwendet. Das Blut wird in Monovetten (Sarstedt, Nümbrecht, Deutschland) die als Antikoagulans Natriumcitrat 3.8% (1 Teil Citrat + 9 Teile Blut) enthalten, aufgenommen.

Herstellung von fibrinogendepletiertem Plasma: Zur Gewinnung von plättchenarmem Plasma wird das Citrat-Vollblut bei 140g für 20 min abzentrifugiert. Das plättchenarme Plasma wird im Verhältnis 1:25 mit Reptilase (Roche Diagnostic, Deutschland) versetzt und vorsichtig invertiert. Es folgen 10 min Inkubation bei 37°C im Wasserbad mit direkt folgender Inkubation auf Eis für 10 min. Das Plasma-Reptilase Gemisch wird bei 1300g für 15 min zentrifugiert und der Überstand (fibrinogendepletiertes Plasma) wird gewonnen.

Thrombozyten-Isolierung: Zur Gewinnung von plättchenreichem Plasma wird das Citrat-Vollblut bei 140g für 20 min zentrifugiert. Zu dem PRP wird ein Viertel des Volumens ACD-Puffer (44.8 mM Natriumcitrat, 20.9 mM Citronensäure, 74.1 mM Glucose und 4 mM Kaliumchlorid) zugegeben und 10 Minuten bei 1300g zentrifugiert. Das Thrombozyten-Pellet wird mit WaschPuffer resuspendiert und 10 Minuten bei 1300g zentrifugiert. Die Thrombozyten werden in Inkubations-Puffer resuspendiert und auf 400000 Z/µl eingestellt und Calciumchloridlösung mit einer Endkonzentration von 5 mM (1/200Verdünnung) zugegeben.

Für die Aggregationsmessungen werden Aliquots (98 µl fibrinogendepletiertes Plasma und 80 µl Thrombozytensuspension) mit aufsteigenden Konzentrationen an Prüfsubstanz 10 min bei RT inkubiert. Anschließend wird die Aggregation durch Zugabe von humanem alpha Thrombin in einem Aggregometer ausgelöst und mittels der turbidimetrischen Methode nach Born (Born, G.V.R., Cross M.J., The Aggregation of Blood Platelets; J. Physiol. 1963, 168, 178-195) bei 37°C bestimmt. Die alpha Thrombin Konzentration, die gerade eben zur maximalen Aggregation führt, wird jeweils für jeden Spender individuell ermittelt.

Zur Berechnung der inhibitorischen Wirkung wird die Zunahme der maximalen Lichttransmission (Amplitude der Aggregationskurve in %) innerhalb 5 Minuten nach Zugabe des Agonisten in Gegenwart und Abwesenheit von Prüfsubstanz ermittelt und die Inhibition berechnet. Aus den Inhibitionskurven wird die Konzentration berechnet, die die Aggregation zu 50% hemmt.

### 1.f) Stimulation gewaschener Thrombozyten und Analyse in der Durchflusszytometrie

Isolierung gewaschener Thrombozyten: Humanes Vollblut wird mittels Venenpunktion von freiwilligen Spendern gewonnen und in Monovetten (Sarstedt, Nümbrecht, Deutschland) überführt, die als Antikoagulans Natriumcitrat enthalten (1 Teil Natriumcitrat 3.8% + 9 Teile Vollblut). Die Monovetten werden bei 900 Umdrehungen pro Minute und 4°C über einen Zeitraum von 20 Minuten zentrifugiert (Heraeus Instruments, Deutschland; Megafuge 1.0RS). Das plättchenreiche Plasma wird vorsichtig abgenommen und in ein 50 ml-Falconröhrchen überführt. Nun wird das Plasma mit ACD-Puffer (44 mM Natriumcitrat, 20.9 mM Zitronensäure, 74.1 mM Glucose) versetzt. Das Volumen des ACD-Puffers entspricht einem Viertel des Plasmavolumens. Durch zehnminütige Zentrifugation bei 2500 Umdrehungen und 4°C werden die Thrombozyten sedimentiert. Danach wird der Überstand vorsichtig abdekantiert und verworfen. Die präzipitierten Thrombozyten werden zunächst vorsichtig mit einem Milliliter Waschpuffer (113 mM Natriumchlorid, 4 mM Dinatriumhydrogenphosphat, 24 mM Natriumdihydrogenphosphat, 4 mM Kaliumchlorid, 0.2 mM Ethylenglycol-bis-(2-aminoethyl)-*N,N,N'N'*-tetraessigsäure, 0.1% Glucose) resuspendiert und dann mit Waschpuffer auf ein Volumen aufgefüllt, das dem der Plasmamenge entspricht. Der Waschvorgang wird ein zweites Mal durchgeführt. Nachdem die Thrombozyten durch eine erneute zehnminütige Zentrifugation bei 2500 Umdrehungen und 4°C präzipitiert worden sind, werden sie vorsichtig in einem Milliliter Inkubationspuffer (134 mM Natriumchlorid, 12 mM Natriumhydrogencarbonat, 2.9 mM Kaliumchlorid, 0.34 mM Natriumdihydrogencarbonat, 5 mM HEPES, 5 mM Glucose, 2 mM Calciumchlorid und 2 mM Magnesiumchlorid) resuspendiert und mit Inkubationspuffer auf eine Konzentration von 300.000 Thrombozyten pro µl eingestellt.

Färbung und Stimulierung der humanen Thrombozyten mit humanem α-Thrombin in Gegenwart oder Abwesenheit eines PAR-1-Antagonisten: Die Thrombozytensuspension wird mit der zu prüfenden Substanz bzw. des entsprechenden Lösungsmittels für 10 Minuten bei 37°C vorinkubiert (Eppendorf, Deutschland; Thermomixer Comfort). Durch Zugabe des Agonisten (0.5 µM bzw. 1 µM α-Thrombin; Kordia, Niederlande, 3281 NIH Units/mg; oder 30µg/ml Thrombin, receptor activating peptide (TRAP6); Bachem, Schweiz) bei 37° und unter Schütteln von 500 Umdrehungen pro Minute wird die Thrombozytenaktivierung ausgelöst. Zu den Zeitpunkten 0, 1, 2.5, 5, 10 und 15 Minuten wird jeweils ein Aliquot von 50µl entnommen und in einen Milliliter einfach-konzentrierte CellFix™-Lösung (Becton Dickinson Immunocytometry Systems, USA) überführt. Zur Fixierung der Zellen werden sie 30 Minuten bei 4°C in der Dunkelheit inkubiert. Durch eine zehnminütige Zentrifugation bei 600 g und 4°C werden die Thrombozyten präzipitiert. Der Überstand wird verworfen und die Thrombozyten werden in 400 µl CellWash™ (Becton Dickinson Immunocytometry Systems, USA) resuspendiert. Ein Aliquot von 100 µl wird in ein neues FACS-Röhrchen überführt. 1 µl des thrombozyten-identifizierenden Antikörpers und 1 µl des aktivierungszustands-detektierenden Antikörpers werden mit CellWash™ auf ein Volumen von 100 µl aufgefüllt. Diese Antikörperlösung wird dann zur Thrombozytensuspension gegeben und 20 Minuten bei 4°C in der Dunkelheit inkubiert. Im Anschluss an die Färbung wird das Ansatzvolumen durch Zugabe von weiteren 400 µl CellWash™ erhöht.

Zur Identifizierung der Thrombozyten wird ein fluorescein-isothiocyanat-konjugierter Antikörper eingesetzt, der gegen das humane Glykoprotein IIb (CD41) gerichtet ist (Immunotech Coulter, Frankreich; Cat. No. 0649). Mit Hilfe des phycoerythrin-konjugierten Antikörpers, der gegen das humane Glykoprotein P-Selektin (Immunotech Coulter, Frankreich; Cat. No. 1759) gerichtet ist, lässt sich der Aktivierungszustand der Thrombozyten bestimmen. P-Selektin (CD62P) ist in den α-Granula ruhender Thrombozyten lokalisiert. Es wird jedoch nach *in-vitro-* bzw. *in-vivo-*Stimulierung zur äußeren Plasmamembran translokalisiert.

Durchflusszytometrie und Auswertung der Daten: Die Proben werden im Gerät FACSCalibur™ Flow Cytometry System der Firma Becton Dickinson Immunocytometry Systems, USA, vermessen und mit Hilfe der Software CellQuest, Version 3.3 (Becton Dickinson Immunocytometry Systems, USA) ausgewertet und graphisch dargestellt. Das Maß der Thrombozytenaktivierung wird durch den Prozentsatz der CD62P-positiven Thrombozyten (CD41-positive Ereignisse) bestimmt. Es werden von jeder Probe 10.000 CD41-positive Ereignisse gezählt.

Die inhibitorische Wirkung der zu prüfenden Substanzen wird anhand der Reduktion der Thrombozytenaktivierung berechnet, die sich auf die Aktivierung durch den Agonisten bezieht.

### 1.g) Thrombozytenaggregationsmessung mit der Parallelplattenflußkammer

Zur Bestimmung der Thrombozytenaggregation wird Blut von gesunden Probanden beiderlei Geschlechts, die innerhalb der letzten zehn Tage keine die Thrombozytenaggregation beeinflussende Medikation erhalten hatten, verwendet. Das Blut wird in Monovetten (Sarstedt, Nümbrecht, Deutschland) die als Antikoagulans Natriumcitrat 3.8% (1 Teil Citrat + 9 Teile Blut) enthalten, aufgenommen. Zur Gewinnung von plättchenreichem Plasma wird das Citrat-Vollblut bei 140g für 20 min zentrifugiert. Zu dem PRP wird ein Viertel des Volumens ACD-Puffer (44.8 mM Natriumcitrat, 20.9 mM Citronensäure, 74.1 mM Glucose und 4 mM Kaliumchlorid) zugegeben und 10 Minuten bei 1000g zentrifugiert. Das Thrombozyten-Pellet wird mit WaschPuffer resuspendiert und 10 Minuten bei 1000g zentrifugiert. Für die Perfusionsstudie wird eine Mischung von 40% Erythrozyten und 60% gewaschene Thrombozyten (200.000/µl) hergestellt und in HEPES-Tyrode Puffer suspendiert. Die Messung der Thrombozytenaggregation unter Flußbedingungen wird mittels der Parallelplattenflußkammer durchgeführt (B. Nieswandt et al., EMBO J. 2001, 20, 2120-2130; C. Weeterings, Arterioscler Thromb. Vasc. Biol. 2006, 26, 670-675; JJ Sixma, Thromb. Res. 1998, 92, 43-46). Glasobjektträger werden mit 100 µl humaner α-Thrombinlösung (gelöst in Tris-Puffer) über Nacht bei 4°C benetzt (α-Thrombin in verschiedenen Konzentrationen, z.B. 10 bis 50 µg/ml) und abschließend mittels 2% BSA abgeblockt.

Rekonstituiertes But wird über die Thrombin-benetzten Glasobjektträger über 5 Minuten mit konstanter Flußrate geleitet (z.B. Scherrate 300/Sekunde) und mittels Mikroskop-Videosystem beobachtet und aufgezeichnet. Die inhibitorische Wirkung der zu prüfenden Substanzen wird morphometrisch anhand der Reduktion der Plättchenaggregatsbildung ermittelt. Alternativ kann die Inhibierung der Plättchenaktivierung durch Durchflußzytometrie, z.B. über p-Selektin-Expression (CD62p), bestimmt werden (siehe Methode 1.f).

### 2.) Ex vivo Assay

### 2.a) Thrombozytenaggregation (Primaten, Meerschweinchen)

Meerschweinchen oder Primaten werden in wachem oder narkotisiertem Zustand oral, intravenös oder intraperitoneal mit Prüfsubstanzen in geeigneter Formulierung behandelt. Als Kontrolle werden andere Meerschweinchen oder Primaten in identischer Weise mit dem entsprechenden Vehikel behandelt. Nach je nach Applikationsart unterschiedlich langer Zeit wird aus den tief narkotisierten Tieren Blut durch Punktion des Herzens oder der Aorta gewonnen. Das Blut wird in Monovetten (Sarstedt, Nümbrecht, Deutschland) die als Antikoagulans Natriumcitrat 3.8% (1 Teil Citratlösung + 9 Teile Blut) enthalten, aufgenommen. Zur Gewinnung von plättchenreichem Plasma wird das Citrat-Vollblut bei 140g für 20 min zentrifugiert.

Die Aggregation wird durch Zugabe eines Thrombin-Rezeptor Agonisten (TRAP6, SFLLRN, 50 µg/ml; Konzentration wird in jedem Experiment je nach Tierart ermittelt) in einem Aggregometer ausgelöst und mittels der turbidimetrischen Methode nach Born (Born, G.V.R., Cross M.J., The Aggregation of Blood Platelets; J. Physiol. 1963, 168, 178-195) bei 37°C bestimmt.

Zur Aggregationsmessung wird die maximale Zunahme der Lichttransmission (Amplitude der Aggregationskurve in %) innerhalb 5 Minuten nach Zugabe des Agonisten ermittelt. Die inhibitorische Wirkung der verabreichten Prüfsubstanzen in den behandelten Tieren wird durch die Reduktion der Aggregation, bezogen auf den Mittelwert der Kontrolltiere, berechnet.

### 3.) In vivo Assays

### 3.a) Thrombosemodelle

Die erfindungsgemäßen Verbindungen können in Thrombosemodellen in geeigneten Tierspezies, in denen die Thrombin-induzierte Plättchenaggregation über den PAR-1-Rezeptor vermittelt wird, untersucht werden. Als Tierspezies eignen sich Meerschweinchen und insbesondere Primaten (vergleiche: Lindahl, A.K., Scarborough, R.M., Naughton, M.A., Harker, L.A., Hanson, S.R., Thromb Haemost 1993, 69, 1196; Cook JJ, Sitko GR, Bednar B, Condra C, Mellott MJ, Feng D-M, Nutt RF, Shager JA, Gould RJ, Connolly TM, Circulation 1995, 91, 2961-2971; Kogushi M, Kobayashi H, Matsuoka T, Suzuki S, Kawahara T, Kajiwara A, Hishinuma I, Circulation 2003, 108 Suppl. 17, IV-280; Derian CK, Damiano BP, Addo MF, Darrow AL, D'Andrea MR, Nedelman M, Zhang H-C, Maryanoff BE, Andrade-Gordon P, J. Pharmacol. Exp. Ther. 2003, 304, 855-861). Alternativ können Meerschweinchen verwendet werden, die mit Inhibitoren von PAR-3 und/oder PAR-4 vorbehandelt werden (Leger AJ et al., Circulation 2006, 113, 1244-1254), oder transgene PAR-3- und/oder PAR-4-Knockdown-Meerschweinchen.

### 3.b) Gerinnungsstörung und Organdysfunktion bei Disseminierter Intravasaler Gerinnung (DIC)

Die erfindungsgemäßen Verbindungen können in Modellen für DIC und/oder Sepsis in geeigneten Tierspezies untersucht werden. Als Tierspezies eignen sich Meerschweinchen und insbesondere Primaten, bei Untersuchung der endothelvermittelten Effekte auch Mäuse und Ratten (vergleiche: Kogushi M, Kobayashi H, Matsuoka T, Suzuki S, Kawahara T, Kajiwara A, Hishinuma I, Circulation 2003, 108 Suppl. 17, IV-280; Derian CK, Damiano BP, Addo MF, Darrow AL, D'Andrea MR, Nedelman M, Zhang H-C, Maryanoff BE, Andrade-Gordon P, J. Pharmacol. Exp. Ther. 2003, 304, 855-861; Kaneider NC et al., Nat Immunol, 2007, 8, 1303-12; Camerer E et al., Blood, 2006, 107, 3912-21; Riewald M et al., J Biol Chem, 2005, 280, 19808-14.). Alternativ können Meerschweinchen verwendet werden, die mit Inhibitoren von PAR-3 und/oder PAR-4 vorbehandelt werden (Leger AJ et al., Circulation 2006, 113, 1244-1254), oder transgene PAR-3-und/oder PAR-4-Knockdown-Meerschweinchen.

### 3.b.1) Thrombin-Antithrombin-Komplexe

Thrombin-Antithrombin-Komplexe (nachfolgend als "TAT" bezeichnet) sind ein Maß für das durch Gerinnungsaktivierung endogen gebildete Thrombin. TAT werden mittels eines ELISA-Assays bestimmt (Enzygnost TAT micro, Dade-Behring). Aus Citratblut wird durch Zentrifugation Plasma gewonnen. Zu 50 µl Plasma wird 50 µl TAT-Probenpuffer gegeben, kurz geschüttelt und 15 min bei Raumtemperatur inkubiert. Die Proben werden abgesaugt, und das Well 3-malig mit Waschpuffer gewaschen (300 µl/Well). Die Platte wird zwischen den Waschgängen abgeklopft. Es wird Konjugatlösung (100 µl) hinzugegeben und 15 min bei Raumtemperatur inkubiert. Die Proben werden abgesaugt, und das Well 3-malig mit Waschpuffer gewaschen (300 µl/Well). Anschließend wird chromogenes Susbtrat hinzugegeben (100 µl/Well), 30 min im Dunkeln bei Raumtemperatur inkubiert, Stopplösung hinzugegeben (100 µl/ Well), und die Farbbildung bei 492 nm gemessen (Saphire Plate reader).

### 3.b.2) Parameter für Organdysfunktion

Es werden verschiedene Parameter bestimmt, aufgrund derer Rückschlüsse auf die Funktionseinschränkung verschiedener innerer Organe durch die LPS-Gabe gezogen werden können, und der therapeutische Effekt von Prüfsubstanzen abgeschätzt werden kann. Citratblut oder ggf. Lithium-Heparin-Blut wird zentrifugiert, und die Parameter aus dem Plasma bestimmt. Folgende Parameter werden typischerweise erhoben: Kreatinin, Harnstoff, Aspartat-Aminotransferase (AST), Alanin-Aminotransferase (ALT), Gesamt-Bilirubin, Laktatdehydrogenase (LDH), Gesamt-Protein, Gesamt-Albumin und Fibrinogen. Die Werte geben Aufschluss auf die Funktion der Niere, der Leber, des Kreislaufes und der Gefäße.

### 3.b.3) Parameter für Entzündung

Das Ausmaß der durch Endotoxin ausgelösten Entzündungsreaktion lässt sich aus dem Anstieg von Entzündungsmediatoren im Plasma nachweisen, z.B. Interleukine (1, 6, 8 und 10), Tumomekrosefaktor alpha oder Monocyte Chemoattractant Protein-1. Hierzu können ELISAs oder das Luminex-System verwendet werden.

### 3.c) Antitumor Wirksamkeit

Die erfindungsgemäßen Verbindungen können in Modellen für Krebs getestet werden, z.B. im humanen Brustkrebs-Modell in immundefizienten Mäusen (vergleiche: S. Even-Ram et. al., Nature Medicine, 1988, 4, 909-914).

### 3.d) Antiangiogenetische Wirksamkeit

Die erfindungsgemäßen Verbindungen können in *in vitro* und *in vivo* Modellen für Angiogenese getestet werden (vergleiche: Caunt et al., Journal of Thrombosis and Haemostasis, 2003, 10, 2097-2102; Haralabopoulos et al., Am JPhysiol, 1997, C239-C245; Tsopanoglou et al., JBC, 1999, 274, 23969-23976; Zania et al., JPET, 2006, 318, 246-254).

### 3.e) Blutdruck- und Herzfrequenz-modulierende Wirkung

Die erfindungsgemäßen Verbindungen können in *in vivo* Modellen hinsichtlich Ihrer Wirkung auf den arteriellen Blutdruck und die Herzfrequenz untersucht werden. Hierzu werden Ratten (z.B. Wistar) mit implantierbaren Radiotelemetrieeinheiten instrumentiert, und es wird ein elektronisches Datenaquisitions- und Speicherungs-System (Data Sciences, MN, USA), bestehend aus einem chronisch implantierbaren Transducer/Transmitter-Einheit in Verbindung mit einem Flüssigkeits-gefüllten Katheter, verwendet. Der Transmitter wird in die Peritonealhöhle implantiert und der Sensor-Katheter in der deszendierenden Aorta positioniert. Die erfindungsgemäßen Verbindungen können appliziert werden (z.B. oral oder intravenös). Vor Behandlung wird der mittlere arterielle Blutdruck und die Herzfrequenz der unbehandelten und behandelten Tiere gemessen und sichergestellt, dass diese im Bereich von ca. 131-142 mmHg und 279-321 Schläge/Minute liegen. PAR-1-aktivierendes Peptid (SFLLRN; z.B. Dosen zwischen 0.1 und 5 mg/kg) wird intravenös verabreicht. Der Blutdruck und die Herzfrequenz werden in verschiedenen Zeitintervallen und Zeiträumen mit und ohne PAR-1-aktivierendem Peptid sowie mit und ohne einer der erfindungsgemäßen Verbindungen gemessen (vergleiche: Cicala C et al., The FASEB Journal, 2001, 15, 1433-5; Stasch JP et al., British Journal of Pharmacology 2002, 135, 344-355).

### 4.) Bestimmung der Löslichkeit

### Herstellung der Ausgangslösung (Urlösung):

Mindestens 1.5 mg der Testsubstanz werden in ein Wide Mouth 10 mm Screw V-Vial (Fa. Glastechnik Gräfenroda GmbH, Art.-Nr. 8004-WM-H/V15µ) mit passender Schraubkappe und Septum genau eingewogen, mit DMSO zu einer Konzentration von 50 mg/ml versetzt und 30 Minuten mittels eines Vortexers geschüttelt.

### Herstellung der Kalibrierlösungen:

Die notwendigen Pipettierschritte erfolgen in 1.2 ml 96er Deep Well Plate (DWP) mittels eines Liquid-Handling-Roboters. Als Lösemittel wird ein Gemisch aus Acetonitril/Wasser 8:2 verwendet.

*Herstellung der Ausgangslösung für Kalibrierlösungen (stammlösung:* 10 µl der Urlösung werden mit 833 µl des Lösemittelgemisch versetzt (Konzentration = 600 µg/ml) und homogenisiert. Es werden von jeder Testsubstanz 1:100 Verdünnungen in separaten DWP's hergestellt und wiederum homogenisiert.

*Kalibrierlösung 5 (600 nglml):* 30 µl der Stammlösung werden mit 270 µl Lösemittelgemisch versetzt und homogenisiert.

*Kalibrierlösung 4 (60 ng*/*ml):* 30 µl der Kalibrierlösung 5 werden mit 270 µl Lösemittelgemisch versetzt und homogenisiert.

*Kalibrierlösung 3 (12 ng*/*ml):* 100 µl der Kalibrierlösung 4 werden mit 400 µl Lösemittelgemisch versetzt und homogenisiert.

*Kalibrierlösung 2 (1.2 ng*/*ml):* 30 µl der Kalibrierlösung 3 werden mit 270 µl Lösemittelgemisch versetzt und homogenisiert.

*Kalibrierlösung 1 (0.6 ng*/*ml):* 150 µl der Kalibrierlösung 2 werden mit 150 µl Lösemittelgemisch versetzt und homogenisiert.

### Herstellung der Probenlösungen:

Die notwendigen Pipettierschritte erfolgen in 1.2 ml 96er DWP mittels eines Liquid-Handling-Roboters. 10.1 µl der Stammlösung werden mit 1000 µl PBS-Puffer pH 6.5 versetzt. (PBS-Puffer pH 6.5: 61.86 g Natriumchlorid, 39.54 g Natriumdihydrogenphosphat und 83.35 g 1 N Natronlauge werden in einen 1 Liter-Messkolben eingewogen, mit Wasser aufgefüllt und ca. 1 Stunde gerührt. Von dieser Lösung werden 500 ml in einen 5 Liter-Messkolben gegeben und mit Wasser aufgefüllt. Es wird mit 1 N Natronlauge auf pH 6.5 einstellen.)

### Durchführung:

Die notwendigen Pipettierschritte erfolgen in 1.2 ml 96er DWP mittels eines Liquid-Handling-Roboters. Die so hergestellten Probenlösungen werden 24 Stunden bei 1400 rpm mittels eines temperierbaren Schüttlers bei 20°C geschüttelt. Von diesen Lösungen werden jeweils 180 µl abgenommen und in Beckman Polyallomer Centrifuge Tubes überführt. Diese Lösungen werden 1 Stunde mit ca. 223.000 x g zentrifugiert. Von jeder Probenlösung werden 100 µl des Überstandes abgenommen und 1:10 und 1:1000 mit PBS-Puffer 6.5 verdünnt.

### Analytik:

Die Proben werden mittels HPLC/MS-MS analysiert. Quantifiziert wird über eine Fünf-Punkt-Kalibrationskurve der Testverbindung. Die Löslichkeit wird in mg/l ausgedrückt. Analysensequenz: 1) Blank (Lösemittelgemisch); 2) Kalibrierlösung 0.6 ng/ml; 3) Kalibrierlösung 1.2 ng/ml; 4) Kalibrierlösung 12 ng/ml; 5) Kalibrierlösung 60 ng/ml; 6) Kalibrierlösung 600 ng/ml; 7) Blank (Lösemittelgemisch); 8) Probenlösung 1:1000; 9) Probenlösung 1:10.

### HPLC/MS-MSMethode:

HPLC: Agilent 1100, quat. Pumpe (G1311A), Autosampler CTC HTS PAL, Degaser (G1322A) und Säulenthermostat (G1316A); Säule: Oasis HLB 20 mm x 2.1 mm, 25 µ; Temperatur: 40°C; Eluent A: Wasser + 0.5 ml Ameisensäure/l; Eluent B: Acetonitril + 0.5 ml Ameisensäure/l; Flussrate: 2.5 ml/min; Stoptime 1.5 min; Gradient: 0 min 95% A, 5% B; Rampe: 0-0.5 min 5% A, 95% B; 0.5-0.84 min 5% A, 95% B; Rampe: 0.84-0.85 min 95% A, 5% B; 0.85-1.5 min 95% A, 5% B.

MS/MS: WATERS Quattro Micro Tandem MS/MS; Z-Spray API-Interface; HPLC-MS-Eingangssplitter 1:20; Messung im ESI-Mode.

### C) Ausführunzsbeispiele für pharmazeutische Zusammensetzungen

Die erfindungsgemäßen Substanzen können folgendermaßen in pharmazeutische Zubereitungen überführt werden:

### Tablette:

### Zusammensetzung:

100 mg der Verbindung des Beispiels 1, 50 mg Lactose (Monohydrat), 50 mg Maisstärke, 10 mg Polyvinylpyrolidon (PVP 25) (Fa. BASF, Deutschland) und 2 mg Magnesiumstearat.

Tablettengewicht 212 mg. Durchmesser 8 mm, Wölbungsradius 12 mm.

### Herstellung:

Die Mischung aus der Verbindung des Beispiels 1, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben).

### Orale Suspension:

### Zusammensetzung:

1000 mg der Verbindung des Beispiels 1, 1000 mg Ethanol (96%), 400 mg Rhodigel (Xanthan gum) (Fa. FMC, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die Verbindung des Beispiels 1 wird der Suspension zugefügt. Unter Rühren erfolgt die Zugabe des Wassers. Bis zum Abschluss der Quellung des Rhodigels wird ca. 6h gerührt.

### Intravenös applizierbare Lösung:

### Zusammensetzung:

1 mg der Verbindung von Beispiel 1, 15 g Polyethylenglykol 400 und 250 g Wasser für Inj ektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 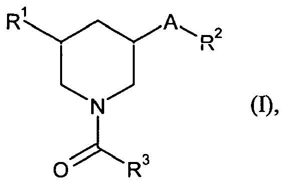 in welcher
A für eine Gruppe der Formel 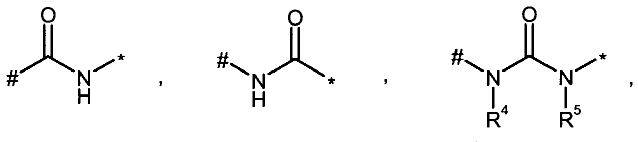 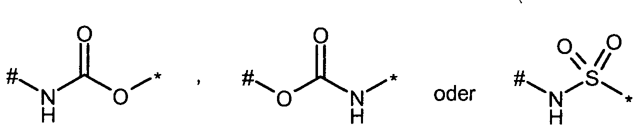 steht,
wobei
# die Anknüpfstelle an den Piperidinring ist,
* die Anknüpfstelle an R² ist,
R⁴ für Wasserstoff oder C₁-C₃-Alkyl steht,
und
R⁵ für Wasserstoff oder C₁-C₃-Alkyl steht,
R¹ für Phenyl steht,
wobei Phenyl substituiert sein kann mit 1 bis 3 Substituenten, unabhängig voneinander ausgewählt aus der Gruppe bestehend aus Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, Methylsulfonyl, C₁-C₄-Alkyl, C₁-C₄-Alkoxy und C₁-C₄-Alkoxycarbonyl,
R² für C₁-C₆-Alkyl, C₃-C₆-Cycloalkyl, 4- bis 6-gliedriges Heterocyclyl, Phenyl, 2,2-Difluor-1,3-benzodioxolyl oder 5- oder 6-gliedrige Heteroaryl steht,
wobei Cycloalkyl, Heterocyclyl, Phenyl und Heteroaryl substituiert sein können mit 1 bis 3 Substituenten, unabhängig voneinander ausgewählt aus der Gruppe bestehend aus Halogen, Cyano, Hydroxy, Amino, Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, C₁-C₄-Alkyl, C₁-C₄-Alkoxy, C₁-C₆-Alkylamino und Phenyl,
worin Phenyl substituiert sein kann mit 1 bis 3 Substituenten, unabhängig voneinander ausgewählt aus der Gruppe bestehend aus Halogen und Trifluormethyl,
und
wobei C₁-C₄-Alkyl substituiert sein kann mit einem Substituenten ausgewählt aus der Gruppe bestehend aus C₃-C₆-Cycloalkyl und Phenyl,
worin Cycloalkyl und Phenyl substituiert sein können mit 1 bis 3 Substituenten, unabhängig voneinander ausgewählt aus der Gruppe bestehend aus Halogen, Cyano, Monofluormethyl, Difluormethyl, Trifluormethyl, Monofluormethoxy, Difluormethoxy, Trifluormethoxy, C₁-C₄-Alkyl und C₁-C₄-Alkoxy,
R³ für C₁-C₆-Alkyl, C₂-C₆-Alkenyl, C₂-C₆-Alkinyl, C₁-C₆-Alkoxy, C₁-C₆-Alkylamino, C₃-C₇-Cycloalkyl, 4- bis 7-gliedriges Heterocyclyl, Phenyl, 5- oder 6-gliedriges Heteroaryl, C₃-C₇-Cycloalkyloxy, C₃-C₇-Cycloalkylamino, 4- bis 7-gliedriges Heterocyclylamino, Phenylamino oder 5- oder 6-gliedriges Heteroarylamino steht,
wobei Alkyl, C₂-C₆-Alkoxy und Alkylamino substituiert sein können mit einem Substituenten ausgewählt aus der Gruppe bestehend aus Halogen, Hydroxy, Amino, Cyano, C₁-C₄-Alkoxy, C₁-C₄-Alkoxycarbonyl, C₃-C₇-Cycloalkyl, 4- bis 6-gliedriges Heterocyclyl, Phenyl und 5- oder 6-gliedriges Heteroaryl,
worin Alkoxy substituiert sein kann mit einem Substituenten C₁-C₄-Alkoxy,
und
wobei Cycloalkyl, Heterocyclyl, Phenyl, Heteroaryl, Cycloalkyloxy, Cycloalkylamino, Heterocyclylamino, Phenylamino und Heteroarylamino substituiert sein können mit 1 bis 3 Substituenten, unabhängig voneinander ausgewählt aus der Gruppe bestehend aus Halogen, Cyano, Oxo, Hydroxy, Amino, Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, Hydroxycarbonyl, Aminocarbonyl, C₁-C₄-Alkyl, C₁-C₄-Alkoxy, C₁-C₆-Alkylamino, C₁-C₄-Alkoxycarbonyl, C₁-C₄-Alkylanünocarbonyl und Cyclopropyl,
worin Alkyl substituiert sein kann mit einem Substituenten Hydroxy, oder eines ihrer Salze, ihrer Solvate oder der Solvate ihrer Salze,
mit Ausnahme der Verbindungen
trans-tert-Butyl-3-{[(benzyloxy)carbonyl]amino}-5-phenylpiperidin-1-carboxylat und cis-tert-Butyl-3-{[(benzyloxy)carbonyl]-amino}-5-phenylpiperidin-1-carboxylat.

2. Verbindung nach Anspruch 1, **dadurch gekennzeichnet, dass**
A für eine Gruppe der Formel 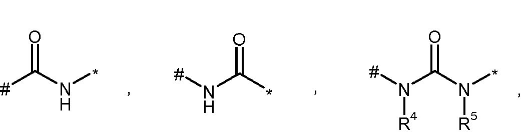 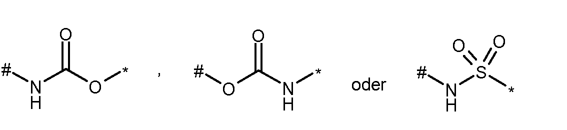 steht,
wobei
# die Anknüpfstelle an den Piperidinring ist,
* die Anknüpfstelle an R² ist,
R⁴ für Wasserstoff oder Methyl steht, und
R⁵ für Wasserstoff oder Methyl steht,
R¹ für Phenyl steht,
wobei Phenyl substituiert ist mit 1 bis 2 Substituenten, unabhängig voneinander ausgewählt aus der Gruppe bestehend aus Trifluormethyl, Trifluormethoxy, Methyl, Ethyl, Isopropyl, Methoxy und Ethoxycarbonyl,
R² für Methyl, Ethyl, Isopropyl, tert-Butyl, Cyclopropyl, Cyclopentyl, Pyrrolidinyl, Piperazinyl, Phenyl, 2,2-Difluor-1,3-benzodioxolyl, Thienyl, Thiazolyl oder Pyridyl steht,
wobei Cyclopentyl, Piperazinyl, Phenyl, Thienyl, Thiazolyl und Pyridyl substituiert sein können mit 1 bis 2 Substituenten, unabhängig voneinander ausgewählt aus der Gruppe bestehend aus Halogen, Cyano, Hydroxy, Trifluormethyl, Difluormethoxy, Trifluormethoxy, Methyl, Ethyl, Methoxy, Ethoxy und Phenyl,
worin Phenyl substituiert sein kann mit 1 bis 2 Substituenten, unabhängig voneinander ausgewählt aus der Gruppe bestehend aus Halogen und Trifluormethyl,
und
wobei Methyl substituiert sein kann mit einem Substituenten Phenyl,
R³ für Methyl, Ethyl, Isopropyl, tert-Butyl, Ethoxy, Ethylamino, tert-Butylamino, N-Methyl-N-ethylamino, Diethylamino, Cyclopropyl, Cyclobutyl, Cyclopentyl, Cyclohexyl, Tetrahydrofuranyl, Tetrahydropyranyl, Morpholin-4-yl, Thiomorpholin-4-yl, 1,1-Dioxidothiomorpholin-4-yl, Azetidiny-1-yl, Pyrrolidin-1-yl, Piperidin-1-yl, Piperazin-1-yl, Phenyl, Pyrrolyl, Furyl, Thiazolyl, Pyrazolyl, Pyridyl, Cyclopentyloxy, Cyclohexylamino, Phenylamino oder Pyridylamino steht,
wobei Methyl, Ethyl, Isopropyl, tert-Butyl, Ethoxy und Ethylamino substituiert sein können mit einem Substituenten ausgewählt aus der Gruppe bestehend aus Halogen, Hydroxy, Methoxy, Cyclopropyl, Phenyl, Furyl, Thienyl und Pyrazolyl,
und
wobei Cyclopropyl, Cyclobutyl, Cyclohexyl, Tetrahydrofuranyl, Morpholin-4-yl, Azetidiny-1-yl, Pyrrolidin-1-yl, Piperidin-1-yl, Piperazin-1-yl, Phenyl, Furyl, Thiazolyl, Pyrazolyl, Pyridyl, Cyclohexylamino und Phenylamino substituiert sein können mit 1 bis 2 Substituenten, unabhängig voneinander ausgewählt aus der Gruppe bestehend aus Halogen, Cyano, Oxo, Hydroxy, Amin, Trifluormethyl, Difluormethoxy, Trifluormethoxy, Hydroxycarbonyl, Aminocarbonyl, Methyl, Ethyl, Methoxy, Ethoxy, Dimethylamino, Methoxycarbonyl, Ethoxycarbonyl, Dimethylaminocarbonyl und Cyclopropyl,
worin Methyl und Ethyl substituiert sein können mit einem Substituenten Hydroxy,
oder eines ihrer Salze, ihrer Solvate oder der Solvate ihrer Salze.

3. Verbindung nach Anspruch 1 oder 2, **dadurch gekennzeichnet, dass**
A für eine Gruppe der Formel 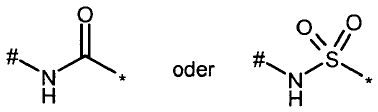 steht,
wobei
# die Anknüpfstelle an den Piperidinring ist,
* die Anknüpfstelle an R² ist,
R¹ für Phenyl steht,
wobei Phenyl substituiert ist mit 1 bis 2 Substituenten, unabhängig voneinander ausgewählt aus der Gruppe bestehend aus Trifluormethyl, Trifluormethoxy, Methyl und Ethyl,
R² für Methyl, Ethyl, Isopropyl, tert-Butyl, Cyclopropyl, Cyclopentyl, Phenyl, Thienyl oder Pyridyl steht,
wobei Cyclopentyl, Phenyl, Thienyl und Pyridyl substituiert sein können mit 1 bis 2 Substituenten, unabhängig voneinander ausgewählt aus der Gruppe bestehend aus Halogen, Cyano, Trifluormethyl, Trifluormethoxy, Methyl, Methoxy und Phenyl,
worin Phenyl substituiert sein kann mit 1 bis 2 Substituenten, unabhängig voneinander ausgewählt aus der Gruppe bestehend aus Chlor, Fluor und Trifluormethyl,
und
wobei Methyl substituiert sein kann mit einem Substituenten Phenyl,
R³ für Morpholin-4-yl, 1,1-Dioxidothiomorpholin-4-yl, 3-Hydroxyazetidiny-1-yl, 3-Hydroxypyrrolidin-1-yl, 4-Cyanopiperidin-1-yl oder 4-Hydroxypiperidin-1-yl steht,
oder eines ihrer Salze, ihrer Solvate oder der Solvate ihrer Salze.

4. Verbindung nach einem der Ansprüche 1 bis 3, **dadurch gekennzeichnet, dass** die Substituenten -R¹ und -A-R² in cis-Position zueinander stehen.

5. Verfahren zur Herstellung einer Verbindung der Formel (I) oder eines ihrer Salze, ihrer Solvate oder der Solvate ihrer Salze nach Anspruch 1, **dadurch gekennzeichnet, dass** entweder
[A] eine Verbindung der Formel 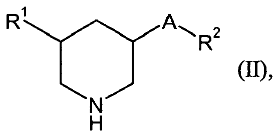 in welcher
A, R¹ und R² die in Anspruch 1 angegebene Bedeutung aufweisen,
mit einer Verbindung der Formel 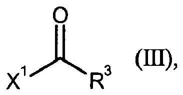 in welcher
R³ die in Anspruch 1 angegebene Bedeutung aufweist, und
X¹ für Halogen, bevorzugt Brom oder Chlor, oder Hydroxy steht,
umgesetzt wird
oder
[B] eine Verbindung der Formel (II) mit einer Verbindung der Formel 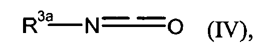 in welcher
R^{3a} für C₁-C₆-Alkyl, C₃-C₇-Cycloalkyl, 4- bis 7-gliedriges Heterocyclyl, Phenyl oder 5-oder 6-gliedriges Heteroaryl steht,
wobei Alkyl substituiert sein kann mit einem Substituenten ausgewählt aus der Gruppe bestehend aus Halogen, Hydroxy, Amino, Cyano, C₁-C₄-Alkoxy, C₁-C₄-Alkoxycarbonyl, C₃-C₇-Cycloalkyl, 4- bis 6-gliedriges Heterocyclyl, Phenyl und 5-oder 6-gliedriges Heteroaryl,
worin Alkoxy substituiert sein kann mit einem Substituenten C₁-C₄-Alkoxy,
und
wobei Cycloalkyl, Heterocyclyl, Phenyl und Heteroaryl substituiert sein können mit 1 bis 3 Substituenten, unabhängig voneinander ausgewählt aus der Gruppe bestehend aus Halogen, Cyano, Oxo, Hydroxy, Amino, Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, Hydroxycarbonyl, Aminocarbonyl, C₁-C₄-Alkyl, C₁-C₄-Alkoxy, C₁-C₆-Alkylamino, C₁-C₄-Alkoxycarbonyl, C₁-C₄-Alkylaminocarbonyl und Cyclopropyl,
worin Alkyl substituiert sein kann mit einem Substituenten Hydroxy,
zu einer Verbindung der Formel 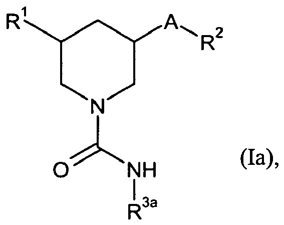 in welcher
A, R¹, R² und R^{3a} die in Anspruch 1 angegebene Bedeutung aufweisen, umgesetzt wird
oder
[C] eine Verbindung der Formel 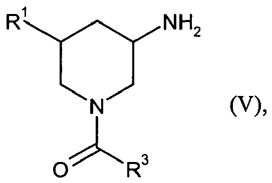 in welcher
R¹ und R³ die in Anspruch 1 angegebene Bedeutung aufweisen,
mit einer Verbindung der Formel 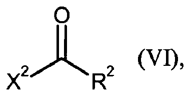 in welcher
R² die in Anspruch 1 angegebene Bedeutung aufweist, und
X² für Halogen, bevorzugt Brom oder Chlor, oder Hydroxy steht,
zu einer Verbindung der Formel 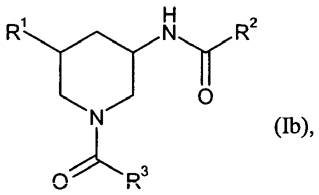 in welcher
R¹, R² und R³ die in Anspruch 1 angegebene Bedeutung aufweisen, umgesetzt wird
oder
[D] eine Verbindung der Formel (V) mit einer Verbindung der Formel 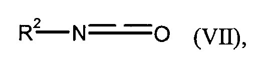 in welcher
R² die in Anspruch 1 angegebene Bedeutung aufweist,
zu einer Verbindung der Formel 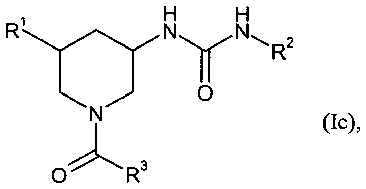 in welcher
R¹, R² und R³ die in Anspruch 1 angegebene Bedeutung aufweisen, umgesetzt wird
oder
[E] eine Verbindung der Formel (V) mit einer Verbindung der Formel 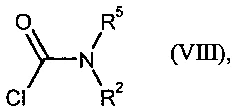 in welcher
R² und R⁵ die in Anspruch 1 angegebene Bedeutung aufweisen,
zu einer Verbindung der Formel 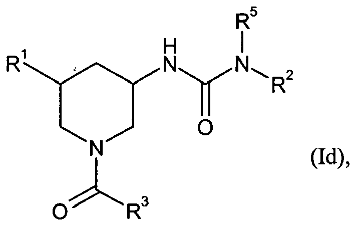 in welcher
R¹, R², R³ und R⁵ die in Anspruch 1 angegebene Bedeutung aufweisen,
umgesetzt wird
oder
[F] eine Verbindung der Formel (Id) mit einer Verbindung der Formel 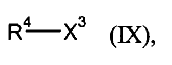 in welcher
R⁴ die in Anspruch 1 angegebene Bedeutung aufweist, und
X³ für Halogen, bevorzugt Iod, Brom oder Chlor, steht,
zu einer Verbindung der Formel 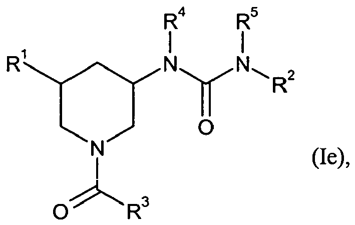 in welcher
R¹, R², R³, R⁴ und R⁵ die in Anspruch 1 angegebene Bedeutung aufweisen, umgesetzt wird
oder
[G] eine Verbindung der Formel (V) mit einer Verbindung der Formel 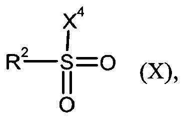 in welcher
R² die in Anspruch 1 angegebene Bedeutung aufweist, und
X⁴ für Chlor oder Hydroxy steht,
zu einer Verbindung der Formel 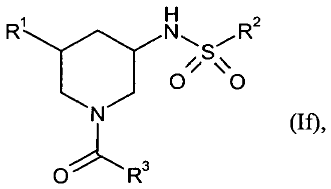 in welcher
R¹, R² und R³ die in Anspruch 1 angegebene Bedeutung aufweisen,
umgesetzt wird
oder
[H] eine Verbindung der Formel 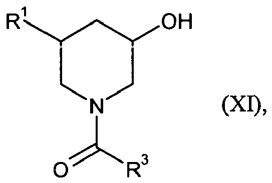 in welcher
R¹ und R³ die in Anspruch 1 angegebene Bedeutung aufweisen,
zunächst mit Disuccinimidylcarbonat und anschließend mit einer Verbindung der Formel
H₂N-R² (XII),
in welcher
R² die in Anspruch 1 angegebene Bedeutung aufweist,
zu einer Verbindung der Formel 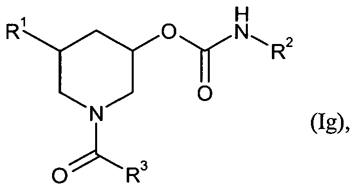 in welcher
R¹, R² und R³ die in Anspruch 1 angegebene Bedeutung aufweisen,
umgesetzt wird.

6. Verbindung nach einem der Ansprüche 1 bis 4 zur Behandlung und/oder Prophylaxe von Krankheiten.

7. Verwendung einer Verbindung nach einem der Ansprüche 1 bis 4 zur Herstellung eines Arzneimittels zur Behandlung und/oder Prophylaxe von Krankheiten.

8. Verwendung einer Verbindung nach einem der Ansprüche 1 bis 4 zur Herstellung eines Arzneimittels zur Behandlung und/oder Prophylaxe von Herz-Kreislauf Erkrankungen, thromboembolischen Erkrankungen und/oder von Tumorerkrankungen.

9. Verwendung einer Verbindung nach einem der Ansprüche 1 bis 4 zur Verhinderung der Blutkoagulation *in vitro*.

10. Arzneimittel enthaltend eine Verbindung nach einem der Ansprüche 1 bis 4 in Kombination mit einem inerten, nichttoxischen, pharmazeutisch geeigneten Hilfsstoff.

11. Arzneimittel enthaltend eine Verbindung nach einem der Ansprüche 1 bis 4 in Kombination mit einem weiteren Wirkstoff.

12. Arzneimittel nach Anspruch 10 oder 11 zur Behandlung und/oder Prophylaxe von Herz-Kreislauf-Erkrankungen, thromboembolischen Erkrankungen und/oder von Tumorerkrankungen.

13. Verfahren zur Verhinderung der Blutkoagulation *in vitro*, **dadurch gekennzeichnet, dass** eine antikoagulatorisch wirksame Menge einer Verbindung nach einem der Ansprüche 1 bis 4 zugegeben wird.

## Claims

1. Compound of the formula 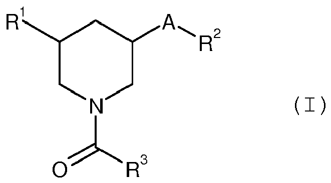 in which
A represents a group of the formula 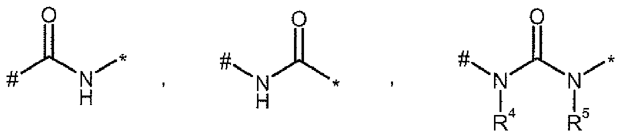 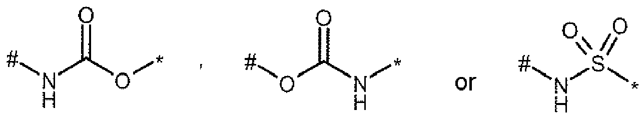 where
# is the point of attachment to the piperidine ring,
* is the point of attachment to R²,
R⁴ represents hydrogen or C₁-C₃-alkyl,
and
R⁵ represents hydrogen or C₁-C₃-alkyl,
R¹ represents phenyl,
where phenyl may be substituted by 1 to 3 substituents independently of one another selected from the group consisting of monofluoromethyl, difluoromethyl, trifluoromethyl, monofluoromethoxy, difluoromethoxy, trifluoromethoxy, monofluoromethylsulphanyl, difluoromethylsulphanyl, trifluoromethylsulphanyl, methylsulphonyl, C₁-C₄-alkyl, C₁-C₄-alkoxy and C₁-C₄-alkoxycarbonyl,
R² represents C₁-C₆-alkyl, C₃-C₆-cycloalkyl, 4- to 6-membered heterocyclyl, phenyl, 2,2-difluoro-1,3-benzodioxolyl or 5- or 6-membered heteroaryl,
where cycloalkyl, heterocyclyl, phenyl and heteroaryl may be substituted by 1 to 3 substituents independently of one another selected from the group consisting of halogen, cyano, hydroxyl, amino, monofluoromethyl, difluoromethyl, trifluoromethyl, monofluoromethoxy, difluoromethoxy, trifluoromethoxy, monofluoromethylsulphanyl, difluoromethylsulphanyl, trifluoromethylsulphanyl, C₁-C₄-alkyl, C₁-C₄-alkoxy, C₁-C₆-alkylamino and phenyl,
where phenyl may be substituted by 1 to 3 substituents independently of one another selected from the group consisting of halogen and trifluoromethyl,
and
where C₁-C₄-alkyl may be substituted by a substituent selected from the group consisting of C₃-C₆-cycloalkyl and phenyl,
where cycloalkyl and phenyl may be substituted by 1 to 3 substituents independently of one another selected from the group consisting of halogen, cyano, monofluoromethyl, difluoromethyl, trifluoromethyl, monofluoromethoxy, difluoromethoxy, trifluoromethoxy, C₁-C₄-alkyl and C₁-C₄-alkoxy,
R³ represents C₁-C₆-alkyl, C₂-C₆-alkenyl, C₂-C₆-alkynyl, C₁-C₆-alkoxy, C₁-C₆-alkylamino, C₃-C₇-cycloalkyl, 4- to 7-membered heterocyclyl, phenyl, 5- or 6-membered heteroaryl, C₃-C₇-cycloalkyloxy, C₃-C₇-cycloalkylamino, 4- to 7-membered heterocyclylamino, phenylamino or 5-or 6-membered heteroarylamino,
where alkyl, C₂-C₆-alkoxy and alkylamino may be substituted by a substituent selected from the group consisting of halogen, hydroxyl, amino, cyano, C₁-C₄-alkoxy, C₁-C₄-alkoxycarbonyl, C₃-C₇-cycloalkyl, 4- to 6-membered heterocyclyl, phenyl and 5- or 6-membered heteroaryl,
where alkoxy may be substituted by a C₁-C₄-alkoxy substituent,
and
where cycloalkyl, heterocyclyl, phenyl, heteroaryl, cycloalkyloxy, cycloalkylamino, heterocyclylamino, phenylamino and heteroarylamino may be substituted by 1 to 3 substituents independently of one another selected from the group consisting of halogen, cyano, oxo, hydroxyl, amino, monofluoromethyl, difluoromethyl, trifluoromethyl, monofluoromethoxy, difluoromethoxy, trifluoromethoxy, monofluoromethylsulphanyl, difluoromethylsulphanyl, trifluoromethylsulphanyl, hydroxycarbonyl, aminocarbonyl, C₁-C₄-alkyl, C₁-C₄-alkoxy, C₁-C₆-alkylamino, C₁-C₄-alkoxycarbonyl, C₁-C₄-alkylaminocarbonyl and cyclopropyl,
where alkyl may be substituted by a hydroxyl substituent,
or one of the salts thereof, solvates thereof or solvates of the salts thereof,
with the exception of the compounds trans-tert-butyl 3-{[benzyloxy)carbonyl]amino}-5-phenylpiperidine-1-carboxylate and cis-tert-butyl 3-{[benzyloxy)carbonyl]amino}-5-phenylpiperidine-1-carboxylate.

2. Compound according to Claim 1, **characterized in that**
A represents a group of the formula 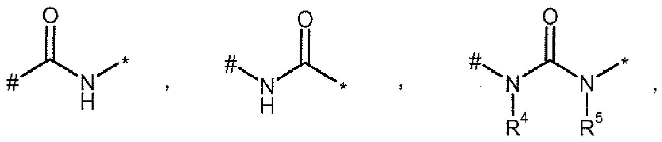 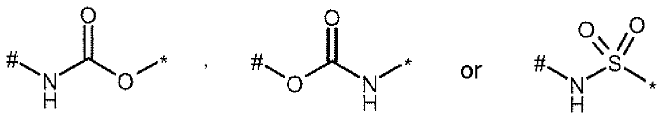 where
# is the point of attachment to the piperidine ring,
* is the point of attachment to R²,
R⁴ represents hydrogen or methyl,
and
R⁵ represents hydrogen or methyl,
R¹ represents phenyl,
where phenyl is substituted by 1 or 2 substituents independently of one another selected from the group consisting of trifluoromethyl, trifluoromethoxy, methyl, ethyl, isopropyl, methoxy and ethoxycarbonyl,
R² represents methyl, ethyl, isopropyl, tert-butyl, cyclopropyl, cyclopentyl, pyrrolidinyl, piperazinyl, phenyl, 2,2-difluoro-1,3-benzodioxolyl, thienyl, thiazolyl or pyridyl,
where cycloalkyl, piperazinyl, phenyl, thienyl, thiazolyl and pyridyl may be substituted by 1 or 2 substituents independently of one another selected from the group consisting of halogen, cyano, hydroxyl, trifluoromethyl, difluoromethoxy, trifluoromethoxy, methyl, ethyl, methoxy, ethoxy and phenyl,
where phenyl may be substituted by 1 or 2 substituents independently of one another selected from the group consisting of halogen and trifluoromethyl,
and
where methyl may be substituted by a phenyl substituent,
R³ represents methyl, ethyl, isopropyl, tert-butyl, ethoxy, ethylamino, tert-butylamino, N-methyl-N-ethylamino, diethylamino, cyclopropyl, cyclobutyl, cyclopentyl, cyclohexyl, tetrahydrofuranyl, tetrahydropyranyl, morpholin-4-yl, thiomorpholin-4-yl, 1,1-dioxidothiomorpholin-4-yl, azetidin-1-yl, pyrrolidin-1-yl, piperidin-1-yl, piperazin-1-yl, phenyl, pyrrolyl, furyl, thiazolyl, pyrazolyl, pyridyl, cyclopentyloxy, cyclohexylamino, phenylamino or pyridylamino,
where methyl, ethyl, isopropyl, tert-butyl, ethoxy and ethylamino may be substituted by a substituent selected from the group consisting of halogen, hydroxyl, methoxy, cyclopropyl, phenyl, furyl, thienyl and pyrazolyl,
and
where cyclopropyl, cyclobutyl, cyclohexyl, tetrahydrofuranyl, morpholin-4-yl, azetidin-1-yl, pyrrolidin-1-yl, piperidin-1-yl, piperazin-1-yl, phenyl, furyl, thiazolyl, pyrazolyl, pyridyl, cyclohexylamino and phenylamino may be substituted by 1 or 2 substituents independently of one another selected from the group consisting of halogen, cyano, oxo, hydroxyl, amino, trifluoromethyl, difluoromethoxy, trifluoromethoxy, hydroxycarbonyl, aminocarbonyl, methyl, ethyl, methoxy, ethoxy, dimethylamino, methoxycarbonyl, ethoxycarbonyl, dimethylaminocarbonyl and cyclopropyl, in which methyl and ethyl may be substituted by a hydroxyl substituent,
or one of the salts thereof, solvates thereof or solvates of the salts thereof.

3. Compound according to Claim 1 or 2, **characterized in that**
A represents a group of the formula 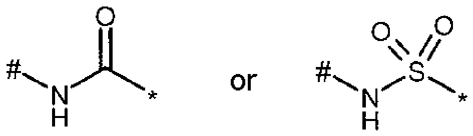 where
# is the point of attachment to the piperidine ring,
* is the point of attachment to R²,
R¹ represents phenyl,
where phenyl is substituted by 1 or 2 substituents independently of one another selected from the group consisting of trifluoromethyl, trifluoromethoxy, methyl and ethyl,
R² represents methyl, ethyl, isopropyl, tert-butyl, cyclopropyl, cyclopentyl, phenyl, thienyl or pyridyl,
where cyclopentyl, phenyl, thienyl and pyridyl may be substituted by 1 or 2 substituents independently of one another selected from the group consisting of halogen, cyano, trifluoromethyl, trifluoromethoxy, methyl, methoxy and phenyl,
where phenyl may be substituted by 1 or 2 substituents independently of one another selected from the group consisting of chlorine, fluorine and trifluoromethyl,
and
where methyl may be substituted by a phenyl substituent,
R³ represents morpholin-4-yl, 1,1-dioxidothiomorpholin-4-yl, 3-hydroxyazetidiny-1-yl, 3-hydroxypyrrolidin-1-yl, 4-cyanopiperidin-1-yl or 4-hydroxypiperidin-1-yl,
or one of the salts thereof, solvates thereof or solvates of the salts thereof.

4. Compound according to any of Claims 1 to 3, **characterized in that** the substituents -R¹ and -A-R² are in the cis-position to one another.

5. Process for preparing a compound of the formula (I) or one of the salts thereof, solvates thereof or solvates of the salts thereof according to Claim 1, **characterized in that** either
[A] a compound of the formula 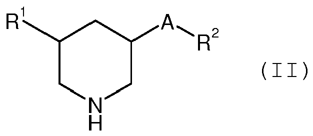 in which
A, R¹ and R² have the meaning given in Claim 1,
is reacted with a compound of the formula 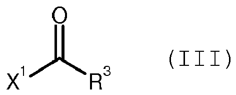 in which
R³ has the meaning given in Claim 1, and
X¹ represents halogen, preferably bromine or chlorine, or hydroxyl,
or
[B] a compound of the formula (II) is reacted with a compound of the formula 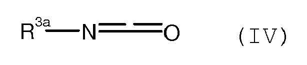 in which
R^{3a} represents C₁-C₆-alkyl, C₃-C₇-cycloalkyl, 4- to 7-membered heterocyclyl, phenyl or 5- or 6-membered heteroaryl,
where alkyl may be substituted by a substituent selected from the group consisting of halogen, hydroxyl, amino, cyano, C₁-C₄-alkoxy, C₁-C₄-alkoxycarbonyl, C₃-C₇-cycloalkyl, 4- to 6-membered heterocyclyl, phenyl and 5- or 6-membered heteroaryl,
where alkoxy may be substituted by a C₁-C₄-alkoxy substituent,
and
where cycloalkyl, heterocyclyl, phenyl and heteroaryl may be substituted by 1 to 3 substituents independently of one another selected from the group consisting of halogen, cyano, oxo, hydroxyl, amino, monofluoromethyl, difluoromethyl, trifluoromethyl, monofluoromethoxy, difluoromethoxy, trifluoromethoxy, monofluoromethylsulphanyl, difluoromethylsulphanyl, trifluoromethylsulphanyl, hydroxycarbonyl, aminocarbonyl, C₁-C₄-alkyl, C₁-C₄-alkoxy, C₁-C₆-alkylamino, Cₗ₁-C₄-alkoxycarbonyl, C₁-C₄-alkylaminocarbonyl and cyclopropyl,
where alkyl may be substituted by a hydroxyl substituent,
to give a compound of the formula 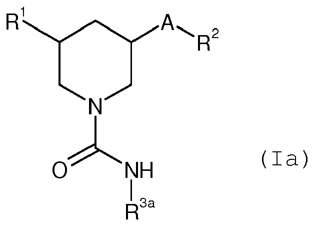 in which
A, R¹, R² and R^{3a} have the meaning given in Claim 1,
or
[C] a compound of the formula 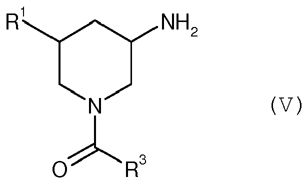 in which
R¹ and R³ have the meaning given in Claim 1,
is reacted with a compound of the formula 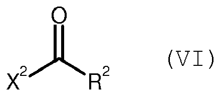 in which
R² has the meaning given in Claim 1, and
X² represents halogen, preferably bromine or chlorine, or hydroxyl,
to give a compound of the formula 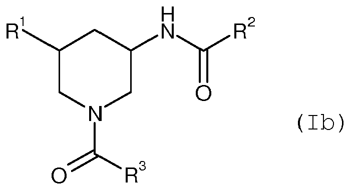 in which
R¹, R² and R³ have the meaning given in Claim 1,
or
[D] a compound of the formula (V) is reacted with a compound of the formula 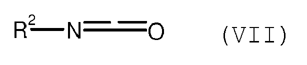 in which
R² has the meaning given in Claim 1,
to give a compound of the formula 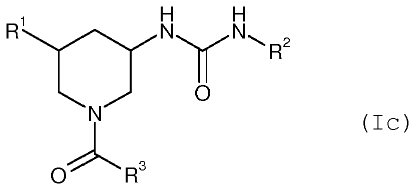 in which
R¹, R² and R³ have the meaning given in Claim 1,
or
[E] a compound of the formula (V) is reacted with a compound of the formula 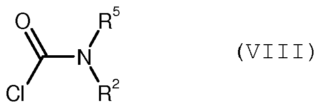 in which
R² and R⁵ have the meaning given in Claim 1,
to give a compound of the formula 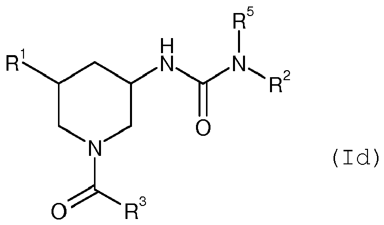 in which
R¹, R², R³ and R⁵ have the meaning given in Claim 1,
or
[F] a compound of the formula (Id) is reacted with a compound of the formula 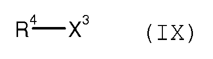 in which
R⁴ has the meaning given in Claim 1, and
X³ represents halogen, preferably iodine, bromine or chlorine,
to give a compound of the formula 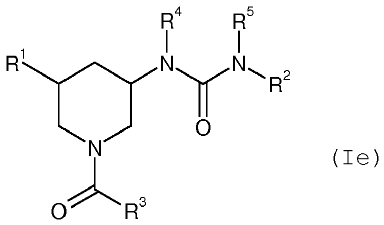 in which
R¹, R², R³, R⁴ and R⁵ have the meaning given in Claim 1,
or
[G] a compound of the formula (V) is reacted with a compound of the formula 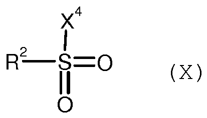 in which
R² has the meaning given in Claim 1, and
X⁴ represents chlorine or hydroxyl,
to give a compound of the formula 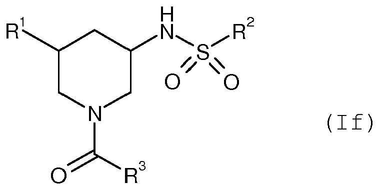 in which
R¹, R² and R³ have the meaning given in Claim 1, or
[H] a compound of the formula 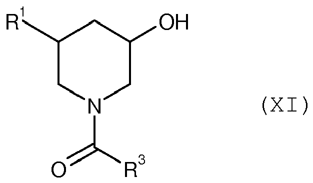 in which
R¹ and R³ have the meaning given in Claim 1,
is reacted initially with disuccinimidyl carbonate and then with a compound of the formula
H₂N-R² (XII),
in which
R² has the meaning given in Claim 1,
to give a compound of the formula 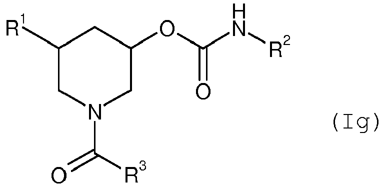 in which
R¹, R² and R³ have the meaning given in Claim 1.

6. Compound according to any of Claims 1 to 4 for the treatment and/or prophylaxis of diseases.

7. Use of a compound according to any of Claims 1 to 4 for producing a medicament for the treatment and/or prophylaxis of diseases.

8. Use of a compound according to any of Claims 1 to 4 for preparing a medicament for the treatment and/or prophylaxis of cardiovascular disorders, thromboembolic disorders and/or tumour disorders.

9. Use of a compound according to any of Claims 1 to 4 for preventing blood coagulation *in vitro.*

10. Medicament comprising a compound according to any of Claims 1 to 4 in combination with an inert, nontoxic, pharmaceutically suitable excipient.

11. Medicament comprising a compound according to any of Claims 1 to 4 in combination with a further active compound.

12. Medicament according to Claim 10 or 11 for the treatment and/or prophylaxis of cardiovascular disorders, thromboembolic disorders and/or tumour disorders.

13. Method for preventing blood coagulation *in vitro,* **characterized in that** an anticoagulatory amount of a compound according to any of Claims 1 to 4 is added.

## Revendications

1. Composé de formule 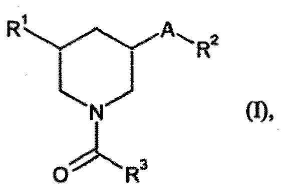 dans laquelle
A représente un groupe de formule 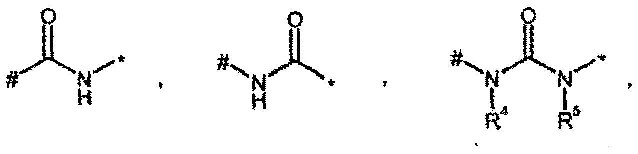 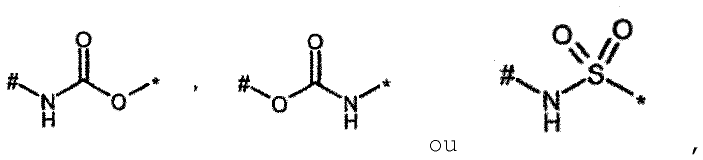
# représentant l'emplacement de liaison au cycle pipéridine,
* représentant l'emplacement de liaison à R²,
R⁴ représentant hydrogène ou alkyle en C₁-C₃, et
R⁵ représentant hydrogène ou alkyle en C₁-C₃,
R¹ représente phényle,
le phényle pouvant être substitué avec 1 à 3 substituants, choisis indépendamment les uns des autres dans le groupe constitué par monofluorométhyle, difluorométhyle, trifluorométhyle, monofluorométhoxy, difluorométhoxy, trifluorométhoxy, monofluorométhylsulfanyle, difluorométhylsulfanyle, trifluorométhylsulfanyle, méthylsulfonyle, alkyle en C₁-C₄, alcoxy en C₁-C₄ et alcoxycarbonyle en C₁-C₄,
R² représente alkyle en C₁-C₆, cycloalkyle en C₃-C₆, hétérocyclyle de 4 à 6 éléments, phényle, 2,2-difluoro-1,3-benzodioxolyle ou hétéroaryle à 5 ou 6 éléments,
le cycloalkyle, l'hétérocyclyle, le phényle et l'hétéroaryle pouvant être substitués avec 1 à 3 substituants, choisis indépendamment les uns des autres dans le groupe constitué par halogène, cyano, hydroxy, amino, monofluorométhyle, difluorométhyle, trifluorométhyle, monofluorométhoxy, difluorométhoxy, trifluorométhoxy, monofluorométhylsulfanyle, difluorométhylsulfanyle, trifluorométhylsulfanyle, alkyle en C₁-C₄, alcoxy en C₁-C₄, alkylamino en C₁-C₆ et phényle,
le phényle pouvant être substitué avec 1 à 3 substituants, choisis indépendamment les uns des autres dans le groupe constitué par halogène et trifluorométhyle,
et
l'alkyle en C₁-C₄ pouvant être substitué avec un substituant choisi dans le groupe constitué par cycloalkyle en C₃-C₆ et phényle,
le cycloalkyle et le phényle pouvant être substitués avec 1 à 3 substituants, choisis indépendamment les uns des autres dans le groupe constitué par halogène, cyano, monofluorométhyle, difluorométhyle, trifluorométhyle, monofluorométhoxy, difluorométhoxy, trifluorométhoxy, alkyle en C₁-C₄ et alcoxy en C₁-C₄,
R³ représente alkyle en C₁-C₆, alcényle en C₂-C₆, alcynyle en C₂-C₆, alcoxy en C₁-C₆, alkylamino en C₁-C₆, cycloalkyle en C₃-C₇, hétérocyclyle de 4 à 7 éléments, phényle, hétéroaryle à 5 ou 6 éléments, cycloalkyloxy en C₃-C₇, cycloalkylamino en C₃-C₇, hétérocyclylamino de 4 à 7 éléments, phénylamino ou hétéroarylamino à 5 ou 6 éléments,
l'alkyle, l'alcoxy en C₂-C₆ et l'alkylamino pouvant être substitués avec un substituant choisi dans le groupe constitué par halogène, hydroxy, amino, cyano, alcoxy en C₁-C₄, alcoxycarbonyle en C₁-C₄, cycloalkyle en C₃-C₇, hétérocyclyle de 4 à 6 éléments, phényle et hétéroaryle à 5 ou 6 éléments,
l'alcoxy pouvant être substitué avec un substituant alcoxy en C₁-C₄,
et
le cycloalkyle, l'hétérocyclyle, le phényle, l'hétéroaryle, le cycloalkyloxy, le cycloalkylamino, l'hétérocyclylamino, le phénylamino et l'hétéroarylamino pouvant être substitués avec 1 à 3 substituants, choisis indépendamment les uns des autres dans le groupe constitué par halogène, cyano, oxo, hydroxy, amino, monofluorométhyle, difluorométhyle, trifluorométhyle, monofluorométhoxy, difluorométhoxy, trifluorométhoxy, monofluorométhylsulfanyle, difluorométhylsulfanyle, trifluorométhylsulfanyle, hydroxycarbonyle, aminocarbonyle, alkyle en C₁-C₄, alcoxy en C₁-C₄, alkylamino en C₁-C₆, alcoxycarbonyle en C₁-C₄, alkylaminocarbonyle en C₁-C₄ et cyclopropyle,
l'alkyle pouvant être substitué avec un substituant hydroxy,
ou un de ses sels, de ses solvates ou des solvates de ses sels,
à l'exception des composés
1-carboxylate de trans-tert-butyl-3-{[(benzyloxy)carbonyl]amino}-5-phénylpipéridine et 1-carboxylate de cis-tert-butyl-3-{[(benzyloxy)carbonyl]amino}-5-phénylpipéridine.

2. Composé selon la revendication 1, **caractérisé en ce que**
A représente un groupe de formule 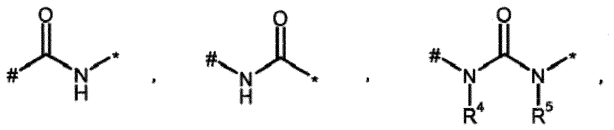 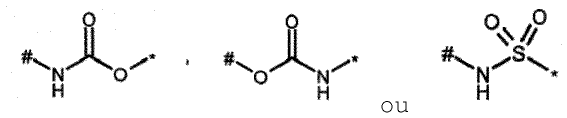
# représentant l'emplacement de liaison au cycle pipéridine,
* représentant l'emplacement de liaison à R²,
R⁴ représentant hydrogène ou méthyle,
et
R⁵ représentant hydrogène ou méthyle,
R¹ représente phényle,
le phényle pouvant être substitué avec 1 à 2 substituants, choisis indépendamment l'un de l'autre dans le groupe constitué par trifluorométhyle, trifluorométhoxy, méthyle, éthyle, isopropyle, méthoxy et éthoxycarbonyle,
R² représente méthyle, éthyle, isopropyle, tert-butyle, cyclopropyle, cyclopentyle, pyrrolidinyle, pipérazinyle, phényle, 2,2-difluoro-1,3-benzodioxolyle, thiényle, thiazolyle ou pyridyle,
le cyclopentyle, le pipérazinyle, le phényle, le thiényle, le thiazolyle et le pyridyle pouvant être substitués avec 1 à 2 substituants, choisis indépendamment l'un de l'autre dans le groupe constitué par halogène, cyano, hydroxy, trifluorométhyle, difluorométhoxy, trifluorométhoxy, méthyle, éthyle, méthoxy, éthoxy et phényle,
le phényle pouvant être substitué avec 1 à 2 substituants, choisis indépendamment l'un de l'autre dans le groupe constitué par halogène et trifluorométhyle,
et
le méthyle pouvant être substitué avec un substituant phényle,
R³ représente méthyle, éthyle, isopropyle, tert-butyle, éthoxy, éthylamino, tert-butylamino, N-méthyl-N-éthylamino, diéthylamino, cyclopropyle, cyclobutyle, cyclopentyle, cyclohexyle, tétrahydrofuranyle, tétrahydropyranyle, morpholin-4-yle, thiomorpholin-4-yle, 1,1-dioxydothiomorpholin-4-yle, azétidiny-1-yle, pyrrolidin-1-yle, pipéridin-1-yle, pipérazin-1-yle, phényle, pyrrolyle, furyle, thiazolyle, pyrazolyle, pyridyle, cyclopentyloxy, cyclohexylamino, phénylamino ou pyridylamino,
le méthyle, l'éthyle, l'isopropyle, le tert-butyle, l'éthoxy et l'éthylamino pouvant être substitués avec un substituant choisi dans le groupe constitué par halogène, hydroxy, méthoxy, cyclopropyle, phényle, furyle, thiényle et pyrazolyle,
et
le cyclopropyle, le cyclobutyle, le cyclohexyle, le tétrahydrofuranyle, le morpholin-4-yle, l'azétidiny-1-yle, le pyrrolidin-1-yle, le pipéridin-1-yle, le pipérazin-1-yle, le phényle, le furyle, le thiazolyle, le pyrazolyle, le pyridyle, le cyclohexylamino et le phénylamino pouvant être substitués avec 1 à 2 substituants, choisis indépendamment l'un de l'autre dans le groupe constitué par halogène, cyano, oxo, hydroxy, amino, trifluorométhyle, difluorométhoxy, trifluorométhoxy, hydroxycarbonyle, aminocarbonyle, méthyle, éthyle, méthoxy, éthoxy, diméthylamino, méthoxycarbonyle, éthoxycarbonyle, diméthylaminocarbonyle et cyclopropyle,
le méthyle et l'éthyle pouvant être substitués avec un substituant hydroxy,
ou un de ses sels, de ses solvates ou des solvates de ses sels.

3. Composé selon la revendication 1 ou 2, **caractérisé en ce que**
A représente un groupe de formule 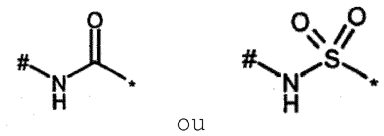
# représentant l'emplacement de liaison au cycle pipéridine,
* représentant l'emplacement de liaison à R²,
R¹ représente phényle,
le phényle pouvant être substitué avec 1 à 2 substituants, choisis indépendamment l'un de l'autre dans le groupe constitué par trifluorométhyle, trifluorométhoxy, méthyle et éthyle,
R² représente méthyle, éthyle, isopropyle, tert-butyle, cyclopropyle, cyclopentyle, phényle, thiényle ou pyridyle,
le cyclopentyle, le phényle, le thiényle et le pyridyle pouvant être substitués avec 1 à 2 substituants, choisis indépendamment l'un de l'autre dans le groupe constitué par halogène, cyano, trifluorométhyle, trifluorométhoxy, méthyle, méthoxy et phényle,
le phényle pouvant être substitué avec 1 à 2 substituants, choisis indépendamment l'un de l'autre dans le groupe constitué par chlore, fluor et trifluorométhyle,
et
le méthyle pouvant être substitué avec un substituant phényle,
R³ représente morpholin-4-yle, 1,1-dioxydothiomorpholin-4-yle, 3-hydroxyazétidiny-1-yle, 3-hydroxypyrrolidin-1-yle, 4-cyanopipéridin-1-yle ou 4-hydroxypipéridin-1-yle,
ou un de ses sels, de ses solvates ou des solvates de ses sels.

4. Composé selon l'une quelconque des revendications 1 à 3, **caractérisé en ce que** les substituants -R¹ et -A-R² sont en position cis l'un par rapport à l'autre.

5. Procédé de fabrication d'un composé de formule (I) ou d'un de ses sels, de ses solvates ou des solvates de ses sels selon la revendication 1, **caractérisé en ce que** soit
[A] un composé de formule 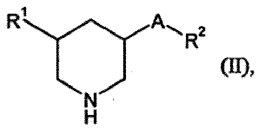 dans laquelle
A, R¹ et R² ont la signification indiquée dans la revendication 1,
est mis en réaction avec un composé de formule 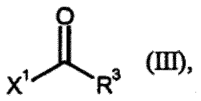 dans laquelle
R³ a la signification indiquée dans la revendication 1, et
X¹ représente halogène, de préférence brome ou chlore, ou hydroxy,
soit
[B] un composé de formule (II) est mis en réaction avec un composé de formule 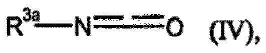 dans laquelle
R^{3a} représente alkyle en C₁-C₆, cycloalkyle en C₃-C₇, hétérocyclyle de 4 à 7 éléments, phényle ou hétéroaryle à 5 ou 6 éléments,
l'alkyle pouvant être substitué avec un substituant choisi dans le groupe constitué par halogène, hydroxy, amino, cyano, alcoxy en C₁-C₄, alcoxycarbonyle en C₁-C₄, cycloalkyle en C₃-C₇, hétérocyclyle de 4 à 6 éléments, phényle et hétéroaryle à 5 ou 6 éléments,
l'alcoxy pouvant être substitué avec un substituant alcoxy en C₁-C₄,
et
le cycloalkyle, l'hétérocyclyle, le phényle et l'hétéroaryle pouvant être substitués avec 1 à 3 substituants, choisis indépendamment les uns des autres dans le groupe constitué par halogène, cyano, oxo, hydroxy, amino, monofluorométhyle, difluorométhyle, trifluorométhyle, monofluorométhoxy, difluorométhoxy, trifluorométhoxy, monofluorométhylsulfanyle, difluorométhylsulfanyle, trifluorométhylsulfanyle, hydroxycarbonyle, aminocarbonyle, alkyle en C₁-C₄, alcoxy en C₁-C₄, alkylamino en C₁-C₆, alcoxycarbonyle en C₁-C₄, alkylaminocarbonyle en C₁-C₄ et cyclopropyle,
l'alkyle pouvant être substitué avec un substituant hydroxy,
pour former un composé de formule 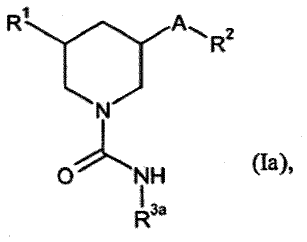 dans laquelle
A, R¹, R² et R^{3a} ont la signification indiquée dans la revendication 1,
soit
[C] un composé de formule 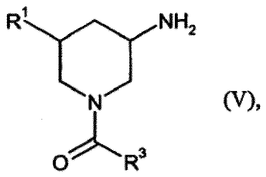 dans laquelle
R¹ et R³ ont la signification indiquée dans la revendication 1,
est mis en réaction avec un composé de formule 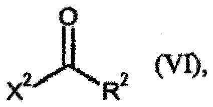 dans laquelle
R² a la signification indiquée dans la revendication 1, et
X² représente halogène, de préférence brome ou chlore, ou hydroxy,
pour former un composé de formule 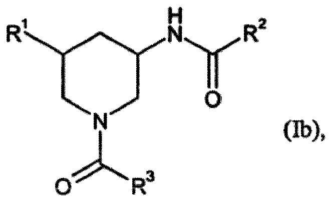 dans laquelle
R¹, R² et R³ ont la signification indiquée dans la revendication 1,
soit
[D] un composé de formule (V) est mis en réaction avec un composé de formule 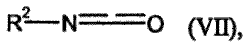 dans laquelle
R² a la signification indiquée dans la revendication 1,
pour former un composé de formule 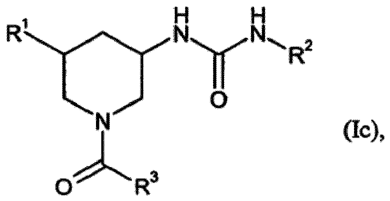 dans laquelle
R¹, R² et R³ ont la signification indiquée dans la revendication 1,
soit
[E] un composé de formule (V) est mis en réaction avec un composé de formule 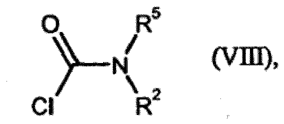 dans laquelle
R² et R⁵ ont la signification indiquée dans la revendication 1,
pour former un composé de formule 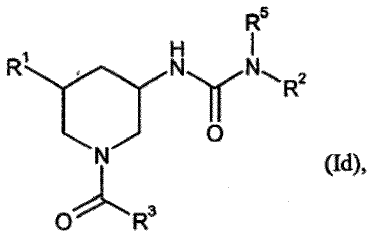 dans laquelle
R¹, R², R³ et R⁵ ont la signification indiquée dans la revendication 1,
soit
[F] un composé de formule (Id) est mis en réaction avec un composé de formule 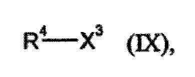 dans laquelle
R⁴ a la signification indiquée dans la revendication 1, et
X³ représente halogène, de préférence iode, brome ou chlore,
pour former un composé de formule 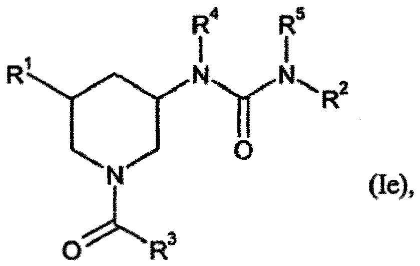 dans laquelle
R¹, R², R³, R⁴ et R⁵ ont la signification indiquée dans la revendication 1,
soit
[G] un composé de formule (V) est mis en réaction avec un composé de formule 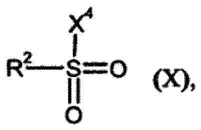 dans laquelle
R² a la signification indiquée dans la revendication 1, et
X⁴ représente chlore ou hydroxy,
pour former un composé de formule 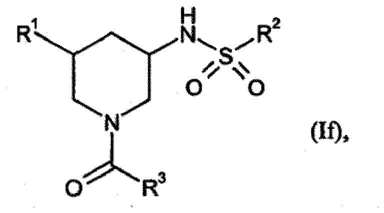 dans laquelle
R¹, R² et R³ ont la signification indiquée dans la revendication 1,
soit
[H] un composé de formule 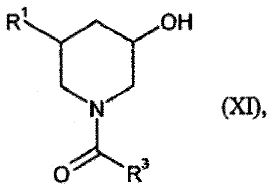 dans laquelle
R¹ et R³ ont la signification indiquée dans la revendication 1
est tout d'abord mis en réaction avec du carbonate de disuccinimidyle, puis avec un composé de formule
**H₂N-R²** **(XII),**
dans laquelle
R² a la signification indiquée dans la revendication 1,
pour former un composé de formule 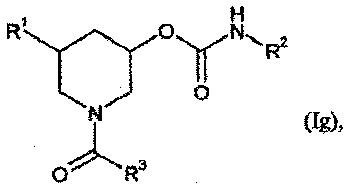 dans laquelle
R¹, R² et R³ ont la signification indiquée dans la revendication 1.

6. Composé selon l'une quelconque des revendications 1 à 4 pour le traitement et/ou la prophylaxie de maladies.

7. Utilisation d'un composé selon l'une quelconque des revendications 1 à 4 pour la fabrication d'un médicament pour le traitement et/ou la prophylaxie de maladies.

8. Utilisation d'un composé selon l'une quelconque des revendications 1 à 4 pour la fabrication d'un médicament pour le traitement et/ou la prophylaxie de maladies cardiovasculaires, de maladies thromboemboliques et/ou de maladies tumorales.

9. Utilisation d'un composé selon l'une quelconque des revendications 1 à 4 pour l'inhibition de la coagulation du sang *in vitro.*

10. Médicament contenant un composé selon l'une quelconque des revendications 1 à 4 en combinaison avec un adjuvant inerte, non toxique, pharmaceutiquement approprié.

11. Médicament contenant un composé selon l'une quelconque des revendications 1 à 4 en combinaison avec un agent actif supplémentaire.

12. Médicament selon la revendication 10 ou 11 pour le traitement et/ou la prophylaxie de maladies cardiovasculaires, de maladies thromboemboliques et/ou de maladies tumorales.

13. Procédé d'inhibition de la coagulation du sang *in vitro,* **caractérisé en ce qu'**une quantité à effet anticoagulant d'un composé selon l'une quelconque des revendications 1 à 4 est administrée.
